# EUROPEAN PATENT SPECIFICATION

(11) **EP 4 434 533 B1**
(45) Date of publication and mention of the grant of the patent: **04.12.2024**
(21) Application number: 23163092.2
(22) Date of filing: 21.03.2023
(51) Int. Cl.: A61K 38/00, C07K 14/435, C12N 15/62, G01N 33/50, C12N 5/075

(54) **ARTIFICIAL COMPLEX FOR TETHERING CHROMATIDS OF CHROMOSOMES**
KÜNSTLICHER KOMPLEX ZUR BINDUNG VON CHROMOSOMCHROMATIDEN
COMPLEXE ARTIFICIEL POUR LA FIXATION DE CHROMATIDES DE CHROMOSOMES

(43) Date of publication of application: 25.09.2024
(73) Proprietor: Max-Planck-Gesellschaft zur Förderung der Wissenschaften e.V., 80539 München (DE)
(72) Inventor: SCHUH, Melina, 37070, Göttingen (DE); WEBSTER, Alexandre, 37070, Göttingen (DE)
(74) Representative: Wichmann, Hendrik

(56) References cited:
- WO-A1-2009/030932
- WO-A1-2017/192847
- WO-A2-2006/036637
- CN-A- 115 656 509
- MUIR KYLE W ET AL: "The structure of the cohesin ATPase elucidates the mechanism of SMC-kleisin ring opening", NATURE STRUCTURAL & MOLECULAR BIOLOGY, NATURE PUBLISHING GROUP US, NEW YORK, vol. 27, no. 3, 17 February 2020 (2020-02-17), pages 233 - 239, XP037057096, ISSN: 1545-9993, [retrieved on 20200217], DOI: 10.1038/S41594-020-0379-7

## Description

### Background of the invention

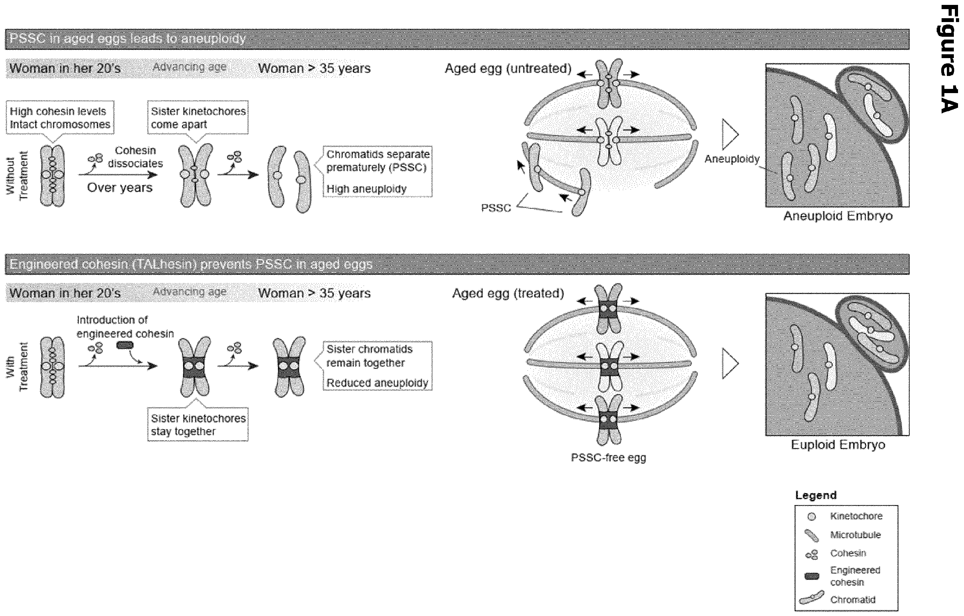

Women become infertile as they advance in age. In particular, fertility begins to decline from approximately 35 years of age onwards^{1,3}. The decline in fertility is caused by an age-related increase in aneuploidy in women's eggs. Immature eggs are stored in the ovary from birth onwards, and can therefore be decades-old at the time of fertilization. As women approach their 40's, the number of aneuploid eggs increases dramatically^{1,4,5,10,11}. Aneuploidy is transferred from the egg to the embryo following fertilization (Fig. 1a). In most cases, aneuploid embryos can neither implant in the uterus nor develop to term. If too many eggs are aneuploid, women can no longer conceive. The high aneuploidy levels in eggs of women in their late 30's and 40's also explain why IVF treatments in this age group often fail, despite the successful retrieval of eggs.

Chromosomes in the egg are made up of individual chromatids linked together by cohesin complexes. Cohesins provide chromosome cohesion that is critical for the accurate segregation of chromosomes throughout meiotic cell divisions¹²⁻¹⁵. During ageing, eggs display a gradual loss of cohesin complexes (upper left panel, Fig. 1a)^{6,8,9,12-15}. The cohesin loss eventually causes sister chromatids to separate prematurely^{1,10,12-14,16-19}. Once separated, sister chromatids segregate randomly during both the first and second meiotic cell divisions (meiosis I and II), leading to aneuploidy (upper right panel, Fig. 1a). WO 2009/030932 A1 discloses methods for reducing chromosome non-disjunctions and aneuploidy during meiosis by administering an effective amount of cdh1 or a variant thereof. CN 115 656 509 A discloses ERK1/2 protein as a marker of chromosome aneuploidy in oocytes and that growth hormone can increase the expression of ERK1/2. WO 2017/192847 A1 discloses female fertility therapies including FSH lowering methodologies and agents that bind to activin an methods of utilizing these agents to prevent and/or treat egg fertility. Methods to prevent premature separation of sister chromatids (PSSC) by preserving cohesin on sister chromatids, or to reverse its loss, do not currently exist to the knowledge of the inventor. As a consequence, there are no treatments available for age-related infertility, and many women over 35 cannot become pregnant.

### Summary of the invention

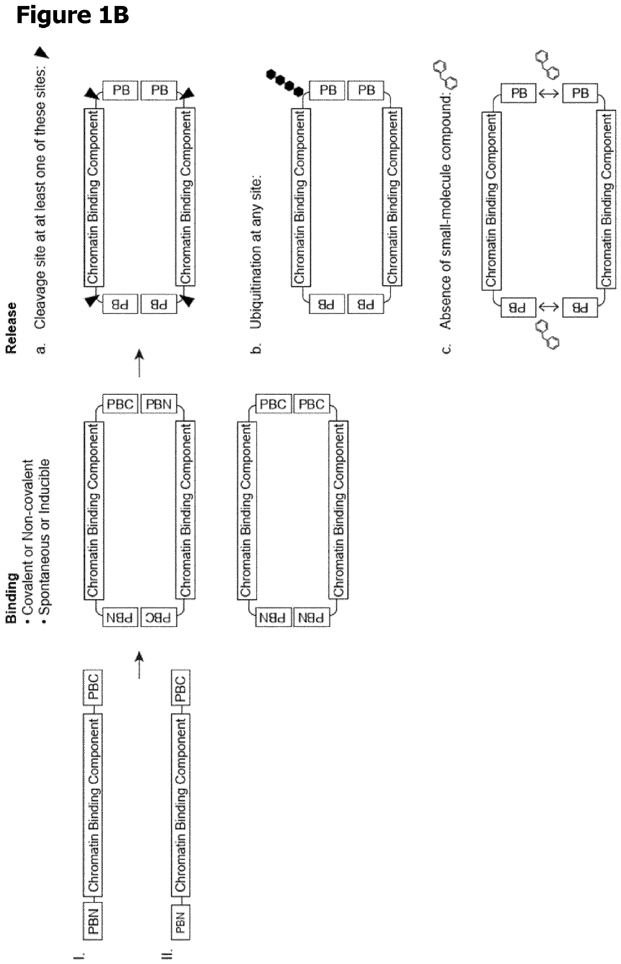

To meet this need, the inventors engineered an artificial cohesion system, a complex, for chromatid or chromosome tethering to reduce, for example, age-related PSSC in eggs (lower panel, Fig. 1a). The parts of the complex and the overall structure of the complex are depicted in Fig. 1b.

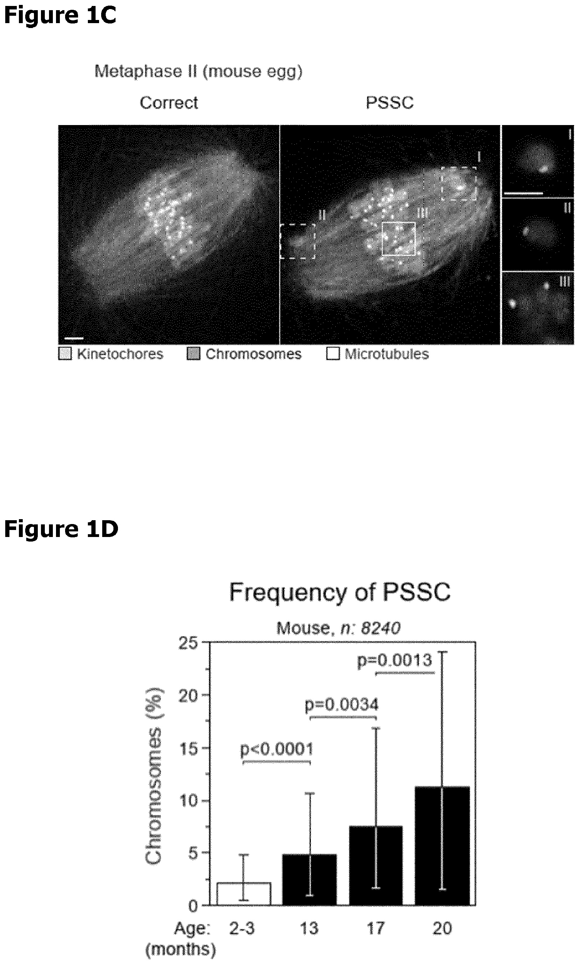

To demonstrate that the artificial cohesion system tethers e.g. sister chromatids, the inventors primarily used mouse oocytes as a model of PSSC (Fig. 1c,d), because human oocytes are difficult to source. Based on the mouse data and data generated in human somatic cells, the invention additionally demonstrates that the artificial cohesion system works in human eggs (see Example 1 and 2). The technical advantage of the present invention is that the artificial cohesion system is a complex tethering chromosomes or chromatids, such as sister chromatids, so that e.g. the risk of aneuploidy is reduced. Said complex comprises (I) one or more first protein(s) and (II) one or more second protein(s), wherein the (I) first and the (II) second protein(s) each comprise (i) a chromatin-binding component, (ii) a protein-binding region being N-terminal of the chromatin-binding component (PBN region), (iii) a protein-binding region being C-terminal of the chromatin-binding component (PBC region), as further defined in the claims. The overall arrangement is depicted in Figure 1b. The complex tethers the chromosomes or chromatids by binding of the chromosome-binding component to the chromosomes or chromatids and by binding of the protein binding regions of the first protein(s) to the protein binding regions of the second protein(s). Furthermore, the said complex tethers chromosomes and chromatids reversibly. In other words, the tethering is releasable to allow chromosome or chromatid separation, e.g. during anaphase I or II of meiosis.

The present invention also includes nucleic acid molecule(s) encoding the complex as further defined in the claim. Furthermore, several *in vitro* methods concerning the complex or the nucleic acid molecule(s) encoding said complex also form part of the invention and as further defined in the claims. The complex or the nucleic acid molecule(s) suitable for use in reproductive medicine is also claimed. In particular, assisted reproductive technology is preferred. The various aspects of the invention are defined in the independent claims. Preferred embodiments are contained in dependent claims.

### Description of the Figures

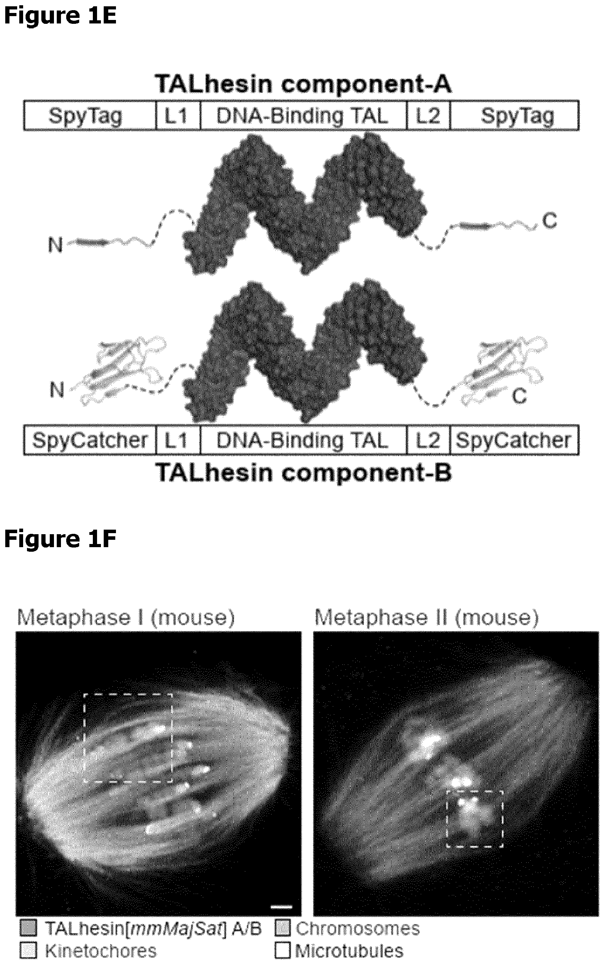

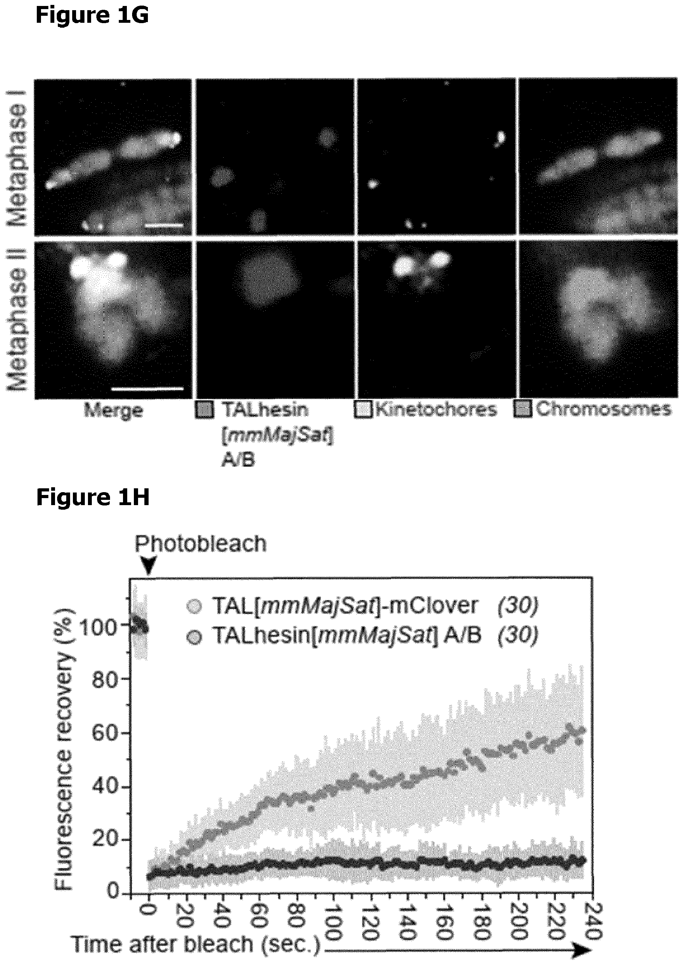

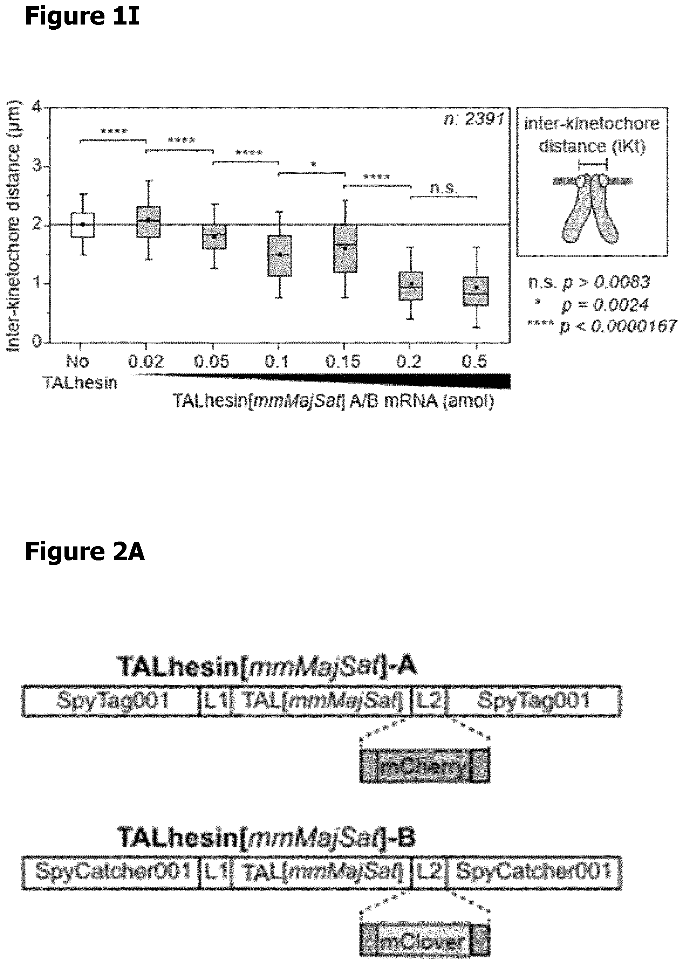

**Fig. 1****. An engineered cohesion system to prevent chromosome errors in eggs. (A)** Schematic of engineered cohesion system on human egg metaphase chromosomes. Cohesin gradually dissociates from chromosomes in eggs during aging, causing Premature Separation of Sister Chromatids (PSSC) (upper panel, left) and aneuploidy (meiosis II upper panel, middle)^{3,49}. An untreated secondary oocyte with PSSC results in an aneuploid embryo with two grey chromosomes and an aneuploid second polar body with no grey chromosomes (upper panel, right). An engineered cohesin system restores cohesion (black) to pericentromeric regions, preventing PSSC (lower panel, left) and aneuploidy (lower panel, middle), leading to a euploid embryo (lower panel, right). **(B)** Overall depiction of the invention. The left cartoon view illustrates the overall arrangement of (I) the first protein(s) and (II) the second protein(s), each comprising (i) a chromatin-binding component, (ii) a protein-binding region being N-terminal of the chromatin-binding component (PBN region), (iii) a protein-binding region being C-terminal of the chromatin-binding component (PBC region). The cartoon in the middle illustrates that the PBN of the first and the PBC of the second protein may bind; and that the PBC of the first and the PBN of the second protein may bind. Alternatively, the PBN regions of the first and the second bind to each other; and the PBC regions of the first and the second protein bind to each other. Said binding may be covalent or non-covalent. In the right panel of the cartoon, three exemplary ways of releasing the binding are depicted. Firstly, (a) cleavage of the complex via a protease cleavage site located between a chromatin binding component and a protein binding region. Secondly, (b) degradation of the complex, wherein the first and/or the second protein(s) of the complex additionally comprise a ubiquitination site. Thirdly, (c) disruption of the binding of the PBN and PBC regions of the first and the second protein(s), wherein said binding is mediated by chemically induced proximity. **(C)** Chromosomes (grey) aligned correctly to the meiotic spindle mid-zone (white) (left panel) or with PSSC (right panel) in metaphase II mouse eggs. In boxes I and II, separated chromatids marked by a single kinetochore (white) indicate PSSC; in box III paired sister chromatids aligned at the mid-zone. Scale bars: 2 µm. **(D)** Frequency of PSSC in eggs from young mice (hollow bars) and mice aged 13, 17, and 20 months (filled bars), n, number of chromosomes analyzed. Statistical significance measure by Fisher's exact test. Bars: 95% CI, 1 degree of freedom. **(E)** Model of TALhesin components A and B. DNA-binding TAL effector (dark grey) is flanked by either SpyTags (component A) or Spy-Catchers (component B) (See Figure 2A for further detail). Dashed lines are linker regions L1 and L2. TAL c-terminal domain not shown. Based on pdb: 3UGM⁵⁰ and 4MLI⁵¹. **(F)** TALhesin[*mmMajSat*]*A*/*B* localizes to chromosomes in metaphase I (left panel), and metaphase II (right panel) spindles (white). Scale bars: 2 µm. **(G)** Magnification of boxed chromosomes in Fig. 1f. TALhesin[*mmMajSat*] localizes to chromosome pericentromeric regions in mouse meiosis I (upper) and meiosis II spindles (lower). Scale bars: 2 µm **(H)** Mobility of TALhesin[*mmMajSat*] chromosome foci (dark grey) compared to TAL[*mmMajSat*] effector alone (light grey) measured by FRAP. Bars: SD from the mean; 30 replicates of each condition. **(I)** Inter-kinetochore distance measurements between sister chromatids of metaphase II chromosomes in mouse eggs injected with the indicated amounts of TALhesin[*mmMajSat*]A/B mRNA. Filled square indicates mean; line indicates median for each condition. Statistics measured by Bonferroni corrected two sample T-test (equal variance not assumed). n, sister kinetochore pairs analyzed: 2391.

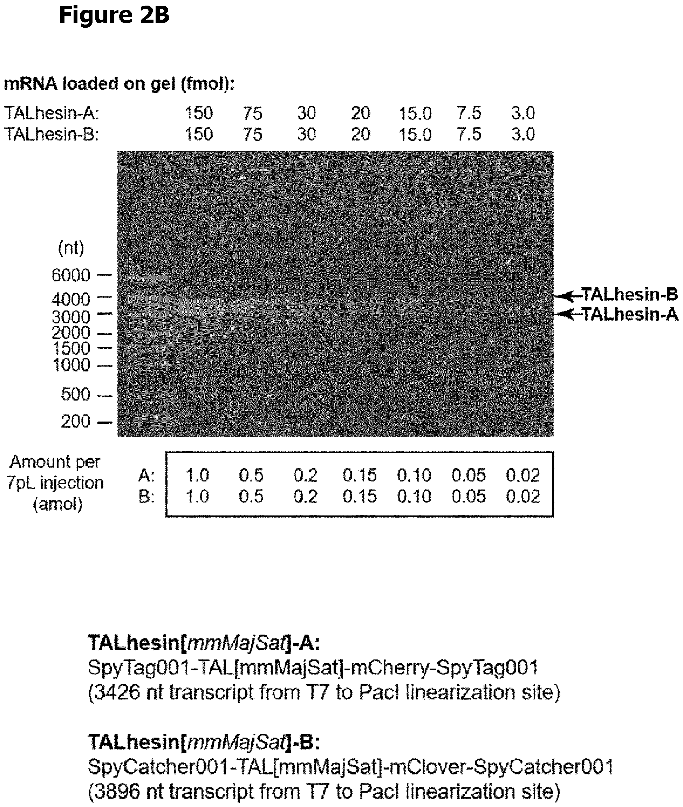

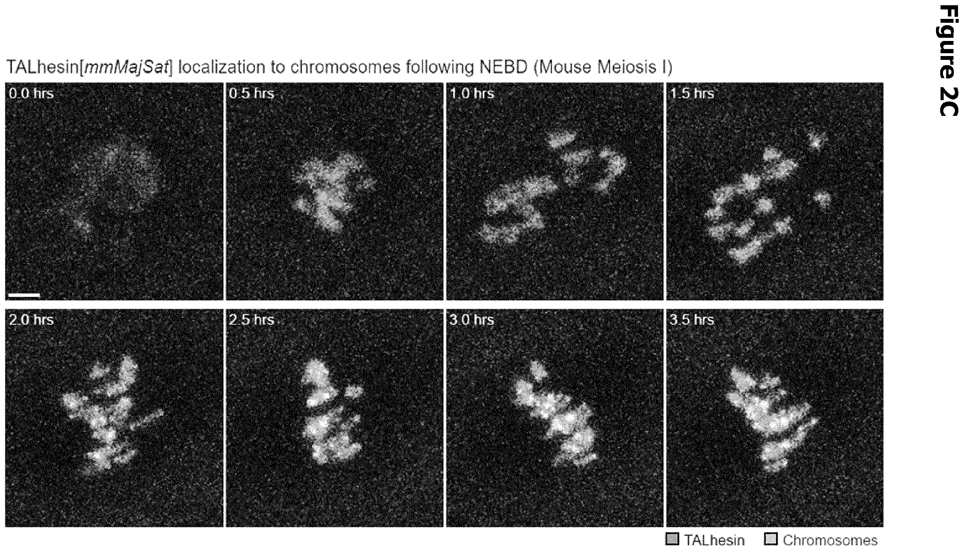

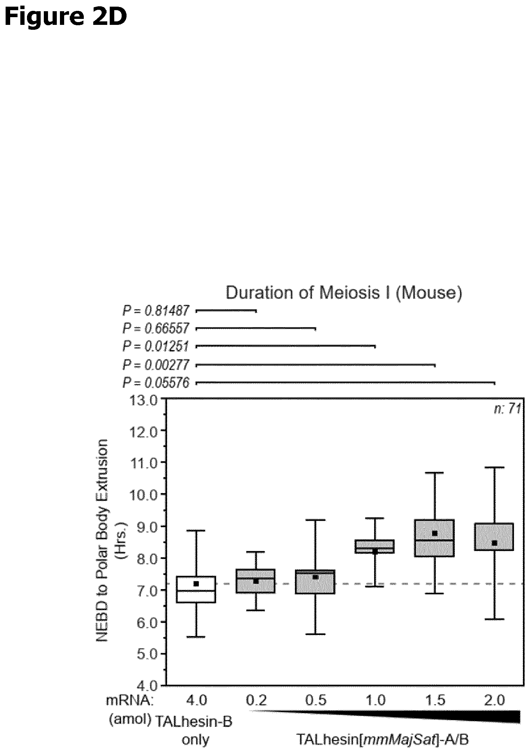

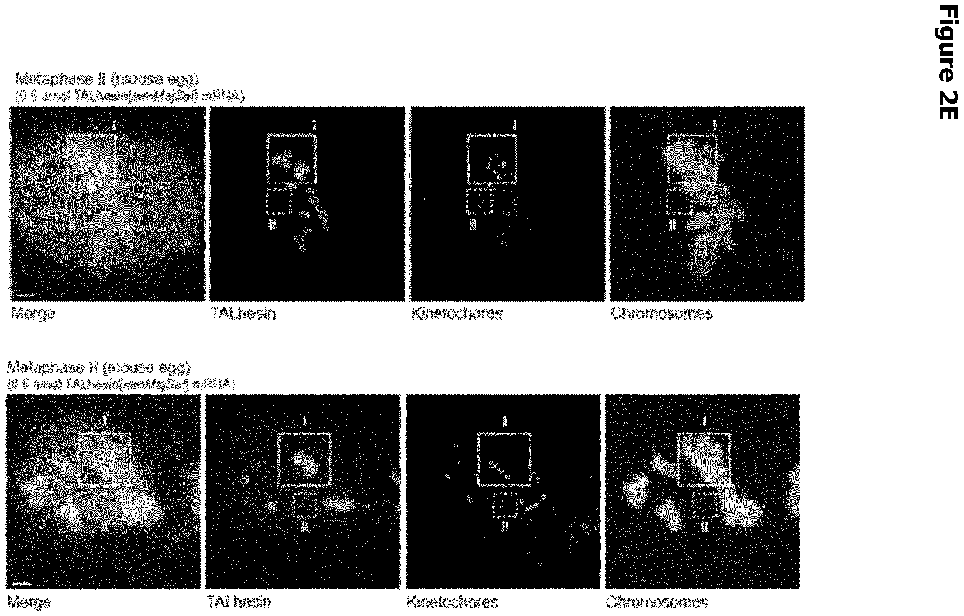

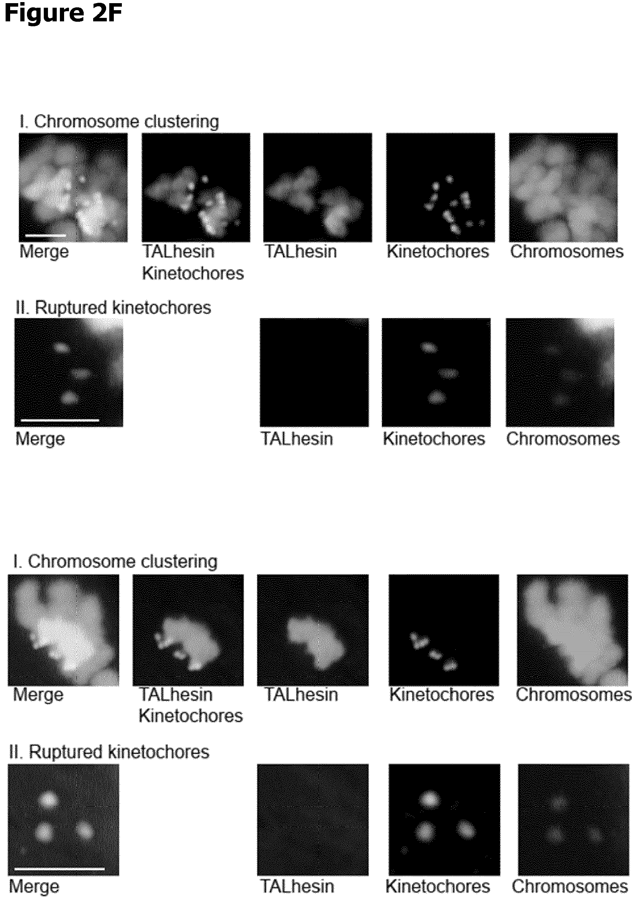

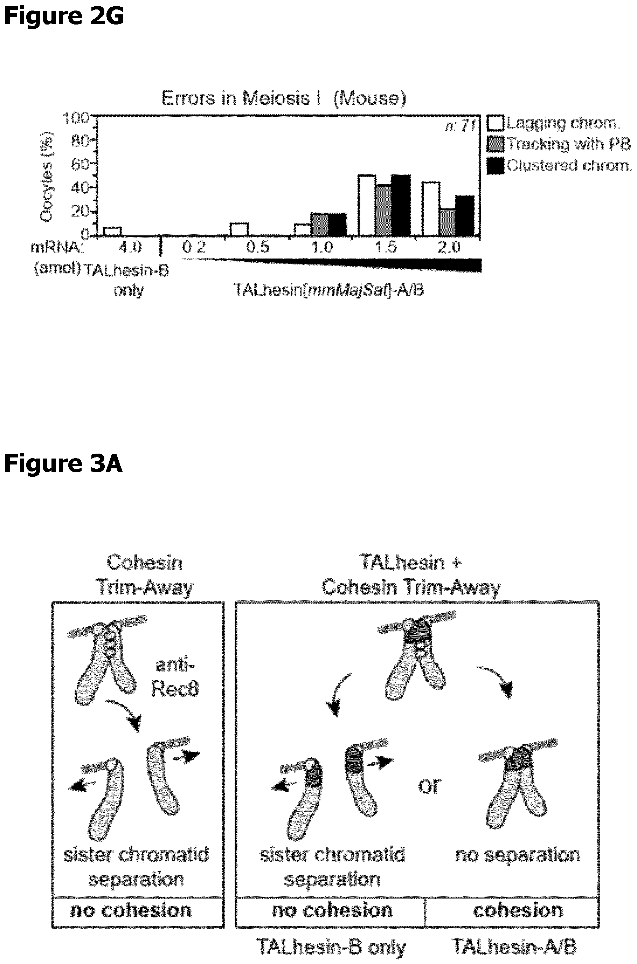

**Fig. 2****. High artificial complex (TALhesin) expression causes meiosis I and II errors.**
   **(A)** Schematic of domain arrangement for TALhesin[*mmMajSat*] targeted to mouse pericentromeric repeats (not drawn to scale). Fluorescent proteins, mCherry or mClover are integrated into L2 linker regions. **(B)** TALhesin[*mmMajSat*]A/B mRNA titration analyzed by 1% agarose gel electrophoresis. The domain composition and expected size of each transcript is indicated next to the annotated image in nucleotides (nt). **(C)** TALhesin[*mmMajSat*]A/B localizes to chromosomes following NEBD. Chromosomes, grey; TALhesin, dark grey (hrs: hours following NEBD). Scale bar: 5 µm. **(D)** Duration of mouse meiosis I measured from NEBD to polar body extrusion, in oocytes micro-injected with TALhesin[*mmMajSat*]-B only (hollow bar), or increasing amounts of TALhesin[*mmMajSat*]A/B mRNA (amol, attomoles; Hrs, hours). Filled square indicates mean; line indicates median for each condition. Bars: SD from the mean. n, number of cells analyzed. P-values measured by Student's T-test. **(E)** Metaphase II spindles of two example mouse eggs expressing high amounts (0.5 amol) of TALhesin[*mmMajSat*] mRNA. Chromosome clustering (box I) and ruptured kinetochores (white dots) (box II) are indicated in each example. **(F)** Magnification of boxed chromosomes in Fig. 2e showing chromosome clustering and ruptured kinetochores. For **(E-F),** TALhesin, dark grey; Kinetochores; Microtubules; Chromosomes. Scale bars: 2 µm. **(G)** Quantification of errors detected by live microscopy of meiosis I in mouse oocytes expressing increasing amounts of TALhesin[*mmMajSat*]A/B mRNA. Lagging chromosomes indicated with hollow bars. Chromosomes in the egg remaining linked to and tracking with polar body are indicated with grey bars. Clustered chromosomes, filled bars.

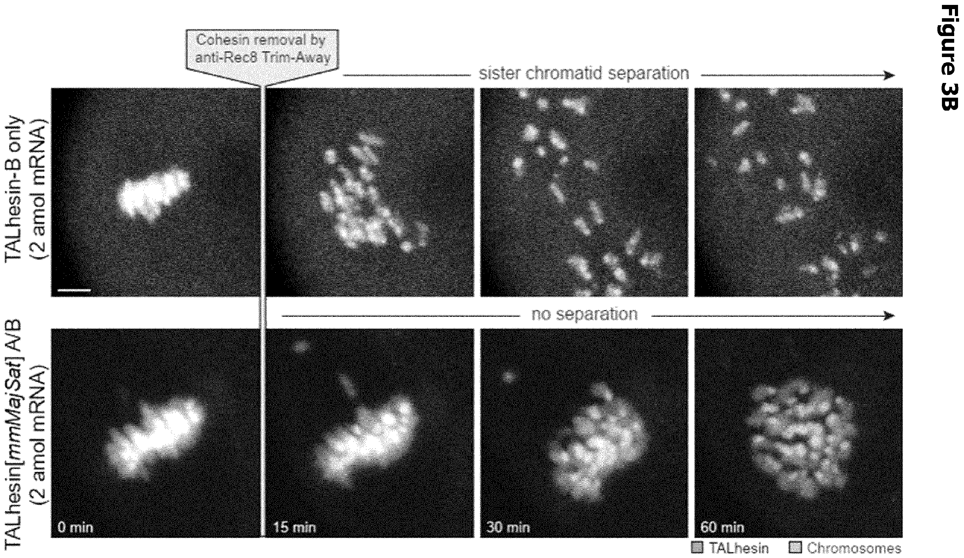

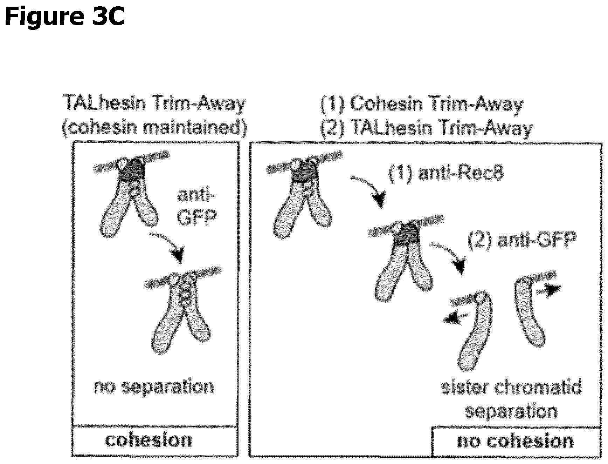

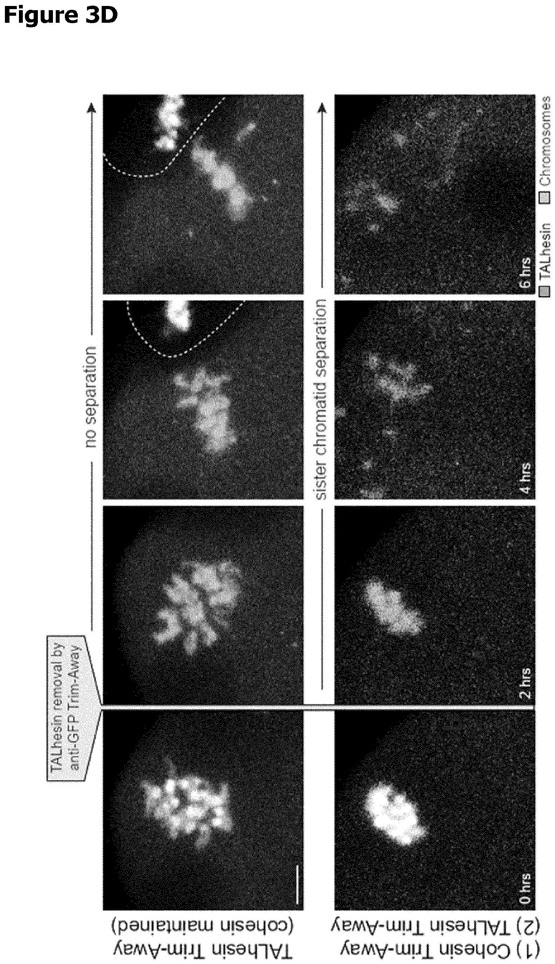

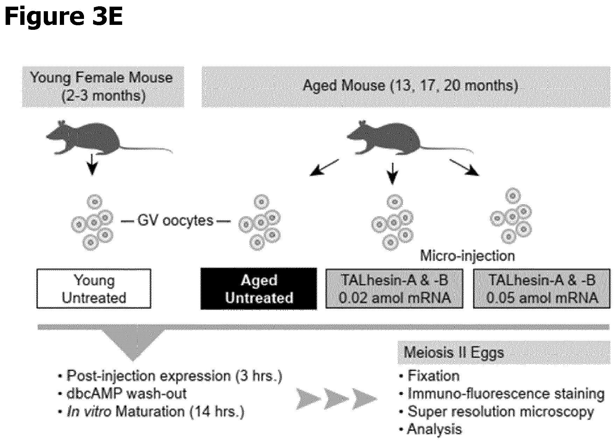

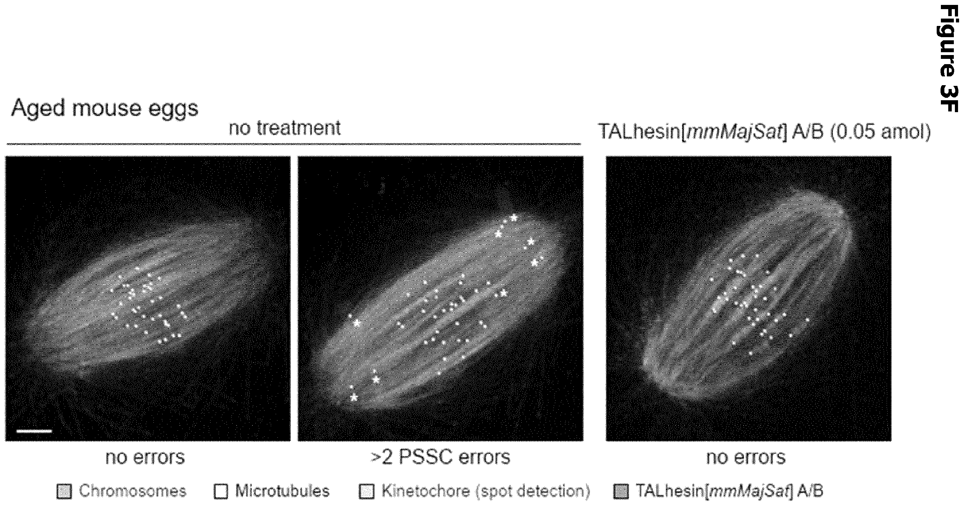

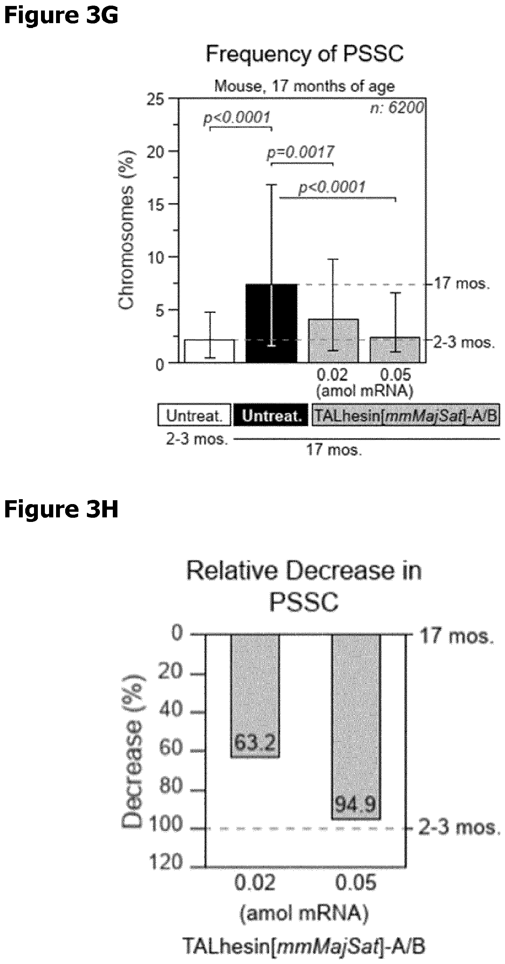

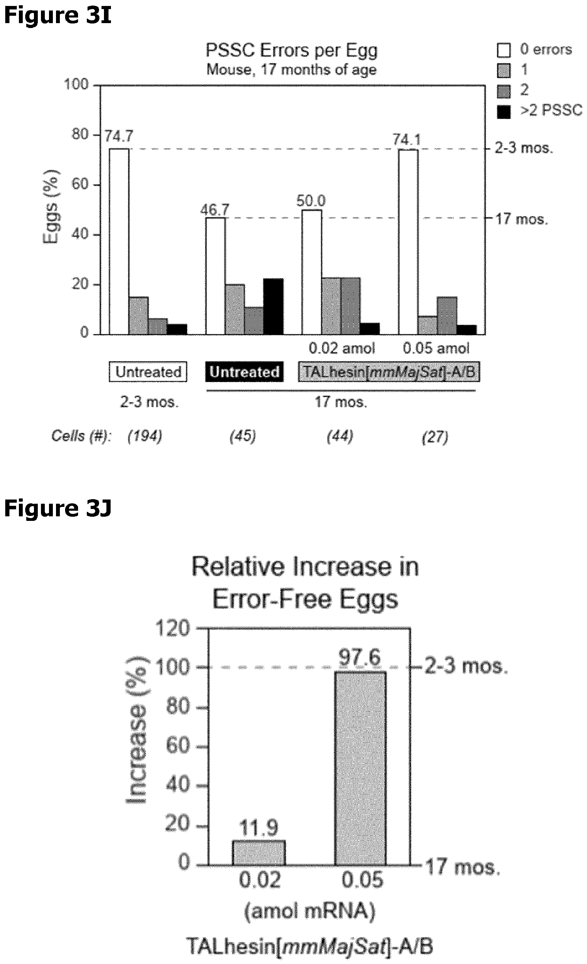

**Fig. 3****. Artificial complex (TALhesin) provides cohesion to mouse chromosomes.**
   **(A)** Schematic of Rec8 Trim-Away depletion outcomes in Fig. 3b. (B) Rec8 Trim-Away depletion in young mouse eggs injected with 2 amol TALhesin[*mmMajSat*]-B component only (upper panel). Rec8 Trim-Away depletion in eggs injected with 2 amol TALhesin[*mmMajSat*]A/B (lower panel) (min: minutes). Min.: minutes. Scale bar: 5 µm. **(C)** Schematic of TALhesin[*mmMajSat*] protein Trim-Away depletion outcomes in Fig. 3d. (D) Depletion of TALhesin[*mmMajSat*] by anti-GFP Trim-Away (upper panel), or following Rec8 depletion (lower panel). Dashed line indicates polar body within field of view. hrs: hours. Scale bar: 5 µm. **(E)** Schematic of chromosome error-reduction assay in mouse eggs. **(F)** Examples of meiosis II spindles from aged mouse eggs used for analysis. Automated spot detection of 40 kinetochores (white spheres) in each example. PSSCs indicated with asterisks (center panel) where >2 errors are detected. TALhesin[*mmMajSat*]A/B was injected at 0.05 amol mRNA (right panel). Microtubules, white. Scale bar: 3 µm. **(G)** Frequency of PSSC in untreated eggs from young (hollow bar) and aged (filled bar) mice, and aged eggs treated with TALhesin[*mmMajSat*]A/B mRNA (grey bars). n, number of chromosomes analyzed. Statistical significance measure by Fisher's exact test. Bars: 95% CI, one degree of freedom. **(H)** PSSC decrease in aged mouse eggs micro-injected with TALhesin[*mmMajSat*] mRNA relative to untreated young and aged eggs. **(I)** Percentage of eggs with zero (hollow bar), one (light grey bar), two (dark grey bar), or more than two (filled bar) PSSC errors from aged mice treated with TALhesin[*mmMajSat*], compared to untreated young and aged eggs. Number of eggs analyzed indicated below each condition. **(J)** Increase in error-free eggs from aged mice treated with TALhesin[*mmMajSat*] mRNA relative to untreated eggs from young and aged mice.

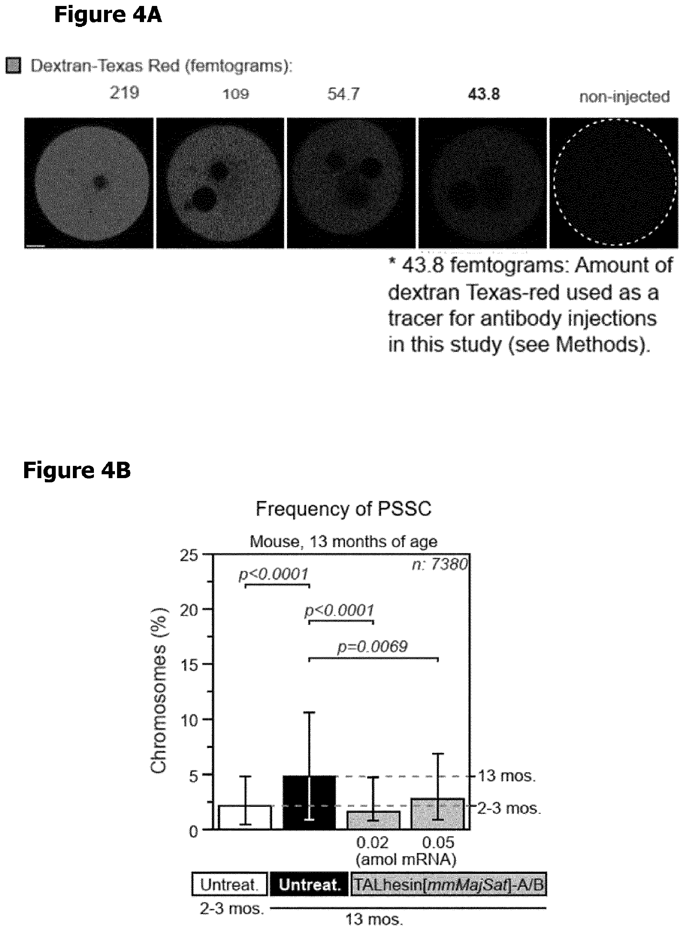

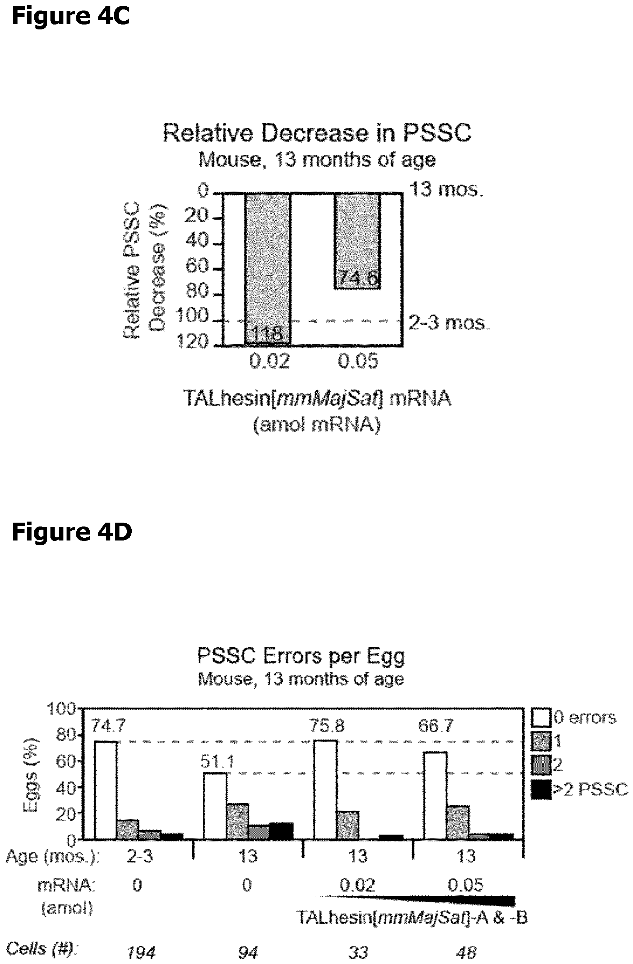

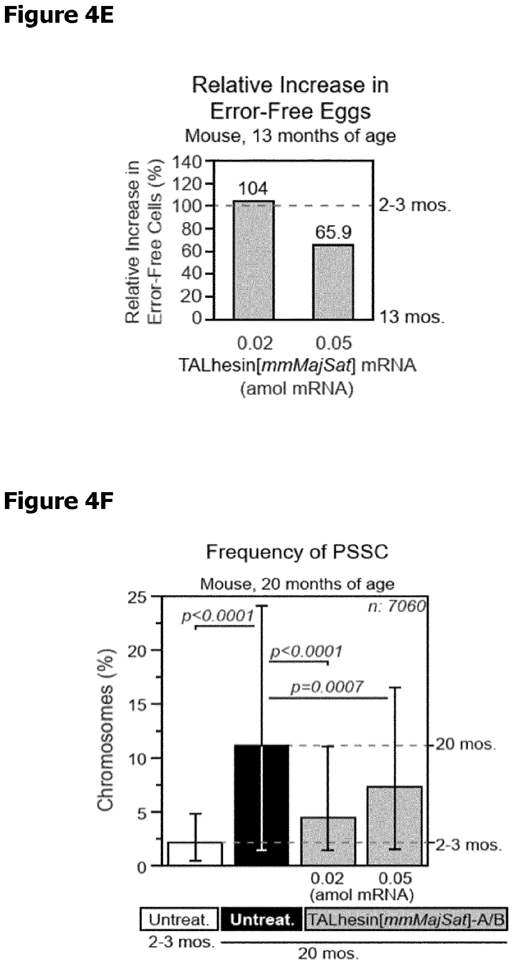

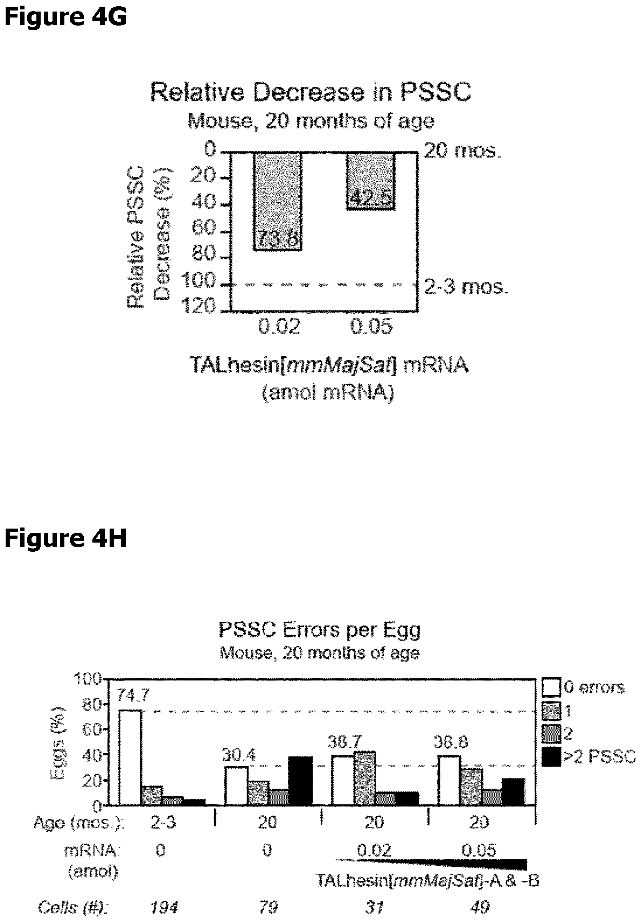

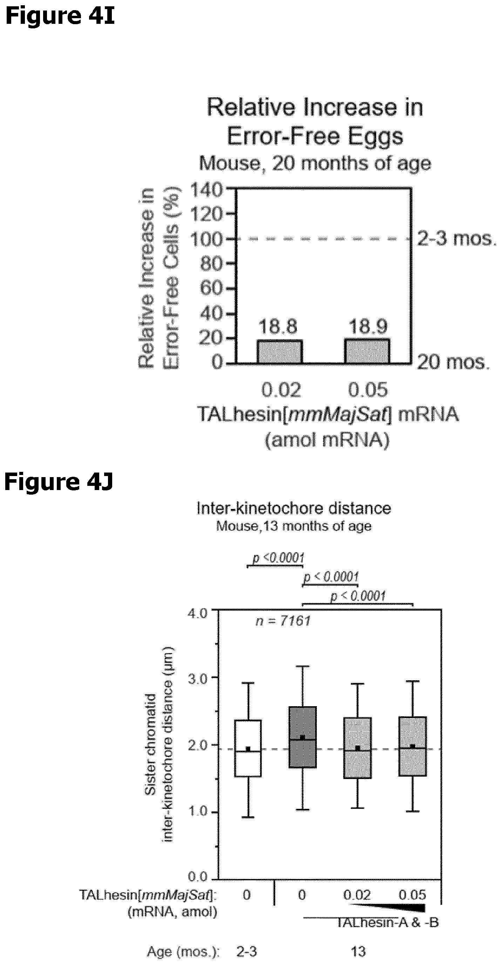

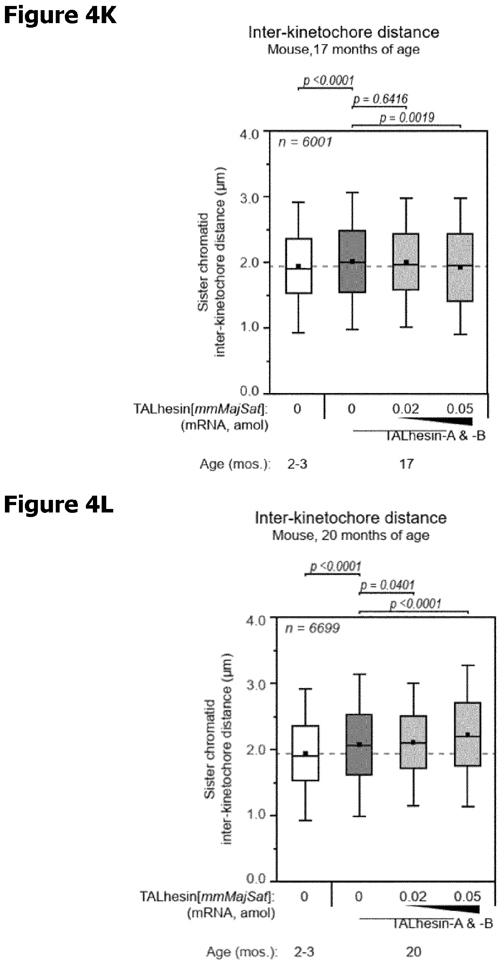

**Fig. 4****. Artificial complex (TALhesin) reduces PSSC errors in aged mice.**
   **(A)** Dextran Texas-Red was used as a tracer for antibody injection in Trim-Away assays. Mouse oocytes injected with different amounts of Dextran Texas-Red (from 219 to 43.8 femtograms), or non-injected (far right). 43.8 femtograms was used as a tracer for anti-Rec8 Trim-Away assays. Dashed line indicates cell membrane. Scale bar: 10 µm. **B-E.** Chromosome error-reduction assay for mouse eggs 13-months of age. **F-I,** Chromosome error-reduction assay for mouse eggs 20-months of age. **B, F,** Frequency of PSSC in untreated eggs from young (hollow bar) and aged (filled bar) mice, and eggs 13-months **(B),** or 20-months **(F)** treated with TALhesin[*mmMajSat*]A/B mRNA (grey bars), n, number of chromosomes analyzed. Statistical significance measure by Fisher's exact test. Bars: 95% CI, 1 degree of freedom. **C,G,** PSSC decrease in eggs from mice aged 13-months **(C)** or 20-months **(G)** treated with TALhesin[*mmMajSat*]A/B mRNA relative to untreated eggs from young and aged mice. **D, H,** Percentage of eggs with zero (hollow bar), one (light grey bar), two (dark grey bar), or more than two (filled bar) PSSC errors from mice aged 13-months **(C)** or 20-months **(H)** treated with TALhesin[*mmMajSat*], compared to untreated eggs from young and aged mice. Number of eggs analyzed are indicated below each condition. **E**, **I,** Increase in error-free eggs from mice aged 13-months **(E)** or 20-months **(I)** treated with TALhesin[*mmMajSat*] mRNA relative to untreated eggs from young and aged mice. **J-L,** Sister chromatid inter-kinetochore distance (iKt) in eggs from young mice (hollow bars), and mice aged 13 **(J),** 17 **(K),** and 20-months **(L)** treated with TALhesin[*mmMajSat*] (light grey bars), or untreated (dark grey bars). Statistics were measured by two sample T-test (equal variance not assumed). Filled square indicates mean; line indicates median for each condition. mos.: months; Bars: SD.

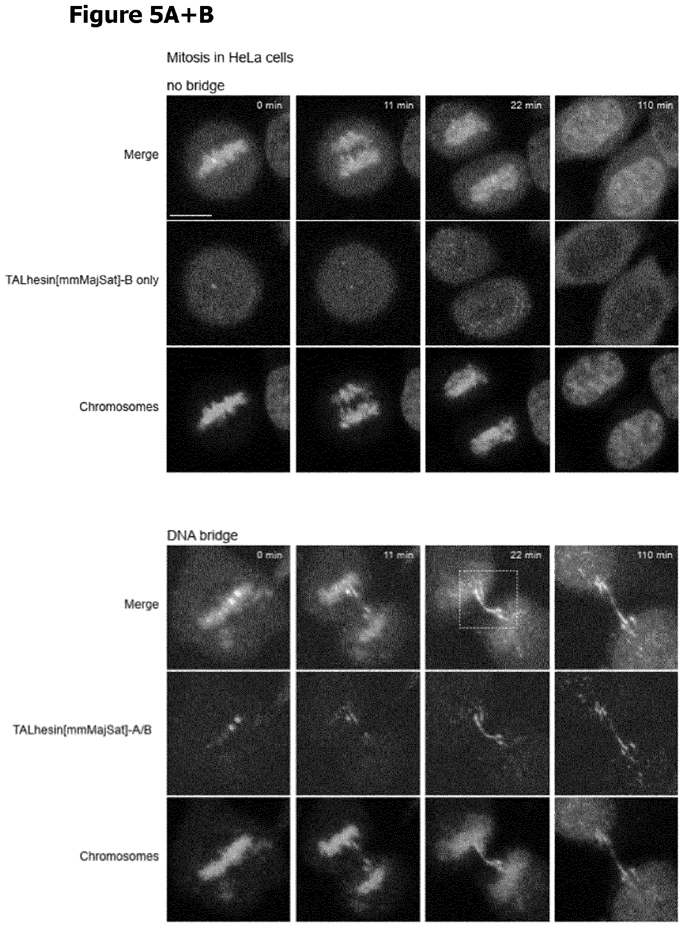

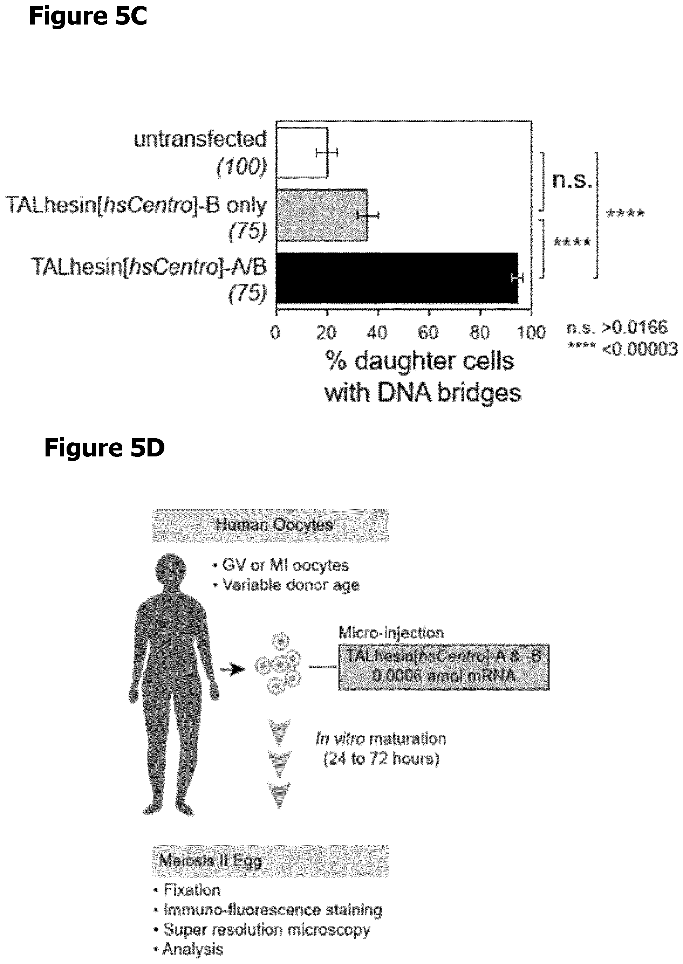

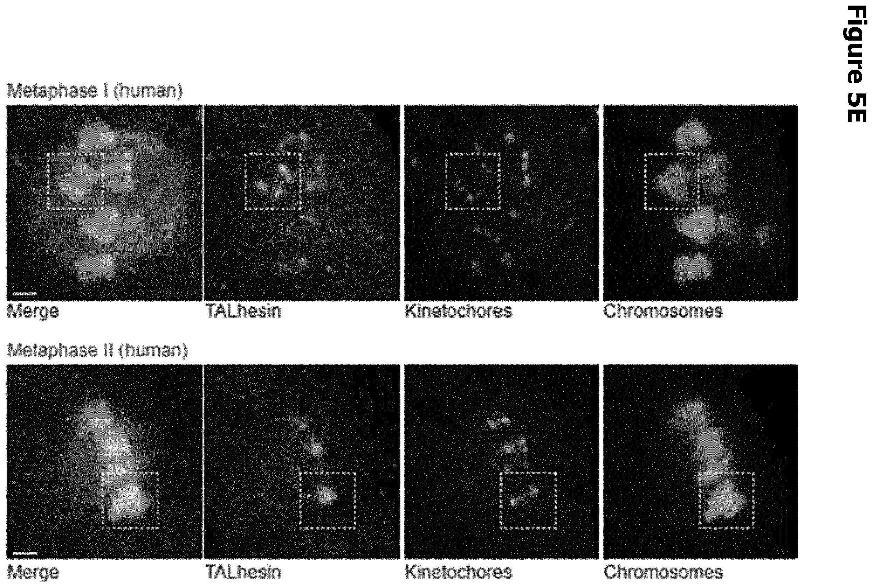

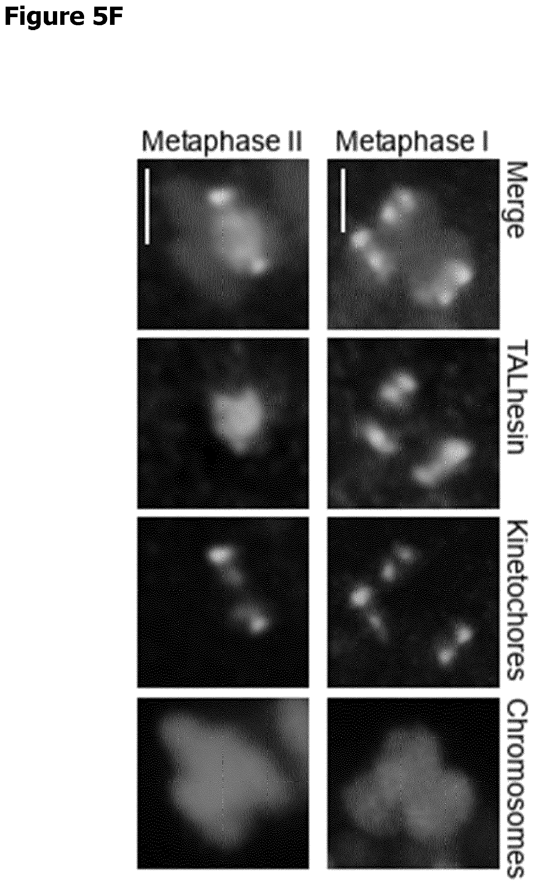

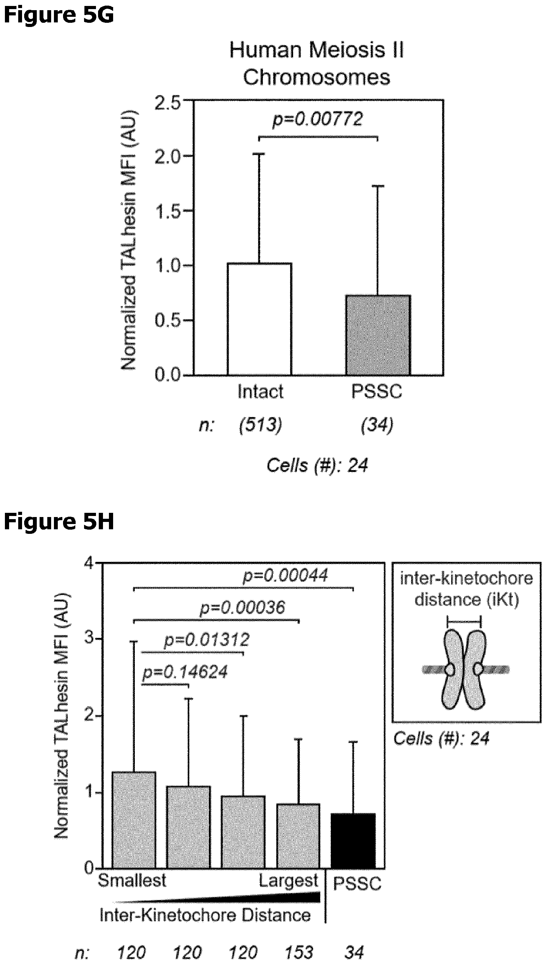

**Fig. 5****. Artificial complex (TALhesin) provides cohesion to human chromosomes**
   **(A)** Representative images of mitotic HeLa cells lacking visible DNA bridges (upper panel), or with apparent DNA bridges (lower panel, Fig. 5B). Arrows indicate *TALhesin[hsCentro]* foci on human chromosomes. Scale bar: 5 µm. **(B)** TALhesin[*hsCentro*] colocalizes with DNA bridging the nuclei of two daughter cells expressing TALhesin[*hsCentro*]A/B. Scale bar: 5 µm. **(C)** Quantification of daughter cells connected by DNA bridges in untransfected HeLa cells (hollow bar), transfected with TALhesin[*hsCentro*]-B only (grey bar), or TALhesin[*hsCentro*]A/B (filled bar). In parentheses, number of daughter cell pairs analyzed for each condition. Statistics measured by two sample T-test (equal variance not assumed) with Bonferroni correction. Bars: SEM. **(D)** Schematic depicting treatment of human oocytes with *TALhesin[hsCentro].* **(E)** TALhesin[*hsCentro*] localization to human meiosis I (upper panel) and meiosis II chromosomes (lower panel). Kinetochores; microtubules; and, chromosomes. Scale bar: 5 µm. **(F)** Magnification of boxed chromosomes in Fig. 5e. *TALhesin[hsCentro]* localizes to pericentromeric regions of human chromosomes in meiosis I (upper) and meiosis II eggs (lower). Scale bars: 2 µm. **(G)** Normalized *TALhesin[hsCentro]* mean fluorescence intensity (MFI) of paired chromosomes and of prematurely separated sister chromatids (PSSC) in human meiosis II eggs. **(H)** Normalized *TALhesin[hsCentro]* MFI of intact human chromosomes grouped according to smallest to largest inter-kinetochore distances, and of PSSCs. For **G** and **H,** number of analyzed sister kinetochore pairs, n, is indicated below each condition. Total number of cells analyzed is 24. Statistics were measured by two sample T-test (equal variance not assumed). mos.: months; Bars: SD.

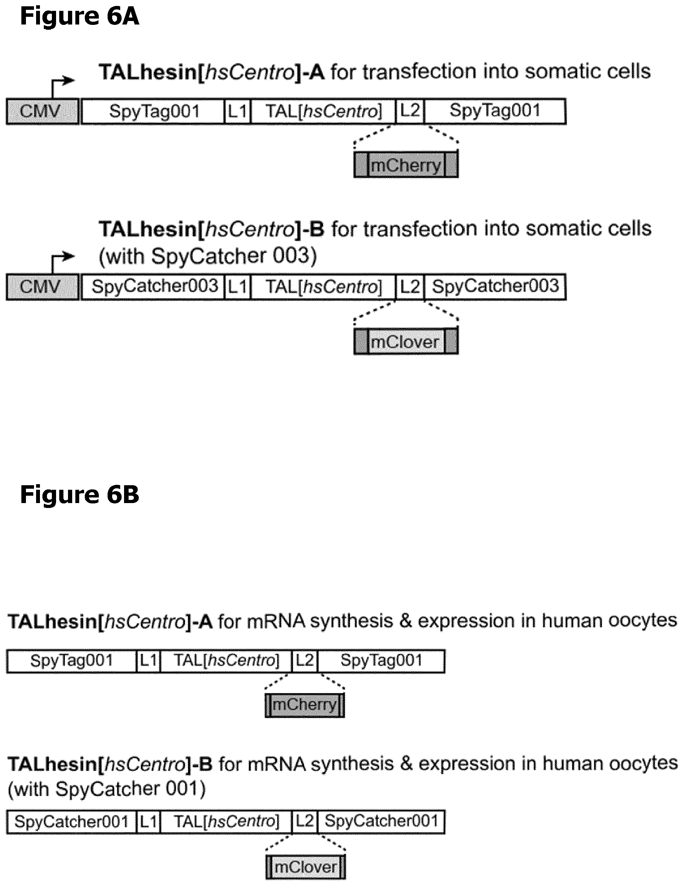

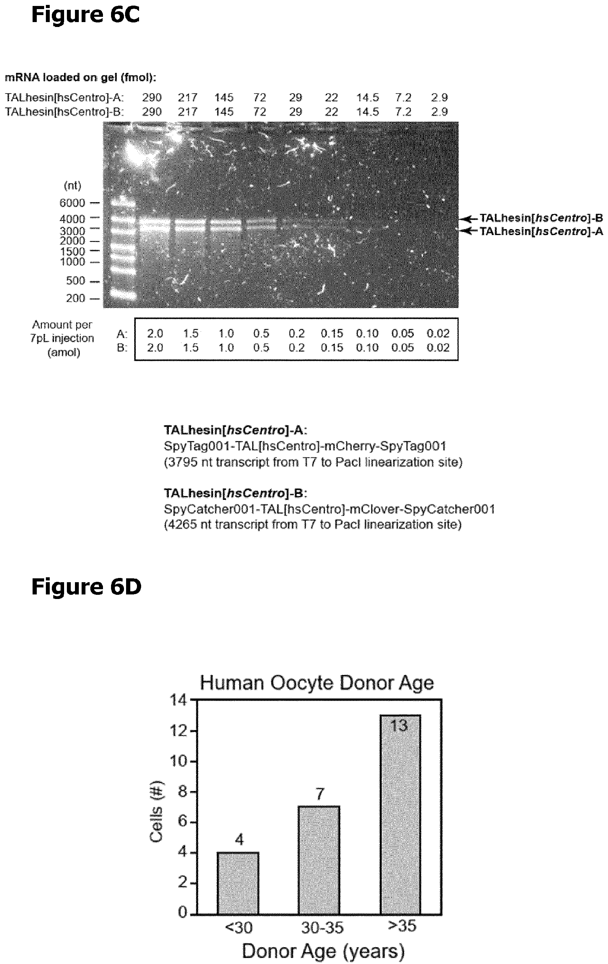

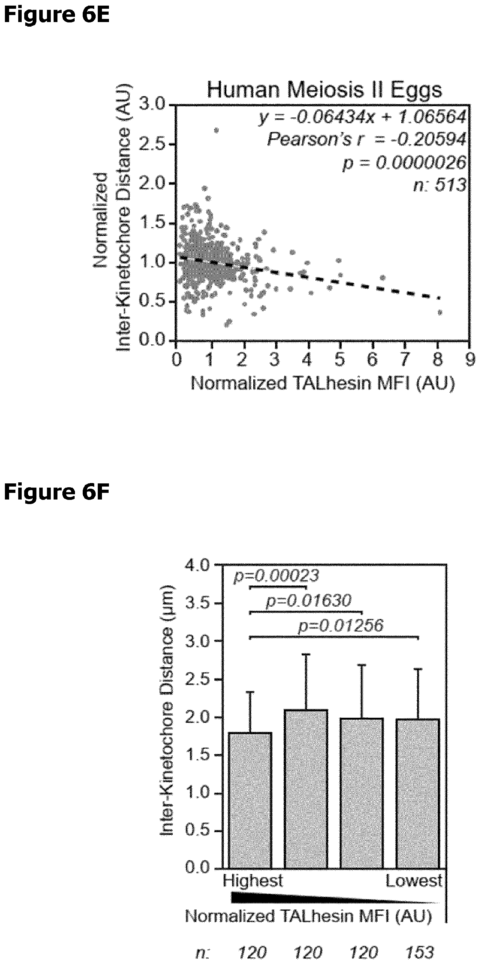

**Fig. 6****. Artificial complex (TALhesin) provides cohesion to human chromosomes.**
   **(A)** Schematic of domain arrangement for *TALhesin[hsCentro]* with Spy-Catcher 003 targeted to human pericentromeric repeats (not drawn to scale) used for expression in HeLa cells. **(B)** Schematic of domain arrangement for TALhesin[*hsCentro*] with Spy-Catcher001 targeted to human pericentromeric repeats (not drawn to scale) used for expression in human oocytes. **(C)** TALhesin[*hsCentro*]A/B (with Spy-Catcher 001) mRNA analyzed by 1% agarose gel electrophoresis used for human oocyte micro-injection. The domain composition and expected size of each transcript is indicated next to the annotated image in nucleotides (nt). **(D)** Distribution of human oocyte donor ages. Number of cells from each age group is indicated at the top of each bar. **(E)** Normalized mean fluorescence intensity (MFI) of TALhesin[*hsCentro*] is inversely related to normalized inter-kinetochore distance in treated human meiosis II eggs. **(F)** In human meiosis II eggs, chromosomes with high TALhesin[*hsCentro*] MFI have lowest interkinetochore distance. Sister kinetochore pairs, n, is indicated below each condition. Total number of cells analyzed is 24. Statistics were measured by two sample T-test (equal variance not assumed). Bars: SD.

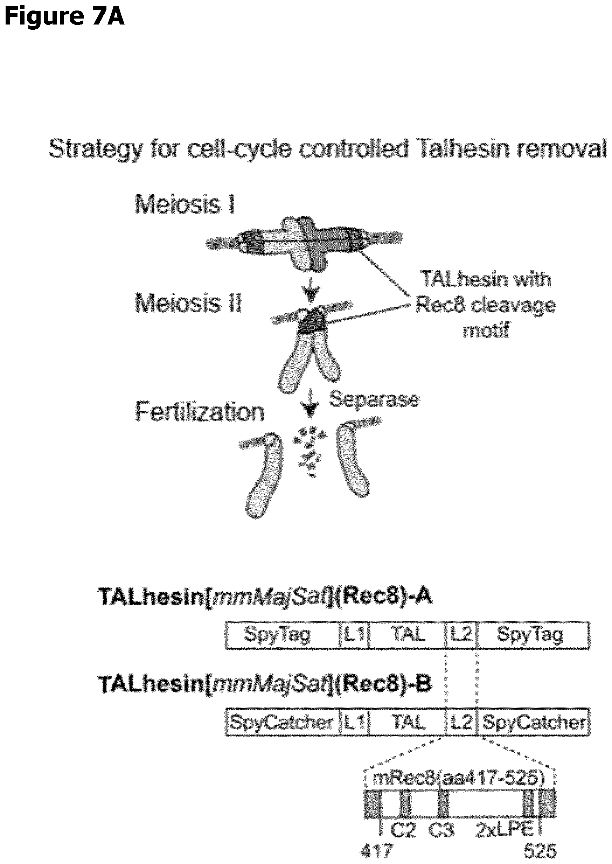

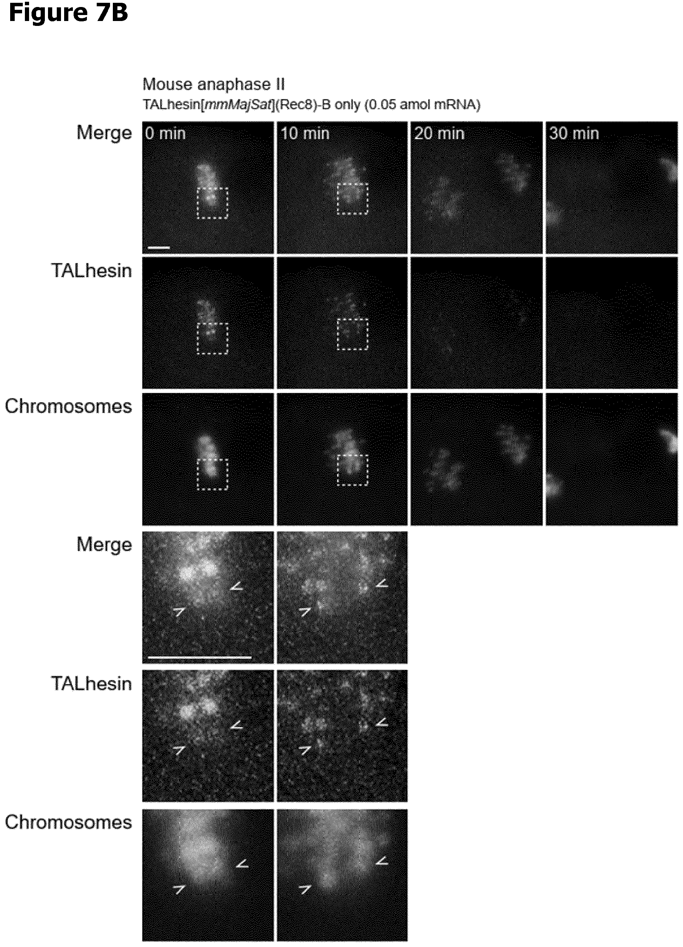

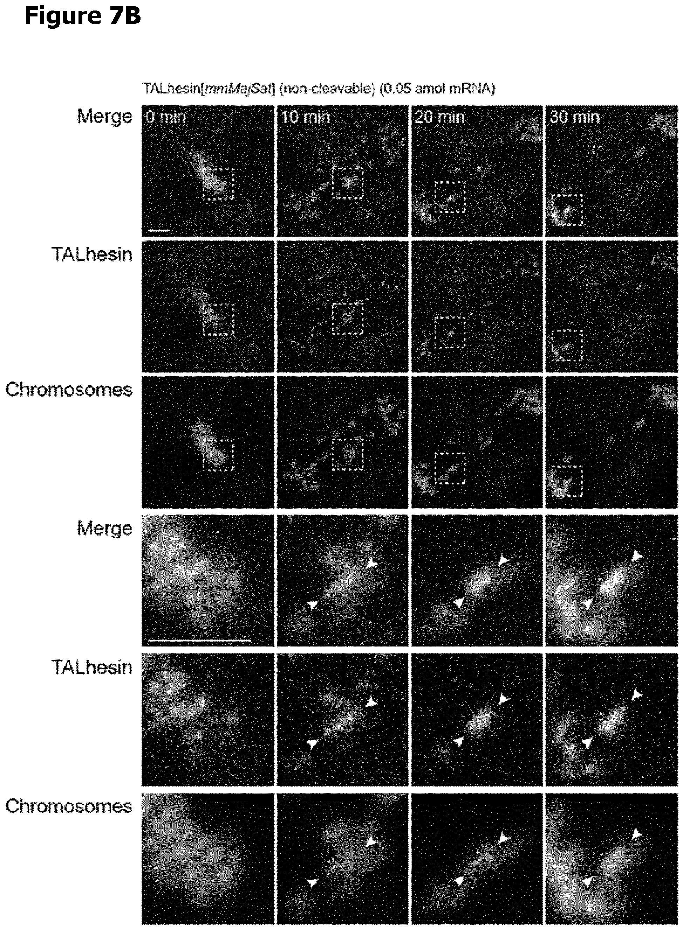

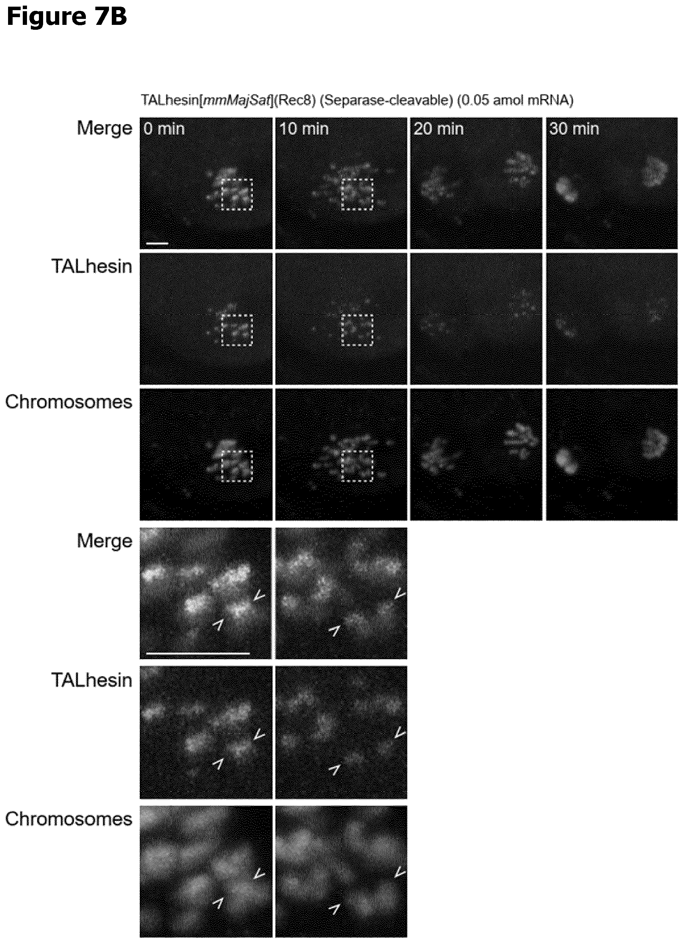

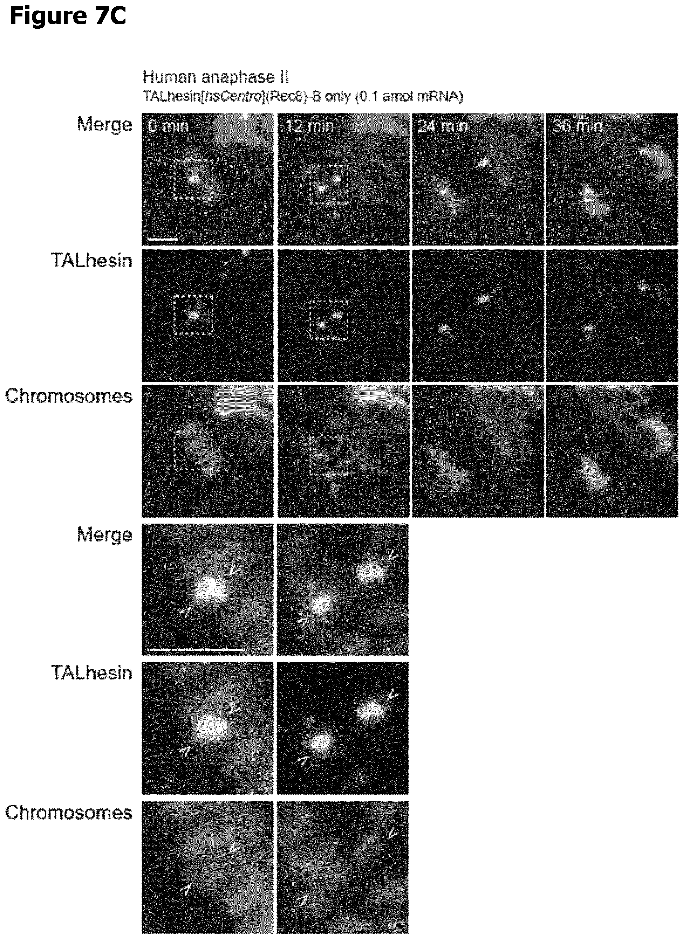

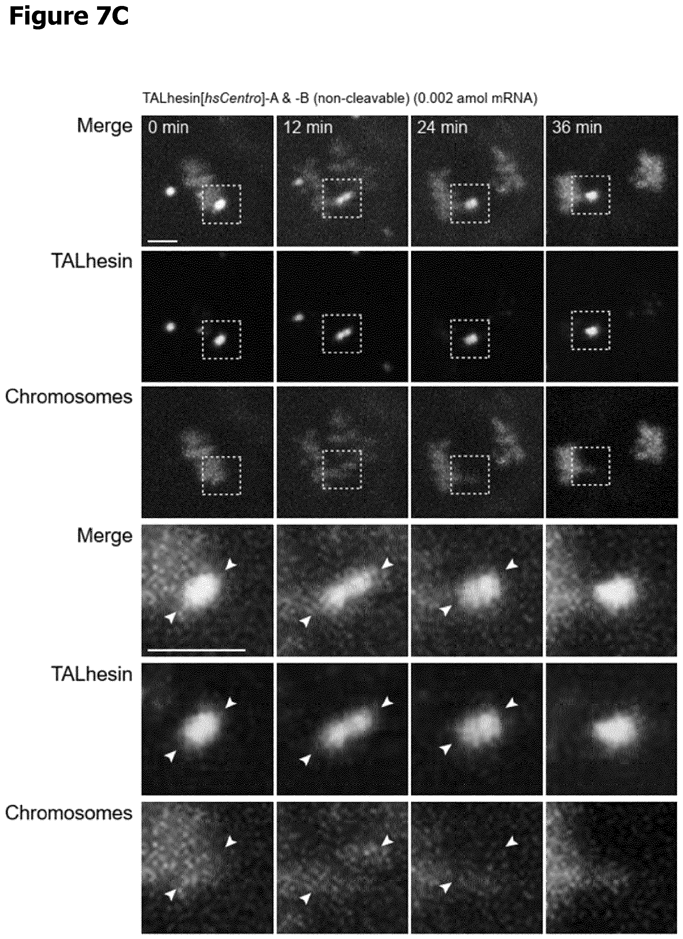

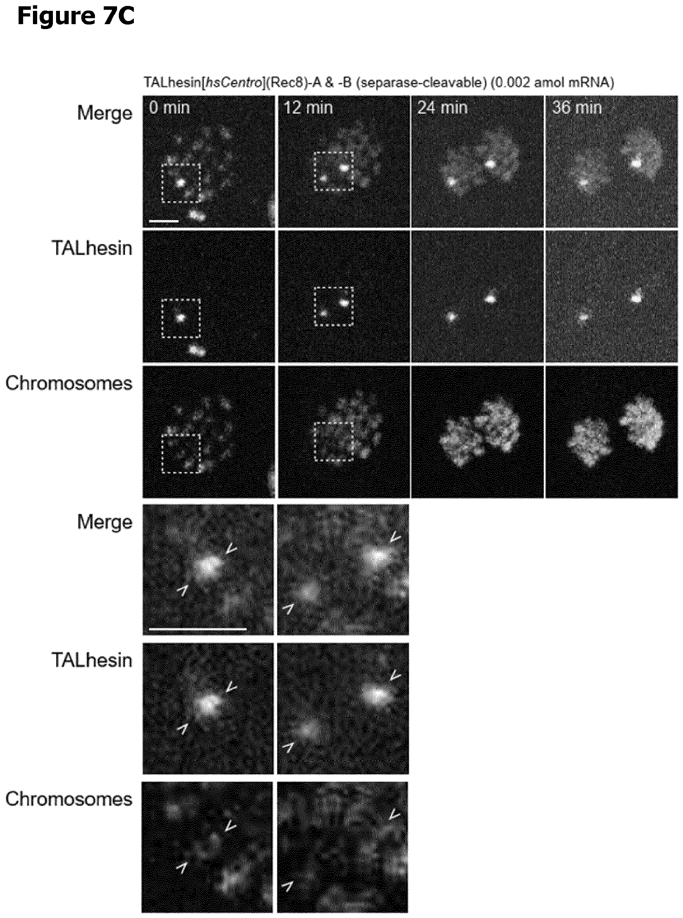

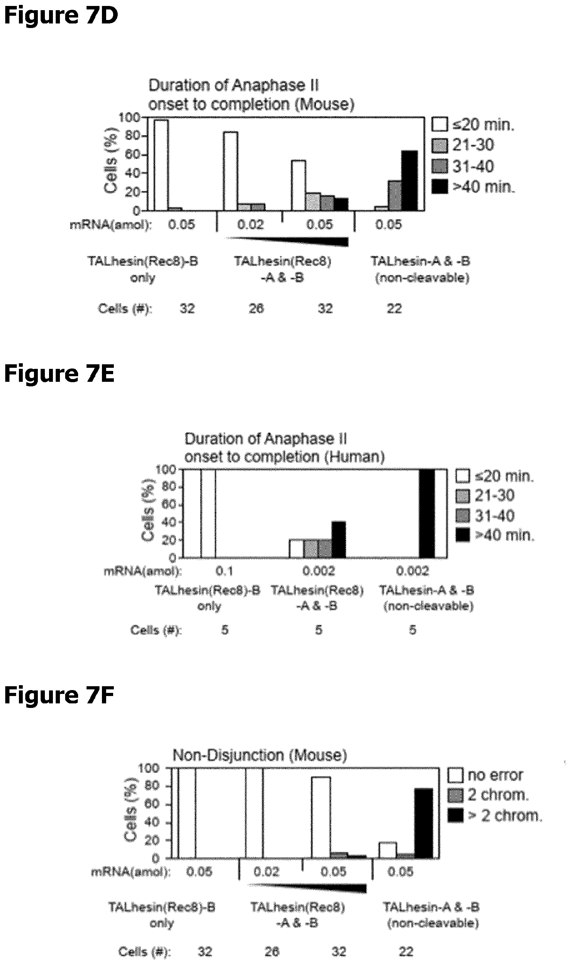

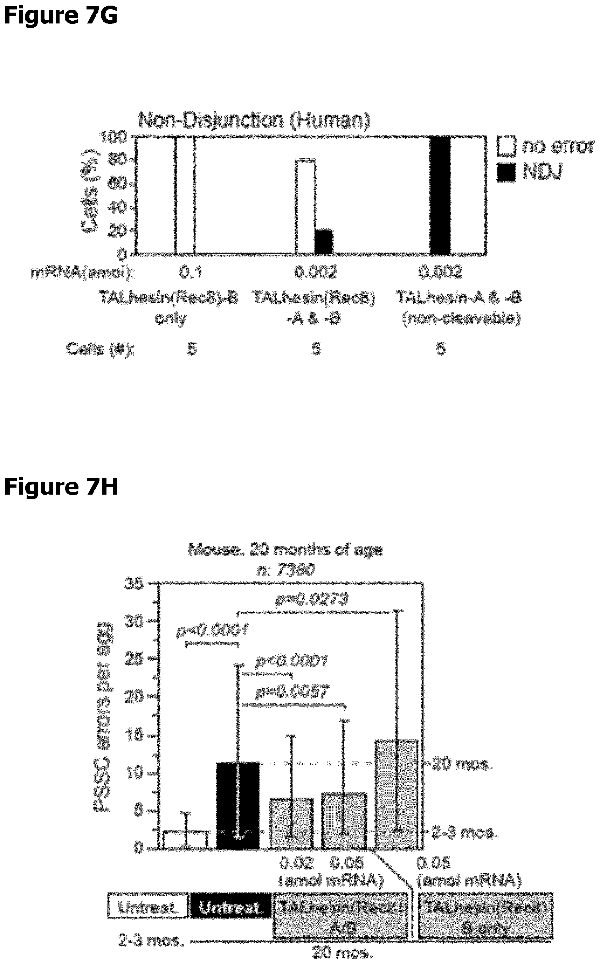

**Fig. 7****. Separase cleavage motifs disengage artificial complex-mediated chromosome cohesion.**
   **(A)** Schematic for deactivation of TALhesin-mediated chromosome cohesion (left) and domain arrangement for TTALhesin[*mmMajSat*](Rec8) with integrated mouse Rec8 fragment into the linker-2 site (right; not drawn to scale). **B-C,** Live imaging of activated mouse **(B)** or human eggs **(C)** micro-injected with mRNA coding TALhesin(Rec8)-B only (upper panels), non-cleavable TALhesin (middle panels), or separase-cleavable TALhesin(Rec8) (lower panels). Boxed regions are magnified below. Filled arrowheads indicate chromosome non-disjunction; hollow arrows indicate chromatid separation. TALhesins; chromosomes; min: minutes. Scale bars: 5 µm. **D-E,** Anaphase II duration of activated mouse **(D)** or human eggs **(E)** measured from anaphase II onset to completion (min: minutes). **F-G,** Percentage of eggs with non-disjoining chromosomes in anaphase II of activated mouse **(F)** or human eggs **(G).** Attomoles (amol) of injected mRNA are listed below each condition. Number of cells analyzed is indicated below for each condition. **(H)** Frequency of PSSC in untreated eggs from young (hollow bar) and aged (filled bar) mice, and aged eggs treated with TALhesin[*mmMajSat*](Rec8)-A/B mRNA (grey bars), n, number of chromosomes analyzed. Statistical significance measure by Fisher's exact test. Bars: 95% CI, 1 degree of freedom.

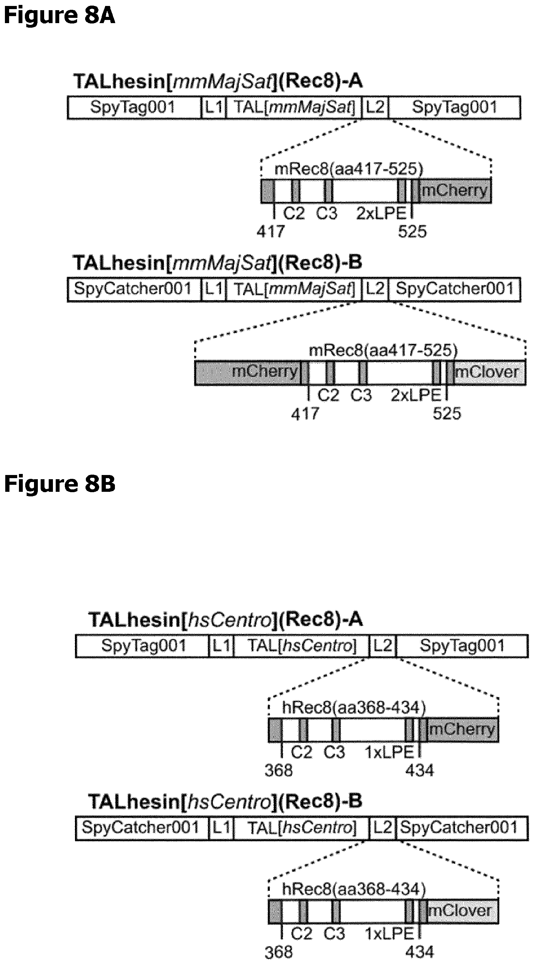

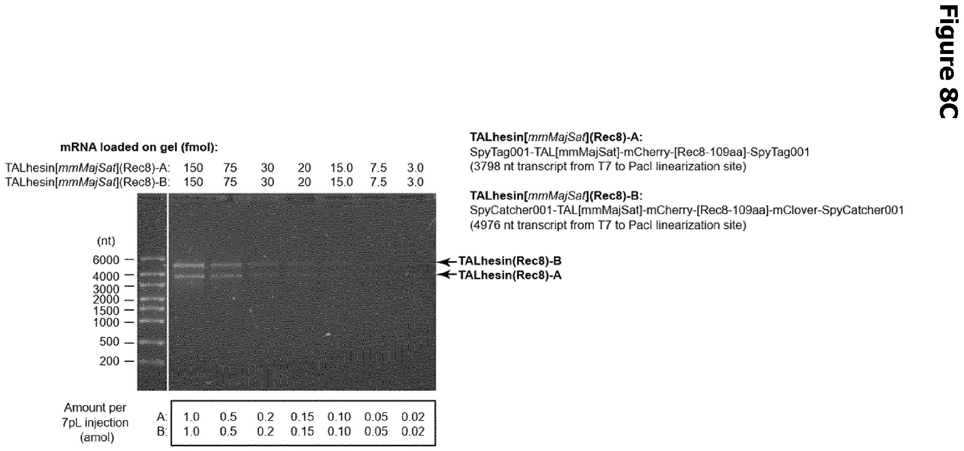

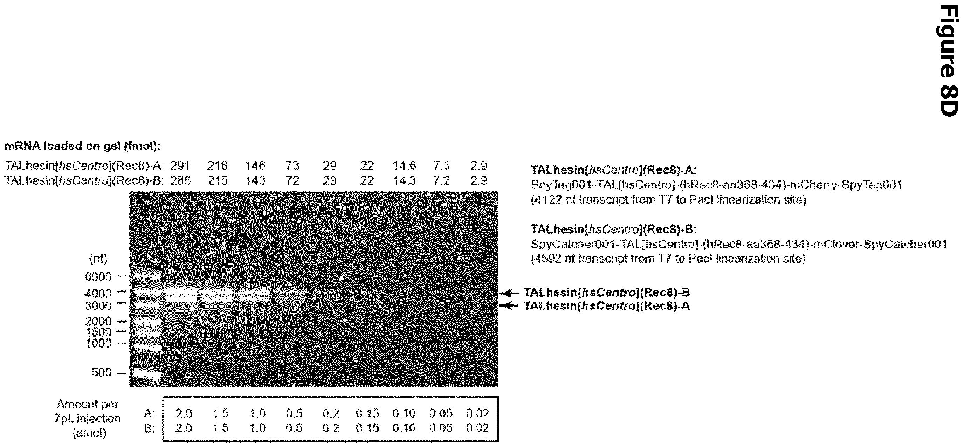

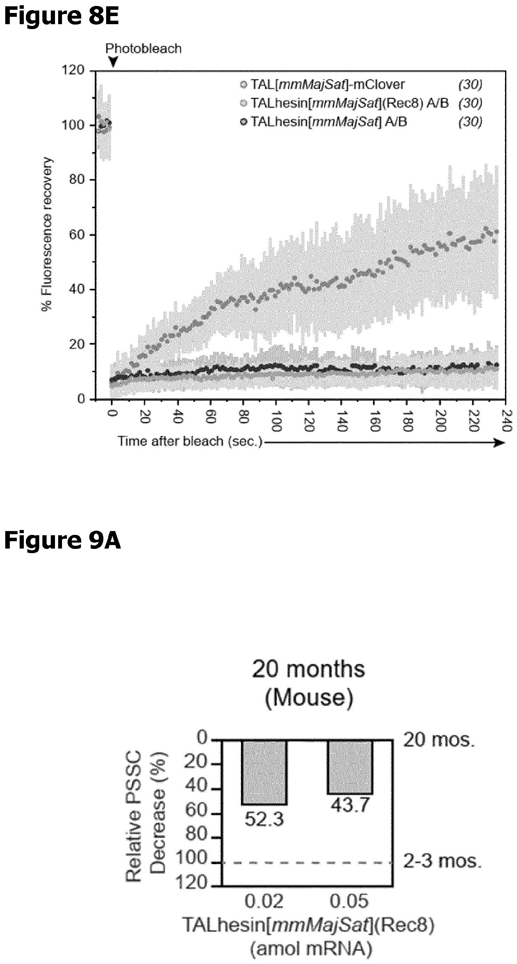

**Fig. 8****. Integrating Rec8 motifs into the artificial complex architecture.**
   **(A)** Schematic of domain arrangement for TALhesin[*mmMajSat*](Rec8) targeted to mouse pericentromeric repeats (not drawn to scale). **(B)** Schematic of domain arrangement for TALhesin[*hsCentro*](Rec8) targeted to human pericentromeric repeats (not drawn to scale). **(C)** TTALhesin[*mmMajSat*](Rec8) components-A and -B mRNA analyzed by 1% agarose gel electrophoresis. **(D)** TALhesin[*hsCentro*](Rec8) (with Spy-Catcher 001) components A and B mRNA analyzed by 1% agarose gel electrophoresis used for human oocyte micro-injection. For **C-D,** the domain composition and expected size of each transcript is indicated next to the annotated image in nucleotides (nt). **(E)** Mobility of TTALhesin[*mmMajSat*](Rec8) chromosome foci compared to TALhesin[*mmMajSat*], and TAL[*mmMajSat*] effector alone measured by FRAP. Bars: SD from the mean for 30 replicates of each condition.

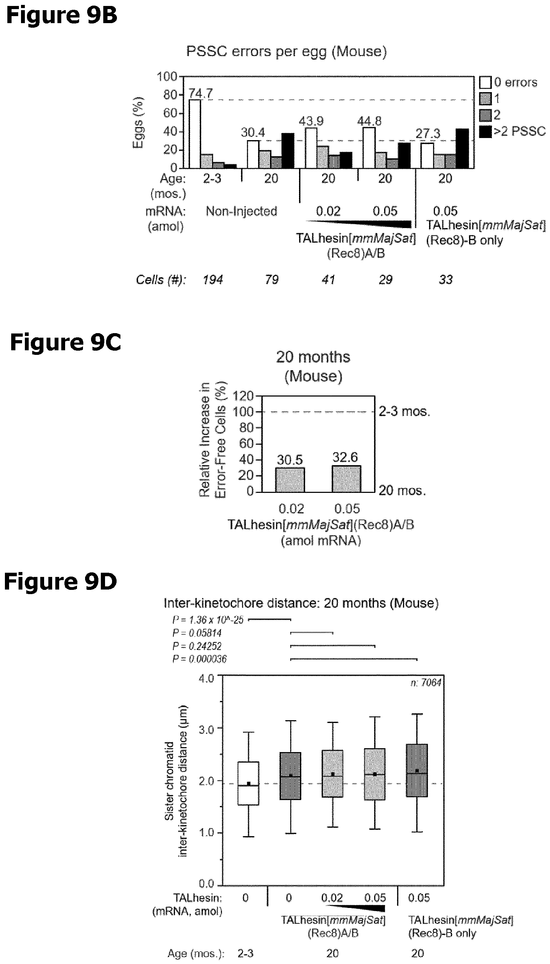

**Fig. 9****. Artificial complex with integrated Rec8 motif reduces PSSC.**
   **(A)** PSSC decrease in eggs from mice aged 20-months treated with 0.02 or 0.05 amol TALhesin[*mmMajSat*](Rec8) mRNA relative to untreated young and eggs from mice aged 20-months. **(B)** Percentage of eggs with zero (hollow bar), one (light grey bar), two (dark grey bar), or more than two (filled bar) PSSC errors from mice aged 20-months treated with TALhesin[*mmMajSat*](Rec8), compared to untreated young and eggs from mice aged 20-months. Eggs expressing 0.05 amol TTALhesin[*mmMajSat*](Rec8) component B mRNA only is shown on far right. Number of eggs analyzed are indicated below each condition. **(C)** Increase in error-free eggs from mice aged 20-months treated with 0.02 or 0.05 amol TALhesin[*mmMajSat*](Rec8) mRNA relative to untreated eggs from young and mice aged 20-months. **(D)** Sister chromatids inter-kinetochore distance (iKt) in eggs from young mice (hollow bars), and mice 20-months treated with TTALhesin[*mmMajSat*](Rec8) (light grey bars), or untreated (dark grey bar, left). iKt for eggs expressing 0.05 amol TALhesin[*mmMajSat*](Rec8) component B mRNA only is shown on right with dark grey bar. Statistics were measured by two sample T-test (equal variance not assumed). Median is indicated with line; mean is indicated by filled square; bars: SD, mos.: months.

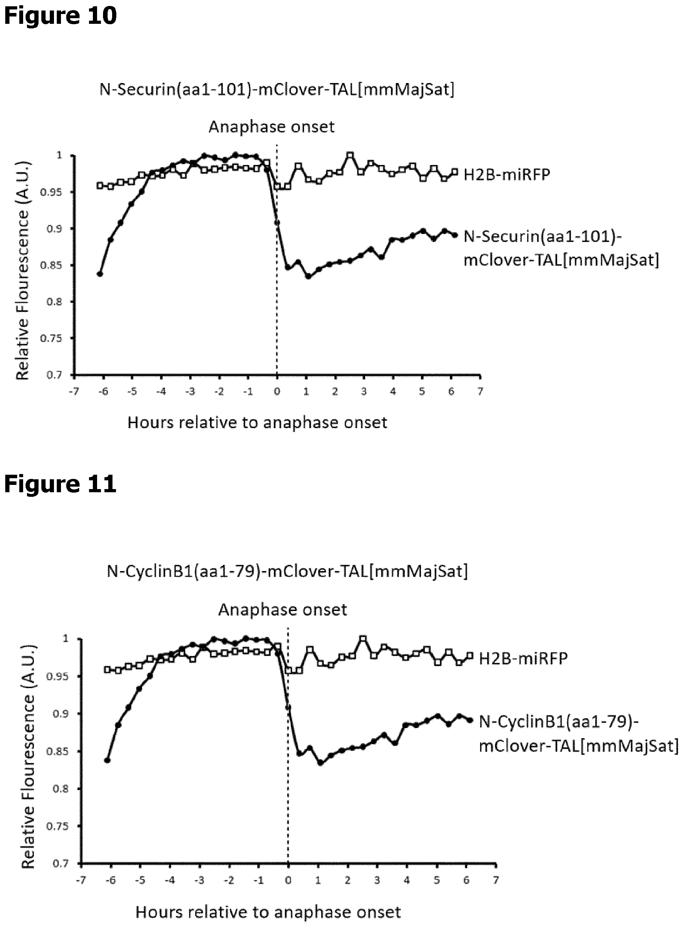

**Fig. 10****. Chromatin binding component (TAL protein) fused with N-Securin(aa1-101) is degraded in anaphase I of a mouse oocyte.**
   Relative fluorescence intensity of pericentromeric N-Securin(aa1-101)-mClover-TAL[mmMajSat] foci (filled circles) decreases around onset of anaphase I (dotted line). Relative fluorescence intensity of the chromosome reporter H2B-miRFP is independent of anaphase I (empty squares). Left of dotted line is hours prior to onset of anaphase I; Right of dotted line is hours after anaphase I onset.
**Fig. 11****. TAL protein fused with N-CyclinB1(aa1-79) is degraded in anaphase I of a mouse oocyte.**
   Relative fluorescence intensity of pericentromeric N-CyclinB1(aa1-79)-mClover-TAL[mmMajSat] foci (filled circles) decreases around onset of anaphase I (dotted line). Relative fluorescence intensity of the chromosome reporter H2B-miRFP is independent of anaphase I (empty squares). Left of dotted line is hours prior to onset of anaphase I; Right of dotted line is hours after anaphase I onset.

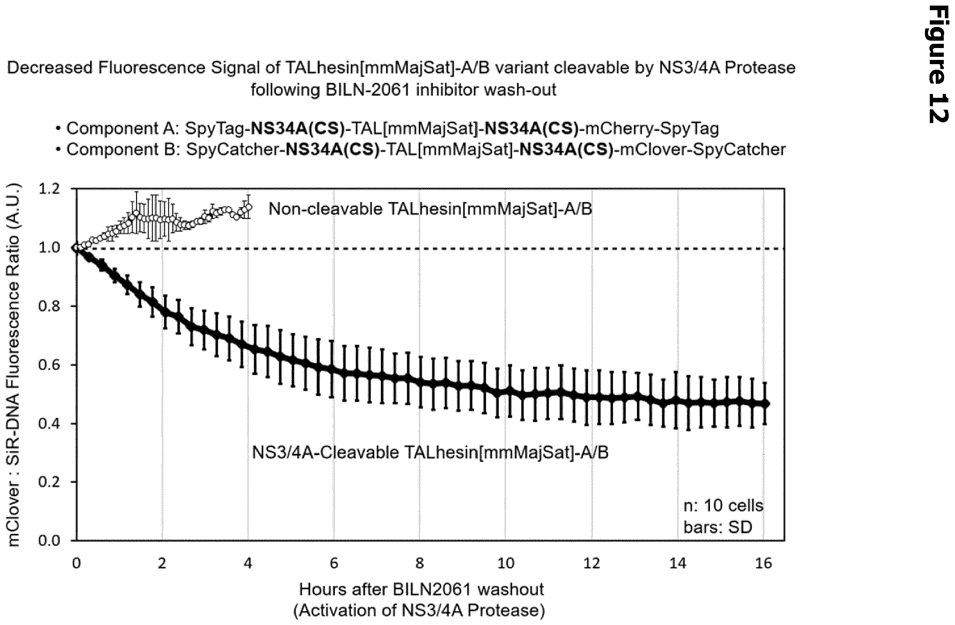

**Fig. 12****. NS3/4A protease degrades artificial complex on mouse oocyte chromosomes.**
   Fluorescence chromosome foci signal of TALhesin[mmMajSat]-A/B containing 2x NS3/4A proteolytic motifs decreases relative to chromosome dye (SiR-DNA) in the hours following BILN-2061 wash-out (filled diamonds). Non-cleavable TALhesin does not decrease after BILN-2061 wash-out (empty circles). Number of cells analyzed, n, is 10. Bars indicate S.D.

### Detailed description of the invention

A complex suitable for cohesion of chromatids or chromosomes comprising (I) one or more first protein(s) and (II) one or more second protein(s) is disclosed, as further defined in the claims. In said complex, the (I) first and the (II) second protein(s) each comprise at least three parts. Specifically, the (I) first and the (II) second protein(s) each comprise **(i)** a chromatin-binding component, **(ii)** a protein-binding region being N-terminal of the chromatin-binding component (PBN region), **(iii)** a protein-binding region being C-terminal of the chromatin-binding component (PBC region), as further defined in the claims. According to this disclosure, (i) the chromatin binding components of the first and the second protein(s) are capable of binding to a target sequence on a chromatid or a chromosome. Furthermore, (I)(ii) the PBN region of the first protein(s) is/are capable of binding to (II)(ii) the PBN region of the second protein(s) and (I)(iii) the PBC region of the first protein(s) is/are capable of binding to (II)(iii) the PBC region of the second protein(s), or wherein (I)(ii) the PBN region of the first protein(s) is/are capable of binding to (II)(iii) the PBC region of the second protein(s) and (I)(iii) the PBC region of the first protein(s) is/are capable of binding to (II)(ii) the PBN region of the second protein(s). As further defined in the claims, the binding to the chromatids or chromosomes and the binding of the PBN and PBC regions of the first and second protein(s) reversibly tether chromatids or chromosomes. The overall arrangement of the complex is illustrated in Figure 1b. Said complex is an artificial protein complex. In other words, said complex is non-naturally occurring. This complex is obtained *ex vivo or* the nucleic acid encoding said complex is obtained *ex vivo.* The complex or the nucleic acid encoding said complex is e.g. obtained by an *in vitro* method. The skilled person is aware that there are commercial providers to obtain purified proteins and/or desired nucleic acid sequences, upon providing the sequence to said commercial providers, such as IDT or ProMab.

The technical advantage of this arrangement of parts is that said complex is capable of, e.g., tethering sister chromatid or chromosomes in order to reduce the risk of premature separation, e.g. premature separation of sister chromatids. As a consequence, the risk of, e.g., aneuploidy is reduced.

As used herein, a 'component' is a part of a larger whole. In the present case, the component is part of the first and the second protein. A 'chromatin binding component' is a part of the first and the second protein, wherein said component is capable of binding to chromatin. As known in the art, chromatin refers to a mixture of DNA and proteins that from the chromosomes found in eukaryotic cells. The chromatin binding component is thus capable of binding to the DNA or the DNA associated proteins. The skilled person is able to assess whether a component is binding to DNA or a DNA associated protein by standard methods in the art. A chromatin binding component may comprise, ideally consist of, one or more domains. Preferably, the chromatin binding component is a chromatin binding domain. In general, a domain is a region of a polypeptide chain that is self-stabilizing and that folds independently from the rest. Generally, each domain forms a compact folded three-dimensional structure.

As used herein, a 'region' is a part of a larger whole. In the present case, the first and the second protein each comprise at least two regions. A'protein binding region' is thus an amino acid sequence, which is capable of binding to another protein. Ideally, the protein binding region is capable of binding to another protein binding region. According to the present invention the protein binding regions are N- terminal and C-terminal of the chromatin binding component. Each of the first and the second protein(s) comprise one protein binding region N-terminal of the chromatin binding component and one protein binding region C-terminal of the chromatin binding region. Ideally, the PBN region is at the N-terminus of the first and the second protein. In particular, the PBC region may be at the C-terminus of the first and the second protein.

### Binding of the PBN and PBC regions

In a first aspect, (I)(ii) the PBN region of the first protein(s) is/are capable of binding to (II)(ii) the PBN region of the second protein(s) and (I)(iii) the PBC region of the first protein(s) is/are capable of binding to (II)(iii) the PBC region of the second protein(s). This arrangement is also depicted in Figure 1b. In said aspect, the protein binding regions bind to each other without induction. This is advantageous as no further trigger for induction is required. Ideally, (I)(ii) the PBN region of the first protein(s) and (I)(iii) PBC region of the first protein(s) have at least 80% sequence identity, preferably at least 83%, more preferably at least 86%, more preferably at least 90%, more preferably at least 93%, even more preferably at least 96%, and most preferably 100% sequence identity. Furthermore, (II)(ii) the PBN region of the second protein(s) and (II)(iii) the PBC region of the second protein(s) may have at least 80% sequence identity, preferably at least 83%, more preferably at least 86%, more preferably at least 90%, more preferably at least 93%, even more preferably at least 96%, and most preferably 100% sequence identity. The skilled person is capable of assessing sequence identity of amino acids sequences, e.g. via online tools such as ExPASy (SIM - Alignment Tool for protein sequences).

In embodiments, the PBN region and the PBC region of the first protein(s) are capable of forming a covalent bond with the PBN region and the PBC region of the second protein(s). As known in the art, covalent bonds are chemical bonds that involve the sharing of electrons to form electron pairs between atoms. For example, an amine bond is a preferred covalent bond. Ideally, the PBN region and the PBC region of the first protein(s) is capable of reconstituting with the PBN region and the PBC region of the second protein(s). 'Reconstituting' as used herein means that two protein binding regions interact to form a covalent amine bond. The PBN region and the PBC region of the first protein(s) is capable of conjugating with the PBN region and the PBC region of the second protein(s). 'Conjugating' as used herein means that the two protein binding regions irreversible interact with each and to form an intermolecular isopeptide bond between. As known in the art, an isopeptide bond is an amide bond that can form between a carboxyl group of one amino acid and an amino group of another and wherein at least one of these joining groups is part of the side chain of one of these amino acids. For example, a spy-tag and a spy-catcher conjugate to form an isopeptide bond. The PBN region and the PBC region of the first and the second protein(s) may by split protein fragments. As known in the art, split protein fragments are capable of reconstituting in order to reconstruct an intact protein from two fragments, i.e. the split protein fragments. In general, splitting bioactive proteins into reconstituting fragments is a powerful strategy for building tools to control biological systems. The skilled person is aware of corresponding split protein fragments and further examples are provided below. For example, the PBN region and the PBC region of the first protein(s) may be a spy-tag. In order to conjugate to the second protein(s), the PBN region and the PBC region of the second protein(s) may be a spy-catcher.

In particular, (I)(ii) the PBN region of the first protein(s) may be selected from a spy-tag, a snoop-tag, a Dog-Tag, and a split intein N-terminal fragment, preferably wherein (I)(ii) the PBN regions of the first protein(s) is a spy-tag. Preferably, (I)(iii) the PBC region of the first protein(s) is selected from a spy-tag, a snoop-tag, a Dog-Tag, and a split intein N-terminal fragment, preferably wherein the (I)(iii) PBC region of the first protein(s) are a spy-tag. Furthermore, the (I)(ii) PBN region of the first protein(s) and the (I)(iii) PBC region of the first protein(s) may be selected from a spy-tag, a snoop-tag, a Dog-Tag, and a split intein N-terminal fragment, preferably wherein the (I)(ii) PBN region of the first protein(s) and the (I)(iii) PBC region of the first protein(s) are a spy-tag.

It is especially preferred that the spy-tag is selected from the spy-tag 001, the spy-tag 002 and the spy-tag 003, and it is even more preferred that the spy-tag is the spy-tag 001. Example 1 shows that a spy-tag 001 is particularly suitable for the complex. In embodiments, wherein the (I)(ii) PBN region of the first protein(s) and/or the (I)(iii) PBC region of the first protein(s) is a split intein N-terminal fragment, the split intein N-terminal fragment may be a split DnaE intein N-terminal fragment, preferably the split DnaE intein N-terminal fragment is from Nostoc punctiforme (Npu).

With regard to the (II)(ii) PBN region of the second protein(s), said protein binding region may be selected from any of a spy-catcher, a snoop-catcher, a Dog-Catcher, and a split intein C-terminal fragment. In embodiments, (II)(iii) PBC region of the second protein(s) is selected from a spy-catcher, a snoop-catcher, a Dog-Catcher, and a split intein C-terminal fragment. Ideally, the (II)(ii) PBN region of the second protein(s) and the (II)(iii) PBC region of the second protein(s) are selected from any of a spy-catcher, a snoop-catcher, a Dog-Catcher, and a split intein C-terminal fragment.

In embodiments wherein the (II)(ii) PBN region of the second protein(s) and/or the (II)(iii) PBC region of the second protein(s) are a spy-catcher, the spy-catcher is ideally selected from the spy-catcher 001, the spy-catcher 002 and the spy-catcher 003. Most preferably the spy-catcher is the spy-catcher 001. In embodiments wherein the (II)(ii) PBN region of the second protein(s) and/or the (II)(iii) PBC region of the second protein(s) are a split intein C-terminal fragment is a split DnaE intein C-terminal fragment, the split DnaE intein C-terminal fragment is preferably from Nostoc punctiforme (Npu).

According to this first aspect of the invention, the (I)(ii) PBN region of the first protein(s) is/are capable of binding to the (II)(ii) PBN region of the second protein(s) and the (I)(iii) PBC region of the first protein(s) is/are capable of binding to the (II)(iii) PBC region of the second protein(s). The corresponding protein binding regions are thus suitable to bind to each other. With regard to split inteins, corresponding split protein fragments, i.e. split intein N-terminal and C-terminal fragments capable of reconstituting, are known in the art. It is known in the art that inteins are protein segments capable of joining adjacent residues via a peptide bond. In this process known as protein splicing, a scarless peptide ligation is achieved. For example, Pinto et al. (2020)⁵² provides 15 examples of split inteins, which are capable of reconstituting. For example, the split intein N-terminal and C-terminal fragments may be selected from any of SspDnaB^{Δ275}M86, Npu^{N}/Ssp^{C}DnaE, gp41-1, gp41-8, NrdJ-1, IMPDH-1, SspDnaX^{Δ297}, SspGyrB^{Δ279}, TerThyX^{Δ134}, TvoVMA, PhoRadA, CIV RIR1, CthATCC27405 TerA, MP-Be DnaB, MP-Catera gp206, PfuRIR1-1, and MjaKIbA.

In preferred embodiments, the (I)(ii) PBN region and (I)(iii) PBC region of the first protein(s) are spy-tags. Specifically, the (I)(ii) PBN region and (I)(iii) PBC region of the first protein(s) may have at least 70% sequence identity to SEQ ID NOs: 4 or 8, preferably at least 80%, more preferably at least 85%, more preferably at least 90%, even more preferably at least 92%, even more preferably at least 94%, even more preferably at least 96%, even more preferably at least 97%, even more preferably at least 98% and most preferably 100%. Furthermore, the (II)(ii) PBN region and (II)(iii) PBC region of the second protein(s) may be spy-catcher. In particular, the (II)(ii) PBN region and (II)(iii) PBC region of the second protein(s) may have at least 70% sequence identity to SEQ ID NOs: 2 or 6, preferably at least 80%, more preferably at least 85%, more preferably at least 90%, even more preferably at least 92%, even more preferably at least 94%, even more preferably at least 96%, even more preferably at least 97%, even more preferably at least 98% and most preferably 100%. As demonstrated in Example 1, spy-tag binds spy-catcher to form a stable covalent isopeptide bond between the first and the second protein(s)²⁴.

In a second aspect of the invention, the (I)(ii) the PBN region of the first protein(s) is/are capable of binding to the (II)(iii) the PBC region of the second protein(s) and the (I)(iii) the PBC region of the first protein(s) is/are capable of binding to the (II)(ii) the PBN region of the second protein(s). Ideally, the said binding of the PBN and PBC regions is inducible. The advantage of this aspect is that the first and the second protein may have the same or a very similar sequence. Hence, only one type of artificial protein, or nucleic acid encoding said artificial protein, needs to be introduced into the cell in order to form a complex. This has the particular advantage that only one introduction is required, e.g. a single injection. Particularly when handling oocytes or egg cells, this is particularly advantageous in order to minimize disturbance of the oocyte or egg cells.

Exemplary inducible protein binding is dependent on the presence of a compound or is light inducible. It is particularly advantageous to have an inducible system as the binding can then be introduced at a desired time point. Ideally, the (I)(ii) PBN region of the first protein(s) is/are capable of reconstituting with the (II)(iii) PBC region of the second protein(s); and the (I)(iii) PBC region of the first protein(s) is capable of reconstituting with the (II)(ii) PBN region of the second protein(s).

In preferred embodiments, the (I)(ii) PBN region of the first protein(s) and (II)(ii) PBN region of the second protein(s) have at least 80% sequence identity, preferably at least 83%, more preferably at least 86%, more preferably at least 90%, more preferably at least 93%, even more preferably at least 96%, and most preferably 100% sequence identity. Preferably, the (I)(iii) PBC region of the first protein(s) and (II)(iii) PBC region of the second protein(s) have at least 80% sequence identity, preferably at least 83%, more preferably at least 86%, more preferably at least 90%, more preferably at least 93%, even more preferably at least 96%, and most preferably 100% sequence identity.

Additionally, the (I)(ii) PBN region and the (I)(iii) PBC region of the first protein(s) may be capable of forming a covalent bond with the (II)(ii) PBN region and the (II)(iii) PBC region of the second protein(s). In said embodiments, the (I)(ii) PBN region of the first protein is capable of forming a covalent bond with the (II)(ii) PBN region of the second protein and the (I)(iii) PBC region of the first protein is capable of forming a covalent bond with the (II)(iii) PBC region of the second protein.

Ideally, the PBN region and the PBC region of the first protein(s) is capable of reconstituting with the PBN region and the PBC region of the second protein(s).

With regard to the inducibility, the binding of the PBN and PBC regions is inducible by light and/or by chemically induced proximity. This has the advantage that an inducer, e.g. light, induces the binding of the protein binding regions. Induced binding has the advantage that the first and the second protein(s) may be present without forming a complex until the inducer is present. Hence, complex formation can be triggered by an inducer at a desired point in time, e.g. a particular time point during the meiosis I or II.

When the binding is induced by light, the binding may be inducible by blue, green or red light, and preferably by blue light. There are light inducible binding systems known in the art. In certain embodiments, the (I)(ii) PBN region and the (I)(iii) PBC region of the first protein(s) are capable of forming a covalent bond with the (II)(ii) PBN region and the (II)(iii) PBC region of the second protein(s); and wherein said binding is inducible by light. There are light inducible binding systems known in the art. For example, the (I)(ii) PBN region of the first protein(s) and/or the (II)(ii) PBN region of the second protein(s) may be a light inducible spy-tag, preferably a blue light inducible spy-tag (BLISS); and the (I)(iii) PBC region of the first protein(s) and/or the (II)(iii) PBC region of the second protein(s) may be a spy-catcher. Exemplary protein and nucleic acid sequences of spy-catchers can be found herein, such as SEQ ID NOs: 1, 2, 5 and 6. Further details on BLISS can be found in Hartzell et al. (2021).⁵³

In certain embodiments, the (I)(ii) PBN region of the first protein(s) has at least 70% sequence identity to SEQ ID NO: 40, preferably at least 80%, more preferably at least 85%, more preferably at least 90%, even more preferably at least 92%, even more preferably at least 94%, even more preferably at least 96%, even more preferably at least 97%, even more preferably at least 98% and most preferably 100%; and the (I)(iii) PBC region of the first protein(s) has at least 70% sequence identity to SEQ ID NO: 40, preferably at least 80%, more preferably at least 85%, more preferably at least 90%, even more preferably at least 92%, even more preferably at least 94%, even more preferably at least 96%, even more preferably at least 97%, even more preferably at least 98% and most preferably 100%. An exemplary nucleic acid sequence encoding the sequence of SEQ ID NO: 40 can be found in SEQ ID NO: 39.

In certain embodiments, the (II)(ii) PBN region of the second protein(s) has at least 70% sequence identity to SEQ ID NO: 40, preferably at least 80%, more preferably at least 85%, more preferably at least 90%, even more preferably at least 92%, even more preferably at least 94%, even more preferably at least 96%, even more preferably at least 97%, even more preferably at least 98% and most preferably 100%; and wherein the (II)(iii) PBC region of the second protein(s) have at least 70% sequence identity to SEQ ID NO: 40, preferably at least 80%, more preferably at least 85%, more preferably at least 90%, even more preferably at least 92%, even more preferably at least 94%, even more preferably at least 96%, even more preferably at least 97%, even more preferably at least 98% and most preferably 100%. An exemplary nucleic acid sequence encoding the sequence of SEQ ID NO: 40 can be found in SEQ ID NO: 39.

In further embodiments, the binding of the PBN and PBC regions is inducible by chemically induced proximity (CIP). As known in the art, the physical distance, or proximity, between molecules often directs biological events. As also known in the art, CIP is generally dependent on membrane-permeable compound that reversibly regulates the proximity of molecules. Numerous examples of CIP are reviewed in Stanton et al. (2018).⁵⁴ The technical advantage of using CIP in the context of artificially tethering chromatids and chromosomes is that the binding as well as the release can be chemically induced by the presence and absence of the compound (see also Figure 1b). Practically, the compound may be added and removed, e.g. washed away, at a desired time points. As the compounds are generally membrane-permeable, the oocyte or egg is minimally disturbed. It is noted that the compound may be added and removed, e.g. by washing away, to/from the cell, when the cell is present *ex vivo.* Hence, any such method is an *in vitro* method. For addition, the compound may be comprised within the medium. For removal the cell may be washed with a medium no longer comprising the compound.

Ideally, the chemically induced proximity is induced by a compound. In particular, said compound may be membrane-permeable. Further in particular, said compound is a small molecule compound. Examples of small molecule compounds for CIP can also be found in Stanton et al. (2018).⁵⁴ In said context, the small molecule compound may be selected from one or two, preferably one, of rapamycin, FK506, FK506-cyclosporin, FK1012, coumermycin, gibberellin, S-(+)-abscisic acid, BisMTX, TMP-Halo (trimethoprim fused to Halo ligand), caffeine and cannabidiol. Based on e.g. Stanton et al. (2018), the skilled person knows which protein pairs show induced binding upon addition of one of the above small molecule compounds.

In addition, examples are provided:

| **(I)(ii) PBN region of the 1st protein(s)** | **(I)(iii) PBC region of the 1st protein** | **(II)(ii) PBN region of the 2nd protein(s)** | **(II)(iii) PBC region of the 2nd protein(s)** | **small molecule compound** |
|---|---|---|---|---|
| FRB | FKBP | FRB | FKBP | rapamycin |
| FKBP | Calciuneurin A | FKBP | Calciuneurin A | FK506 |
| FKBP | Cyclophilin | FKBP | Cyclophilin | FK506-Cyclosporin |
| FKBP | FKBP | FKBP | FKBP | FK1012 |
| GyrB | GyrB | GyrB | GyrB | Coumermycin |
| GID1 | GAI | GID1 | GAI | Gibberellin |
| PYL1 | ABI1 | PYL1 | ABI1 | S-(+)-abscisic acid (ABA) |
| DHFR | DHFR | DHFR | DHFR | BisMTX |
| AcVHH | AcVHH | AcVHH | AcVHH | caffeine |
| FKBP | FRB | FKBP | FRB | rapamycin |
| Calciuneurin A | FKBP | Calciuneurin A | FKBP | FK506 |
| Cyclophilin | FKBP | Cyclophilin | FKBP | FK506-Cyclosporin |
| GAI | GID1 | GAI | GID1 | Gibberellin |
| ABI1 | PYL1 | ABI1 | PYL1 | S-(+)-abscisic acid (ABA) |
| nanobodies capable of heterodimerization | | | | cannabidiol |

Specifically, the (I)(ii) PBN region of the first protein(s) and the (II)(iii) and PBC region of the second protein may be selected from any of FRB and FKBP; FKBP and Calciuneurin A; FKBP and Cyclophilin; FKBP and FKBP; GyrB and GyrB; GID1 and GAI; PYL1 and ABI1; DHFR and DHFR; AcVHH and AcVHH; or nanobodies capable of heterodimerization by cannabidiol; respectively.

In particular, the (I)(iii) PBC region of the first protein(s) and the (II)(ii) and PBN region of the second protein may be selected from FRB and FKBP; FKBP and Calciuneurin A; FKBP and Cyclophilin; FKBP and FKBP; GyrB and GyrB; GID1 and GAI; PYL1 and ABI1; DHFR and DHFR; AcVHH and AcVHH; or nanobodies capable of heterodimerization by cannabidiol; respectively.

With regard to nanobodies capable of heterodimerization by cannabidiol, further information is provided in Kang et al. (2019).⁶¹

It is further preferred that the PBN and PBC regions is inducible by chemically induced dimerization. In general 'dimerization' refers to a process wherein, two identical parts, e.g. monomers, react to form a dimer, i.e. a molecule comprising two identical or similar parts. As shown in the above table, there are examples of chemically induced dimerization, i.e. the chemically induced binding of two identical or similar proteins, such as DHFR, AcVHH, FKBP and GyrB.

For example, AcVHH is a single domain antibody, which dimerizes upon chemically induced dimerization with caffeine. Further information about the AcVHH dimerization upon caffeine binding can be found in Lesne et al. (2019).⁵⁵

In certain embodiments, the chemically induced proximity is a photoswitchable. The skilled person is aware of photoswitchable chemically induced proximity in general. In photoswitchable chemically induced proximity, the compound is usually photoswitchable. Such proximity is induced by 'switching on'the compound, i.e. obtaining a compound capable of inducing proximity. The skilled person is aware that photoswitchable compounds are generally commercially available. For example, the compound is selected from one or two, preferably one, of TMP-Halo (trimethoprim fused to Halo ligand; chemical name: 4,5-Dimethoxy-2-nitrobenzyl(4-amino-5-(4-((21-chloro-5,8-dioxo-12,15-dioxa-4,9-diazahenicosyl)oxy)-3,5-dimethoxybenzyl)pyrimidin-2-yl)carbamate), photo-caged rapamycin, and coumarin cage-TMP-Halo (CTH; chemical Name: CTH; (7-(Diethylamino)-2-oxo-2H-chromen-4-yl)methyl (4-amino-5-(4-((21-chloro-5,8-dioxo-12,15-dioxa-4,9-diazahenicosyl)oxy)-3,5-dimethoxybenzyl)pyrimidin-2-yl)carbamate). For example, photo-caged rapamycin has also been further described in Kargi-nov et al (2011).⁵⁶

Alternatively, the compound may be NVOC cage-TMP-Halo (NTH). The chemical name of NVOC cage-TMP-Halo (NTH) is 4,5-Dimethoxy-2-nitrobenzyl (4-amino-5-(4-((21-chloro-5,8-dioxo-12,15-dioxa-4,9-diazahenicosyl)oxy)-3,5-dimethoxybenzyl)pyrimidin-2-yl)carbamate. In particular, light removes the NVOC cage from TMP-Halo. TMP then binds a DHFR protein domain and Halo-moiety binds to Halo-tag. Further information on NVOC cage-TMP-Halo induced binding can be found in Ballister et al. (2014).⁶⁷

Specific examples of photoswitchable chemically induced proximity in the context of the present disclosure is provided as follows:

| **(I)(ii) PBN region of the 1st protein(s)** | **(I)(iii) PBC region of the 1st protein** | **(II)(ii) PBN region of the 2nd protein(s)** | **(II)(iii) PBC region of the 2nd protein(s)** | **small molecule compound** |
|---|---|---|---|---|
| FRB | FKBP | FRB | FKBP | photo-caged rapamycin |
| DHFR | Halo-Taq | DHFR | Halo-Taq | TMP-Halo |
| DHFR | Halo-Tag | DHFR | Halo-Tag | coumarin cage-TMP-Halo |
| DHFR | Halo-Tag | DHFR | Halo-Tag | NVOC cage-TMP-Halo (NTH) |
| FKBP | FRB | FKBP | FRB | photo-caged rapamycin |
| Halo-Taq | DHFR | Halo-Taq | DHFR | TMP-Halo |
| Halo-Tag | DHFR | Halo-Tag | DHFR | coumarin cage-TMP-Halo |
| Halo-Tag | DHFR | Halo-Tag | DHFR | NVOC cage-TMP-Halo (NTH) |

In certain embodiments, the (I)(ii) PBN region of the first protein(s) and the (II)(iii) and PBC region of the second protein are selected from FRB and FKBP; and DHFR and Halo-tag; respectively. In further certain embodiment, the (I)(iii) PBC region of the first protein(s) and the (II)(ii) and PBN region of the second protein are selected from FRB and FKBP; and DHFR and Halo-tag; respectively. Furthermore, photoswitchable chemically induced dimerization is also further described in Chen et al (2018).⁶⁰

According to the second aspect of the disclosure, the first and the second protein(s) may have a sequence identity of at least 70%, preferably at least 75%, more preferably at least 80%, more preferably at least 85%, even more preferably at least 90%, even more preferably at least 95%, and even more preferably 100% sequence identity.

### Reversible tethering

According to the first and the second aspect of the invention, the binding of the PBN and PBC regions of the first and second protein(s) reversibly tethers chromatids or chromosomes. Ideally, said tethering is reversible by
(a) cleavage of the complex, wherein the first and the second protein(s) of complex additionally comprise a protease cleavage site; and/or
(b) degradation of the complex, wherein the first and/or the second protein(s) of complex additionally comprise a ubiquitination site; and/or
(c) disruption of the binding of the PBN and PBC regions of the first and the second protein(s), wherein said binding is mediated by chemically induced proximity.

In certain embodiments, the tethering is reversible by one of the options (a) to (c), e.g. by cleavage of the complex via a protease cleavage site. In other embodiments, the tethering is reversible by two of the options (a) to (c). In some embodiments, all three options (a) to (c) are present. Experimental data for using a protease cleavage site is provided in Example 1 herein. Experimental data for using a ubiquitination site is provided in Example 2.

The term 'reversible'/'reversibly' as used herein means that something is capable of being reversed so that the previous state is restored. With regard to the present disclosure this means that a 'reversible' tethering means that the tethering is capable of being reversed. In other words, the tethering is capable of being released. After release, the chromosomes or chromatids are no longer tethered.

### Protease cleavage site

It is preferred that tethering of the chromatids or chromosomes is reversible by cleavage of the complex. Said cleavage preferably occurs at a protease cleavage site. Ideally, the (a) protease cleavage site is located between the (ii) PBN region and the (i) chromatin binding component or between the (i) chromatin binding component and the (iii) PBC region of the first or the second protein. In particular, the (a) protease cleavage site is located between the (ii) PBN region and the (i) chromatin binding component or between the (i) chromatin binding component and the (iii) PBC region of the first or the second protein.

Ideally, the complex is cleavable within a naturally occurring environment by a naturally occurring protease. In certain embodiments, the protease cleavage site is cleavable by naturally occurring protease in a fertilized zygote. It is additionally preferred that the protease cleavage site is a separase cleavable site, and it is even more preferred that said separase cleavable site is a Rec8 site. The skilled person is capable of identifying Rec8 sites by using online tools such as Uniprot. Additionally, Kudo et al. (2009)⁵⁷ provides further information regarding Rec8 cleavage sites. For example, Kudo et a. (2009) describes a variety of separase cleavable sites, including mouse Rec8 sites.

Preferably, the Rec8 site is a mammalian Rec8 site. The Rec8 site may be from a veterinary animal. In particular, the mammalian Rec8 site may originate from a human, cat, dog, horse, cattle, sheep, goat or pig. The Rec8 site may be from an endangered animal such as a panda, an orangutan, a saola, a tiger, a rhinoceros, a leopard, a gorilla, an elephant, a bonobo, or a dolphin. Even more preferably, the Rec8 site originates from a human, cat, dog or horse. Ideally, the Rec8 site is a human Rec8 site.

It is also preferred that the first and the second protein each comprise at least two Rec8 cleavage sites. Ideally, the first and the second protein each comprise two Rec8 cleavage sites. Experimental data in support of having two Rec8 cleavage sites is provided in Example 1 herein. In particular, wherein the first and the second protein each comprise the protein cleavable site additionally comprises a Rec8 leucine-proline-glutamic acid motif (LPE-motif). The skilled person is provided with additional information concerning said motif in Rosen et al. (2019).³⁷ In some embodiments, at least one protease cleavage site has at least 75% identity to SEQ ID Nos: 30 or 32. Corresponding nucleic acid sequences can be found in SEQ ID Nos: 29 and 31.

The first and the second protein(s) may comprise two or more protease cleavage sites. It is preferred that the two or more protease cleavage sites are two Rec8 cleavage sites. As also shown in Example 1, it is preferred that said two Rec8 cleavage sites additionally comprise a Rec8 LPE-motif.

It is preferred that the first and the second protein each comprise two protease cleavage sites which have at least 70% sequence identity to SEQ ID NO: 28, preferably at least 75%, more preferably at least 80%, more preferably at least 85%, even more preferably at least 90%, even more preferably at least 93%, even more preferably at least 96%, even more preferably at least 97%, even more preferably at least 98% and most preferably 100% sequence identity. An exemplary nucleic acid sequence is provided in SEQ ID NO: 27.

In certain embodiments, the protease is a non-naturally occurring enzyme in a fertilized zygote. In other words, such protease cleavage sites are not cleaved naturally in a fertilized zygote. Such protease cleavage site may cleavable by a cysteine or serine protease. Exemplary proteases are a TEV or an HCV NS3/4 protease.

In some embodiments, the protease is capable of being inhibited by a small molecule inhibitor. An exemplary inhibitor is BILN-2061. Such inhibitors are commercially available. Example 2 provides experimental evidence that NS3/4 protease cleavage sites as well as the inhibitor BILN-2061 work in the context of artificial cohesin.

### Ubiquitination site

It is preferred that tethering of the chromatids or chromosomes is reversible by the presence of (b) a ubiquitination site within the first or the second protein(s). The tethering of the chromatids or chromosomes may be reversible by the presence of (b) a ubiquitination site within both the first and the second protein(s). In such a case, the first and/or the second protein(s) of complex additionally comprise(s) a ubiquitination site. This arrangement is also depicted in Figure 1b. Experimental evidence is provided in Example 2.

Ubiquitination can be applied to a variety of amino acids within the first and/or the second protein. For example, the ubiquitination site may comprise one or more lysines. Preferably, the ubiquitination site comprises one lysine. Alternatively, the ubiquitination site may comprise two lysines.

During meiosis, securin inhibits separase, which cleaves the cohesin rings holding chromosomes together. Securin must be depleted before anaphase to ensure chromosome segregation occurs with anaphase. In cells, securin is naturally depleted by proteolysis, i.e. the securin becomes ubiquitinated and thus degraded. Naturally, securin is ubiquitinated by APC/C. The skilled person is aware that APC/C is the abbreviated term for Anaphase-promoting complex/cyclosome. APC/C is a E3 ubiquitin ligase that marks target cell cycle proteins for degradation. Ubiquitination sites being ubiquitinated by APC/C will be degraded by the 26S proteasome in cells. One major target for degradation by the APC/C is securin and cyclin, including cyclin B. Securin releases separase, a protease. Separase then triggers the cleavage of cohesin, the protein complex that binds sister chromatids together.

The inventors transferred and adapted this idea to artificial cohesin and importantly provided proof-of-concept data in Example 2 herein. Specifically, the first and/or the second protein(s) of the complex may comprise a ubiquitination site. According to the disclosure, said ubiquitination site may be ubiquitinated and thus degraded. Specifically, said ubiquitination site may be capable of being ubiquitinated by APC/C. According to this disclosure, the ubiquitination site is ideally an amino acid sequence of securin that is capable of being ubiquitinated by APC/C. According to this disclosure, the ubiquitination site is ideally an amino acid sequence of a cyclin, preferably cyclin B, more preferably cyclin B2, that is capable of being ubiquitinated by APC/C. The skilled person is capable of tracing securin and cyclin sequences via Online Tools such as Uniprot. The sequence of human securin can for example be found via the Uniprot identifier 095997. The sequence of human cyclin B1 can for example be found via the Uniprot identifier P14635. Similarly, the corresponding sequence for mouse, cat, dog and other animals can be found. If a ubiquitination site is included in the artificial complex, the ubiquitination site should match the animal in which the artificial cohesin is used. The particular advantage of a release via a ubiquitin site becoming ubiquitinated naturally, e.g. by APC/C, has the advantage that the artificial cohesin is released at the onset of anaphase by the natural enzymes in the cell. Hence, the complex becomes degraded at the same or similar time as the natural cohesion system would become degraded. Therefore, no further intervention is required and thus these embodiments are saving the step of induction of the release.

Example 2 provides experimental evidence that artificial cohesin is degraded at the onset of anaphase when it comprises a ubiquitination site, which is capable of being ubiquitinated by APC/C. Upon degradation of artificial cohesin, the artificial cohesin no longer tethers chromosomes or chromatids.

It is preferred that the amino acid sequence of securin has at least 25 amino acids, preferably at least 40 amino acids, more preferably at least 60 amino acids, more preferably wherein at least 80 amino acids, and even more preferably from 80 amino acids to 150 amino acids of securin. Ideally, the amino acid sequences of securin is located at the N-terminus of securin. Ideally, the securin sequence originates from human, cat, dog, horse, cattle, sheep, goat or pig. The securin sequence may originate from a veterinary animal. The securin sequence may be from an endangered animal such as a panda, an orangutan, a saola, a tiger, a rhinoceros, a leopard, a gorilla, an elephant, a bonobo, or a dolphin. Even more preferably, the securin sequence originates from a human, cat, dog or horse. Ideally, the securin sequence is a human securin sequence.

In particular, the sequence may originate from human, cat, dog or horse. In certain embodiments, the sequence originates from human. It is preferred that the amino acid sequence of securin is ubiquitinated at entry into anaphase.

It is preferred that the amino acid sequence of cyclin has at least 25 amino acids, preferably at least 40 amino acids, more preferably at least 60 amino acids, and even more preferably from 60 amino acids to 300 amino acids of cyclin. Ideally, the amino acid sequences of cyclin is located at the N-terminus of cyclin. In particular, cyclin B is preferred, while cyclin B1 is even more preferred. Ideally, the cyclin sequence originates from human, cat, dog, horse, cattle, sheep, goat or pig. The cyclin sequence may originate from a veterinary animal. The cyclin sequence may be from an endangered animal such as a panda, an orangutan, a saola, a tiger, a rhinoceros, a leopard, a gorilla, an elephant, a bonobo, or a dolphin. Even more preferably, the cyclin sequence originates from a human, cat, dog or horse. Ideally, the cyclin sequence is a human cyclin sequence. It is preferred that the amino acid sequence of cyclin is ubiquitinated at entry into anaphase.

As noted above, it is preferred that tethering of the chromatids or chromosomes is reversible by the presence of (b) a ubiquitination site within the first and/or the second protein(s). Said tethering may be reversible by targeted protein degradation. Targeted protein degradation is the overall process of targeting a protein for destruction. Hence, the first and/or the second protein(s) of the complex may comprise a ubiquitination site, which becomes ubiquitinated via targeted protein degradation. Ideally, the first and/or the second protein(s) further comprises a PROTAC binding site. A proteolysis targeting chimera (PROTAC) is a heterobifunctional molecule composed of two active domains and a linker, capable of binding to the PROTAC binding site and to an E3 ubiquitin ligase. Upon presence of the PROTAC, the first and/or the second protein(s) becomes ubiquitinated by an E3 ligase and thus the first and/or the second protein is proteolyzed. In these embodiments, the target of said targeted protein degradation is the first and/or the second protein(s). In embodiments of this disclosure, the complex further comprises a PROTAC binding site. In other words, the PROTAC binds to said PROTAC binding site and thereby recruits an E3 ubiquitin ligase. In preferred embodiments, the presence of a PROTAC results in the ubiquitination of the complex. Targeted protein degradation has the particular advantage that the degradation can be induced at a desired time point, e.g. at the onset of anaphase, by the presence of the PROTAC. Said PROTAC may be membrane-permeable so that said PROTAC may be added to the medium at a desired time point in order to release tethering. Hence, the release can be induced at any desired time point.

For example, the PROTAC binding site may be selected from a group consisting of an androgen receptor domain, oestrogen receptor domain, a BRD9 domain, a B-cell extra-large domain (BCL-xL), an IRAK4 domain, a STAT3 domain, a BTK domain, an EGFR domain, and a tropo-myosin receptor domain. Further information on PROTACs has been reviewed in Bekes et al (2022).⁵⁸

### Chemically induced proximity

It is preferred that tethering of the chromatids or chromosomes is reversible by disruption of the binding of the PBN and PBC regions of the first and the second protein(s), wherein said binding is mediated by chemically induced proximity. As described above, the principle of chemically induced proximity (CID) is known in the art. However, CID has so far not been used in the context of artificial cohesin.

In particular, the binding of the PBN and PBC regions of the first protein(s) to the PBN and PBC regions of the second protein(s) may be mediated by chemically induced proximity and wherein the binding of the PBN and PBC regions of the first and the second protein(s) is disrupted by the absence of a small molecule compound or by functionally inactivating said small molecule compound. The skilled person is aware of examples of chemically induced proximity. The examples as provided in the above Table may also be used for release. In this case, the release is achieved by e.g. washing out the compound mediating the chemically induced proximity. Hence, the protein binding regions of the first and the second protein(s) release the binding and thus the tethering is reversed. Ideally, the compound mediating the chemically induced proximity is a small molecule compound.

It is preferred that the small molecule compound is a photo-switchable small molecule compound. Photoswitchable compounds can be inactivated by light. Hence, said inactivated compound is no longer be capable of mediated CID. In other words, the CID is disrupted by inactivating the compound. Consequently, the tethering of the chromosomes or chromatids is released. Examples of such compounds are known in the art. Preferably, the compound is selected from one of MeNV-HaXS, and TMP-NVOC linker-Halo.

Examples of photoswitchable chemically induced proximity in the context of the present disclosure for releasing the tethering are provided as follows:

| **(I)(ii) PBN region of the 1st protein(s)** | **(I)(iii) PBC region of the 1st protein** | **(II)(ii) PBN region of the 2nd protein(s)** | **(II)(iii) PBC region of the 2nd protein(s)** | **small molecule compound** |
|---|---|---|---|---|
| Snap-tag | Halo-taq | Snap-tag | Halo-taq | MeNV-HaXS |
| DHFR | Halo-Tag | DHFR | Halo-Tag | TMP-NVOC linker-Halo |
| Halo-tag | Snap-tag | Halo-tag | Snap-tag | MeNV-HaXS |
| Halo-Tag | DHFR | Halo-Tag | DHFR | TMP-NVOC linker-Halo, wherein NVOC is 6-nitroveratryl oxycarbonyl (TNH) |

In general, these photoswitchable compounds are commercially available. Further information on MeNV-HaXS can, for example, be found in Zimmermann et al. (2014).⁵⁹

The chemical name of TMP-NVOC linker-Halo (TNH) is 4-((18-Chloro-3,6,9,12-tetraoxaoctadecyl)oxy)-5-methoxy-2-nitrobenzyl (3-(4-((2,4-diaminopyrimidin-5-yl)methyl)-2,6-dimethoxyphenoxy)propyl)carbamate.

### Chromatin-binding component

As defined above, the 'chromatin binding component' is a part of the first and the second protein(s), wherein said component is capable of binding to chromatin. As known in the art, chromatin refers to a mixture of DNA and proteins that from the chromosomes found in eukaryotic cells. The chromatin binding component is thus capable of binding to the DNA or the DNA associated proteins. Ideally, the chromatin-binding component is a DNA binding domain. The chromatin-binding component may be capable of binding to a DNA-associated protein.

If the chromatin-binding component is a DNA binding domain, the DNA binding domain may be selected from a group consisting of a TAL protein domain, a Zinc finger protein, a leucine zipper, and a dCas9 complexed with a gRNA. It is preferred that DNA-binding component is selected from a group consisting of a TAL protein domain and a Zinc finger protein. As shown in Example, 1, the DNA binding component may be a TAL protein domain. As shown in Figure 1e, the first protein(s) comprised a TAL protein domain flanked on both sides (N-terminal and C-terminal) by spy-tags, and the second protein(s) comprised a TAL protein domain flanked on both sides (N-terminal and C-terminal) by spy-catchers.

In some embodiments, the chromatin-binding component is a DNA binding domain suitable to corkscrew around the chromatid or chromosome for at least half a turn. For example, a TAL protein domain corkscrews around the DNA. Ideally, the DNA binding domain corkscrews around the chromatid or chromosome for at least three-quarters of a turn, preferably a full turn, even more preferably at least 1.5 turns, and more preferably at least 2 turns. In particular, the DNA binding domain may corkscrew around the chromatid or chromosome for at most 20 turns, preferably at most 10 turns.

Alternative, the chromatin-binding component may be capable of binding to a DNA-associated protein. The skilled person is aware of DNA-associated proteins. Exemplary DNA-associated protein are CENP-C, CENP-T, CENP-H, TRF1, TRF2, Dsn1 and histones. The chromatin-binding component may bind to any of these exemplary proteins.

As further defined in the claims, the chromatin binding component is capable of binding to a target sequence on a chromatid of a chromosome. Such a target sequence is ideally at a particular location at the chromosome or chromatid. For example, such a particular location may be the centromere or the telomere. Ideally, the target sequence on each chromatid or chromosome is a repetitive DNA sequence. The skilled person is aware of repetitive sequences along the chromatids or chromosomes. For example, said repetitive sequence may be a pericentromeric satellite repeat, a centromeric satellite repeat, or a telomeric repeat. It is preferred that said repetitive sequence is a pericentromeric satellite repeat. As experimentally demonstrated in Example 1, artificial cohesin successfully tethers chromatids when using a pericentromeric satellite repeat.

Pericentromeric satellite repeats are particularly preferred as premature separation of sister chromatids (PSSC) is caused by a loss of cohesin at pericentromeric regions^{12,15,20,21}. In Example 1, the inventors hence targeted the artificial cohesion system to a 15-bp sequence within repetitive pericentromeric major satellite DNA. The complex according to this disclosure binds efficiently to the pericentromeric region of mouse chromosomes, and is well tolerated by meiotically dividing oocytes^{22,23}.

Ideally, the repetitive DNA sequence originates from a human, cat, dog, horse, cattle, sheep, goat or pig. Ideally, the repetitive DNA sequence originates from human, cat, dog, horse, cattle, sheep, goat or pig. The repetitive DNA sequence may originate from a veterinary animal. The repetitive DNA sequence may be from an endangered animal such as a panda, an orangutan, a saola, a tiger, a rhinoceros, a leopard, a gorilla, an elephant, a bonobo, or a dolphin. Even more preferably, the repetitive DNA sequence originates from a human, cat, dog or horse. Ideally, the repetitive DNA sequence is a human repetitive DNA sequence.

In certain embodiments, the chromatin binding component of the first and the second protein(s) binds to the identical target sequence, e.g. a pericentromeric repeat as demonstrated in Example 1.

The chromatin-binding component of the first and the second protein(s) may have at least 70% sequence identity to SEQ ID NO: 25. Ideally, said sequence identity is at least 80%, preferably at least 85%, more preferably at least 90%, even more preferably at least 92%, even more preferably at least 94%, even more preferably at least 96%, even more preferably at least 97%, even more preferably at least 98% and most preferably 100%.

It is also disclosed that the chromatin-binding component of the first and the second protein(s) may have an amino acid sequence identity of at least 90%, preferably at least 92%, more preferably at least 94%, even more preferably at least 96% and most preferably, wherein the first and the second protein(s) have 100% amino acid the sequence identity.

As noted above, the complex according to this disclosure is an artificial complex. In other words, said complex is non-naturally occurring.

With regard to the number of the first and the second protein within one complex, said number is at least one of first and at least one of the second protein. Ideally, the complex comprises at least two first proteins and at least two second proteins, preferably the complex comprises at least three first proteins and at least three second protein(s), more preferably the complex comprises at least four first proteins and at least four second protein(s), more preferably the complex comprises at least five first proteins and at least five second protein(s). Practically, the complex or the nucleic acid encoding said complex may be delivered into a cell via an *in vitro* method. Hence, many first and second proteins are present within a cell. Additionally, two, three, four, five, six, seven, eight, nine, ten or more first and second proteins may form one complex tethering the chromosomes or chromatids together. In such a case, several first and second proteins from an artificial cohesion ring of four to possibly 400 000 proteins. In other words, the first and the second proteins may bind polymerically. This means that several first and second protein(s) tether the chromosomes or chromatids. A single complex for tethering chromosomes or chromatids may comprise at most 1 000 000 first proteins and at most 1 000 000 second protein(s).

As also demonstrated in Examples 1 and 2, the complex may additionally comprises a fluorescent marker. Said marker may be located between the PBN region and the chromatin-binding component or between the PBC region and the chromatin-binding component. For example, said fluorescent marker may be detectable by fluorescence microscopy.

### Nucleic acid molecule(s)

As noted above, the disclosure also includes a nucleic acid molecule encoding the complex as disclosed herein. Alternatively, two nucleic acid molecules may encode the complex as disclosed herein. For example a first nucleic acid may encode the first protein of the complex and a second nucleic acid may encode the second protein of the complex. Alternatively, a single nucleic acid molecule may encode the first and the second protein. This may be, for example, achieved by encoding the first and the second protein one after the other. Alternatively, the amino acid sequence of the first and the second protein may be identical. Hence, the nucleic acid sequence may also be identical so that only one nucleic acid molecule encodes the first and the second protein.

Preferably, the nucleic acid molecule(s) is/are RNA or DNA molecule(s). RNA molecule(s) are more preferred. This is because RNA molecule(s) can be directly translated in oocytes or eggs into protein. Ideally, the RNA molecule(s) are mRNA molecule(s). In other words, the RNA molecule(s) are translatable to form a complex according to this disclosure.

The RNA molecule(s) may comprise(s) stabilizing modifications. The skilled person is aware of stabilizing modifications. Generally said modifications inhibit endonucleases and/or exonucle-ases. In other words, said stabilizing modifications inhibit enzymatic degradation within the cell. Hence, (a) stabilized RNA molecule(s) has/have a longer half-life within the cell. Thus, said RNA molecule(s) can on average be translated for a longer time period resulting in more first and second proteins expression over a longer time. Exemplary stabilizing modifications are pseudouridine, 2-thiouridine, 5-methyluridine, 5-methylcytidine, N6-methyladenosine, N-methylguanosine and/or 1-methyl-pseudouridine. One or more of these modifications may be comprised within the RNA molecule(s). Preferably, the RNA molecule(s) comprise(s) 5' end stabilizing modification. For example, said 5' end stabilizing modification may be a 7-methyl guanosine (m⁷G).

Ideally, the total size of the nucleic acid molecule(s) encoding the first and/or the second protein of the complex is at most 10 kb, preferably at most 9 kb, more preferably at most 8 kb, more preferably at most 7 kb, and most preferably at most 6kb. Said maximal sizes have the advantage that they are easier to produce by in vitro methods and that they are more easily introduced into a cell. Hence, the overall handling of the nucleic acid becomes less tedious.

In particular, the complex or the nucleic acid molecule(s) as described herein may be comprised within an oocyte or egg cell.

### Further embodiments of the complex and/or the nucleic acid molecule(s) encoding said complex

In certain embodiments, the complex tethers the chromatids or chromosomes during meiosis. For example said tethering is before fertilization. The tethering may be during meiosis I.

The complex or the nucleic acid molecule(s) encoding said complex may have been introduced into the oocyte or the egg by microinjection, transfection, transduction, or electroporation. It is preferred that the complex is introduced by microinjection. The skilled person is aware of the methodological steps of microinjection of oocytes and/or egg cells, such as using a microinjection needle. Said microinjection needle may introduce 1-25 picoliters into an oocyte or egg cell. Other delivery methods may also be used to introduce the protein and/or nucleic acid molecule(s) to the oocyte and/or egg. In general, electroporation is a method whereby protein or nucleic acid molecule(s) are introduced into cells by electrical current. According to the present disclose, said cell may be an oocyte or egg cell.

Alternatively, the complex or the nucleic acid molecule(s) encoding said complex may be introduced by transfection using a liposome. In general, transfection is the process of deliberately introducing nucleic acid molecule(s) or protein into eukaryotic cells. Furthermore, the skilled person knows that liposomes fuse with the cell membrane and release their cargo into the cell. According to the present disclosure, liposomes may fuse with the oocyte or egg cell membrane and release the complex or the nucleic acid molecule(s) encoding said complex. In other words, the complex or the nucleic acid molecule(s) may be the cargo. The liposome may be capable of fusion with the oocyte or egg cell. The liposome may further encapsulate the complex or the nucleic acid molecule(s) encoding said complex. An exemplary liposome comprises Sendai virus coat protein. For example, the liposome may be a Hemagglutinating virus of Japan (HVJ) envelope vector. Ideally, the Hemagglutinin Virus of Japan (HVJ) Envelope Cell Fusion Kit may be used for introduction of the complex.

In particular, the complex may be suitable for use during meiosis. Meiosis can be subdivided in meiosis I and meiosis II. Ideally, the complex is suitable for use before fertilization. Fertilization is when sperm joins the egg cell. More in particular, the complex may be suitable for use during meiosis I. In meiosis I, chromosomes in a diploid cell re-segregate, producing haploid daughter cells. In haploid cells only a single set of chromosomes is present. In other words, homologue pairs separate during a first round of cell division, i.e. meiosis I. Using the complex during meiosis I has the technical advantage that the chromosomes are tethered and released during said process to ensure that all haploid daughter cells comprise one complete set of chromosomes. If an error occurs during meiosis, too many or too few chromosomes are comprised within a daughter cells. These resulting daughter cells are prone to aneuploidy. In order to reduce the risk of aneuploidy, the complex as disclosed herein tethers chromosomes and releases the tether during anaphase I. In meiosis II, sister chromatids separate.

A complex or (a) nucleic acid molecule(s) suitable for use in reproductive medicine is also disclosed herein. Ideally, said complex or (a) nucleic acid molecule(s) may be for use in assisted reproductive technology (ART). ART includes fertility treatments in which either eggs or zygotes are handled. ART does not include somatic nuclear transfer or the like. According to the present disclosure, ART may be performed *in vitro,* when e.g. introducing the complex or the nucleic acid molecule(s) encoding said complex into the oocyte or egg cell. Alternatively, ART may be performed, e.g., when the complex or the nucleic acid molecule(s) encoding said complex may be for use in reducing the risk of aneuploidy in a zygote. Additionally, the complex or the nucleic acid molecule(s) as disclosed herein may be for use in chromatid or chromosome cohesion in a zygote. Specifically, the complex or the nucleic acid molecule(s) as disclosed herein may be for use in reducing the risk of aneuploidy in a zygote. Aneuploidy is the occurrence of one or more extra or missing chromosomes leading to an unbalanced chromosome complement, or any chromosome number that is not an exact multiple of the haploid number. The haploid number in humans is 23. In other words, aneuploidy is the presence of an abnormal number of chromosomes in a cell. Aneuploidy is a major cause of infertility and inherited birth defects. Hence, reducing the risk of aneuploidy has the medical benefit of having more fertile oocytes or egg cells and having a lower risk if birth defects.

The oocyte or egg cell may be obtained from, e.g., a woman. In other words, said oocyte or egg cell may be *ex vivo.* Said oocyte or egg cell may be *in vitro.* Starting from said oocyte or egg cell, the complex or the nucleic acid(s) as disclosed herein may be introduced into the oocyte or egg cell. An *in vitro* method of introducing the complex or the nucleic acid molecule(s) as disclosed herein into an oocyte or an egg cell is disclosed herein. Said method may comprise the step of introducing the complex or the nucleic acid molecule(s) into the oocyte or the egg cell. The term 'introduce' refers to any process of transferring the complex or the nucleic acid molecule(s) into the oocyte or egg cell, such as microinjection or electroporation.

The nucleic acid molecule(s) may be translated to form the complex as disclosed herein. Ideally, the complex or the translated nucleic acid molecule(s) as disclosed herein stabilize chromatid or chromosome cohesion in the oocyte or the egg cell. In certain embodiments, a suitable amount of the complex or the nucleic acid molecule(s) is introduced into the human oocyte or the human egg cell. As shown in Example 1 the amount of the complex or nucleic acid molecule(s) changes the outcome. When too much of the complex or nucleic acid molecule(s) is introduced, e.g. chromatin clustering may be a consequence. Specifically, the suitable amount may be assessed by a titration experiment. For example, the amount is too large when detecting chromosome clustering, ruptured kinetochores, reduction in chromatid or chromosome inter-kinetochore distances, decreased recovery of chromosome-localized fluorescent signal after photo-bleaching, resistance of sister chromosome separation after acute depletion of cohesin subunits, a reduction in chromosome errors in metaphase II eggs, and/or other chromatid or chromosome cohesion disturbances using fluorescence microscopy or polarized light microscopy. For example, the amount is too little when premature separation of sister chromatids is not reduced compared to cells not having introduced the complex or nucleic acid molecule(s) encoding said complex.

Exemplary suitable amounts of the nucleic acid molecule(s) ranges between 0.0002 to 0.02 attomoles, preferably 0.0004 to 0.01 attomoles, more preferably 0.0008 attomoles to 0.005 attomoles, more preferably 0.0009 to 0.004 attomoles, more preferably 0.001 to 0.003 attomoles, and most preferably around 0.002 attomoles. Example 1 provides an exemplary titration of attomoles of nucleic acid(s) for introduction into an oocyte or egg cell.

Exemplary suitable amounts of the complex range between 0.001-25 pg per oocyte, preferably 0.005-10 pg, more preferably 0.01-8 pg, more preferably 0.02-5 pg, more preferably 0.04-3 pg, and even more preferably 0.06-2 pg.

An *in vitro* method of stabilizing chromatid or chromosome cohesion in an oocyte or egg cell comprising the step of introducing the complex or the nucleic acid molecule(s) according to the present disclosure into an oocyte or egg cell is also disclosed herein. Ideally, the complex or the nucleic acid molecule(s) is introduced into a human oocyte or human egg cell. In particular, said *in vitro* methods do not modify the germline identity of human beings.

The method may reduce premature separation of sister chromatids (PSSC) in an oocyte compared to an oocyte without having introduced the complex or the nucleic acid molecule(s) as disclosed herein. Specifically, the PSSC may be reduced by at least 10%, preferably at least 20%, more preferably at least 30%, more preferably at least 40%, more preferably at least 50%, more preferably at least 60%, more preferably at least 70%, more preferably at least 80%, more preferably at least 90%, and even more preferably by at least 100%. Example 1 demonstrates that PSSC has been reduced by at least 100% in mouse oocytes. A similar level is expected for human oocytes, as said process is largely conserved. In particular, the PSSC may be reduced by at most 1000%. As shown in Example 1, for example Figures 7D and 7E, the duration of anaphase II may take at least 15 minutes. The duration of anaphase II may take at most 40 minutes. Preferably, the duration of anaphase II is less than 20 minutes.

Ideally, the complex or the nucleic acid molecule(s) as disclosed herein is delivered before metaphase II arrest. In particular, the complex or the nucleic acid molecule(s) as disclosed herein may be delivered during meiosis I. The term 'deliver' refers to any process of transferring the complex or the nucleic acid molecule(s) into the oocyte or egg cell, such as microinjection or electroporation. Ideally, the complex or the nucleic acid molecule(s) as disclosed herein is delivered before nuclear envelope breakdown of germinal vesicle-stage oocytes, wherein meiotic resumption is determined by nuclear envelope breakdown. It is further preferred that the complex or the nucleic acid molecule(s) as disclosed herein is delivered after nuclear envelope breakdown before anaphase I. In particular, the complex or the nucleic acid molecule(s) as disclosed herein may be delivered in metaphase I. Delivery after nuclear envelope breakdown but before anaphase I has the particular advantage that the complex is capable of functioning during chromosome segregation of meiosis I in order to reduce the risk of aneuploidy in meiosis I.

An *in vitro* method for stabilizing chromatid or chromosome cohesion of an oocyte or an egg cell is also disclosed. Said method comprises the step of delivering the complex or the nucleic acid molecule(s) as disclosed herein into the human oocyte or egg cell, wherein said nucleic acid molecule(s) are translated into the complex as disclose herein, whereby the complex stabilizes the chromatid or chromosome cohesion in the human oocyte or the egg cell. Preferably, said method comprises an additional step, which is carried out as a first step. In said step, the oocyte or egg cell is provided *in vitro.*

In particular, the method does not modify the germline identity of human or animal beings. Ideally, the oocyte or the egg cell originates from a human, cat, dog, horse, cattle, sheep, goat or pig. In particular, the oocyte or the egg cell may originate from a human, cat, dog or horse. It is preferred that the oocyte or the egg cell originates from a human

The *in vitro* method may reduce premature separation of sister chromatids (PSSC) in an oocyte compared to an oocyte without having introduced the complex as disclosed herein or the nucleic acid molecule(s) as disclosed herein. The method may reduce premature separation of sister chromatids (PSSC) in an oocyte compared to an oocyte without having introduced the complex or the nucleic acid molecule(s) as disclosed herein. Specifically, the PSSC may be reduced by at least 10%, preferably at least 20%, more preferably at least 30%, more preferably at least 40%, more preferably at least 50%, more preferably at least 60%, more preferably at least 70%, more preferably at least 80%, more preferably at least 90%, and even more preferably by at least 100%. Example 1 demonstrates that PSSC has been reduced by at least 100% in mouse oocytes. A similar level is expected for human oocytes, as said process is largely conserved. In particular, the PSSC may be reduced by at most 1000%.

The complex or the nucleic acid molecule(s) as disclosed herein is delivered before metaphase II arrest. In particular, the complex or the nucleic acid molecule(s) as disclosed herein may be delivered during meiosis I. Ideally, the complex or the nucleic acid molecule(s) as disclosed herein is delivered before nuclear envelope breakdown of germinal vesicle-stage oocytes, wherein meiotic resumption is determined by nuclear envelope breakdown. It is further preferred that the complex or the nucleic acid molecule(s) as disclosed herein is delivered after nuclear envelope breakdown before anaphase I. In particular, the complex or the nucleic acid molecule(s) as disclosed herein may be delivered in metaphase I.

The following methods are also disclosed and the embodiments described herein with regard to the complex, the nucleic acid molecule(s) as well as the methods and medical uses apply also to the following methods.

A method in reproductive medicine comprising the step of introducing the complex or the nucleic acid molecule(s) as described herein into an oocyte, an egg cell or a zygote are also disclosed. Specifically, said method may be an assisted reproductive technology method.

A method of increasing chromatid or chromosome cohesion in an oocyte, an egg cell, or zygote comprising the step of introducing the complex or the nucleic acid molecule(s) as described herein into the oocyte, the egg cell or the zygote is also disclosed. Specifically, chromatid or chromosome cohesion is increased compared to an oocyte, an egg cell or a zygote without having introduced the complex or the nucleic acid molecule(s) as described herein.

A method of reducing the risk of aneuploidy in an oocyte, an egg cell or a zygote comprising the step of introducing the complex or the nucleic acid molecule(s) as described herein into the oocyte, the egg cell or the zygote, wherein the risk of aneuploidy is reduced compared to an oocyte or a zygote without having introduced the complex or the nucleic acid molecule(s) as described herein.

A method of reducing the probability of aneuploidy in an oocyte, an egg cell or a zygote comprising the step of introducing the complex or the nucleic acid molecule(s) as described herein into the oocyte, the egg cell or the zygote, wherein the risk of aneuploidy is reduced compared to an oocyte or a zygote without having introduced the complex or the nucleic acid molecule(s) as described herein.

Finally, the present disclosure also describes a kit comprising the complex or the nucleic acid molecule(s) as described herein. A 'kit' as used herein is generally a set of parts. In other words, the kit may comprise several parts. Said complex or said nucleic acid molecule(s) may be suitable for introduction into an oocyte. The complex or the nucleic acid molecule(s) may be suitable for introduction into an egg cell. The kit may comprise a device for introduction of the complex or the nucleic acid molecule(s) encoding said complex into the egg cell or oocyte. For example, the device may be suitable for microinjection. Said device may be a microinjection needle. Ideally, the microinjection needle is equipped with a compatible pump suitable for transferring the complex or the nucleic acid molecule(s) encoding said complex into an oocyte or an egg cell. Preferably, the compatible pump is suitable for transferring 1 to 25 picoliters. In certain embodiments, the kit comprises liposomes suitable for transfection of the complex or the nucleic acid molecule(s). Said liposomes may encapsulate the complex or the nucleic acid molecule(s) encoding said complex. Specifically, the liposomes may comprise Sendai virus coat protein. The liposomes may be a Hemagglutinating virus of Japan (HVJ) envelope vector.

Additionally, the kit may comprise a container. Ideally, the complex or the nucleic acid molecule(s) are stored within the container. This is the advantage that the complex or nucleic acid molecule(s) may be easily shipped. Preferably, the complex or the nucleic acid molecule(s) are stored at a temperature of at most 4°C. In particular, the complex or the nucleic acid molecule(s) may be stored at a temperature of at most -20 °C. Preferably, the complex or the nucleic acid molecule(s) may be stored at a temperature of at least -196 °C.

### Examples

The following examples are intended to illustrate the invention further, but are not limited to it. The examples describe technical features, and the invention also relates to combinations of the technical features presented in this section.

### Example 1

As aneuploidy is an increased risk in ageing women for infertility and birth defects, there is a need to prevent premature separation of sister chromatids and chomosomes by preserving cohesin, or to reverse its loss. To the knowledge of the inventors, there is currently no system available, which could improve chromosome or chromatid cohesion. As a consequence, there are no treatments available for age-related infertility, and many women over 35 cannot become pregnant.

To meet this need, the inventors engineered an artificial cohesion system to reduce age-related PSSC in eggs (lower panel, Fig. 1a). The inventors primarily used mouse oocytes as a model of PSSC (Fig. 1c,d), because human oocytes are difficult to source. PSSC is caused by a loss of cohesin at pericentromeric regions^{12,15,20,21}. In this example, the artificial cohesion system is targeted to these regions by taking advantage of a Transcription Activator-Like (TAL) effector protein that targets a 15-bp sequence within repetitive pericentromeric major satellite DNA (Fig. 1e). This TAL protein binds efficiently to the pericentromeric region of mouse chromosomes, and is well tolerated by meiotically dividing oocytes^{22,23}. The inventors modified the TAL protein to create the TALhesin (*TAL cohesin*) system, which consists of TALhesin component-A, flanked on both sides by Spy-Tag domains, and TALhesin component-B, flanked on both sides by Spy-Catcher domains (Fig. 1e; Fig. 2a,b). Spy-Tag binds Spy-Catcher to form a stable covalent isopeptide bond between TALhesin-A and -B components²⁴. The inventors named the TALhesin according to its designated DNA target for the purpose of this example. TALhesin[*mmMajSat*]A/B is hence the TALhesin component-A and -B pair that targets the mouse (*mus musculus*) major satellite sequence²².

To characterize the TALhesin system, the inventors isolated and arrested immature mouse oocytes before meiotic resumption, injected them with mRNAs encoding TALhesin[*mmMajSat*]A/B, released them from arrest, and allowed them to progress through the first meiotic division (see Methods). TALhesin[*mmMajSat*]A/B localized to pericentromeric chromosome regions within 1.5 to 2 hours following nuclear envelope breakdown (NEBD) and remained on sister chromatids throughout the first meiotic division, until metaphase II arrest (Fig. 1f, f; Fig. 2 c). TALhesin[*mmMajSat*]A/B did not prevent progression through the first meiotic division (Fig. 2d), nor did it interfere with the unpairing of sister-kinetochores, which occurs as oocytes progress from meiosis I to meiosis II (Fig. 1g)^{25,26}. Fluorescence recovery after photo-bleaching (FRAP) revealed low turn-over of TALhesin[*mmMajSat*]A/B on meiosis II chromosomes compared to a TAL-mClover fusion protein lacking any Spy-interacting domains (Fig. 1h). Thus, TALhesin[*mmMajSat*]A/B stably associates with sister chromatids, similar to cohesin^{6,8,9,27}.

To determine if TALhesin[*mmMajSat*]A/B reduces sister chromatid separation, the inventors first examined the distance between sister kinetochores (inter-kinetochore distance) of mouse meiosis II chromosomes. Low levels of cohesion in the pericentromeric region, as observed in aged oocytes, result in a large inter-kinetochore distance, whereas high levels of cohesion, as observed in young oocytes, correlate with a small interkinetochore distance^{12,15,20,28}. The inventors injected different quantities of TALhesin[*mmMajSat*]A/B mRNAs into young eggs, and found that at moderate levels of TALhesin[*mmMajSat*]A/B mRNA (0.5 - 0.2 amol), the mean inter-kinetochore distance decreased by more than half compared to non-injected young eggs (Fig. 1i). At low mRNA concentrations (0.02-0.05 amol), the inter-kinetochore distances were similar to non-injected cells, whereas at 10-fold and higher TALhesin[*mmMajSat*]A/B mRNA levels (≥ 0.2 amol), chromosomes formed clustered groups that were so tightly held together that kinetochores were sometimes pulled off chromosomes during anaphase I (Fig. 2e,f). Occasionally, TALhesin[*mmMajSat*]A/B-labelled chromosomes in the first polar body remained linked to those in the egg (Fig. 2g). Overall, it is concluded that TALhesin[*mmMajSat*]A/B can create stable links between sister chromatids, and that moderate levels of TALhesin[*mmMajSat*]A/B have the capacity to bring sister kinetochores into closer proximity.

Next, it was asked if TALhesin[*mmMajSat*]A/B can compensate for acute depletion of cohesin²⁹. Rec8 is an essential component of the cohesin complex, and depleting Rec8 using Trim-Away causes sister chromatids to separate²⁹. To determine if the TALhesin system can mediate cohesion on its own, the inventors depleted Rec8 from young eggs that had been injected with high amounts (2 amol) of TALhesin[*mmMajSat*]A/B mRNA or TALhesin[*mmMajSat*]-B only, and examined sister chromatid separation (Fig. 3a). Indeed, most sister chromatids remained together after Rec8 was depleted in cells expressing TALhesin[*mmMajSat*]A/B, but not in those expressing TALhesin[*mmMajSat*] only (Fig. 3b; Fig. 4a). To test if cohesion mediated by TALhesin[*mmMajSat*]A/B can be relieved, TALhesin[*mmMajSat*]A/B was depleted using anti-GFP Trim-Away. Depletion of TALhesin[*mmMajSat*]A/B alone should not affect chromosome cohesion when endogenous cohesin remains intact (Fig. 3c, left panel), but should abolish cohesion and trigger sister chromatid separation in the absence of Rec8 (Fig. 3c, right panel). Consistent with this hypothesis, the inventors found that depletion of Rec8 followed by TALhesin[*mmMajSat*]A/B depletion resulted in sister chromatid separation over six hours (lower panel, Fig. 3d), whereas sister chromatids remained intact over the same period in cells depleted for TALhesin[*mmMajSat*]A/B only (upper panel, Fig. 3d). Overall, these data indicate that TALhesin[*mmMajSat*]A/B maintains sister chromatid cohesion after depletion of Rec8-cohesin, confirming that TALhesin can functionally replace endogenous cohesin.

Given that the TALhesin system can maintain sister chromatid cohesion, it was hypothesized that TALhesin[*mmMajSat*]A/B could reduce PSSC errors in eggs from older mice. To this end, the inventors compared the number of chromosomes with PSSC in eggs from older mice that were untreated or treated with TALhesin[*mmMajSat*]A/B mRNA. As controls, untreated eggs from young mice (2-3 months of age) (Fig. 3e) were examined. Aged eggs were treated with two low doses of TALhesin[*mmMajSat*]A/B mRNA (0.02 and 0.05 amol), which had maintained inter-kinetochore distances similar to young eggs without triggering chromosome clustering, ruptured kinetochores or other effects observed at higher (≥ 0.2 amol) mRNA amounts (Fig. 1h, Fig. 2d-g).

PSSC errors were defined as sister chromatids with kinetochores that were separated by distances of ≥3 µm, or that appeared as a single arm with a single kinetochore (Fig. 3f). Strikingly, the frequency of PSSC was significantly decreased in TALhesin[*mmMajSat*]A/B-treated eggs isolated from mice aged 13, 17 and 20 months, approaching the low PSSC error rates observed in young eggs (Fig. 3g,h; Fig. 4b-i). Moreover, the percentage of aged eggs lacking any PSSC error increased from 46.7% to 74.1% upon treatment with TALhesin[*mmMajSat*]A/B (Fig. 3i), similar to the percentage of eggs lacking PSSC errors from young mice. Treatment with 0.05 amol of TALhesin[*mmMajSat*]A/B mRNAs led to a 97.6% restoration of error-free eggs from 17 month-old mice relative to eggs from young mice (Fig. 3j). Similar effects were observed in TALhesin[*mmMajSat*]A/B-treated eggs from mice that were 13-months of age (Fig. 4b-e).

The effects were less pronounced but still significant for eggs in the 20-months age group (Fig. 4f-i). Consistent with the results from young oocytes, injection of low amounts (0.02 - 0.05 amol) of TALhesin[*mmMajSat*]A/B mRNA did not have a prominent effect on inter-kinetochore distances (Fig. 4j-l). Overall, TALhesin[*mmMajSat*]A/B treatment reduces the frequency of PSSC in eggs from mice of advancing maternal age, yielding more eggs with no PSSC errors.

Next, the inventors aimed to apply the TALhesin system to human eggs. To this end, they used a similar strategy as for mouse oocytes, but employed a TAL effector domain targeting a 19-bp sequence within a human centromeric satellite repeat³⁰. The inventors initially examined the consequences of TALhesin in mitotic HeLa cells. Because the duration of mitosis in somatic cells is only ~30 minutes, compared to the ~12 hours to complete the first meiotic division in mouse oocytes, the inventors used Spy-Catcher003 domains in TALhesin-B to improve interaction kinetics with Spy-Tag³¹. These modifications generated TALhesin[*hsCen-tro*]A/B (TALhesin directed against *homo sapiens* centromeric repeats; Fig. 5a). The inventors transfected HeLa cells and observed TALhesin[*hsCentro*]A/B enrichment on several chromosomes (Arrows, Fig. 5a). Some chromosomes had lower TALhesin occupancy, which likely reflects chromosome-specific densities and copy numbers of the targeted satellite element (Fig. 5a)³². They noticed that newly divided HeLa cells expressing TALhesin[*hsCentro*]A/B formed distinct inter-cellular bridges that consisted of TALhesin-labelled DNA (Fig. 6b). HeLa cells are well known to spontaneously form chromosome bridges during mitosis³³, and the inventors observed a substantial increase in these bridges, from approximately 20% in non-transfected cells, and 32% in cells expressing only TALhesin[*hsCentro*]-B, to 94.7% of cells expressing both TALhesin[*hsCentro*]A/B components (Fig. 5c). It is concluded that TALhesin[*hsCentro*]A/B can stably link chromosomes in human mitotic cells.

To determine if the TALhesin system could tether sister chromatids in human eggs, again the kinetics of isopeptide bond formation were modified by incorporating Spy-Catcher001 domains in TALhesin[*hsCentro*]-B (Fig. 6b,c). Donated human oocytes were injected with 0.0006 amol TALhesin[*hsCentro*]A/B mRNA, and matured *in vitro* (Fig. 5d). TALhesin[*hsCentro*]A/B localized to pericentromeric regions on most human meiosis I and II chromosomes and did not impede progression through the first meiotic division (Fig. 5e, f). Variability between donor ages (Fig. 6d), in addition to oocyte-specific variation in expression and maturation timing, resulted in heterogeneous levels of TALhesin[*hsCentro*] proteins that constrained the analyses. It was hypothesized that paired sister chromatids would display higher levels of TALhesin[*hsCentro*]A/B than chromosomes that had undergone PSSC, which would be consistent with TALhesin[*hsCentro*]A/B helping to prevent PSSC in human eggs. The mean fluorescence intensity of TALhesin[*hsCentro*]A/B was measured on sister chromatid pairs that were intact and compared them to chromatids that were dissociated. Indeed, intact chromosomes had higher TALhesin[*hsCentro*]A/B mean fluorescence intensity compared to those that had experienced PSSC (Fig. 5g). Additionally, chromosomes with smaller inter-kinetochore distances had higher TALhesin[*hsCentro*]A/B mean fluorescence intensity compared to those with larger inter-kinetochore distances, or those that had experienced PSSC (Fig. 5h, Fig. 6e,f). These data suggest that TALhesin[*hsCentro*]A/B reduces inter-kinetochore distances and may protect human oocytes from PSSC.

Sister chromatids segregate when anaphase II is activated by fertilization (Fig. 7a). To evaluate sister chromatid segregation, the inventors treated mouse and human eggs expressing the appropriate TALhesin system with compounds that activate anaphase II, thus simulating fertilization by a sperm^{34,35}. The inventors found that even low levels of non-cleavable TALhesin led to inefficient sister chromatid segregation in anaphase II of activated mouse (0.05 amol TALhesin[*mmMajSat*]A/B) (Fig. 7b, middle panels) and human eggs (0.0006 amol TALhesin[*hsCentro*]A/B) (Fig. 7c, middle panels), associated with a delayed completion of anaphase II and an elevated frequency of chromosome non-disjunction events (Fig. 7d-g).

The inventors considered that the TALhesin system must be deactivated for efficient chromosome segregation in anaphase II. To disengage TALhesin from tethering sister chromatids during anaphase, the inventors took advantage of the cell cycle-dependence of separase activity (Fig. 7a). The protease separase is activated in anaphase to cleave the cohesin subunit Rec8 at defined proteolytic sites ³⁶⁻³⁸. The inventors reasoned that integrating Rec8 proteolytic motifs into TALhesin could potentiate separase cleavage at anaphase II. Using mouse and human Rec8 fragments, respectively, the inventors generated TALhesin[*mmMajSat*](Rec8)A/B and TALhesin[*hsCentro*](Rec8)A/B (Fig. 8a-d). In contrast with non-cleavable TALhesin proteins, separase-cleavable TALhesin[*mmMajSat*](Rec8)A/B or TALhesin[*hsCentro*](Rec8)A/B supplementation led to synchronous chromatid segregation in activated young mouse eggs and human eggs, respectively (lower panels, Fig. 7b, c), similar to eggs expressing TALhesin(Rec8)-B only (upper panels, Fig. 7b, c). In addition, the time to anaphase II completion and the frequency of chromosome non-disjunction events were reduced in activated eggs expressing TALhesin(Rec8) compared to non-cleavable TALhesin, for both mouse and human (Fig. 7d-g). TALhesin[*mmMajSat*](Rec8)-A/B stably associated to chromosome pericentromeric regions (Fig. 8e), similar to non-cleavable TALhesin[*mmMajSat*] (Fig. 1h). Importantly, eggs from 20-month old mice treated with TALhesin[*mmMajSat*](Rec8)A/B had a reduced frequency of PSSC errors (Fig. 7h; Fig. 9a) and an increase in the percentage of cells without any PSSC errors (Fig. 9b-d). Thus, the integration of a separase-cleavable Rec8 fragment into eggs allows deactivation at anaphase II while still reducing PSSC errors in mouse eggs.

### Example 2

TALhesin functions to tether chromosomes together. However, the timed disabling of TALhesin is necessary to allow chromosomes to segregate freely during anaphase. The inventorstested several approaches to disable TALhesin. In the first approach, they tested if the introduction of a cell-cycle dependent degradation sequence causes TAL[mmMajSat] protein to disappear from chromosomes. In one example, the inventors introduced the first 101 amino acids from mus musculus securin protein⁶³ at the N-terminus of mClover-TAL[*mmMajSat*] (N-Securin-mClover-TAL[*mmMajSat*]). Additionally, the inventors engineered and tested an approach where the first 79 amino acids from mus musculus cyclinB1 protein⁶⁴ are fused at the N-terminus of mClover-TAL[*mmMajSat*] (N-CyclinB1-mClover-TAL[*mmMajSat*]). Both the N-terminal domains of securin and cyclinB1 contain well-characterized ubiquitin pathway-dependent destruction motifs that are targeted for degradation prior to and during anaphase I and II. The inventors evaluated fluorescence signal in mouse oocytes expressing either of these constructs as they proceeded through anaphase I (Fig. 11,12). When either of the degradation signals are added to mClover-TAL[*mmMajSat*], fluorescence signal decreases prior to and during anaphase I. Conversely, a fluorescent protein reporter H2B-miRFP that labels chromosomes in the same cells did not decrease during anaphase I. These data show that the N-terminal domains of both securin and cyclinB1 target TAL[mmMajSat] for cell cycle-dependent destruction by the ubiquitin pathway.

Another approach using recombinant NS3/4A protease was also tested. This approach relies on expressing the protease from synthesized mRNA alongside TALhesin with internal NS3/4A cleavage motifs. Importantly, the compound BILN-2061 (Ciluprevir) reversibly inhibits NS3/4A.⁶⁵ Wash-out of BILN-2061 allows for timed control of protease activity.⁶⁶ Thereby TALhesin was deactivated. Cleavage motifs on both the N and C terminal flanks of TAL[*mmMajSat*] for both A and B components were introduced. The addition of two cleavage motifs on both TALhesin components generated TALhesin[*mmMajSat*](2×CS)-A and -B. In total, each component contains two NS3/4A cleavage motifs, thus maximizing the possibility of deactivating TALhesin. Oocytes injected with mRNA coding for TALhesin[*mmMajSat*](2×CS)-A and -B, in addition to mRNA coding for NS3/4A, were matured in vitro in the presence of BILN-2061, a specific inhibitor compound of HCV NS3/4A protease. Following wash-out of BILN-2061, fluorescence signal originating from TALhesin[*mmMajSat*](2×CS)-A/B foci on chromosomes steadily decreased over the course of 16 hours (Figure 12). These data show that TALhesin[*mmMajSat*] can be deactivated in mouse oocytes by using the NS3/4A protease and BILN-2061 inhibitor system.

### Materials and Methods

### Isolation and Culturing of Mouse oocytes

The Animal Facility of the Max Planck Institute for Multidisciplinary Sciences maintained all mouse lines used in this study. Mice were housed in a specific pathogen-free environment in accordance with international animal welfare rules (Federation for Laboratory Animal Science Associations guidelines and recommendations) and German Welfare Act (*Tierschutzgesetz TSchG*) guidelines.

Mouse strains CD1, 129s6, and B6/CBA-F1 were used in this study. B6/CBA-F1 hybrid females were used to generate Fig. 1i. For aging experiments in Fig. 3e-j and Fig. 3b-l, 129s6 strain mice 2 - 3 months of age were used alongside 129s6 strain mice 13, 17, or 20 months of age, as indicated. Young CD1 mice 2 - 3 months of age were used all other mouse experiments. GV-stage oocytes were cultured in homemade M2-medium supplemented with dibutyryl cyclic AMP (dbcAMP, Sigma-Aldrich: D0627) under NidOil paraffin oil (Nidacon: NO-100). Oocytes from CD1 and 129s6 strains were cultured with 250 µM dbcAMP and B6/CBA-F1 oocytes with 750 µM dbcAMP. Only mature oocytes ~ 70 µm in diameter with centered nuclei and thick zona pelucida (~5 µm) were selected for study.

### Cloning & mRNA synthesis

All constructs were assembled into the pGEM-HE vector backbone⁴⁴. To generate TALhesin-A, a custom synthesized 'chassis' containing terminal Spy-Tag sequences and internal restriction sites (Eurofins) was inserted into pGEM-HE. A Gateway cassette (Thermo-Fisher) and the sequence for mCherry fluorescent protein were then inserted in-frame with Spy-Tag sequences. Plasmids containing Gateway cassettes were propagated in ccdB Survival 2 T1^{R} Competent Cells (Thermo-Fisher: A10460) according to manufacturer's instructions. The TALhesin-B chassis was generated by replacing terminal Spy-Tag sequences of TALhesin-A with 5' and 3' terminal Spy-Catcher sequences, while mClover3 fluorescent protein replaced mCherry. For somatic cell expression, the TALhesin-A and -B chassis containing an internal Gateway cassette and fluorescent proteins were inserted within the pCMV vector by restriction digest and ligation. Spy-Catcher001 (Addgene plasmid #72324) and Spy-Catcher003 (Addgene plasmid #133447) were generous gifts from Dr. Mark Howarth^{24,31}.

The transcription Activator-like (TAL) sequence targeting to *mus musculus* major satellite repeats, TAL[*mmMajSat*], pTALYM3B15, was a generous gift from Dr. Maria-Elena Torres-Padilla (TALYM3B15; Addgene plasmid #47878)²². The TAL sequence targeting *Homo sapiens* centromeric repeats (*TAL*[*hsCentro*]) was a generous gift from Dr. Guang-Hui Liu³⁰. TAL sequences lacking N-terminal NLS were PCR amplified with primers [fwd: 5'-CACCA TGCAG GTGGA TCTAC GCACG CTCGG CTA-3' and rev: 5'-GGAGG AGGCG GTGCC GGA-3'] and cloned into pENTR/D-Topo (Thermo-Fisher) according to the manufacturer's instructions. To generate the complete constructs, Gateway cloning using LR clonase II plus (Thermo-Fisher: 12538120) was used to insert TAL sequences within pGEMHE-TALhesin-A and pGEMHE-TALhesin-B. The same reaction was used to generate the complete pCMV-based expression constructs.

TALhesin[*mmMajSat*](Rec8) was generated by inserting *Mus musculus* Rec8(aa417-525) into TALhesin-A and -B. This region contains 2xLPE pseudo-binding sites in addition to cleavage motifs C2 and C3³⁷. Rec8(aa417-525) was PCR amplified from pET45b(+) His-tagged FL Rec8 plasmid and inserted with Gibson Assembly master mix (New England BioLabs, E2611) into pGEMHE-TALhesin-A and -B in the linker-2 (L2) region. TALhesin[hsCentro](Rec8) was generated similarly by inserting *Homo sapiens* Rec8(aa368-434) into *TALhesin[hsCentro].* This region contains a 1×LPE pseudo-binding site in addition to cleavage motifs C2 and C3³⁷. pET45b(+) His-tagged FL Rec8 (Addgene plasmid # 45243) and H2B-mScarlet-hRec8(297-506)-mNeonGreen (Addgene plasmid #174717) were generous gifts from Dr. Michael Lampson^{12,45}. The inventors noticed that expression of TALhesin[*MajSat*](Rec8)-B was enhanced by introducing mCherry between the Gateway cassette and the Rec8 fragment (data not shown). Therefore, TALhesin[*MajSat*](Rec8)-B contains two fluorescent proteins flanking the Rec8 fragment.

Constructs were transcribed *in vitro* using HiScribe T7 ARCA kit (New England BioLabs, E2065S). Briefly, template plasmids within the pGEM-HE backbone were linearized overnight at 37°C using PacI restriction endonuclease (New England Biolabs: R0547), phenol/chloroform extracted, ethanol precipitated in the presence of sodium acetate, washed, and resuspended in endonuclease-free water. pTALYM3B15 was linearized similarly but using AgeI-HF restriction endonuclease (New England Biolabs: R3552). *In vitro* mRNA transcription was performed according to manufacturer's instructions, followed by DNase treatment, phenol/chloroform extraction, ethanol precipitated in the presence of ammonium acetate, washed, and resuspended in endonuclease-free water. mRNA concentration was determined by high-sensitivity RNA Qubit 4 fluorometry (Thermo-Fisher) and used to calculate moles of mRNA injected into oocytes. All RNA were analyzed by gel electrophoresis prior to injection. Briefly, RNA was incubated at 70°C for 5 min in formamide loading buffer (Thermo-Fisher: R0641) and run alongside RiboRuler HR RNA ladder (Thermo-Fisher: SM1821). Gels were visualized with SYBR-safe stain (Thermo-Fisher: S33102).

### mRNA Microinjection

A mercury needle-based injection set-up was used to inject precisely 7 picoliters of mRNA into mouse oocytes, as previously described⁴⁶. All mRNA amounts reported in this study were calibrated to this injection volume. Briefly, dbcAMP-arrested GV-stage mouse oocytes were loaded into a custom-made injection shelf containing fresh warm M2 medium. Oocytes were injected in small batches to avoid cooling of medium and evaporation. After injection, oocytes were washed in three droplets of fresh M2 medium containing dbcAMP under oil (NidOil) and incubated at 37.5°C for three hours to allow protein expression. Cells were then washed into fresh M2 medium lacking dbcAMP and matured for 15 hours either in a 37.5°C incubator, or imaged on an incubated confocal microscope (Zeiss) in M2 medium containing 200 nM SiR-DNA (SpyroChrome: SC007).

### Rec8 antibody generation, Trim-Away & Parthenogenic Activation

Antigen protein corresponding to *Mus musculus* Rec8(aa25-286) was expressed from pET28a(+)-N-Rec8-6×His plasmid⁴⁷. Rec8(aa25-286) was PCR amplified from pET45b+ His-tagged FL Rec8 plasmid (Addgene plasmid #45243) and inserted by Gibson assembly into the pET28a(+)-6×His backbone (Merck-Millipore: #69864). Bacteria were cultured in Magic Media (Thermo-Fisher: K6803) at 37°C for 16 hours. Protein was extracted from inclusion bodies by nutation in buffer containing 50 mM Tris, pH 8.0, 250 mM NaCl, and 6 M guanidine HCl for 18 hours at 4°C, followed by centrifugation at 39,000 xg at 4°C for 2 hours, and supernatant passed through a 0.22 µm syringe filter. The supernatant was diluted 1:10 with dilution buffer (50 mM Tris, pH 8.0; 250 mM NaCl; 15 mM imidazole; 5 mM 2-mercaptoethanol; 6 M urea) before loading on a pre-equilibrated Ni-NTA bead column. The column was washed in dilution buffer, followed by elution in elution buffer (50 mM Tris, pH 8.0; 250 mM NaCl; 500 mM imidazole; 5 mM 2-mercaptoethanol; 6 M urea). Protein was then dialyzed against buffer containing 25 mM Tris, pH 8.0, 250 mM NaCl, 5% glycerol, 5 mM DTT, 5 M urea for 1 hour, followed by buffer exchange at a rate of 3 mL/min with 3 L of refolding buffer (25 mM Tris, pH 8.0, 250 mM NaCl, 5% glycerol, 5 mM DTT). Protein was then concentrated to 1.91 mg/mL using an Amicon Ultra-4 10 kDa mwco (Merck-Millipore: UFC801024). Cambridge Research Biochemicals (Cambridge, UK) performed antibody generation in rabbits and affinity column purification of antibody.

For Trim-away, GV-stage oocytes were microinjected with a cocktail containing 2.9 amol of *in vitro* synthesized TRIM-21, TALhesin-A, and -B mRNA as described above (see Methods: mRNA Injection). Mature meiosis II eggs were first incubated in M2 medium containing 200 nM SiR-DNA (SpyroChrome: SC007). Polyclonal rabbit anti-Rec8 antibody (for anti-Rec8 trim-away), or polyclonal rabbit anti-GFP antibody (AbCam: 6556) (for anti-GFP trim-away targeting the depletion of TALhesin) were buffer exchanged in phosphate-buffered saline (PBS) for Trim-away as previously described²⁹. For anti-Rec8 Trim-away, MII oocytes were then injected with 7 pg of 1.01 mg / mL anti-Rec8 antibody in PBS with 0.03% NP-40, alongside 43.8 femtograms of dextran-Texas Red, 70 kDa (Thermo-Fisher: D1830) as a tracer for antibody injection (Fig. 4a). For TALhesin Trim-away, antibodies were injected with 7 pg of 0.5 mg / mL rabbit anti-GFP antibody in PBS with 0.03% NP-40. Cells were then directly imaged in M2 medium containing SiR-DNA.

For parthenogenic activation of mouse eggs, *in vitro* matured eggs were incubated for one hour in M2 medium containing 200 nM SiR-DNA to label chromosomes. They were then washed in nine 20 µL droplets under oil of calcium-free M2 medium with SiR-DNA. The eggs were then washed through nine 20 µL droplets under oil of calcium-free M2 medium supplemented with 10 mM SrCl₂ (Sigma-Aldrich: 255521) with SiR-DNA then directly imaged in the same medium 34

For parthenogenic activation of human eggs, GV or MI stage oocytes were injected with 0.0002 amol mRNA and *in vitro* matured to MII eggs. Cells were incubated at least 3 hours with 100 nM SiR-DNA in GMOPS-PLUS media (VitroLife: 10130). They were then washed in nine 20 µL droplets under oil of SiR-DNA/GMOPS-PLUS media containing 10 µM ionomycin (calcium ionophore, MP Biomedicals) and incubated 10 minutes in the final droplet ³⁵. The cells were then washed through nine 20 µL droplets of SiR-DNA/GMOPS-PLUS media under oil and immediately prepared for imaging. Human activation was visualized with a Viventis LS1 Live lightsheet microscope (Viventis, Lausanne, Switzerland) in SiR-DNA/GMOPS-PLUS media under oil 37.5°C.

### HeLa cell culture

HeLa (CCL2) cells were cultured in high-glucose Dulbecco's modification of Eagles media (Thermo-Fisher: 11965084) supplemented with 10% fetal bovine serum (Thermo-Fisher: 26140079) at 37°C with 5% carbon dioxide in air. Cells were plated at 120,000 cells/ml on 4-well dishes (Ibidi: 80427). Cells were cultured for 24 hours before transfection using Lipofectamine-LTX (Thermo-Fisher: 15338100), according to manufacturer's instructions. Briefly, each well was transfected with either 0.125 µg of TALhesin[*hsCentro*]-B or 0.125 µg of both TALhesin[*hsCentro*]-A and TALhesin[*hsCentro*]-B diluted in Opti-MEM (Thermo Fisher Scientific: 31985062) supplemented with PLUS reagent (volume equal to plasmid DNA) and 1 µl of lipofectamine LTX. After 8 hours of transfection, cells were synchronized through treatment with 9 µM RO-3306 (Sigma-Aldrich: SML0569) for 16 hours. After synchronization, cells were prepared for live-cell microscopy or for immunostaining. For live-cell microscopy, cells were released in media supplemented with 10 µM verampril and 1:10,000 SiR-DNA (Spirochrome: SC007). For immunostaining, cells were either fixed immediately after 16 hours of RO-3306 treatment or released for 24 hours in culture media before fixation.

### Immunofluorescence imaging of HeLa cells

HeLa cells were fixed in 4% methanol-free formaldehyde (Polysciences: 040181) in PBS for 30 minutes at room temperature. Fixed cells were permeabilized in 0.1% triton X-100 (Sigma-Aldrich: 93443) in PBS (0.1-PBT) for 1 hour at room temperature. Cells were blocked in 3% BSA / 0.1-PBT for 30 minutes at room temperature. Primary and secondary antibody solutions were prepared in blocking buffer. Cells were stained with the primary antibodies, anti-GFP (Abcam: Ab6556, 1:100), anti-Centromere (Antibodies Incorporated: 15234, 1:200), and anti-Tubulin (Bio-Rad: MCA78G, 1:100) for one hour at room temperature. CREST antibody was centrifuged at 16,000 × g for 30 minutes at 4°C to remove antibody aggregates. Cells were washed in PBS and incubated with secondary antibodies, Alexa-Fluor 488 conjugated donkey anti-rabbit (Thermo-Fisher: A21206, 1:400), Alexa-Fluor 568 conjugated goat anti-rat (Thermo-Fisher: A11077, 1:400), and Alexa-Fluor 647 conjugated goat anti human (Thermo-Fisher: A21445, 1:400), for one hour at room temperature. Cells were incubated with Hoechst solution (Thermo-Fisher: 62249) at 1:333 dilution in PBS for 15 minutes at room temperature. Cells were then washed in PBS and mounted in 50% glycerol.

### Immunofluorescence imaging of oocytes and eggs

Oocytes and eggs from mouse and human were fixed in 100 mM HEPES, pH 7.0, 50 mM EGTA, pH 7.0, 10 mM MgSO₄, 2% methanol-free formaldehyde, and 0.2% triton X-100 at 37°C for 30 min. Fixed cells were then extracted in PBS with 0.5% triton X-100 (0.5-PBT) for overnight at 4°C and blocked for at least 6 hours in 0.5% BSA / 0.5-PBT (BSA-0.5PBT) passed through a 0.22 µm syringe filter. All primary antibody incubations were performed for 18 hours at 4°C in BSA-0.5PBT. Primary antibodies used were human anti-Centromere (Antibodies Incorporated: 15234, 1:10 for mouse cells; 1:20 for human cells), rabbit anti-GFP (Abcam: Ab6556, 1:100 for mouse cells; 1:50 for human cells), and rat anti-Tubulin (Bio-Rad: MCA78G, 1:200). Secondary antibody incubations were performed at a 1:200 concentration for 1 hour at room temperature. Secondary antibodies used were Alexa-Fluor 488 conjugated goat anti-human (Thermo-Fisher: A11013), Alexa-Fluor conjugated donkey anti-rat (Thermo-Fisher: A10042), and Alexa-Fluor 647 conjugated goat anti-rat (Thermo-Fisher: A2124). Primary and secondary antibody incubations were each followed by five washes in 15 µL BSA-0.5PBT over two hours at room temperature. Cells were stained with 400 µM Hoechst 33342 (Thermo-Fisher: 62249) for one hour, washed in PBS, then directly imaged in PBS.

### Microscopy

Live confocal microscopy was performed using LSM-800, LSM-880, LSM-900, or LSM-980 equipped with a 40×C-Apochromat 1.2 NA water-immersion objective at 37.0°C. Images were acquired with an optical slice thickness of 2.0 µm confocal section with a z-stack interval of 1.0 µm covering approximately 20 µm. mClover, mCherry, and SiR-DNA were imaged simultaneously with 488, 561, and 647 nm wavelength laser, respectively, with a 2.53 sec frame imaging time with averaging set to two. Super-resolution of fixed immuno-fluorescently labelled cells were acquired using Airyscan modules and processed using ZEN software. Normally, 100 to 120 z-sections were acquired to ensure all chromosomes within the meiotic spindle were detected.

### Fluorescence Recovery After Photo-bleaching (FRAP)

MII mouse eggs expressing 0.1 amol mRNA coding TAL[*mmMajSat*] or TALhesin proteins were used for determining protein mobility. Square regions of interest (ROIs) corresponding to 10 µm² were photo-bleached after five pre-bleaching time points using 488 and 561 nm excitation laser lines at 100% power. The temporal interval between images was 1.26 sec with averaging set to two. Analysis was performed as previously described⁴⁸.

### In situ chromosome analysis

Airy-processed super-resolution images were analyzed using Imaris software version 9.1.2 (Bitplane). Automated spot detection was used to identify kinetochore centers based on local maxima (spot diameter of 360 nm). For some cells, chromosomes were segmented using automated surface function followed by masking of channels corresponding to kinetochores and mClover foci. Inter-sister kinetochore (iKt) distances were obtained by manual pairwise measurements between detected spots. Aneuploidy was recorded when more or less than 20 and 23 iKt distances could be measured, for mouse and human respectively. PSSC was recorded when an iKt distance ≥3.0 µm was measured, and/or when single chromatids with a single kinetochore were observed.

### Semi-Automated Chromosome Segmentation

Chromosome segmentation was performed in Imaris 9.3 using scripts written in Matlab R2018b. Segmentation combines Otsu thresholding with the watershed algorithm and a 'greedy' method addressing over-segmentation. First, sister kinetochores were manually paired as filaments to initiate the segmentation algorithm. The image was interpolated linearly to achieve almost equidistance between voxels in each dimension, addressing anisotropy. A mask using the maximum expected volume of 800 µm³ was applied regularly to remove artifacts. Next, deconvolution of the image to improve segmentation was applied with an estimated point-spread function of one micrometer using the Matlab function deconvblind. This image was used to create an image that is smoothed with a 0.2 µm Gaussian filter, in addition to a five micrometer Gaussian smoothed image for background subtraction. Otsu thresholding was then attempted up to five times with an increasing number of simultaneous thresholds to find a segmentation that satisfies the boundary conditions of at least 50 µm³ and at most 800 µm³ volume. Small fragments caused by noise were removed with the function bwareaopen set to a size limit of 0.0080 µm³. The watershed algorithm uses a distance map from the image to create 'connected components' (CCs). The watershed segmentation is morphologically closed with a one pixel radius. Any resulting CC of the watershed result sized 20 voxels or smaller was removed. CCs that contain a single seed are assigned to that seed. CCs that contain multiple seeds are divided among those seeds based on a distance map. CCs that do not contain any seeds are assigned to neighboring CCs based on contact areas. This 'greedy' assignment method starts with the largest contact area in the entire dataset and continues until no contact areas are left. CCs that remain isolated and unassigned are assigned to the nearest CC based on the average distance of the pixels on their perimeter. Finally, holes are filled using the Matlab function imfill. After the filling of holes, the CCs are assigned to seeds a second time. On occasion, some segmented chromosomes required manual segmentation in Imaris. Fluorescence background signal from outside of pericentromeric foci was then subtracted from mean fluorescence intensity measurements. This was achieved by segmenting high intensity signal region within a chromosome and setting intensity measurements to zero. The mean fluorescence intensity of these regions was then subtracted from the total mean fluorescence intensity of each segmented chromosomes.

### Statistical analysis

Statistical analyses were performed using Origin2019b, GraphPad, or R. Data from chromosome error-reduction assays were first analyzed using the Shapiro-Wilks test for normality. All conditions returned p-values of < 0.05, rejecting a normal distribution. The inventors therefore used a one-tailed Fisher's Exact test to determine the significance value for TALhesin reducing the frequency of PSSC. Here, the inventors tested total intact sister chromatids versus total PSSC errors between two conditions as indicated in each figure. Error bars indicated 95% confidence interval, calculated using the R function, prop.test, implemented with one degree of freedom. For inter-kinetochore distance measurements and Fig. 2d, statistical significance was calculated using unpaired two-tailed Student's t-test with Welsh correction in Origin2019b. Boxes indicate the 25 and 75^{th} percentile range and bars set to a standard deviation (SD) coefficient of 1.5, or indicate standard error from the mean (SEM) where indicated. Mean inter-kinetochore (iKt) distance is marked by a square, and line indicates median.

### Tables

Amino acid sequences are displayed in the standard single letter amino acid code. RNA and DNA sequences are displayed in the standard single letter code for nucleic acids. The skilled person is aware that DNA molecules contain thymine (T), whereas RNA contain uracil (U). The following table reports DNA and RNA sequences accordingly. The skilled person is also aware that a RNA sequence may be converted into a DNA sequence by replacing the U residues by T residues, and *vice versa.*

| **Sequences of Components** | | |
|---|---|---|
| SEQ ID NO | Name (Description) | Sequences |
| 1 | >SpyCatcher001 | AUGGAUAGUGCUACCCAUAUUAAAUUCUCAAAACGUGAUGAGGACGGCAAAGAGUU AGCUGGUGCAACUAUGGAGUUGCGUGAUUCAUCUGGUAAAACUAUUAGUACAUGGA UUUCAGAUGGACAAGUGAAAGAUUUCUACCUGUAUCCAGGAAAAUAUACAUUUGUC GAAACCGCAGCACCAGACGGUUAUGAGGUAGCAACUGCUAUUACCUUUACAGUUAA UGAGCAAGGUCAGGUUACUGUAAAUGGCAAAGCAACUAAAGGUGACGCUCAUAUUG GAUCC |
| | >mRNA transcript | |
| 2 | >SpyCatcher001 | MDSATHIKFSKRDEDGKELAGATMELRDSSGKTISTWISDGQVKDFYLYPGKYTFVETAA PDGYEVATAITFTVNEQGQVTVNGKATKGDAHIGS |
| | >Protein Sequence | |
| 3 | >SpyTag001 | GGUUCCGCCCACAUCGUGAUGGUGGACGCCUACAAGCCGACGAAG |
| | >mRNA transcript | |
| 4 | >SpyTag001 | GSAHIVMVDAYKPTK |
| | >Protein Sequence | |
| 5 | >SpyCatcher003 | AUGACAACUGAAGAAGAUAGUGCUACCCAUAUUAAAUUCUCAAAACGUGAUGAGGAC GGCCGUGAGUUAGCUGGUGCAACUAUGGAGUUGCGUGAUUCAUCUGGUAAAACUAU UAGUACAUGGAUUUCAGAUGGACAUGUGAAGGAUUUCUACCUGUAUCCAGGAAAAU AUACAUUUGUCGAAACCGCAGCACCAGACGGUUAUGAGGUAGCAACUCCAAUUGAAU UUACAGUUAAUGAGGACGGUCAGGUUACUGUAGAUGGUGAAGCAACUGAAGGUGAC GCUCAUACUGGAUCCAGUGGUAGC |
| | >mRNA transcript | |
| 6 | >SpyCatcher003 | MTTEEDSATHIKFSKRDEDGRELAGATMELRDSSGKTISTWISDGHVKDFYLYPGKYTFV ETAAPDGYEVATPIEFTVNEDGQVTVDGEATEGDAHTGSSGS |
| | >Protein Sequence | |
| 7 | >SpyTag003 | AGAGGAGUACCUCACAUCGUGAUGGUGGACGCCUACAAGCGGUACAAG |
| | >mRNA transcript | |
| 8 | >SpyTag003 | RGVPHIVMVDAYKRYK |
| | >Protein Sequence | |
| 9 | >TAL[mmMajSat] | AUGCAGGUGGAUCUACGCACGCUCGGCUACAGUCAGCAGCAAGAGAAGAUCAAA CCGAAGGUGCGUUCGACAGUGGCGCAGCACCACGAGGCACUGGUGGGCCAUGGGUU UACACACGCGCACAUCGUUGCGCUCAGCCAACACCCGGCAGCGUUAGGGACCGUCGC UGUCACGUAUCAGCACAUAAUCACGGCGUUGCCAGAGGCGACACACGAAGACAUCGU UGGCGUCGGCAAACAGUGGUCCGGCGCACGCGCCCUGGAGGCCUUGCUCACGGAUG CGGGGGAGUUGAGAGGUCCGCCGUUACAGUUGGACACAGGCCAACUUGUGAAGAUU GCAAAACGUGGCGGCGUGACCGCAAUGGAGGCAGUGCAUGCAUCGCGCAAUGCACU GACGGGUGCCCCCCUGAACCUGACCCCGGACCAAGUGGUGGCUAUCGCCAGCAACG GUGGCGGCAAGCAAGCGCUCGAAACGGUGCAGCGGCUGUUGCCGGUGCUGUGCCAG GACCAUGGCCUGACCCCGGACCAAGUGGUGGCUAUCGCCAGCAACAAGGGCGGCAA GCAAGCGCUCGAAACGGUGCAGCGGCUGUUGCCGGUGCUGUGCCAGGACCAUGGCC UGACUCCGGACCAAGUGGUGGCUAUCGCCAGCCACGAUGGCGGCAAGCAAGCGCUC GAAACGGUGCAGCGGCUGUUGCCGGUGCUGUGCCAGGACCAUGGCCUGACUCCGGA CCAAGUGGUGGCUAUCGCCAGCCACGAUGGCGGCAAGCAAGCGCUCGAAACGGUGC AGCGGCUGUUGCCGGUGCUGUGCCAGGACCAUGGCCUGACCCCGGACCAAGUGGUG GCUAUCGCCAGCAACAUUGGCGGCAAGCAAGCGCUCGAAACGGUGCAGCGGCUGUU GCCGGUGCUGUGCCAGGACCAUGGCCUGACCCCGGACCAAGUGGUGGCUAUCGCCA GCAACGGUGGCGGCAAGCAAGCGCUCGAAACGGUGCAGCGGCUGUUGCCGGUGCUG UGCCAGGACCAUGGCCUGACCCCGGACCAAGUGGUGGCUAUCGCCAGCAACAUUGG CGGCAAGCAAGCGCUCGAAACGGUGCAGCGGCUGUUGCCGGUGCUGUGCCAGGACC AUGGCCUGACCCCGGACCAAGUGGUGGCUAUCGCCAGCAACGGUGGCGGCAAGCAA GCGCUCGAAACGGUGCAGCGGCUGUUGCCGGUGCUGUGCCAGGACCAUGGCCUGAC CCCGGACCAAGUGGUGGCUAUCGCCAGCAACGGUGGCGGCAAGCAAGCGCUCGAAA CGGUGCAGCGGCUGUUGCCGGUGCUGUGCCAGGACCAUGGCCUGACUCCGGACCAA GUGGUGGCUAUCGCCAGCCACGAUGGCGGCAAGCAAGCGCUCGAAACGGUGCAGCG GCUGUUGCCGGUGCUGUGCCAGGACCAUGGCCUGACCCCGGACCAAGUGGUGGCUA UCGCCAGCCACGAUGGCGGCAAGCAAGCGCUCGAAACGGUGCAGCGGCUGUUGCCG GUGCUGUGCCAGGACCAUGGCCUGACCCCGGACCAAGUGGUGGCUAUCGCCAGCAA CAUUGGCGGCAAGCAAGCGCUCGAAACGGUGCAGCGGCUGUUGCCGGUGCUGUGCC AGGACCAUGGCCUGACUCCGGACCAAGUGGUGGCUAUCGCCAGCCACGAUGGCGGC AAGCAAGCGCUCGAAACGGUGCAGCGGCUGUUGCCGGUGCUGUGCCAGGACCAUGG CCUGACCCCGGACCAAGUGGUGGCUAUCGCCAGCAACAAGGGCGGCAAGCAAGCGCU CGAAACGGUGCAGCGGCUGUUGCCGGUGCUGUGCCAGGACCAUGGCCUGACCCCGG ACCAAGUGGUGGCUAUCGCCAGCAACGGUGGCGGCAAGCAAGCGCUCGAAAGCAUU GUGGCCCAGCUGAGCCGGCCUGAUCCGGCGUUGGCCGCGUUGACCAACGACCACCU CGUCGCCUUGGCCUGCCUCGGCGGACGUCCUGCCAUGGAUGCAGUGAAAAAGGGAU UGCCGCACGCGCCGGAAUUGAUCAGAAGAGUCAAUCGCCGUAUUGGCGAACGCACG UCCCAUCGCGUUGCGGGAUCCACUGGAGGCGGAGGUGGCUCCGGCACCGCCUCCUC C |
| | >mRNA transcript | |
| 10 | >TAL[mmMajSat] | MQVDLRTLGYSQQQQEKIKPKVRSTVAQHHEALVGHGFTHAHIVALSQHPAALGTVAVT YQHIITALPEATHEDIVGVGKQWSGARALEALLTDAGELRGPPLQLDTGQLVKIAKRGGV TAMEAVHASRNALTGAPLNLTPDQVVAIASNGGGKQALETVQRLLPVLCQDHGLTPDQV VAIASNKGGKQALETVQRLLPVLCQDHGLTPDQVVAIASHDGGKQALETVQRLLPVLCQ DHGLTPDQVVAIASHDGGKQALETVQRLLPVLCQDHGLTPDQVVAIASNIGGKQALETV QRLLPVLCQDHGLTPDQVVAIASNGGGKQALETVQRLLPVLCQDHGLTPDQVVAIASNIG GKQALETVQRLLPVLCQDHGLTPDQVVAIASNGGGKQALETVQRLLPVLCQDHGLTPDQ VVAIASNGGGKQALETVQRLLPVLCQDHGLTPDQVVAIASHDGGKQALETVQRLLPVLC QDHGLTPDQVVAIASHDGGKQALETVQRLLPVLCQDHGLTPDQVVAIASNIGGKQALET VQRLLPVLCQDHGLTPDQVVAIASHDGGKQALETVQRLLPVLCQDHGLTPDQVVAIASN KGGKQALETVQRLLPVLCQDHGLTPDQVVAIASNGGGKQALESIVAQLSRPDPALAALTN DHLVALACLGGRPAMDAVKKGLPHAPELIRRVNRRIGERTSHRVAGSTGGGGGSGTASS |
| | >Protein Sequence | |
| 11 | >TAL[mmMajSat] Target Sequence | TGCCATATTCCACGT |
| | >DNA Sequence | |
| 12 | >mRec8 | ACAAGUUUGUACAAAAAAGCAGGCUCCGCGGCCGCCCCCUUCACCAUGCUCAGACAA AGGCCACAGCUGGAGACUGAGGAGACAGUGGAGGAAGAGAGAGCAGCAGAUGAGGA AGAGAGGAGAAAGACUGAAGCUCUGAGCGAGAUCGAGGUUCUGAGGGAGGCCCAGG AGCCCAGUGGUCCCCUCAUGCUGUCUUCAGAGCUCUCCCUAGAAGCAGCAGAAGAU GAAAAGUCUCGUACUAGCCUCAUCCCCCCAGAAUGGUGGGCCUGGUCUGAGGAGGG GCAGCCCGAGCCCCCUGCACUGCCCAUGCUGCCUGAACUUCCUGAAGUGCCCAUGGA GAUGCCCCCAAGGCCUGAACUCUCUUCAGAGGCUAAGGGUGGGCGCGCCGACCCAG CUUUCUUGUACAAAGUG |
| | >Mouse Rec8 Fragment (aa417-525) | |
| | >Rec8 sequence inserted for mouse | |
| | >mRNA transcript | |
| 13 | >mRec8 | TSLYKKAGSAAAPFTMLRQRPQLETEETVEEERAADEEERRKTEALSEIEVLREAQEPSGP LMLSSELSLEAAEDEKSRTSLIPPEWWAWSEEGQPEPPALPMLPELPEVPMEMPPRPELS SEAKGGRADPAFLYKV |
| | >Mouse Rec8 Fragment (aa417-525) | |
| | >Rec8 sequence inserted for mouse | |
| | >Amino acid sequence | |
| 14 | >mRec8 C2 sequence | GAGACAGUGGAGGAAGAGAGAGCA |
| | >Mouse Rec8 Second Cleavage motif | |
| | >Within Mouse Rec8 Fragment (aa417-525) inserted for Mouse TALhesin | |
| | >mRNA transcript | |
| 15 | >mRec8 C2 sequence | ETVEEERA |
| | >Mouse Rec8 Second Cleavage motif | |
| | >Within Mouse Rec8 Fragment (aa417-525) inserted for Mouse TALhesin >Protein Sequence | |
| 16 | >mRec8 C3 sequence | GAGAUCGAGGUUCUGAGGGAG |
| | >Mouse Rec8 Third Cleavage motif | |
| | >Within Mouse Rec8 Fragment (aa417-525) inserted for Mouse TALhesin | |
| | >mRNA transcript | |
| 17 | >mRec8 C3 sequence | EIEVLRE |
| | >Mouse Rec8 Third Cleavage motif | |
| | >Within Mouse Rec8 Fragment (aa417-525) inserted for Mouse TALhesin | |
| | >Protein Sequence | |
| 18 | >L1 Linker inserted into TALhesin[mmMajSat] (Rec8)-A/B | ACCGGUACAAGUUUGUACAAAAAAGCAGGCUCCGCGGCCGCCCCCUUCACC |
| | >Figure 7; Figure 8a | |
| | >Inserted into TALhesin[mmMajSat] (Rec8)-A and B | |
| | >mRNA transcript | |
| 19 | >L1 Linker inserted into TALhesin[mmMajSat] (Rec8)-A/B | TGTSLYKKAGSAAAPFT |
| | >Figure 7; 8a | |
| | >Inserted into TALhesin[mmMajSat] (Rec8)-A and B | |
| | >Protein Sequence | |
| 20 | >L2 Linker inserted into TALhesin[mmMajSat] (Rec8)-A | ACCGGUAUGGUGAGCAAGGGCGAGGAGGAUAACAUGGCCAUCAUCAAGGAGUUCAU GCGCUUCAAGGUGCACAUGGAGGGCUCCGUGAACGGCCACGAGUUCGAGAUCGAGG GCGAGGGCGAGGGCCGCCCCUACGAGGGCACCCAGACCGCCAAGCUGAAGGUGACCA AGGGUGGCCCCCUGCCCUUCGCCUGGGACAUCCUGUCCCCUCAGUUCAUGUACGGC UCCAAGGCCUACGUGAAGCACCCCGCCGACAUCCCCGACUACUUGAAGCUGUCCUUC CCCGAGGGCUUCAAGUGGGAGCGCGUGAUGAACUUCGAGGACGGCGGCGUGGUGAC CGUGACCCAGGACUCCUCCCUGCAGGACGGCGAGUUCAUCUACAAGGUGAAGCUGC GCGGCACCAACUUCCCCUCCGACGGCCCCGUAAUGCAGAAGAAGACCAUGGGCUGGG AGGCCUCCUCCGAGCGGAUGUACCCCGAGGACGGCGCCCUGAAGGGCGAGAUCAAG CAGAGGCUGAAGCUGAAGGACGGCGGCCACUACGACGCUGAGGUCAAGACCACCUAC AAGGCCAAGAAGCCCGUGCAGCUGCCCGGCGCCUACAACGUCAACAUCAAGUUGGAC AUCACCUCCCACAACGAGGACUACACCAUCGUGGAACAGUACGAACGCGCCGAGGGC CGCCACUCCACCGGCGGCAUGGACGAGCUGUACAAGGGUACAAGUUUGUACAAAAAA GCAGGCUCCGCGGCCGCCCCCUUCACCAUGCUCAGACAAAGGCCACAGCUGGAGACU GAGGAGACAGUGGAGGAAGAGAGAGCAGCAGAUGAGGAAGAGAGGAGAAAGACUGA AGCUCUGAGCGAGAUCGAGGUUCUGAGGGAGGCCCAGGAGCCCAGUGGUCCCCUCA UGCUGUCUUCAGAGCUCUCCCUAGAAGCAGCAGAAGAUGAAAAGUCUCGUACUAGCC UCAUCCCCCCAGAAUGGUGGGCCUGGUCUGAGGAGGGGCAGCCCGAGCCCCCUGCA CUGCCCAUGCUGCCUGAACU14UCCUGAAGUGCCCAUGGAGAUGCCCCCAAGGCCUG AACUCUCUUCAGAGGCUAAGGGUGGGCGCGCCGACCCAGCUUUCUUGUACAAAGUG GUAAGCUUA |
| | >Figure 7, 8a; | |
| | >mRNA transcript | |
| 21 | >L2 Linker inserted into TALhesin[mmMajSat] (Rec8)-A | TGMVSKGEEDNMAIIKEFMRFKVHMEGSVNGHEFEIEGEGEGRPYEGTQTAKLKVTKGG PLPFAWDILSPQFMYGSKAYVKHPADIPDYLKLSFPEGFKWERVMNFEDGGVVTVTQDS SLQDGEFIYKVKLRGTNFPSDGPVMQKKTMGWEASSERMYPEDGALKGEIKQRLKLKDG GHYDAEVKTTYKAKKPVQLPGAYNVNIKLDITSHNEDYTIVEQYERAEGRHSTGGMDELY KGTSLYKKAGSAAAPFTMLRQRPQLETEETVEEERAADEEERRKTEALSEIEVLREAQEPS GPLMLSSELSLEAAEDEKSRTSLIPPEWWAWSEEGQPEPPALPMLPELPEVPMEMPPRPE LSSEAKGGRADPAFLYKVVSL |
| | >Figure 7; 8a >Protein Sequence | |
| 22 | >L2 Linker inserted into TALhesin[mmMajSat] (Rec8)-B | ACCGGUAUGGUGAGCAAGGGCGAGGAGGAUAACAUGGCCAUCAUCAAGGAGUUCAU GCGCUUCAAGGUGCACAUGGAGGGCUCCGUGAACGGCCACGAGUUCGAGAUCGAGG GCGAGGGCGAGGGCCGCCCCUACGAGGGCACCCAGACCGCCAAGCUGAAGGUGACCA AGGGUGGCCCCCUGCCCUUCGCCUGGGACAUCCUGUCCCCUCAGUUCAUGUACGGC UCCAAGGCCUACGUGAAGCACCCCGCCGACAUCCCCGACUACUUGAAGCUGUCCUUC CCCGAGGGCUUCAAGUGGGAGCGCGUGAUGAACUUCGAGGACGGCGGCGUGGUGAC CGUGACCCAGGACUCCUCCCUGCAGGACGGCGAGUUCAUCUACAAGGUGAAGCUGC GCGGCACCAACUUCCCCUCCGACGGCCCCGUAAUGCAGAAGAAGACCAUGGGCUGGG AGGCCUCCUCCGAGCGGAUGUACCCCGAGGACGGCGCCCUGAAGGGCGAGAUCAAG CAGAGGCUGAAGCUGAAGGACGGCGGCCACUACGACGCUGAGGUCAAGACCACCUAC AAGGCCAAGAAGCCCGUGCAGCUGCCCGGCGCCUACAACGUCAACAUCAAGUUGGAC AUCACCUCCCACAACGAGGACUACACCAUCGUGGAACAGUACGAACGCGCCGAGGGC CGCCACUCCACCGGCGGCAUGGACGAGCUGUACAAGGGUACAAGUUUGUACAAAAAA GCAGGCUCCGCGGCCGCCCCCUUCACCAUGCUCAGACAAAGGCCACAGCUGGAGACU GAGGAGACAGUGGAGGAAGAGAGAGCAGCAGAUGAGGAAGAGAGGAGAAAGACUGA AGCUCUGAGCGAGAUCGAGGUUCUGAGGGAGGCCCAGGAGCCCAGUGGUCCCCUCA UGCUGUCUUCAGAGCUCUCCCUAGAAGCAGCAGAAGAUGAAAAGUCUCGUACUAGCC UCAUCCCCCCAGAAUGGUGGGCCUGGUCUGAGGAGGGGCAGCCCGAGCCCCCUGCA CUGCCCAUGCUGCCUGAACUUCCUGAAGUGCCCAUGGAGAUGCCCCCAAGGCCUGAA CUCUCUUCAGAGGCUAAGGGUGGGCGCGCCGACCCAGCUUUCUUGUACAAAGUGGU AAGCUUCGAAUUCAUGGUGAGCAAGGGCGAGGAGCUGUUCACCGGGGUGGUGCCCA UCCUGGUCGAGCUGGACGGCGACGUAAACGGCCACAAGUUCAGCGUCCGCGGCGAG GGCGAGGGCGAUGCCACCAACGGCAAGCUGACCCUGAAGUUCAUCUGCACCACCGGC AAGCUGCCCGUGCCCUGGCCCACCCUCGUGACCACCUUCGGCUACGGCGUGGCCUG CUUCAGCCGCUACCCCGACCACAUGAAGCAGCACGACUUCUUCAAGUCCGCCAUGCC CGAAGGCUACGUCCAGGAGCGCACCAUCUCUUUCAAGGACGACGGUACCUACAAGAC CCGCGCCGAGGUGAAGUUCGAGGGCGACACCCUGGUGAACCGCAUCGAGCUGAAGG GCAUCGACUUCAAGGAGGACGGCAACAUCCUGGGGCACAAGCUGGAGUACAACUUCA ACAGCCACAACGUCUAUAUCACGGCCGACAAGCAGAAGAACGGCAUCAAGGCUAACU UCAAGAUCCGCCACAACGUUGAGGACGGCAGCGUGCAGCUCGCCGACCACUACCAGC AGAACACCCCCAUCGGCGACGGCCCCGUGCUGCUGCCCGACAACCACUACCUGAGCC AUCAGUCCAAGCUGAGCAAAGACCCCAACGAGAAGCGCGAUCACAUGGUCCUGCUGG AGUUCGUGACCGCCGCCGGGAUUACACAUGGCAUGGACGAGCUGUACAAGGUCGAC |
| | >Figure 7; 8a; | |
| | >mRNA transcript | |
| 23 | >L2 Linker inserted into TALhesin[mmMajSat] (Rec8)-B | TGMVSKGEEDNMAIIKEFMRFKVHMEGSVNGHEFEIEGEGEGRPYEGTQTAKLKVTKGG PLPFAWDILSPQFMYGSKAYVKHPADIPDYLKLSFPEGFKWERVMNFEDGGVVTVTQDS SLQDGEFIYKVKLRGTNFPSDGPVMQKKTMGWEASSERMYPEDGALKGEIKQRLKLKDG GHYDAEVKTTYKAKKPVQLPGAYNVNIKLDITSHNEDYTIVEQYERAEGRHSTGGMDELY KGTSLYKKAGSAAAPFTMLRQRPQLETEETVEEERAADEEERRKTEALSEIEVLREAQEPS GPLMLSSELSLEAAEDEKSRTSLIPPEWWAWSEEGQPEPPALPMLPELPEVPMEMPPRPE LSSEAKGGRADPAFLYKVVSFEFMVSKGEELFTGVVPILVELDGDVNGHKFSVRGEGEGD ATNGKLTLKFICTTGKLPVPWPTLVTTFGYGVACFSRYPDHMKQHDFFKSAMPEGYVQE RTISFKDDGTYKTRAEVKFEGDTLVNRIELKGIDFKEDGNILGHKLEYNFNSHNVYITADK QKNGIKANFKIRHNVEDGSVQLADHYQQNTPIGDGPVLLPDNHYLSHQSKLSKDPNEKR DHMVLLEFVTAAGITHGMDELYKVD |
| | >Figure 7; 8a | |
| | >Protein Sequence | |
| **24** | >TAL[**hs**CENTRO] | AUGGUAGAUUUGAGAACUUUGGGAUAUUCACAGCAGCAGGAAAAGAU CAAGCCCAAAGUGAGGUCGACAGUCGCGCAGCAUCACGAAGCGCUGGUGG GUCAUGGGUUUACACAUGCCCACAUCGUAGCCUUGUCGCAGCACCCUGCA GCCCUUGGCACGGUCGCCGUCAAGUACCAGGACAUGAUUGCGGCGUUGCC GGAAGCCACACAUGAGGCGAUCGUCGGUGUGGGGAAACAGUGGAGCGGAG CCCGAGCGCUUGAGGCCCUGUUGACGGUCGCGGGAGAGCUGAGAGGGCCU CCCCUUCAGCUGGACACGGGCCAGUUGCUGAAGAUCGCGAAGCGGGGAGG AGUCACGGCGGUCGAGGCGGUGCACGCGUGGCGCAAUGCGCUCACGGGAG CACCCCUCAACCUGACCCCAGAGCAGGUCGUGGCAAUUGCGAGCCAUGACG GGGGAAAGCAGGCACUCGAAACCGUCCAGAGGUUGCUGCCUGUGCUGUGC CAAGCGCACGGACUUACGCCAGAGCAGGUCGUGGCAAUUGCGAGCCAUGA CGGGGGAAAGCAGGCACUCGAAACCGUCCAGAGGUUGCUGCCUGUGCUGU GCCAAGCGCACGGACUAACCCCAGAGCAGGUCGUGGCAAUUGCGAGCAAC AUCGGGGGAAAGCAGGCACUCGAAACCGUCCAGAGGUUGCUGCCUGUGCU GUGCCAAGCGCACGGGUUGACCCCAGAGCAGGUCGUGGCAAUUGCGAGCA ACGGAGGGGGAAAGCAGGCACUCGAAACCGUCCAGAGGUUGCUGCCUGUG CUGUGCCAAGCGCACGGGUUGACCCCAGAGCAGGUCGUGGCAAUUGCGAG CAACGGAGGGGGAAAGCAGGCACUCGAAACCGUCCAGAGGUUGCUGCCUG UGCUGUGCCAAGCGCACGGACUGACACCAGAGCAGGUCGUGGCAAUUGCG AGCCAUGACGGGGGAAAGCAGGCACUCGAAACCGUCCAGAGGUUGCUGCC UGUGCUGUGCCAAGCGCACGGACUUACACCCGAACAAGUCGUGGCAAUUG CGAGCCAUGACGGGGGAAAGCAGGCACUCGAAACCGUCCAGAGGUUGCUG CCUGUGCUGUGCCAAGCGCACGGACUAACCCCAGAGCAGGUCGUGGCAAU UGCGAGCAACAUCGGGGGAAAGCAGGCACUCGAAACCGUCCAGAGGUUGC UGCCUGUGCUGUGCCAAGCGCACGGGUUGACCCCAGAGCAGGUCGUGGCA AUUGCGAGCAACGGAGGGGGAAAGCAGGCACUCGAAACCGUCCAGAGGUU GCUGCCUGUGCUGUGCCAAGCGCACGGGUUGACCCCAGAGCAGGUCGUGG CAAUUGCGAGCAACGGAGGGGGAAAGCAGGCACUCGAAACCGUCCAGAGG UUGCUGCCUGUGCUGUGCCAAGCGCACGGACUGACACCAGAGCAGGUCGU GGCAAUUGCGAGCCAUGACGGGGGAAAGCAGGCACUCGAAACCGUCCAGA GGUUGCUGCCUGUGCUGUGCCAAGCGCACGGACUUACACCCGAACAAGUC GUGGCAAUUGCGAGCCAUGACGGGGGAAAGCAGGCACUCGAAACCGUCCA GAGGUUGCUGCCUGUGCUGUGCCAAGCGCACGGCCUCACCCCAGAGCAGG UCGUGGCAAUUGCGAGCAACAUCGGGGGAAAGCAGGCACUCGAAACCGUC CAGAGGUUGCUGCCUGUGCUGUGCCAAGCGCACGGACUUACGCCAGAGCA GGUCGUGGCAAUUGCGAGCAACGGAGGGGGAAAGCAGGCACUCGAAACCG UCCAGAGGUUGCUGCCUGUGCUGUGCCAAGCGCACGGACUAACCCCAGAG CAGGUCGUGGCAAUUGCGAGCAACGGAGGGGGAAAGCAGGCACUCGAAAC CGUCCAGAGGUUGCUGCCUGUGCUGUGCCAAGCGCACGGGUUGACCCCAG AGCAGGUCGUGGCAAUUGCGAGCCAUGACGGGGGAAAGCAGGCACUCGAA ACCGUCCAGAGGUUGCUGCCUGUGCUGUGCCAAGCGCACGGCCUGACCCC AGAGCAGGUCGUGGCAAUUGCGAGCCAUGACGGGGGAAAGCAGGCACUCG AAACCGUCCAGAGGUUGCUGCCUGUGCUGUGCCAAGCGCACGGACUGACA CCAGAGCAGGUCGUGGCAAUUGCGAGCAACAUCGGGGGAAAGCAGGCACU CGAAACCGUCCAGAGGUUGCUGCCUGUGCUGUGCCAAGCGCACGGACUCA CGCCUGAGCAGGUAGUGGCUAUUGCAUCCAACGGAGGGGGCAGACCCGCA CUGGAGUCAAUCGUGGCCCAGCUUUCGAGGCCGGACCCCGCGCUGGCCGC ACUCACUAAUGAUCAUCUUGUAGCGCUGGCCUGCCUCGGCGGACGACCCG CCUUGGAUGCGGUGAAGAAGGGGCUCCCGCACGCGCCUGCAUUGAUUAAG CGGACCAACAGAAGGAUUCCCGAGAGGACAUCACAUCGAGUGGCA |
| | >mRNA transcript | |
| **25** | >TAL[**hs**CENTRO] | MVDLRTLGYSQQQQEKIKPKVRSTVAQHHEALVGHGFTHAHIVALSQHPAALGTVAVKY QDMIAALPEATHEAIVGVGKQWSGARALEALLTVAGELRGPPLQLDTGQLLKIAKRGGVT AVEAVHAWRNALTGAPLNLTPEQVVAIASHDGGKQALETVQRLLPVLCQAHGLTPEQVV AIASHDGGKQALETVQRLLPVLCQAHGLTPEQVVAIASNIGGKQALETVQRLLPVLCQAH GLTPEQVVAIASNGGGKQALETVQRLLPVLCQAHGLTPEQVVAIASNGGGKQALETVQRL LPVLCQAHGLTPEQVVAIASHDGGKQALETVQRLLPVLCQAHGLTPEQVVAIASHDGGK QALETVQRLLPVLCQAHGLTPEQVVAIASNIGGKQALETVQRLLPVLCQAHGLTPEQVVAI ASNGGGKQALETVQRLLPVLCQAHGLTPEQVVAIASNGGGKQALETVQRLLPVLCQAHG LTPEQVVAIASHDGGKQALETVQRLLPVLCQAHGLTPEQVVAIASHDGGKQALETVQRLL PVLCQAHGLTPEQVVAIASNIGGKQALETVQRLLPVLCQAHGLTPEQVVAIASNGGGKQA LETVQRLLPVLCQAHGLTPEQVVAIASNGGGKQALETVQRLLPVLCQAHGLTPEQVVAIA SHDGGKQALETVQRLLPVLCQAHGLTPEQVVAIASHDGGKQALETVQRLLPVLCQAHGL TPEQVVAIASNIGGKQALETVQRLLPVLCQAHGLTPEQVVAIASNGGGRPALESIVAQLSR PDPALAALTNDHLVALACLGGRPALDAVKKGLPHAPALIKRTNRRIPERTSHRVA |
| | >Protein Sequence | |
| 26 | >TAL[hsCENTRO] Target Sequence | CCATTCCATTCCATTCCAT |
| | >DNA Sequence | |
| 27 | >hRec8 | CAUUGUGCCCAGCCACCCCCAAAAGCCCUCAGGCGAGAGCUGCCUGAGGAGGCAGCC GCUGAGGAGGAAAGGAGAAAGAUUGAAGUUCCAAGUGAGAUUGAGGUCCCGAGGGA GGCCCUGGAGCCCAGUGUUCCCCUUAUGGUGUCUUUAGAGAUCUCCCUAGAGGCAG CUGAAGAGGAGAAGUCCCGCAUCAGCCUCAUCCCACCAGAAGAACGGUGGGCCUGGC CUGAGGUGGAGGCGCCAGAAGCUCCUGCAUUGCCCGUGGUGCCUGAACUCCCUGAG GUGCCCAUGGAGAUGCCUUUGGUGCUGCCCCCAGAGCUCGAGCUGCUCAUC |
| | >Human Rec8 Fragment (aa368-434) | |
| | >Rec8 sequence inserted for Human TALhesin | |
| | >mRNA transcript | |
| 28 | >hRec8 | HCAQPPPKALRRELPEEAAAEEERRKIEVPSEIEVPREALEPSVPLMVSLEISLEAAEEEKSR ISLIPPEERWAWPEVEAPEAPALPVVPELPEVPMEMPLVLPPELELLI |
| | >Human Rec8 Fragment (aa368-434) | |
| | >Rec8 sequence inserted for Human TALhesin | |
| | >Protein Sequence | |
| 29 | >hRec8 C2 sequence | GAGGAGGAAAGG |
| | >Human Rec8 Second Cleavage motif | |
| | >Within Human Rec8 Fragment (aa368-434)inserted for Human TALhesin | |
| 30 | >hRec8 C2 sequence | EEER |
| | >Human Rec8 Second Cleavage motif | |
| | >Within Human Rec8 Fragment (aa368-434)inserted for Human TALhesin | |
| 31 | >hRec8 C3 sequence | GAGGUCCCGAGG |
| | >Human Rec8 Third Cleavage motif | |
| | >Within Human Rec8 Fragment (aa368-434)inserted for Human TALhesin | |
| 32 | >hRec8 C3 sequence | EVPR |
| | >Human Rec8 Third Cleavage motif | |
| | >Within Human Rec8 Fragment (aa368-434)inserted for Human TALhesin | |
| 33 | >L1 Linker inserted into TALhesin[*hsCEN-TRO*](Rec8)-A/B | ACCGGUACAAGUUUGUACAAAAAAGCAGGCUCCGCGGCCGCCCCCUUCACC |
| | >Figure 7; 8b | |
| | >Inserted into TALhesin[hsCEN-TRO](Rec8)-A and B | |
| | >mRNA transcript | |
| 34 | >L1 Linker inserted into TALhesin[*hsCEN-TRO*](Rec8)-A/B | TGTSLYKKAGSAAAPFT |
| | >Figure 7; 8b | |
| | >Inserted into TALhesin[hsCEN-TRO](Rec8)-A and B | |
| | >Protein Sequence | |
| 35 | >L2 Linker inserted into TALhesin[*hsCEN-TRO*](Rec8)-A | AAAGUGGUCCAUUGUGCCCAGCCACCCCCAAAAGCCCUCAGGCGAGAGCUGCCUGAG GAGGCAGCCGCUGAGGAGGAAAGGAGAAAGAUUGAAGUUCCAAGUGAGAUUGAGGU CCCGAGGGAGGCCCUGGAGCCCAGUGUUCCCCUUAUGGUGUCUUUAGAGAUCUCCC UAGAGGCAGCUGAAGAGGAGAAGUCCCGCAUCAGCCUCAUCCCACCAGAAGAACGGU GGGCCUGGCCUGAGGUGGAGGCGCCAGAAGCUCCUGCAUUGCCCGUGGUGCCUGAA CUCCCUGAGGUGCCCAUGGAGAUGCCUUUGGUGCUGCCCCCAGAGCUCGAGCUGCU CAGAAUGGUGAGCAAGGGCGAGGAGGAUAACAUGGCCAUCAUCAAGGAGUUCAUGC GCUUCAAGGUGCACAUGGAGGGCUCCGUGAACGGCCACGAGUUCGAGAUCGAGGGC GAGGGCGAGGGCCGCCCCUACGAGGGCACCCAGACCGCCAAGCUGAAGGUGACCAAG GGUGGCCCCCUGCCCUUCGCCUGGGACAUCCUGUCCCCUCAGUUCAUGUACGGCUC CAAGGCCUACGUGAAGCACCCCGCCGACAUCCCCGACUACUUGAAGCUGUCCUUCCC CGAGGGCUUCAAGUGGGAGCGCGUGAUGAACUUCGAGGACGGCGGCGUGGUGACCG UGACCCAGGACUCCUCCCUGCAGGACGGCGAGUUCAUCUACAAGGUGAAGCUGCGC GGCACCAACUUCCCCUCCGACGGCCCCGUAAUGCAGAAGAAGACCAUGGGCUGGGAG GCCUCCUCCGAGCGGAUGUACCCCGAGGACGGCGCCCUGAAGGGCGAGAUCAAGCA GAGGCUGAAGCUGAAGGACGGCGGCCACUACGACGCUGAGGUCAAGACCACCUACAA GGCCAAGAAGCCCGUGCAGCUGCCCGGCGCCUACAACGUCAACAUCAAGUUGGACAU CACCUCCCACAACGAGGACUACACCAUCGUGGAACAGUACGAACGCGCCGAGGGCCG CCACUCCACCGGCGGCAUGGACGAGCUGUACAAGAAA |
| | >Figure 7; 8b | |
| | >mRNA transcript | |
| 36 | >L2 Linker inserted into TALhesin[*hsCEN-TRO*](Rec8)-A | KVVHCAQPPPKALRRELPEEAAAEEERRKIEVPSEIEVPREALEPSVPLMVSLEISLEAAEEE KSRISLIPPEERWAWPEVEAPEAPALPVVPELPEVPMEMPLVLPPELELLRMVSKGEEDNM AIIKEFMRFKVHMEGSVNGHEFEIEGEGEGRPYEGTQTAKLKVTKGGPLPFAWDILSPQF MYGSKAYVKHPADIPDYLKLSFPEGFKWERVMNFEDGGVVTVTQDSSLQDGEFIYKVKL RGTNFPSDGPVMQKKTMGWEASSERMYPEDGALKGEIKQRLKLKDGGHYDAEVKTTYK AKKPVQLPGAYNVNIKLDITSHNEDYTIVEQYERAEGRHSTGGMDELYKK |
| | >Figure 7; 8b | |
| | >Protein Sequence | |
| 37 | >L2 Linker inserted into TALhesin[*hsCEN-TRO*](Rec8)-B | AAGGGUGGGCGCGCCGACCCAGCUUUCUUGUACAAAGUGGUCUCGAGCUCACAUUG UGCCCAGCCACCCCCAAAAGCCCUCAGGCGAGAGCUGCCUGAGGAGGCAGCCGCUGA GGAGGAAAGGAGAAAGAUUGAAGUUCCAAGUGAGAUUGAGGUCCCGAGGGAGGCCC UGGAGCCCAGUGUUCCCCUUAUGGUGUCUUUAGAGAUCUCCCUAGAGGCAGCUGAA GAGGAGAAGUCCCGCAUCAGCCUCAUCCCACCAGAAGAACGGUGGGCCUGGCCUGAG GUGGAGGCGCCAGAAGCUCCUGCAUUGCCCGUGGUGCCUGAACUCCCUGAGGUGCC CAUGGAGAUGCCUUUGGUGCUGCCCCCAGAGCUCGAGCUGCUCAUCGAAUUCAUGG UGAGCAAGGGCGAGGAGCUGUUCACCGGGGUGGUGCCCAUCCUGGUCGAGCUGGAC GGCGACGUAAACGACCACAAGUUCAGCGUCCGCGGCGAGGGCGAGGGCGAUGCCAC CAACGGCAAGCUGACCCUGAAGUUCAUCUGCACCACCGGCAAGCUGCCCGUGCCCUG GCCCACCCUCGUGACCACCUUCGGCUACGGCGUGGCCUGCUUCAGCCGCUACCCCGA CCACAUGAAGCAGCACGACUUCUUCAAGUCCGCCAUGCCCGAAGGCUACGUCCAGGA GCGCACCAUCUCUUUCAAGGACGACGGUACCUACAAGACCCGCGCCGAGGUGAAGUU CGAGGGCGACACCCUGGUGAACCGCAUCGAGCUGAAGGGCAUCGACUUCAAGGAGG ACGGCAACAUCCUGGGGCACAAGCUGGAGUACAACUUCAACAGCCACAACGUCUAUA UCACGGCCGACAAGCAGAAGAACGGCAUCAAGGCUAACUUCAAGAUCCGCCACAACG UUGAGGACGGCAGCGUGCAGCUCGCCGACCACUACCAGCAGAACACCCCCAUCGGCG ACGGCCCCGUGCUGCUGCCCGACAACCACUACCUGAGCCAUCAGUCCAAGCUGAGCA AAGACCCCAACGAGAAGCGCGAUCACAUGGUCCUGCUGGAGUUCGUGACCGCCGCCG GGAUUACACAUGGCAUGGACGAGCUGUACAAGGUCGAC |
| | >Figure 7; 8b | |
| | >mRNA transcript | |
| 38 | >L2 Linker inserted into TALhesin[*hsCEN-TRO*](Rec8)-B | KGGRADPAFLYKVVSSSHCAQPPPKALRRELPEEAAAEEERRKIEVPSEIEVPREALEPSVP LMVSLEISLEAAEEEKSRISLIPPEERWAWPEVEAPEAPALPVVPELPEVPMEMPLVLPPEL ELLIEFMVSKGEELFTGVVPILVELDGDVNDHKFSVRGEGEGDATNGKLTLKFICTTGKLP VPWPTLVTTFGYGVACFSRYPDHMKQHDFFKSAMPEGYVQERTISFKDDGTYKTRAEVK FEGDTLVNRIELKGIDFKEDGNILGHKLEYNFNSHNVYITADKQKNGIKANFKIRHNVED GSVQLADHYQQNTPIGDGPVLLPDNHYLSHQSKLSKDPNEKRDHMVLLEFVTAAGITHG MDELYKVD |
| | >Figure 7; 8b | |
| | >Protein Sequence | |
| 39 | >inducible spy-tag (BLISS) | AUGGAGUUUCUGGCAACCACACUGGAACGGAUCGAGAAAAAUUUCGUGAUUACUGA UCCGAGACUGCCUGACAACCCAAUCAUUUUUGCGAGCGAUUCCUUCCUGCAGCUGAC AGAAUAUUCUCGGGAAGAGAUCCUGGGGCGCAAUUGCCGUUUUCUGCAGGGACCCG AGACAGACCGUGCCACUGUUCGGAAAAUCAGAGAUGCUAUUGACAACCAGACUGAAG UGACCGUUCAGCUGAUCAAUUAUACCAAGAGCGGCAAGAAGUUCUGGAACGUGUUC CACCUGCAGCCGAUGCGCGAUUAUAAGGGCGACGUCCAGUACUUCAUUGGCGUGCA GCUGGAUGGCACCGAACGUCUUCAUGGCGCCGCUGAGCGUGAGGCGGUCAUGCUGA UCAAAAAGACAGCCGCCCACAUCGUGAUGGUGGACGCCUACAAGCCGACGAAG |
| | >mRNA transcript | |
| 40 | >inducible spy-tag (BLISS) | MEFLATTLERIEKNFVITDPRLPDNPIIFASDSFLQLTEYSREEILGRNCRFLQGPETDRAT VRKIRDAIDNQTEVTVQLINYTKSGKKFWNVFHLQPMRDYKGDVQYFIGVQLDGTERLH GAAEREAVMLIKKTAAHIVMVDAYKPTK |
| | > Protein Sequence¹ | |
| 41 | >mClover3 | AUGGUGAGCAAGGGCGAGGAGCUGUUCACCGGGGUGGUGCCCAUCCUGGUCGAGCU GGACGGCGACGUAAACGGCCACAAGUUCAGCGUCCGCGGCGAGGGCGAGGGCGAUG CCACCAACGGCAAGCUGACCCUGAAGUUCAUCUGCACCACCGGCAAGCUGCCCGUGC CCUGGCCCACCCUCGUGACCACCUUCGGCUACGGCGUGGCCUGCUUCAGCCGCUACC CCGACCACAUGAAGCAGCACGACUUCUUCAAGUCCGCCAUGCCCGAAGGCUACGUCC AGGAGCGCACCAUCUCUUUCAAGGACGACGGUACCUACAAGACCCGCGCCGAGGUGA AGUUCGAGGGCGACACCCUGGUGAACCGCAUCGAGCUGAAGGGCAUCGACUUCAAG GAGGACGGCAACAUCCUGGGGCACAAGCUGGAGUACAACUUCAACAGCCACAACGUC UAUAUCACGGCCGACAAGCAGAAGAACGGCAUCAAGGCUAACUUCAAGAUCCGCCAC AACGUUGAGGACGGCAGCGUGCAGCUCGCCGACCACUACCAGCAGAACACCCCCAUC GGCGACGGCCCCGUGCUGCUGCCCGACAACCACUACCUGAGCCAUCAGUCCAAGCUG AGCAAAGACCCCAACGAGAAGCGCGAUCACAUGGUCCUGCUGGAGUUCGUGACCGCC GCCGGGAUUACACAUGGCAUGGACGAGCUGUACAAG |
| | >mRNA transcript | |
| 42 | >mClover3 | MVSKGEELFTGVVPILVELDGDVNGHKFSVRGEGEGDATNGKLTLKFICTTGKLPVPWPT LVTTFGYGVACFSRYPDHMKQHDFFKSAMPEGYVQERTISFKDDGTYKTRAEVKFEGDT LVNRIELKGIDFKEDGNILGHKLEYNFNSHNVYITADKQKNGIKANFKIRHNVEDGSVQLA DHYQQNTPIGDGPVLLPDNHYLSHQSKLSKDPNEKRDHMVLLEFVTAAGITHGMDELYK |
| | >Protein Sequence | |
| 43 | >mCherry | AUGGUGAGCAAGGGCGAGGAGGAUAACAUGGCCAUCAUCAAGGAGUUCAUGCGCUU CAAGGUGCACAUGGAGGGCUCCGUGAACGGCCACGAGUUCGAGAUCGAGGGCGAGG GCGAGGGCCGCCCCUACGAGGGCACCCAGACCGCCAAGCUGAAGGUGACCAAGGGU GGCCCCCUGCCCUUCGCCUGGGACAUCCUGUCCCCUCAGUUCAUGUACGGCUCCAA GGCCUACGUGAAGCACCCCGCCGACAUCCCCGACUACUUGAAGCUGUCCUUCCCCGA GGGCUUCAAGUGGGAGCGCGUGAUGAACUUCGAGGACGGCGGCGUGGUGACCGUG ACCCAGGACUCCUCCCUGCAGGACGGCGAGUUCAUCUACAAGGUGAAGCUGCGCGG CACCAACUUCCCCUCCGACGGCCCCGUAAUGCAGAAGAAGACCAUGGGCUGGGAGGC CUCCUCCGAGCGGAUGUACCCCGAGGACGGCGCCCUGAAGGGCGAGAUCAAGCAGA GGCUGAAGCUGAAGGACGGCGGCCACUACGACGCUGAGGUCAAGACCACCUACAAG GCCAAGAAGCCCGUGCAGCUGCCCGGCGCCUACAACGUCAACAUCAAGUUGGACAUC ACCUCCCACAACGAGGACUACACCAUCGUGGAACAGUACGAACGCGCCGAGGGCCGC CACUCCACCGGCGGCAUGGACGAGCUGUACAAG |
| | >mRNA transcript | |
| 44 | >mCherry | MVSKGEEDNMAIIKEFMRFKVHMEGSVNGHEFEIEGEGEGRPYEGTQTAKLKVTKGGPL PFAWDILSPQFMYGSKAYVKHPADIPDYLKLSFPEGFKWERVMNFEDGGVVTVTQDSSL QDGEFIYKVKLRGTNFPSDGPVMQKKTMGWEASSERMYPEDGALKGEIKQRLKLKDGG HYDAEVKTTYKAKKPVQLPGAYNVNIKLDITSHNEDYTIVEQYERAEGRHSTGGMDELYK |
| | >Protein Sequence | |
| 45 | >Mouse N-Securin(aa1-101) Degron | AUGGCUACUCUUAUCUUUGUUGAUAAGGAUAAUGAAGAACCCGGCCGCCGUUUGGC AUCUAAGGAUGGGUUGAAGCUGGGCACUGGUGUCAAGGCCUUAGAUGGGAAAUUGC AGGUUUCAACGCCUCGAGUCGGCAAAGUGUUCAAUGCUCCAGCCGUGCCUAAAGCC AGCAGAAAGGCUUUGGGGACAGUCAACAGAGUUGCCGAAAAGCCUAUGAAGACUGG CAAACCCCUCCAACCAAAACAGCCGACCUUGACUGGGAAAAAGAUCACCGAGAAGUC UACUAAGACACAAAGUUCUGUU |
| | >mRNA Sequence | |
| 46 | >Mouse N-Securin(aa1-101) Degron | MATLIFVDKDNEEPGRRLASKDGLKLGTGVKALDGKLQVSTPRVGKVFNAPAVPKASRKA LGTVNRVAEKPMKTGKPLQPKQPTLTGKKITEKSTKTQSSV |
| | >Protein Sequence | |
| 47 | >Mouse N-CyclinB1(aa1-79) Degron | AUGGCGCUCAGGGUCACUAGGAACACGAAAAUUAACGCAGAAAAUAAGGCCAAGGUC AGUAUGGCAGGCGCCAAGCGUGUGCCUGUGACAGUUACUGCUGCUUCCAAGCCCGG GCUGAGACCGAGAACUGCUCUUGGAGACAUUGGUAAUAAAGUCAGCGAAGAGCUAC AGGCAAGAGUGCCUCUGAAAAGGGAAGCAAAAACGCUAGGUACUGGAAAAGGUACU GUUAAAGCCCUA |
| | >mRNA Sequence | |
| 48 | >Mouse N-CyclinB1(aa1-79) Degron | MALRVTRNTKINAENKAKVSMAGAKRVPVTVTAASKPGLRPRTALGDIGNKVSEELQARV PLKREAKTLGTGKGTVKAL |
| | >Protein Sequence | |
| 49 | > NS3/4A Cleavage Motif | GACACCGAGGACGUGGUGUGCUGCAGCAUGAGCUACACCUGGACCGGCAAGA |
| | >mRNA Sequence | |
| 50 | > NS3/4A Cleavage Motif | DTEDWCCSMSYTWTGK |
| | >Protein Sequence | |

| | | |
|---|---|---|
| ¹ The underlined sequence is the SpyTag sequence. The N-terminal part of the sequence is iLID domain, based on AsLOV2 based on Guntas et al. (2014) PNAS.⁶² | | |

| | | **FULL LENGTH SEQUENCES** Bold sequences are the coding sequences |
|---|---|---|
| 51 | >TALhesin[*mm MajSat*]-A | GCGAAUUAAUUCGAGCUCGGUACCCAGCUUGCUUGUUCUUUUUGCAGAAGCUCAGAAUAAACGC UCAACUUUGGCAGAUCAAUUCCCCGGUGCUAGCAUGGGUUCCGCCCACAUCGUGAUGGUG GACGCCUACAAGCCGACGAAGACCGGUACAAGUUUGUACAAAAAAGCAGGCUCCGCG GCCGCCCCCUUCACCAUGCAGGUGGAUCUACGCACGCUCGGCUACAGUCAGCAGCAG CAAGAGAAGAUCAAACCGAAGGUGCGUUCGACAGUGGCGCAGCACCACGAGGCACUG GUGGGCCAUGGGUUUACACACGCGCACAUCGUUGCGCUCAGCCAACACCCGGCAGCG UUAGGGACCGUCGCUGUCACGUAUCAGCACAUAAUCACGGCGUUGCCAGAGGCGACA |
| | > Figure 2A | |
| | >SpyTag001-TAL[mmMajSat] -mClover-SpyTaq001 | |
| | >mRNA transcript full-length includes 5' and 3' UTRs. | CACGAAGACAUCGUUGGCGUCGGCAAACAGUGGUCCGGCGCACGCGCCCUGGAGGCC UUGCUCACGGAUGCGGGGGAGUUGAGAGGUCCGCCGUUACAGUUGGACACAGGCCA ACUUGUGAAGAUUGCAAAACGUGGCGGCGUGACCGCAAUGGAGGCAGUGCAUGCAU CGCGCAAUGCACUGACGGGUGCCCCCCUGAACCUGACCCCGGACCAAGUGGUGGCUA UCGCCAGCAACGGUGGCGGCAAGCAAGCGCUCGAAACGGUGCAGCGGCUGUUGCCGG UGCUGUGCCAGGACCAUGGCCUGACCCCGGACCAAGUGGUGGCUAUCGCCAGCAACA AGGGCGGCAAGCAAGCGCUCGAAACGGUGCAGCGGCUGUUGCCGGUGCUGUGCCAG GACCAUGGCCUGACUCCGGACCAAGUGGUGGCUAUCGCCAGCCACGAUGGCGGCAAG CAAGCGCUCGAAACGGUGCAGCGGCUGUUGCCGGUGCUGUGCCAGGACCAUGGCCU GACUCCGGACCAAGUGGUGGCUAUCGCCAGCCACGAUGGCGGCAAGCAAGCGCUCGA AACGGUGCAGCGGCUGUUGCCGGUGCUGUGCCAGGACCAUGGCCUGACCCCGGACCA AGUGGUGGCUAUCGCCAGCAACAUUGGCGGCAAGCAAGCGCUCGAAACGGUGCAGCG GCUGUUGCCGGUGCUGUGCCAGGACCAUGGCCUGACCCCGGACCAAGUGGUGGCUA UCGCCAGCAACGGUGGCGGCAAGCAAGCGCUCGAAACGGUGCAGCGGCUGUUGCCGG UGCUGUGCCAGGACCAUGGCCUGACCCCGGACCAAGUGGUGGCUAUCGCCAGCAACA UUGGCGGCAAGCAAGCGCUCGAAACGGUGCAGCGGCUGUUGCCGGUGCUGUGCCAG GACCAUGGCCUGACCCCGGACCAAGUGGUGGCUAUCGCCAGCAACGGUGGCGGCAAG CAAGCGCUCGAAACGGUGCAGCGGCUGUUGCCGGUGCUGUGCCAGGACCAUGGCCU GACCCCGGACCAAGUGGUGGCUAUCGCCAGCAACGGUGGCGGCAAGCAAGCGCUCGA AACGGUGCAGCGGCUGUUGCCGGUGCUGUGCCAGGACCAUGGCCUGACUCCGGACCA AGUGGUGGCUAUCGCCAGCCACGAUGGCGGCAAGCAAGCGCUCGAAACGGUGCAGCG GCUGUUGCCGGUGCUGUGCCAGGACCAUGGCCUGACCCCGGACCAAGUGGUGGCUA UCGCCAGCCACGAUGGCGGCAAGCAAGCGCUCGAAACGGUGCAGCGGCUGUUGCCGG UGCUGUGCCAGGACCAUGGCCUGACCCCGGACCAAGUGGUGGCUAUCGCCAGCAACA UUGGCGGCAAGCAAGCGCUCGAAACGGUGCAGCGGCUGUUGCCGGUGCUGUGCCAG GACCAUGGCCUGACUCCGGACCAAGUGGUGGCUAUCGCCAGCCACGAUGGCGGCAAG CAAGCGCUCGAAACGGUGCAGCGGCUGUUGCCGGUGCUGUGCCAGGACCAUGGCCU GACCCCGGACCAAGUGGUGGCUAUCGCCAGCAACAAGGGCGGCAAGCAAGCGCUCGA AACGGUGCAGCGGCUGUUGCCGGUGCUGUGCCAGGACCAUGGCCUGACCCCGGACCA AGUGGUGGCUAUCGCCAGCAACGGUGGCGGCAAGCAAGCGCUCGAAAGCAUUGUGG CCCAGCUGAGCCGGCCUGAUCCGGCGUUGGCCGCGUUGACCAACGACCACCUCGUCG CCUUGGCCUGCCUCGGCGGACGUCCUGCCAUGGAUGCAGUGAAAAAGGGAUUGCCGC ACGCGCCGGAAUUGAUCAGAAGAGUCAAUCGCCGUAUUGGCGAACGCACGUCCCAUC GCGUUGCGGGAUCCACUGGAGGCGGAGGUGGCUCCGGCACCGCCUCCUCCAAGGGU GGGCGCGCCGACCCAGCUUUCUUGUACAAAGUGGUCUCGAGAAUGGUGAGCAAGGG CGAGGAGGAUAACAUGGCCAUCAUCAAGGAGUUCAUGCGCUUCAAGGUGCACAUGGA GGGCUCCGUGAACGGCCACGAGUUCGAGAUCGAGGGCGAGGGCGAGGGCCGCCCCUA CGAGGGCACCCAGACCGCCAAGCUGAAGGUGACCAAGGGUGGCCCCCUGCCCUUCGC CUGGGACAUCCUGUCCCCUCAGUUCAUGUACGGCUCCAAGGCCUACGUGAAGCACCC CGCCGACAUCCCCGACUACUUGAAGCUGUCCUUCCCCGAGGGCUUCAAGUGGGAGCG CGUGAUGAACUUCGAGGACGGCGGCGUGGUGACCGUGACCCAGGACUCCUCCCUGCA GGACGGCGAGUUCAUCUACAAGGUGAAGCUGCGCGGCACCAACUUCCCCUCCGACGG CCCCGUAAUGCAGAAGAAGACCAUGGGCUGGGAGGCCUCCUCCGAGCGGAUGUACCC CGAGGACGGCGCCCUGAAGGGCGAGAUCAAGCAGAGGCUGAAGCUGAAGGACGGCGG CCACUACGACGCUGAGGUCAAGACCACCUACAAGGCCAAGAAGCCCGUGCAGCUGCCC GGCGCCUACAACGUCAACAUCAAGUUGGACAUCACCUCCCACAACGAGGACUACACCA UCGUGGAACAGUACGAACGCGCCGAGGGCCGCCACUCCACCGGCGGCAUGGACGAGC UGUACAAGAAAAGCUUAGGUUCCGCCCAUAUAGUUAUGGUGGACGCCUAUAAACCCA CUAAGAGUCGACGGCCCGGGAUCCUGUACAAGUAAAGCGGCCGCUCUAGAUAAAUGAUAG CUUGAUCUGGUUACCACUAAACCAGCCUCAAGAACACCCGAAUGGAGUCUCUAAGCUACAUAAUA CCAACUUACACUUUACAAAAUGUUGUCCCCCAAAAUGUAGCCAUUCGUAUCUGCUCCUAAUAAAA AGAAAGUUUCUUCACAUUCUAAAAAAAAAAAAAAAAAAAAAAAAAAAAAAAAAAAACCCCCCCCCCC CCCCCUGCAGGCGCGCCGCCGGCGAUUUAAAUUAAU |
| 52 | >TALhesin[*mm MajSat*]-A | MGSAHIVMVDAYKPTKTGTSLYKKAGSAAAPFTMQVDLRTLGYSQQQQEKIKPKVRSTVAQHHEALV GHGFTHAHIVALSQHPAALGTVAVTYQHIITALPEATHEDIVGVGKQWSGARALEALLTDAGELRGPP LQLDTGQLVKIAKRGGVTAMEAVHASRNALTGAPLNLTPDQVVAIASNGGGKQALETVQRLLPVLCQ DHGLTPDQVVAIASNKGGKQALETVQRLLPVLCQDHGLTPDQVVAIASHDGGKQALETVQRLLPVLC QDHGLTPDQVVAIASHDGGKQALETVQRLLPVLCQDHGLTPDQVVAIASNIGGKQALETVQRLLPVL CQDHGLTPDQVVAIASNGGGKQALETVQRLLPVLCQDHGLTPDQVVAIASNIGGKQALETVQRLLPV LCQDHGLTPDQVVAIASNGGGKQALETVQRLLPVLCQDHGLTPDQVVAIASNGGGKQALETVQRLLP VLCQDHGLTPDQVVAIASHDGGKQALETVQRLLPVLCQDHGLTPDQVVAIASHDGGKQALETVQRLL PVLCQDHGLTPDQVVAIASNIGGKQALETVQRLLPVLCQDHGLTPDQVVAIASHDGGKQALETVQRL LPVLCQDHGLTPDQVVAIASNKGGKQALETVQRLLPVLCQDHGLTPDQVVAIASNGGGKQALESIVA |
| | > Figure 2A | |
| | >SpyTag001-TAL[mmMajSat] -mClover-SpyTag001 | |
| | >Full Length Protein Sequence | |
| | | QLSRPDPALAALTNDHLVALACLGGRPAMDAVKKGLPHAPELIRRVNRRIGERTSHRVAGSTGGGGG SGTASSKGGRADPAFLYKVVSRMVSKGEEDNMAIIKEFMRFKVHMEGSVNGHEFEIEGEGEGRPYEG TQTAKLKVTKGGPLPFAWDILSPQFMYGSKAYVKHPADIPDYLKLSFPEGFKWERVMNFEDGGVVTV TQDSSLQDGEFIYKVKLRGTNFPSDGPVMQKKTMGWEASSERMYPEDGALKGEIKQRLKLKDGGHY DAEVKTTYKAKKPVQLPGAYNVNIKLDITSHNEDYTIVEQYERAEGRHSTGGMDELYKKSLGSAHIVM VDAYKPTKSRRPGILYK- |
| 53 | >TALhesin[*mm MajSat*]*-*B | GCGAAUUAAUUCGAGCUCGGUACCCAGCUUGCUUGUUCUUUUUGCAGAAGCUCAGAAUAAACGC UCAACUUUGGCAGAUCAAUUCCCCGGUGCUAGCAUGGAUAGUGCUACCCAUAUUAAAUUC UCAAAACGUGAUGAGGACGGCAAAGAGUUAGCUGGUGCAACUAUGGAGUUGCGUGA UUCAUCUGGUAAAACUAUUAGUACAUGGAUUUCAGAUGGACAAGUGAAAGAUUUCU ACCUGUAUCCAGGAAAAUAUACAUUUGUCGAAACCGCAGCACCAGACGGUUAUGAGG UAGCAACUGCUAUUACCUUUACAGUUAAUGAGCAAGGUCAGGUUACUGUAAAUGGCA AAGCAACUAAAGGUGACGCUCAUAUUGGAUCCACCGGUACAAGUUUGUACAAAAAAG CAGGCUCCGCGGCCGCCCCCUUCACCAUGCAGGUGGAUCUACGCACGCUCGGCUACA GUCAGCAGCAGCAAGAGAAGAUCAAACCGAAGGUGCGUUCGACAGUGGCGCAGCACC ACGAGGCACUGGUGGGCCAUGGGUUUACACACGCGCACAUCGUUGCGCUCAGCCAAC ACCCGGCAGCGUUAGGGACCGUCGCUGUCACGUAUCAGCACAUAAUCACGGCGUUGC CAGAGGCGACACACGAAGACAUCGUUGGCGUCGGCAAACAGUGGUCCGGCGCACGCG CCCUGGAGGCCUUGCUCACGGAUGCGGGGGAGUUGAGAGGUCCGCCGUUACAGUUG GACACAGGCCAACUUGUGAAGAUUGCAAAACGUGGCGGCGUGACCGCAAUGGAGGCA GUGCAUGCAUCGCGCAAUGCACUGACGGGUGCCCCCCUGAACCUGACCCCGGACCAA GUGGUGGCUAUCGCCAGCAACGGUGGCGGCAAGCAAGCGCUCGAAACGGUGCAGCG GCUGUUGCCGGUGCUGUGCCAGGACCAUGGCCUGACCCCGGACCAAGUGGUGGCUA UCGCCAGCAACAAGGGCGGCAAGCAAGCGCUCGAAACGGUGCAGCGGCUGUUGCCGG UGCUGUGCCAGGACCAUGGCCUGACUCCGGACCAAGUGGUGGCUAUCGCCAGCCACG AUGGCGGCAAGCAAGCGCUCGAAACGGUGCAGCGGCUGUUGCCGGUGCUGUGCCAG GACCAUGGCCUGACUCCGGACCAAGUGGUGGCUAUCGCCAGCCACGAUGGCGGCAAG CAAGCGCUCGAAACGGUGCAGCGGCUGUUGCCGGUGCUGUGCCAGGACCAUGGCCU GACCCCGGACCAAGUGGUGGCUAUCGCCAGCAACAUUGGCGGCAAGCAAGCGCUCGA AACGGUGCAGCGGCUGUUGCCGGUGCUGUGCCAGGACCAUGGCCUGACCCCGGACCA AGUGGUGGCUAUCGCCAGCAACGGUGGCGGCAAGCAAGCGCUCGAAACGGUGCAGCG GCUGUUGCCGGUGCUGUGCCAGGACCAUGGCCUGACCCCGGACCAAGUGGUGGCUA UCGCCAGCAACAUUGGCGGCAAGCAAGCGCUCGAAACGGUGCAGCGGCUGUUGCCGG UGCUGUGCCAGGACCAUGGCCUGACCCCGGACCAAGUGGUGGCUAUCGCCAGCAACG GUGGCGGCAAGCAAGCGCUCGAAACGGUGCAGCGGCUGUUGCCGGUGCUGUGCCAG GACCAUGGCCUGACCCCGGACCAAGUGGUGGCUAUCGCCAGCAACGGUGGCGGCAAG CAAGCGCUCGAAACGGUGCAGCGGCUGUUGCCGGUGCUGUGCCAGGACCAUGGCCU GACUCCGGACCAAGUGGUGGCUAUCGCCAGCCACGAUGGCGGCAAGCAAGCGCUCGA AACGGUGCAGCGGCUGUUGCCGGUGCUGUGCCAGGACCAUGGCCUGACCCCGGACCA AGUGGUGGCUAUCGCCAGCCACGAUGGCGGCAAGCAAGCGCUCGAAACGGUGCAGCG GCUGUUGCCGGUGCUGUGCCAGGACCAUGGCCUGACCCCGGACCAAGUGGUGGCUA UCGCCAGCAACAUUGGCGGCAAGCAAGCGCUCGAAACGGUGCAGCGGCUGUUGCCGG UGCUGUGCCAGGACCAUGGCCUGACUCCGGACCAAGUGGUGGCUAUCGCCAGCCACG AUGGCGGCAAGCAAGCGCUCGAAACGGUGCAGCGGCUGUUGCCGGUGCUGUGCCAG GACCAUGGCCUGACCCCGGACCAAGUGGUGGCUAUCGCCAGCAACAAGGGCGGCAAG CAAGCGCUCGAAACGGUGCAGCGGCUGUUGCCGGUGCUGUGCCAGGACCAUGGCCU GACCCCGGACCAAGUGGUGGCUAUCGCCAGCAACGGUGGCGGCAAGCAAGCGCUCGA AAGCAUUGUGGCCCAGCUGAGCCGGCCUGAUCCGGCGUUGGCCGCGUUGACCAACGA CCACCUCGUCGCCUUGGCCUGCCUCGGCGGACGUCCUGCCAUGGAUGCAGUGAAAAA GGGAUUGCCGCACGCGCCGGAAUUGAUCAGAAGAGUCAAUCGCCGUAUUGGCGAACG CACGUCCCAUCGCGUUGCGGGAUCCACUGGAGGCGGAGGUGGCUCCGGCACCGCCUC CUCCAAGGGUGGGCGCGCCGACCCAGCUUUCUUGUACAAAGUGGUCUCGAGCUCAAG CUUCGAAUUCAUGGUGAGCAAGGGCGAGGAGCUGUUCACCGGGGUGGUGCCCAUCC UGGUCGAGCUGGACGGCGACGUAAACGACCACAAGUUCAGCGUCCGCGGCGAGGGCG AGGGCGAUGCCACCAACGGCAAGCUGACCCUGAAGUUCAUCUGCACCACCGGCAAGC UGCCCGUGCCCUGGCCCACCCUCGUGACCACCUUCGGCUACGGCGUGGCCUGCUUCA GCCGCUACCCCGACCACAUGAAGCAGCACGACUUCUUCAAGUCCGCCAUGCCCGAAGG CUACGUCCAGGAGCGCACCAUCUCUUUCAAGGACGACGGUACCUACAAGACCCGCGC CGAGGUGAAGUUCGAGGGCGACACCCUGGUGAACCGCAUCGAGCUGAAGGGCAUCGA CUUCAAGGAGGACGGCAACAUCCUGGGGCACAAGCUGGAGUACAACUUCAACAGCCA CAACGUCUAUAUCACGGCCGACAAGCAGAAGAACGGCAUCAAGGCUAACUUCAAGAU CCGCCACAACGUUGAGGACGGCAGCGUGCAGCUCGCCGACCACUACCAGCAGAACACC CCCAUCGGCGACGGCCCCGUGCUGCUGCCCGACAACCACUACCUGAGCCAUCAGUCCA |
| | > Figure 2A | |
| | > SpyCatcher001-TAL[mmMajSat] -mClover-SpyCatcher001 | |
| | >mRNA transcript full-length includes 5' and 3' UTRs. | |
| | | AGCUGAGCAAAGACCCCAACGAGAAGCGCGAUCACAUGGUCCUGCUGGAGUUCGUGA CCGCCGCCGGGAUUACACAUGGCAUGGACGAGCUGUACAAGGUCGACAUGGAUAGUG CUACCCAUAUUAAAUUCUCAAAACGUGAUGAGGACGGCAAAGAGUUAGCUGGUGCAA CUAUGGAGUUGCGUGAUUCAUCUGGUAAAACUAUUAGUACAUGGAUUUCAGAUGGA CAAGUGAAAGAUUUCUACCUGUAUCCAGGAAAAUAUACAUUUGUCGAAACCGCAGCA CCAGACGGUUAUGAGGUAGCAACUGCUAUUACCUUUACAGUUAAUGAGCAAGGUCAG GUUACUGUAAAUGGCAAAGCAACUAAAGGUGACGCUCAUAUUGGAUCCUAAGCGGCC GCUCUAGAUAAAUGAUAGCUUGAUCUGGUUACCACUAAACCAGCCUCAAGAACACCCGAAUGGA GUCUCUAAGCUACAUAAUACCAACUUACACUUUACAAAAUGUUGUCCCCCAAAAUGUAGCCAUUC GUAUCUGCUCCUAAUAAAAAGAAAGUUUCUUCACAUUCUAAAAAAAAAAAAAAAAAAAAAAAAA AAAAAAAACCCCCCCCCCCCCCCCUGCAGGCGCGCCGCCGGCGAUUUAAAUUAAU |
| 54 | >TALhesin[*mm MajSat*]*-B* | MDSATHIKFSKRDEDGKELAGATMELRDSSGKTISTWISDGQVKDFYLYPGKYTFVETAAPDGYEVA TAITFTVNEQGQVTVNGKATKGDAHIGSTGTSLYKKAGSAAAPFTMQVDLRTLGYSQQQQEKIKPKV RSTVAQHHEALVGHGFTHAHIVALSQHPAALGTVAVTYQHIITALPEATHEDIVGVGKQWSGARALE ALLTDAGELRGPPLQLDTGQLVKIAKRGGVTAMEAVHASRNALTGAPLNLTPDQVVAIASNGGGKQA LETVQRLLPVLCQDHGLTPDQVVAIASNKGGKQALETVQRLLPVLCQDHGLTPDQVVAIASHDGGKQ ALETVQRLLPVLCQDHGLTPDQVVAIASHDGGKQALETVQRLLPVLCQDHGLTPDQVVAIASNIGGK QALETVQRLLPVLCQDHGLTPDQVVAIASNGGGKQALETVQRLLPVLCQDHGLTPDQVVAIASNIGG KQALETVQRLLPVLCQDHGLTPDQVVAIASNGGGKQALETVQRLLPVLCQDHGLTPDQVVAIASNGG GKQALETVQRLLPVLCQDHGLTPDQVVAIASHDGGKQALETVQRLLPVLCQDHGLTPDQVVAIASHD GGKQALETVQRLLPVLCQDHGLTPDQVVAIASNIGGKQALETVQRLLPVLCQDHGLTPDQVVAIASH DGGKQALETVQRLLPVLCQDHGLTPDQVVAIASNKGGKQALETVQRLLPVLCQDHGLTPDQVVAIAS NGGGKQALESIVAQLSRPDPALAALTNDHLVALACLGGRPAMDAVKKGLPHAPELIRRVNRRIGERTS HRVAGSTGGGGGSGTASSKGGRADPAFLYKVVSSSSFEFMVSKGEELFTGVVPILVELDGDVNDHKFS VRGEGEGDATNGKLTLKFICTTGKLPVPWPTLVTTFGYGVACFSRYPDHMKQHDFFKSAMPEGYVQE RTISFKDDGTYKTRAEVKFEGDTLVNRIELKGIDFKEDGNILGHKLEYNFNSHNVYITADKQKNGIKAN FKIRHNVEDGSVQLADHYQQNTPIGDGPVLLPDNHYLSHQSKLSKDPNEKRDHMVLLEFVTAAGITH GMDELYKVDMDSATHIKFSKRDEDGKELAGATMELRDSSGKTISTWISDGQVKDFYLYPGKYTFVET AAPDGYEVATAITFTVNEQGQVTVNGKATKGDAHIGS- |
| | > Figure 2A | |
| | > SpyCatcher001-TAL[mmMajSat] -mClover-SpyCatcher001 | |
| | >Full Length Protein Sequence | |
| 55 | >TALhesin[*mm MajSat*](mRec8) -A | GCGAAUUAAUUCGAGCUCGGUACCCAGCUUGCUUGUUCUUUUUGCAGAAGCUCAGAAUAAACGC UCAACUUUGGCAGAUCAAUUCCCCGGUGCUAGCAUGGGUUCCGCCCACAUCGUGAUGGUG GACGCCUACAAGCCGACGAAGACCGGUACAAGUUUGUACAAAAAAGCAGGCUCCGCG GCCGCCCCCUUCACCAUGCAGGUGGAUCUACGCACGCUCGGCUACAGUCAGCAGCAG CAAGAGAAGAUCAAACCGAAGGUGCGUUCGACAGUGGCGCAGCACCACGAGGCACUG GUGGGCCAUGGGUUUACACACGCGCACAUCGUUGCGCUCAGCCAACACCCGGCAGCG UUAGGGACCGUCGCUGUCACGUAUCAGCACAUAAUCACGGCGUUGCCAGAGGCGACA CACGAAGACAUCGUUGGCGUCGGCAAACAGUGGUCCGGCGCACGCGCCCUGGAGGCC UUGCUCACGGAUGCGGGGGAGUUGAGAGGUCCGCCGUUACAGUUGGACACAGGCCA ACUUGUGAAGAUUGCAAAACGUGGCGGCGUGACCGCAAUGGAGGCAGUGCAUGCAU CGCGCAAUGCACUGACGGGUGCCCCCCUGAACCUGACCCCGGACCAAGUGGUGGCUA UCGCCAGCAACGGUGGCGGCAAGCAAGCGCUCGAAACGGUGCAGCGGCUGUUGCCGG UGCUGUGCCAGGACCAUGGCCUGACCCCGGACCAAGUGGUGGCUAUCGCCAGCAACA AGGGCGGCAAGCAAGCGCUCGAAACGGUGCAGCGGCUGUUGCCGGUGCUGUGCCAG GACCAUGGCCUGACUCCGGACCAAGUGGUGGCUAUCGCCAGCCACGAUGGCGGCAAG CAAGCGCUCGAAACGGUGCAGCGGCUGUUGCCGGUGCUGUGCCAGGACCAUGGCCU GACUCCGGACCAAGUGGUGGCUAUCGCCAGCCACGAUGGCGGCAAGCAAGCGCUCGA AACGGUGCAGCGGCUGUUGCCGGUGCUGUGCCAGGACCAUGGCCUGACCCCGGACCA AGUGGUGGCUAUCGCCAGCAACAUUGGCGGCAAGCAAGCGCUCGAAACGGUGCAGCG GCUGUUGCCGGUGCUGUGCCAGGACCAUGGCCUGACCCCGGACCAAGUGGUGGCUA UCGCCAGCAACGGUGGCGGCAAGCAAGCGCUCGAAACGGUGCAGCGGCUGUUGCCGG UGCUGUGCCAGGACCAUGGCCUGACCCCGGACCAAGUGGUGGCUAUCGCCAGCAACA UUGGCGGCAAGCAAGCGCUCGAAACGGUGCAGCGGCUGUUGCCGGUGCUGUGCCAG GACCAUGGCCUGACCCCGGACCAAGUGGUGGCUAUCGCCAGCAACGGUGGCGGCAAG CAAGCGCUCGAAACGGUGCAGCGGCUGUUGCCGGUGCUGUGCCAGGACCAUGGCCU GACCCCGGACCAAGUGGUGGCUAUCGCCAGCAACGGUGGCGGCAAGCAAGCGCUCGA AACGGUGCAGCGGCUGUUGCCGGUGCUGUGCCAGGACCAUGGCCUGACUCCGGACCA AGUGGUGGCUAUCGCCAGCCACGAUGGCGGCAAGCAAGCGCUCGAAACGGUGCAGCG GCUGUUGCCGGUGCUGUGCCAGGACCAUGGCCUGACCCCGGACCAAGUGGUGGCUA UCGCCAGCCACGAUGGCGGCAAGCAAGCGCUCGAAACGGUGCAGCGGCUGUUGCCGG UGCUGUGCCAGGACCAUGGCCUGACCCCGGACCAAGUGGUGGCUAUCGCCAGCAACA UUGGCGGCAAGCAAGCGCUCGAAACGGUGCAGCGGCUGUUGCCGGUGCUGUGCCAG GACCAUGGCCUGACUCCGGACCAAGUGGUGGCUAUCGCCAGCCACGAUGGCGGCAAG |
| | > Figure 8A | |
| | >SpyTag001-TALMajSat-mCherry-mRec8-SpyTag001 | |
| | >mRNA transcript full-length includes 5' and 3' UTRs. | |
| | | CAAGCGCUCGAAACGGUGCAGCGGCUGUUGCCGGUGCUGUGCCAGGACCAUGGCCU GACCCCGGACCAAGUGGUGGCUAUCGCCAGCAACAAGGGCGGCAAGCAAGCGCUCGA AACGGUGCAGCGGCUGUUGCCGGUGCUGUGCCAGGACCAUGGCCUGACCCCGGACCA AGUGGUGGCUAUCGCCAGCAACGGUGGCGGCAAGCAAGCGCUCGAAAGCAUUGUGG CCCAGCUGAGCCGGCCUGAUCCGGCGUUGGCCGCGUUGACCAACGACCACCUCGUCG CCUUGGCCUGCCUCGGCGGACGUCCUGCCAUGGAUGCAGUGAAAAAGGGAUUGCCGC ACGCGCCGGAAUUGAUCAGAAGAGUCAAUCGCCGUAUUGGCGAACGCACGUCCCAUC GCGUUGCGGGAUCCACUGGAGGCGGAGGUGGCUCCGGCACCGCCUCCUCCACCGGUA UGGUGAGCAAGGGCGAGGAGGAUAACAUGGCCAUCAUCAAGGAGUUCAUGCGCUUC AAGGUGCACAUGGAGGGCUCCGUGAACGGCCACGAGUUCGAGAUCGAGGGCGAGGG CGAGGGCCGCCCCUACGAGGGCACCCAGACCGCCAAGCUGAAGGUGACCAAGGGUGG CCCCCUGCCCUUCGCCUGGGACAUCCUGUCCCCUCAGUUCAUGUACGGCUCCAAGGC CUACGUGAAGCACCCCGCCGACAUCCCCGACUACUUGAAGCUGUCCUUCCCCGAGGG CUUCAAGUGGGAGCGCGUGAUGAACUUCGAGGACGGCGGCGUGGUGACCGUGACCC AGGACUCCUCCCUGCAGGACGGCGAGUUCAUCUACAAGGUGAAGCUGCGCGGCACCA ACUUCCCCUCCGACGGCCCCGUAAUGCAGAAGAAGACCAUGGGCUGGGAGGCCUCCU CCGAGCGGAUGUACCCCGAGGACGGCGCCCUGAAGGGCGAGAUCAAGCAGAGGCUGA AGCUGAAGGACGGCGGCCACUACGACGCUGAGGUCAAGACCACCUACAAGGCCAAGA AGCCCGUGCAGCUGCCCGGCGCCUACAACGUCAACAUCAAGUUGGACAUCACCUCCCA CAACGAGGACUACACCAUCGUGGAACAGUACGAACGCGCCGAGGGCCGCCACUCCACC GGCGGCAUGGACGAGCUGUACAAGGGUACAAGUUUGUACAAAAAAGCAGGCUCCGCG GCCGCCCCCUUCACCAUGCUCAGACAAAGGCCACAGCUGGAGACUGAGGAGACAGUG GAGGAAGAGAGAGCAGCAGAUGAGGAAGAGAGGAGAAAGACUGAAGCUCUGAGCGA GAUCGAGGUUCUGAGGGAGGCCCAGGAGCCCAGUGGUCCCCUCAUGCUGUCUUCAG AGCUCUCCCUAGAAGCAGCAGAAGAUGAAAAGUCUCGUACUAGCCUCAUCCCCCCAG AAUGGUGGGCCUGGUCUGAGGAGGGGCAGCCCGAGCCCCCUGCACUGCCCAUGCUGC CUGAACUUCCUGAAGUGCCCAUGGAGAUGCCCCCAAGGCCUGAACUCUCUUCAGAGG CUAAGGGUGGGCGCGCCGACCCAGCUUUCUUGUACAAAGUGGUAAGCUUAGGUUCC GCCCAUAUAGUUAUGGUGGACGCCUAUAAACCCACUAAGAGUCGACGGCCCGGGAUC CUGUACAAGUAAAGCGGCCGCUCUAGAUAAAUGAUAGCUUGAUCUGGUUACCACUAAACCAGC CUCAAGAACACCCGAAUGGAGUCUCUAAGCUACAUAAUACCAACUUACACUUUACAAAAUGUUGU CCCCCAAAAUGUAGCCAUUCGUAUCUGCUCCUAAUAAAAAGAAAGUUUCUUCACAUUCUAAAAAA AAAAAAAAAAAAAAAAAAAAAAAAAAAAACCCCCCCCCCCCCCCUGCAGGCGCGCCGCCGGCGAU UUAAAUUAAU |
| 56 | >TALhesin[*mm MajSat*](mRec8) -A | MGSAHIVMVDAYKPTKTGTSLYKKAGSAAAPFTMQVDLRTLGYSQQQQEKIKPKVRSTVAQHHEALV GHGFTHAHIVALSQHPAALGTVAVTYQHIITALPEATHEDIVGVGKQWSGARALEALLTDAGELRGPP LQLDTGQLVKIAKRGGVTAMEAVHASRNALTGAPLNLTPDQVVAIASNGGGKQALETVQRLLPVLCQ DHGLTPDQVVAIASNKGGKQALETVQRLLPVLCQDHGLTPDQVVAIASHDGGKQALETVQRLLPVLC QDHGLTPDQVVAIASHDGGKQALETVQRLLPVLCQDHGLTPDQVVAIASNIGGKQALETVQRLLPVL CQDHGLTPDQVVAIASNGGGKQALETVQRLLPVLCQDHGLTPDQVVAIASNIGGKQALETVQRLLPV LCQDHGLTPDQVVAIASNGGGKQALETVQRLLPVLCQDHGLTPDQVVAIASNGGGKQALETVQRLLP VLCQDHGLTPDQVVAIASHDGGKQALETVQRLLPVLCQDHGLTPDQVVAIASHDGGKQALETVQRLL PVLCQDHGLTPDQVVAIASNIGGKQALETVQRLLPVLCQDHGLTPDQVVAIASHDGGKQALETVQRL LPVLCQDHGLTPDQVVAIASNKGGKQALETVQRLLPVLCQDHGLTPDQVVAIASNGGGKQALESIVA QLSRPDPALAALTNDHLVALACLGGRPAMDAVKKGLPHAPELIRRVNRRIGERTSHRVAGSTGGGGG SGTASSTGMVSKGEEDNMAIIKEFMRFKVHMEGSVNGHEFEIEGEGEGRPYEGTQTAKLKVTKGGPL PFAWDILSPQFMYGSKAYVKHPADIPDYLKLSFPEGFKWERVMNFEDGGVVTVTQDSSLQDGEFIYK VKLRGTNFPSDGPVMQKKTMGWEASSERMYPEDGALKGEIKQRLKLKDGGHYDAEVKTTYKAKKPV QLPGAYNVNIKLDITSHNEDYTIVEQYERAEGRHSTGGMDELYKGTSLYKKAGSAAAPFTMLRQRPQL ETEETVEEEERAADEEERRKTEALSEIEVLREAQEPSGPLMLSSELSLEAAEDEKSRTSLIPPEWWAWSE EGQPEPPALPMLPELPEVPMEMPPRPELSSEAKGGRADPAFLYKVVSLGSAHIVMVDAYKPTKSRRPGI LYK- |
| | > Figure 8A | |
| | >SpyTag001-TALMajSat-mCherry-mRec8-SpyTag001 | |
| | >Full Length Protein Sequence | |
| 57 | >TALhesin[*mm MajSat*](mRec8) -B | GCGAAUUAAUUCGAGCUCGGUACCCAGCUUGCUUGUUCUUUUUGCAGAAGCUCAGAAUAAACGC UCAACUUUGGCAGAUCAAUUCCCCGGUGCUAGCAUGGAUAGUGCUACCCAUAUUAAAUUC UCAAAACGUGAUGAGGACGGCAAAGAGUUAGCUGGUGCAACUAUGGAGUUGCGUGA UUCAUCUGGUAAAACUAUUAGUACAUGGAUUUCAGAUGGACAAGUGAAAGAUUUCU ACCUGUAUCCAGGAAAAUAUACAUUUGUCGAAACCGCAGCACCAGACGGUUAUGAGG UAGCAACUGCUAUUACCUUUACAGUUAAUGAGCAAGGUCAGGUUACUGUAAAUGGCA AAGCAACUAAAGGUGACGCUCAUAUUGGAUCCACCGGUACAAGUUUGUACAAAAAAG CAGGCUCCGCGGCCGCCCCCUUCACCAUGCAGGUGGAUCUACGCACGCUCGGCUACA GUCAGCAGCAGCAAGAGAAGAUCAAACCGAAGGUGCGUUCGACAGUGGCGCAGCACC ACGAGGCACUGGUGGGCCAUGGGUUUACACACGCGCACAUCGUUGCGCUCAGCCAAC ACCCGGCAGCGUUAGGGACCGUCGCUGUCACGUAUCAGCACAUAAUCACGGCGUUGC |
| | > Figure 8A | |
| | > SpyCatcher001-TAL[mmMajSat] -mCherry-mRec8-mClover-SpyCatcher001 | |
| | >mRNA | |
| | transcript full-length includes 5' and 3' UTRs. | CAGAGGCGACACACGAAGACAUCGUUGGCGUCGGCAAACAGUGGUCCGGCGCACGCG CCCUGGAGGCCUUGCUCACGGAUGCGGGGGAGUUGAGAGGUCCGCCGUUACAGUUG GACACAGGCCAACUUGUGAAGAUUGCAAAACGUGGCGGCGUGACCGCAAUGGAGGCA GUGCAUGCAUCGCGCAAUGCACUGACGGGUGCCCCCCUGAACCUGACCCCGGACCAA GUGGUGGCUAUCGCCAGCAACGGUGGCGGCAAGCAAGCGCUCGAAACGGUGCAGCG GCUGUUGCCGGUGCUGUGCCAGGACCAUGGCCUGACCCCGGACCAAGUGGUGGCUA UCGCCAGCAACAAGGGCGGCAAGCAAGCGCUCGAAACGGUGCAGCGGCUGUUGCCGG UGCUGUGCCAGGACCAUGGCCUGACUCCGGACCAAGUGGUGGCUAUCGCCAGCCACG AUGGCGGCAAGCAAGCGCUCGAAACGGUGCAGCGGCUGUUGCCGGUGCUGUGCCAG GACCAUGGCCUGACUCCGGACCAAGUGGUGGCUAUCGCCAGCCACGAUGGCGGCAAG CAAGCGCUCGAAACGGUGCAGCGGCUGUUGCCGGUGCUGUGCCAGGACCAUGGCCU GACCCCGGACCAAGUGGUGGCUAUCGCCAGCAACAUUGGCGGCAAGCAAGCGCUCGA AACGGUGCAGCGGCUGUUGCCGGUGCUGUGCCAGGACCAUGGCCUGACCCCGGACCA AGUGGUGGCUAUCGCCAGCAACGGUGGCGGCAAGCAAGCGCUCGAAACGGUGCAGCG GCUGUUGCCGGUGCUGUGCCAGGACCAUGGCCUGACCCCGGACCAAGUGGUGGCUA UCGCCAGCAACAUUGGCGGCAAGCAAGCGCUCGAAACGGUGCAGCGGCUGUUGCCGG UGCUGUGCCAGGACCAUGGCCUGACCCCGGACCAAGUGGUGGCUAUCGCCAGCAACG GUGGCGGCAAGCAAGCGCUCGAAACGGUGCAGCGGCUGUUGCCGGUGCUGUGCCAG GACCAUGGCCUGACCCCGGACCAAGUGGUGGCUAUCGCCAGCAACGGUGGCGGCAAG CAAGCGCUCGAAACGGUGCAGCGGCUGUUGCCGGUGCUGUGCCAGGACCAUGGCCU GACUCCGGACCAAGUGGUGGCUAUCGCCAGCCACGAUGGCGGCAAGCAAGCGCUCGA AACGGUGCAGCGGCUGUUGCCGGUGCUGUGCCAGGACCAUGGCCUGACCCCGGACCA AGUGGUGGCUAUCGCCAGCCACGAUGGCGGCAAGCAAGCGCUCGAAACGGUGCAGCG GCUGUUGCCGGUGCUGUGCCAGGACCAUGGCCUGACCCCGGACCAAGUGGUGGCUA UCGCCAGCAACAUUGGCGGCAAGCAAGCGCUCGAAACGGUGCAGCGGCUGUUGCCGG UGCUGUGCCAGGACCAUGGCCUGACUCCGGACCAAGUGGUGGCUAUCGCCAGCCACG AUGGCGGCAAGCAAGCGCUCGAAACGGUGCAGCGGCUGUUGCCGGUGCUGUGCCAG GACCAUGGCCUGACCCCGGACCAAGUGGUGGCUAUCGCCAGCAACAAGGGCGGCAAG CAAGCGCUCGAAACGGUGCAGCGGCUGUUGCCGGUGCUGUGCCAGGACCAUGGCCU GACCCCGGACCAAGUGGUGGCUAUCGCCAGCAACGGUGGCGGCAAGCAAGCGCUCGA AAGCAUUGUGGCCCAGCUGAGCCGGCCUGAUCCGGCGUUGGCCGCGUUGACCAACGA CCACCUCGUCGCCUUGGCCUGCCUCGGCGGACGUCCUGCCAUGGAUGCAGUGAAAAA GGGAUUGCCGCACGCGCCGGAAUUGAUCAGAAGAGUCAAUCGCCGUAUUGGCGAACG CACGUCCCAUCGCGUUGCGGGAUCCACUGGAGGCGGAGGUGGCUCCGGCACCGCCUC CUCCACCGGUAUGGUGAGCAAGGGCGAGGAGGAUAACAUGGCCAUCAUCAAGGAGUU CAUGCGCUUCAAGGUGCACAUGGAGGGCUCCGUGAACGGCCACGAGUUCGAGAUCGA GGGCGAGGGCGAGGGCCGCCCCUACGAGGGCACCCAGACCGCCAAGCUGAAGGUGAC CAAGGGUGGCCCCCUGCCCUUCGCCUGGGACAUCCUGUCCCCUCAGUUCAUGUACGG CUCCAAGGCCUACGUGAAGCACCCCGCCGACAUCCCCGACUACUUGAAGCUGUCCUU CCCCGAGGGCUUCAAGUGGGAGCGCGUGAUGAACUUCGAGGACGGCGGCGUGGUGA CCGUGACCCAGGACUCCUCCCUGCAGGACGGCGAGUUCAUCUACAAGGUGAAGCUGC GCGGCACCAACUUCCCCUCCGACGGCCCCGUAAUGCAGAAGAAGACCAUGGGCUGGG AGGCCUCCUCCGAGCGGAUGUACCCCGAGGACGGCGCCCUGAAGGGCGAGAUCAAGC AGAGGCUGAAGCUGAAGGACGGCGGCCACUACGACGCUGAGGUCAAGACCACCUACA AGGCCAAGAAGCCCGUGCAGCUGCCCGGCGCCUACAACGUCAACAUCAAGUUGGACA UCACCUCCCACAACGAGGACUACACCAUCGUGGAACAGUACGAACGCGCCGAGGGCC GCCACUCCACCGGCGGCAUGGACGAGCUGUACAAGGGUACAAGUUUGUACAAAAAAG CAGGCUCCGCGGCCGCCCCCUUCACCAUGCUCAGACAAAGGCCACAGCUGGAGACUG AGGAGACAGUGGAGGAAGAGAGAGCAGCAGAUGAGGAAGAGAGGAGAAAGACUGAA GCUCUGAGCGAGAUCGAGGUUCUGAGGGAGGCCCAGGAGCCCAGUGGUCCCCUCAU GCUGUCUUCAGAGCUCUCCCUAGAAGCAGCAGAAGAUGAAAAGUCUCGUACUAGCCU CAUCCCCCCAGAAUGGUGGGCCUGGUCUGAGGAGGGGCAGCCCGAGCCCCCUGCACU GCCCAUGCUGCCUGAACUUCCUGAAGUGCCCAUGGAGAUGCCCCCAAGGCCUGAACU CUCUUCAGAGGCUAAGGGUGGGCGCGCCGACCCAGCUUUCUUGUACAAAGUGGUAA GCUUCGAAUUCAUGGUGAGCAAGGGCGAGGAGCUGUUCACCGGGGUGGUGCCCAUC CUGGUCGAGCUGGACGGCGACGUAAACGGCCACAAGUUCAGCGUCCGCGGCGAGGGC GAGGGCGAUGCCACCAACGGCAAGCUGACCCUGAAGUUCAUCUGCACCACCGGCAAG CUGCCCGUGCCCUGGCCCACCCUCGUGACCACCUUCGGCUACGGCGUGGCCUGCUUC AGCCGCUACCCCGACCACAUGAAGCAGCACGACUUCUUCAAGUCCGCCAUGCCCGAAG GCUACGUCCAGGAGCGCACCAUCUCUUUCAAGGACGACGGUACCUACAAGACCCGCG CCGAGGUGAAGUUCGAGGGCGACACCCUGGUGAACCGCAUCGAGCUGAAGGGCAUCG ACUUCAAGGAGGACGGCAACAUCCUGGGGCACAAGCUGGAGUACAACUUCAACAGCC ACAACGUCUAUAUCACGGCCGACAAGCAGAAGAACGGCAUCAAGGCUAACUUCAAGA |
| | | UCCGCCACAACGUUGAGGACGGCAGCGUGCAGCUCGCCGACCACUACCAGCAGAACA CCCCCAUCGGCGACGGCCCCGUGCUGCUGCCCGACAACCACUACCUGAGCCAUCAGUC CAAGCUGAGCAAAGACCCCAACGAGAAGCGCGAUCACAUGGUCCUGCUGGAGUUCGU GACCGCCGCCGGGAUUACACAUGGCAUGGACGAGCUGUACAAGGUCGACAUGGAUAG UGCUACCCAUAUUAAAUUCUCAAAACGUGAUGAGGACGGCAAAGAGUUAGCUGGUGC AACUAUGGAGUUGCGUGAUUCAUCUGGUAAAACUAUUAGUACAUGGAUUUCAGAUG GACAAGUGAAAGAUUUCUACCUGUAUCCAGGAAAAUAUACAUUUGUCGAAACCGCAG CACCAGACGGUUAUGAGGUAGCAACUGCUAUUACCUUUACAGUUAAUGAGCAAGGUC AGGUUACUGUAAAUGGCAAAGCAACUAAAGGUGACGCUCAUAUUGGAUCCUAAGCGG CCGCUCUAGAUAAAUGAUAGCUUGAUCUGGUUACCACUAAACCAGCCUCAAGAACACCCGAAUG GAGUCUCUAAGCUACAUAAUACCAACUUACACUUUACAAAAUGUUGUCCCCCAAAAUGUAGCCAU UCGUAUCUGCUCCUAAUAAAAAGAAAGUUUCUUCACAUUCUAAAAAAAAAAAAAAAAAAAAAAA AAAAAAAAAAACCCCCCCCCCCCCCCCCUGCAGGCGCGCCGCCGGCGAUUUAAAUUAAU |
| 58 | >TALhesin[*mm MajSat*](mRec8) -B | MDSATHIKFSKRDEDGKELAGATMELRDSSGKTISTWISDGQVKDFYLYPGKYTFVETAAPDGYEVA TAITFTVNEQGQVTVNGKATKGDAHIGSTGTSLYKKAGSAAAPFTMQVDLRTLGYSQQQQEKIKPKV RSTVAQHHEALVGHGFTHAHIVALSQHPAALGTVAVTYQHIITALPEATHEDIVGVGKQWSGARALE ALLTDAGELRGPPLQLDTGQLVKIAKRGGVTAMEAVHASRNALTGAPLNLTPDQVVAIASNGGGKQA LETVQRLLPVLCQDHGLTPDQVVAIASNKGGKQALETVQRLLPVLCQDHGLTPDQVVAIASHDGGKQ ALETVQRLLPVLCQDHGLTPDQVVAIASHDGGKQALETVQRLLPVLCQDHGLTPDQVVAIASNIGGK QALETVQRLLPVLCQDHGLTPDQVVAIASNGGGKQALETVQRLLPVLCQDHGLTPDQVVAIASNIGG KQALETVQRLLPVLCQDHGLTPDQVVAIASNGGGKQALETVQRLLPVLCQDHGLTPDQVVAIASNGG GKQALETVQRLLPVLCQDHGLTPDQVVAIASHDGGKQALETVQRLLPVLCQDHGLTPDQVVAIASHD GGKQALETVQRLLPVLCQDHGLTPDQVVAIASNIGGKQALETVQRLLPVLCQDHGLTPDQVVAIASH DGGKQALETVQRLLPVLCQDHGLTPDQVVAIASNKGGKQALETVQRLLPVLCQDHGLTPDQVVAIAS NGGGKQALESIVAQLSRPDPALAALTNDHLVALACLGGRPAMDAVKKGLPHAPELIRRVNRRIGERTS HRVAGSTGGGGGSGTASSTGMVSKGEEDNMAIIKEFMRFKVHMEGSVNGHEFEIEGEGEGRPYEGT QTAKLKVTKGGPLPFAWDILSPQFMYGSKAYVKHPADIPDYLKLSFPEGFKWERVMNFEDGGVVTVT QDSSLQDGEFIYKVKLRGTNFPSDGPVMQKKTMGWEASSERMYPEDGALKGEIKQRLKLKDGGHYD AEVKTTYKAKKPVQLPGAYNVNIKLDITSHNEDYTIVEQYERAEGRHSTGGMDELYKGTSLYKKAGSA AAPFTMLRQRPQLETEETVEEERAADEEERRKTEALSEIEVLREAQEPSGPLMLSSELSLEAAEDEKSR TSLIPPEWWAWSEEGQPEPPALPMLPELPEVPMEMPPRPELSSEAKGGRADPAFLYKVVSFEFMVSKG EELFTGVVPILVELDGDVNGHKFSVRGEGEGDATNGKLTLKFICTTGKLPVPWPTLVTTFGYGVACFS RYPDHMKQHDFFKSAMPEGYVQERTISFKDDGTYKTRAEVKFEGDTLVNRIELKGIDFKEDGNILGHK LEYNFNSHNVYITADKQKNGIKANFKIRHNVEDGSVQLADHYQQNTPIGDGPVLLPDNHYLSHQSKL SKDPNEKRDHMVLLEFVTAAGITHGMDELYKVDMDSATHIKFSKRDEDGKELAGATMELRDSSGKTI STWISDGQVKDFYLYPGKYTFVETAAPDGYEVATAITFTVNEQGQVTVNGKATKGDAHIGS- |
| | > Figure 8A | |
| | > SpyCatcher001-TAL[mmMajSat] -mCherry-mRec8-mClover-SpyCatcher001 | |
| | >Full Length Protein Sequence | |
| 59 | >TALhesin[*hsCE NTRO*]-A | ATGGGTTCCGCCCACATCGTGATGGTGGACGCCTACAAGCCGACGAAGACCGGTACAA GTTTGTACAAAAAAGCAGGCTCCGCGGCCGCCCCCTTCACCATGGTAGATTTGAGAACT TTGGGATATTCACAGCAGCAGCAGGAAAAGATCAAGCCCAAAGTGAGGTCGACAGTCG CGCAGCATCACGAAGCGCTGGTGGGTCATGGGTTTACACATGCCCACATCGTAGCCTTG TCGCAGCACCCTGCAGCCCTTGGCACGGTCGCCGTCAAGTACCAGGACATGATTGCGGC GTTGCCGGAAGCCACACATGAGGCGATCGTCGGTGTGGGGAAACAGTGGAGCGGAGCC CGAGCGCTTGAGGCCCTGTTGACGGTCGCGGGAGAGCTGAGAGGGCCTCCCCTTCAGC TGGACACGGGCCAGTTGCTGAAGATCGCGAAGCGGGGAGGAGTCACGGCGGTCGAGG CGGTGCACGCGTGGCGCAATGCGCTCACGGGAGCACCCCTCAACCTGACCCCAGAGCA GGTCGTGGCAATTGCGAGCCATGACGGGGGAAAGCAGGCACTCGAAACCGTCCAGAGG TTGCTGCCTGTGCTGTGCCAAGCGCACGGACTTACGCCAGAGCAGGTCGTGGCAATTGC GAGCCATGACGGGGGAAAGCAGGCACTCGAAACCGTCCAGAGGTTGCTGCCTGTGCTG TGCCAAGCGCACGGACTAACCCCAGAGCAGGTCGTGGCAATTGCGAGCAACATCGGGG GAAAGCAGGCACTCGAAACCGTCCAGAGGTTGCTGCCTGTGCTGTGCCAAGCGCACGG GTTGACCCCAGAGCAGGTCGTGGCAATTGCGAGCAACGGAGGGGGAAAGCAGGCACTC GAAACCGTCCAGAGGTTGCTGCCTGTGCTGTGCCAAGCGCACGGGTTGACCCCAGAGCA GGTCGTGGCAATTGCGAGCAACGGAGGGGGAAAGCAGGCACTCGAAACCGTCCAGAGG TTGCTGCCTGTGCTGTGCCAAGCGCACGGACTGACACCAGAGCAGGTCGTGGCAATTGC GAGCCATGACGGGGGAAAGCAGGCACTCGAAACCGTCCAGAGGTTGCTGCCTGTGCTG TGCCAAGCGCACGGACTTACACCCGAACAAGTCGTGGCAATTGCGAGCCATGACGGGG GAAAGCAGGCACTCGAAACCGTCCAGAGGTTGCTGCCTGTGCTGTGCCAAGCGCACGG ACTAACCCCAGAGCAGGTCGTGGCAATTGCGAGCAACATCGGGGGAAAGCAGGCACTC GAAACCGTCCAGAGGTTGCTGCCTGTGCTGTGCCAAGCGCACGGGTTGACCCCAGAGCA GGTCGTGGCAATTGCGAGCAACGGAGGGGGAAAGCAGGCACTCGAAACCGTCCAGAGG TTGCTGCCTGTGCTGTGCCAAGCGCACGGGTTGACCCCAGAGCAGGTCGTGGCAATTGC GAGCAACGGAGGGGGAAAGCAGGCACTCGAAACCGTCCAGAGGTTGCTGCCTGTGCTG TGCCAAGCGCACGGACTGACACCAGAGCAGGTCGTGGCAATTGCGAGCCATGACGGGG |
| | > Figure 6A | |
| | >SpyTag001-TAL[hsCENTRO] -mCherry-SpyTag001 | |
| | >DNA Coding Sequence (mammalian expression vector not included) | |
| | | GAAAGCAGGCACTCGAAACCGTCCAGAGGTTGCTGCCTGTGCTGTGCCAAGCGCACGG ACTTACACCCGAACAAGTCGTGGCAATTGCGAGCCATGACGGGGGAAAGCAGGCACTC GAAACCGTCCAGAGGTTGCTGCCTGTGCTGTGCCAAGCGCACGGCCTCACCCCAGAGCA GGTCGTGGCAATTGCGAGCAACATCGGGGGAAAGCAGGCACTCGAAACCGTCCAGAGG TTGCTGCCTGTGCTGTGCCAAGCGCACGGACTTACGCCAGAGCAGGTCGTGGCAATTGC GAGCAACGGAGGGGGAAAGCAGGCACTCGAAACCGTCCAGAGGTTGCTGCCTGTGCTG TGCCAAGCGCACGGACTAACCCCAGAGCAGGTCGTGGCAATTGCGAGCAACGGAGGGG GAAAGCAGGCACTCGAAACCGTCCAGAGGTTGCTGCCTGTGCTGTGCCAAGCGCACGG GTTGACCCCAGAGCAGGTCGTGGCAATTGCGAGCCATGACGGGGGAAAGCAGGCACTC GAAACCGTCCAGAGGTTGCTGCCTGTGCTGTGCCAAGCGCACGGCCTGACCCCAGAGCA GGTCGTGGCAATTGCGAGCCATGACGGGGGAAAGCAGGCACTCGAAACCGTCCAGAGG TTGCTGCCTGTGCTGTGCCAAGCGCACGGACTGACACCAGAGCAGGTCGTGGCAATTGC GAGCAACATCGGGGGAAAGCAGGCACTCGAAACCGTCCAGAGGTTGCTGCCTGTGCTG TGCCAAGCGCACGGACTCACGCCTGAGCAGGTAGTGGCTATTGCATCCAACGGAGGGG GCAGACCCGCACTGGAGTCAATCGTGGCCCAGCTTTCGAGGCCGGACCCCGCGCTGGC CGCACTCACTAATGATCATCTTGTAGCGCTGGCCTGCCTCGGCGGACGACCCGCCTTGG ATGCGGTGAAGAAGGGGCTCCCGCACGCGCCTGCATTGATTAAGCGGACCAACAGAAG GATTCCCGAGAGGACATCACATCGAGTGGCAAAGGGTGGGCGCGCCGACCCAGCTTTCT TGTACAAAGTGGTCTCGAGAATGGTGAGCAAGGGCGAGGAGGATAACATGGCCATCAT CAAGGAGTTCATGCGCTTCAAGGTGCACATGGAGGGCTCCGTGAACGGCCACGAGTTC GAGATCGAGGGCGAGGGCGAGGGCCGCCCCTACGAGGGCACCCAGACCGCCAAGCTG AAGGTGACCAAGGGTGGCCCCCTGCCCTTCGCCTGGGACATCCTGTCCCCTCAGTTCAT GTACGGCTCCAAGGCCTACGTGAAGCACCCCGCCGACATCCCCGACTACTTGAAGCTGT CCTTCCCCGAGGGCTTCAAGTGGGAGCGCGTGATGAACTTCGAGGACGGCGGCGTGGT GACCGTGACCCAGGACTCCTCCCTGCAGGACGGCGAGTTCATCTACAAGGTGAAGCTGC GCGGCACCAACTTCCCCTCCGACGGCCCCGTAATGCAGAAGAAGACCATGGGCTGGGA GGCCTCCTCCGAGCGGATGTACCCCGAGGACGGCGCCCTGAAGGGCGAGATCAAGCAG AGGCTGAAGCTGAAGGACGGCGGCCACTACGACGCTGAGGTCAAGACCACCTACAAGG CCAAGAAGCCCGTGCAGCTGCCCGGCGCCTACAACGTCAACATCAAGTTGGACATCACC TCCCACAACGAGGACTACACCATCGTGGAACAGTACGAACGCGCCGAGGGCCGCCACTC CACCGGCGGCATGGACGAGCTGTACAAGAAAAGCTTAGGTTCCGCCCATATAGTTATGG TGGACGCCTATAAACCCACTAAGAGTCGACGGCCCGGGATCCTGTACAAGTAA |
| 60 | >TALhesin[*hsCE NTRO*]-A | MGSAHIVMVDAYKPTKTGTSLYKKAGSAAAPFTMVDLRTLGYSQQQQEKIKPKVRSTVAQHHEALVG HGFTHAHIVALSQHPAALGTVAVKYQDMIAALPEATHEAIVGVGKQWSGARALEALLTVAGELRGPPL QLDTGQLLKIAKRGGVTAVEAVHAWRNALTGAPLNLTPEQVVAIASHDGGKQALETVQRLLPVLCQA HGLTPEQVVAIASHDGGKQALETVQRLLPVLCQAHGLTPEQVVAIASNIGGKQALETVQRLLPVLCQA HGLTPEQVVAIASNGGGKQALETVQRLLPVLCQAHGLTPEQVVAIASNGGGKQALETVQRLLPVLCQA HGLTPEQVVAIASHDGGKQALETVQRLLPVLCQAHGLTPEQVVAIASHDGGKQALETVQRLLPVLCQA HGLTPEQVVAIASNIGGKQALETVQRLLPVLCQAHGLTPEQVVAIASNGGGKQALETVQRLLPVLCQA HGLTPEQVVAIASNGGGKQALETVQRLLPVLCQAHGLTPEQVVAIASHDGGKQALETVQRLLPVLCQA HGLTPEQVVAIASHDGGKQALETVQRLLPVLCQAHGLTPEQVVAIASNIGGKQALETVQRLLPVLCQA HGLTPEQVVAIASNGGGKQALETVQRLLPVLCQAHGLTPEQVVAIASNGGGKQALETVQRLLPVLCQA HGLTPEQVVAIASHDGGKQALETVQRLLPVLCQAHGLTPEQVVAIASHDGGKQALETVQRLLPVLCQA HGLTPEQVVAIASNIGGKQALETVQRLLPVLCQAHGLTPEQVVAIASNGGGRPALESIVAQLSRPDPAL AALTNDHLVALACLGGRPALDAVKKGLPHAPALIKRTNRRIPERTSHRVAKGGRADPAFLYKVVSRMV SKGEEDNMAIIKEFMRFKVHMEGSVNGHEFEIEGEGEGRPYEGTQTAKLKVTKGGPLPFAWDILSPQF MYGSKAYVKHPADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKKPVQLPGAYNVNIK LDITSHNEDYTIVEQYERAEGRHSTGGMDELYKKSLGSAHIVMVDAYKPTKSRRPGILYK- |
| | > Figure 6A | |
| | >SpyTag001-TAL[hsCENTRO] -mCherry-SpyTag001 | |
| | >Full Length Protein Sequence | |
| 61 | >TALhesin[*hsCE NTRO*]*-*B (with SpyCatcher 003) | ATGACAACTGAAGAAGATAGTGCTACCCATATTAAATTCTCAAAACGTGATGAGGACGG CCGTGAGTTAGCTGGTGCAACTATGGAGTTGCGTGATTCATCTGGTAAAACTATTAGTAC ATGGATTTCAGATGGACATGTGAAGGATTTCTACCTGTATCCAGGAAAATATACATTTGT CGAAACCGCAGCACCAGACGGTTATGAGGTAGCAACTCCAATTGAATTTACAGTTAATG AGGACGGTCAGGTTACTGTAGATGGTGAAGCAACTGAAGGTGACGCTCATACTGGATCC AGTGGTAGCACCGGTACAAGTTTGTACAAAAAAGCAGGCTCCGCGGCCGCCCCCTTCAC CATGGTAGATTTGAGAACTTTGGGATATTCACAGCAGCAGCAGGAAAAGATCAAGCCCA AAGTGAGGTCGACAGTCGCGCAGCATCACGAAGCGCTGGTGGGTCATGGGTTTACACAT GCCCACATCGTAGCCTTGTCGCAGCACCCTGCAGCCCTTGGCACGGTCGCCGTCAAGTA CCAGGACATGATTGCGGCGTTGCCGGAAGCCACACATGAGGCGATCGTCGGTGTGGGG AAACAGTGGAGCGGAGCCCGAGCGCTTGAGGCCCTGTTGACGGTCGCGGGAGAGCTGA GAGGGCCTCCCCTTCAGCTGGACACGGGCCAGTTGCTGAAGATCGCGAAGCGGGGAGG AGTCACGGCGGTCGAGGCGGTGCACGCGTGGCGCAATGCGCTCACGGGAGCACCCCTC |
| | > Figure 6A | |
| | > SpyCatcher003-TAL[hsCENTRO] -mClover-SpyCatcher003 | |
| | >DNA Coding Sequence (mammalian | |
| | expression vector not included) | AACCTGACCCCAGAGCAGGTCGTGGCAATTGCGAGCCATGACGGGGGAAAGCAGGCAC TCGAAACCGTCCAGAGGTTGCTGCCTGTGCTGTGCCAAGCGCACGGACTTACGCCAGAG CAGGTCGTGGCAATTGCGAGCCATGACGGGGGAAAGCAGGCACTCGAAACCGTCCAGA GGTTGCTGCCTGTGCTGTGCCAAGCGCACGGACTAACCCCAGAGCAGGTCGTGGCAATT GCGAGCAACATCGGGGGAAAGCAGGCACTCGAAACCGTCCAGAGGTTGCTGCCTGTGC TGTGCCAAGCGCACGGGTTGACCCCAGAGCAGGTCGTGGCAATTGCGAGCAACGGAGG GGGAAAGCAGGCACTCGAAACCGTCCAGAGGTTGCTGCCTGTGCTGTGCCAAGCGCAC GGGTTGACCCCAGAGCAGGTCGTGGCAATTGCGAGCAACGGAGGGGGAAAGCAGGCAC TCGAAACCGTCCAGAGGTTGCTGCCTGTGCTGTGCCAAGCGCACGGACTGACACCAGAG CAGGTCGTGGCAATTGCGAGCCATGACGGGGGAAAGCAGGCACTCGAAACCGTCCAGA GGTTGCTGCCTGTGCTGTGCCAAGCGCACGGACTTACACCCGAACAAGTCGTGGCAATT GCGAGCCATGACGGGGGAAAGCAGGCACTCGAAACCGTCCAGAGGTTGCTGCCTGTGC TGTGCCAAGCGCACGGACTAACCCCAGAGCAGGTCGTGGCAATTGCGAGCAACATCGG GGGAAAGCAGGCACTCGAAACCGTCCAGAGGTTGCTGCCTGTGCTGTGCCAAGCGCAC GGGTTGACCCCAGAGCAGGTCGTGGCAATTGCGAGCAACGGAGGGGGAAAGCAGGCAC TCGAAACCGTCCAGAGGTTGCTGCCTGTGCTGTGCCAAGCGCACGGGTTGACCCCAGAG CAGGTCGTGGCAATTGCGAGCAACGGAGGGGGAAAGCAGGCACTCGAAACCGTCCAGA GGTTGCTGCCTGTGCTGTGCCAAGCGCACGGACTGACACCAGAGCAGGTCGTGGCAATT GCGAGCCATGACGGGGGAAAGCAGGCACTCGAAACCGTCCAGAGGTTGCTGCCTGTGC TGTGCCAAGCGCACGGACTTACACCCGAACAAGTCGTGGCAATTGCGAGCCATGACGG GGGAAAGCAGGCACTCGAAACCGTCCAGAGGTTGCTGCCTGTGCTGTGCCAAGCGCAC GGCCTCACCCCAGAGCAGGTCGTGGCAATTGCGAGCAACATCGGGGGAAAGCAGGCAC TCGAAACCGTCCAGAGGTTGCTGCCTGTGCTGTGCCAAGCGCACGGACTTACGCCAGAG CAGGTCGTGGCAATTGCGAGCAACGGAGGGGGAAAGCAGGCACTCGAAACCGTCCAGA GGTTGCTGCCTGTGCTGTGCCAAGCGCACGGACTAACCCCAGAGCAGGTCGTGGCAATT GCGAGCAACGGAGGGGGAAAGCAGGCACTCGAAACCGTCCAGAGGTTGCTGCCTGTGC TGTGCCAAGCGCACGGGTTGACCCCAGAGCAGGTCGTGGCAATTGCGAGCCATGACGG GGGAAAGCAGGCACTCGAAACCGTCCAGAGGTTGCTGCCTGTGCTGTGCCAAGCGCAC GGCCTGACCCCAGAGCAGGTCGTGGCAATTGCGAGCCATGACGGGGGAAAGCAGGCAC TCGAAACCGTCCAGAGGTTGCTGCCTGTGCTGTGCCAAGCGCACGGACTGACACCAGAG CAGGTCGTGGCAATTGCGAGCAACATCGGGGGAAAGCAGGCACTCGAAACCGTCCAGA GGTTGCTGCCTGTGCTGTGCCAAGCGCACGGACTCACGCCTGAGCAGGTAGTGGCTATT GCATCCAACGGAGGGGGCAGACCCGCACTGGAGTCAATCGTGGCCCAGCTTTCGAGGC CGGACCCCGCGCTGGCCGCACTCACTAATGATCATCTTGTAGCGCTGGCCTGCCTCGGC GGACGACCCGCCTTGGATGCGGTGAAGAAGGGGCTCCCGCACGCGCCTGCATTGATTA AGCGGACCAACAGAAGGATTCCCGAGAGGACATCACATCGAGTGGCAAAGGGTGGGCG CGCCGACCCAGCTTTCTTGTACAAAGTGGTCTCGAGCTCAAGCTTCGAATTCATGGTGA GCAAGGGCGAGGAGCTGTTCACCGGGGTGGTGCCCATCCTGGTCGAGCTGGACGGCGA CGTAAACGACCACAAGTTCAGCGTCCGCGGCGAGGGCGAGGGCGATGCCACCAACGGC AAGCTGACCCTGAAGTTCATCTGCACCACCGGCAAGCTGCCCGTGCCCTGGCCCACCCT CGTGACCACCTTCGGCTACGGCGTGGCCTGCTTCAGCCGCTACCCCGACCACATGAAGC AGCACGACTTCTTCAAGTCCGCCATGCCCGAAGGCTACGTCCAGGAGCGCACCATCTCT TTCAAGGACGACGGTACCTACAAGACCCGCGCCGAGGTGAAGTTCGAGGGCGACACCC TGGTGAACCGCATCGAGCTGAAGGGCATCGACTTCAAGGAGGACGGCAACATCCTGGG GCACAAGCTGGAGTACAACTTCAACAGCCACAACGTCTATATCACGGCCGACAAGCAGA AGAACGGCATCAAGGCTAACTTCAAGATCCGCCACAACGTTGAGGACGGCAGCGTGCA GCTCGCCGACCACTACCAGCAGAACACCCCCATCGGCGACGGCCCCGTGCTGCTGCCCG ACAACCACTACCTGAGCCATCAGTCCAAGCTGAGCAAAGACCCCAACGAGAAGCGCGAT CACATGGTCCTGCTGGAGTTCGTGACCGCCGCCGGGATTACACATGGCATGGACGAGCT GTACAAGGTCGACATGACAACTGAAGAAGATAGTGCTACCCATATTAAATTCTCAAAAC GTGATGAGGACGGCCGTGAGTTAGCTGGTGCAACTATGGAGTTGCGTGATTCATCTGGT AAAACTATTAGTACATGGATTTCAGATGGACATGTGAAGGATTTCTACCTGTATCCAGGA AAATATACATTTGTCGAAACCGCAGCACCAGACGGTTATGAGGTAGCAACTCCAATTGA ATTTACAGTTAATGAGGACGGTCAGGTTACTGTAGATGGTGAAGCAACTGAAGGTGACG CTCATACTGGATCCAGTGGTAGCTAA |
| 62 | >TALhesin[hsCE *NTRO*]*-*B (with SpyCatcher 003) | MTTEEDSATHIKFSKRDEDGRELAGATMELRDSSGKTISTWISDGHVKDFYLYPGKYTFVETAAPDGY EVATPIEFTVNEDGQVTVDGEATEGDAHTGSSGSTGTSLYKKAGSAAAPFTMVDLRTLGYSQQQQEK IKPKVRSTVAQHHEALVGHGFTHAHIVALSQHPAALGTVAVKYQDMIAALPEATHEAIVGVGKQWSG ARALEALLTVAGELRGPPLQLDTGQLLKIAKRGGVTAVEAVHAWRNALTGAPLNLTPEQVVAIASHDG GKQALETVQRLLPVLCQAHGLTPEQVVAIASHDGGKQALETVQRLLPVLCQAHGLTPEQVVAIASNIG GKQALETVQRLLPVLCQAHGLTPEQVVAIASNGGGKQALETVQRLLPVLCQAHGLTPEQVVAIASNGG GKQALETVQRLLPVLCQAHGLTPEQVVAIASHDGGKQALETVQRLLPVLCQAHGLTPEQVVAIASHDG GKQALETVQRLLPVLCQAHGLTPEQVVAIASNIGGKQALETVQRLLPVLCQAHGLTPEQVVAIASNGG |
| | > Figure 6A | |
| | > SpyCatcher003-TAL[hsCENTRO] | |
| | -mClover-SpyCatcher003 | GKQALETVQRLLPVLCQAHGLTPEQVVAIASNGGGKQALETVQRLLPVLCQAHGLTPEQVVAIASHDG GKQALETVQRLLPVLCQAHGLTPEQVVAIASHDGGKQALETVQRLLPVLCQAHGLTPEQVVAIASNIG GKQALETVQRLLPVLCQAHGLTPEQVVAIASNGGGKQALETVQRLLPVLCQAHGLTPEQVVAIASNGG GKQALETVQRLLPVLCQAHGLTPEQVVAIASHDGGKQALETVQRLLPVLCQAHGLTPEQVVAIASHDG GKQALETVQRLLPVLCQAHGLTPEQVVAIASNIGGKQALETVQRLLPVLCQAHGLTPEQVVAIASNGG GRPALESIVAQLSRPDPALAALTNDHLVALACLGGRPALDAVKKGLPHAPALIKRTNRRIPERTSHRVA KGGRADPAFLYKVVSSSSFEFMVSKGEELFTGVVPILVELDGDVNDHKFSVRGEGEGDATNGKLTLKFI CTTGKLPVPWPTLVTTFGYGVACFSRYPDHMKQHDFFKSAMPEGYVQERTISFKDDGTYKTRAEVKF EGDTLVNRIELKGIDFKEDGNILGHKLEYNFNSHNVYITADKQKNGIKANFKIRHNVEDGSVQLADHY QQNTPIGDGPVLLPDNHYLSHQSKLSKDPNEKRDHMVLLEFVTAAGITHGMDELYKVDMTTEEDSAT HIKFSKRDEDGRELAGATMELRDSSGKTISTWISDGHVKDFYLYPGKYTFVETAAPDGYEVATPIEFT VNEDGQVTVDGEATEGDAHTGSSGS- |
| | >Full Length Protein Sequence | |
| 63 | >TALhesin[*hsCE NTRO*]-A | GCGAAUUAAUUCGAGCUCGGUACCCAGCUUGCUUGUUCUUUUUGCAGAAGCUCAGAAUAAACGC UCAACUUUGGCAGAUCAAUUCCCCGGUGCUAGCAUGGGUUCCGCCCACAUCGUGAUGGUG GACGCCUACAAGCCGACGAAGACCGGUACAAGUUUGUACAAAAAAGCAGGCUCCGCG GCCGCCCCCUUCACCAUGGUAGAUUUGAGAACUUUGGGAUAUUCACAGCAGCAGCAG GAAAAGAUCAAGCCCAAAGUGAGGUCGACAGUCGCGCAGCAUCACGAAGCGCUGGUG GGUCAUGGGUUUACACAUGCCCACAUCGUAGCCUUGUCGCAGCACCCUGCAGCCCUU GGCACGGUCGCCGUCAAGUACCAGGACAUGAUUGCGGCGUUGCCGGAAGCCACACAU GAGGCGAUCGUCGGUGUGGGGAAACAGUGGAGCGGAGCCCGAGCGCUUGAGGCCCU GUUGACGGUCGCGGGAGAGCUGAGAGGGCCUCCCCUUCAGCUGGACACGGGCCAGU UGCUGAAGAUCGCGAAGCGGGGAGGAGUCACGGCGGUCGAGGCGGUGCACGCGUGG CGCAAUGCGCUCACGGGAGCACCCCUCAACCUGACCCCAGAGCAGGUCGUGGCAAUU GCGAGCCAUGACGGGGGAAAGCAGGCACUCGAAACCGUCCAGAGGUUGCUGCCUGUG CUGUGCCAAGCGCACGGACUUACGCCAGAGCAGGUCGUGGCAAUUGCGAGCCAUGAC GGGGGAAAGCAGGCACUCGAAACCGUCCAGAGGUUGCUGCCUGUGCUGUGCCAAGCG CACGGACUAACCCCAGAGCAGGUCGUGGCAAUUGCGAGCAACAUCGGGGGAAAGCAG GCACUCGAAACCGUCCAGAGGUUGCUGCCUGUGCUGUGCCAAGCGCACGGGUUGACC CCAGAGCAGGUCGUGGCAAUUGCGAGCAACGGAGGGGGAAAGCAGGCACUCGAAACC GUCCAGAGGUUGCUGCCUGUGCUGUGCCAAGCGCACGGGUUGACCCCAGAGCAGGU CGUGGCAAUUGCGAGCAACGGAGGGGGAAAGCAGGCACUCGAAACCGUCCAGAGGUU GCUGCCUGUGCUGUGCCAAGCGCACGGACUGACACCAGAGCAGGUCGUGGCAAUUGC GAGCCAUGACGGGGGAAAGCAGGCACUCGAAACCGUCCAGAGGUUGCUGCCUGUGCU GUGCCAAGCGCACGGACUUACACCCGAACAAGUCGUGGCAAUUGCGAGCCAUGACGG GGGAAAGCAGGCACUCGAAACCGUCCAGAGGUUGCUGCCUGUGCUGUGCCAAGCGCA CGGACUAACCCCAGAGCAGGUCGUGGCAAUUGCGAGCAACAUCGGGGGAAAGCAGGC ACUCGAAACCGUCCAGAGGUUGCUGCCUGUGCUGUGCCAAGCGCACGGGUUGACCCC AGAGCAGGUCGUGGCAAUUGCGAGCAACGGAGGGGGAAAGCAGGCACUCGAAACCGU CCAGAGGUUGCUGCCUGUGCUGUGCCAAGCGCACGGGUUGACCCCAGAGCAGGUCG UGGCAAUUGCGAGCAACGGAGGGGGAAAGCAGGCACUCGAAACCGUCCAGAGGUUGC UGCCUGUGCUGUGCCAAGCGCACGGACUGACACCAGAGCAGGUCGUGGCAAUUGCGA GCCAUGACGGGGGAAAGCAGGCACUCGAAACCGUCCAGAGGUUGCUGCCUGUGCUGU GCCAAGCGCACGGACUUACACCCGAACAAGUCGUGGCAAUUGCGAGCCAUGACGGGG GAAAGCAGGCACUCGAAACCGUCCAGAGGUUGCUGCCUGUGCUGUGCCAAGCGCACG GCCUCACCCCAGAGCAGGUCGUGGCAAUUGCGAGCAACAUCGGGGGAAAGCAGGCAC UCGAAACCGUCCAGAGGUUGCUGCCUGUGCUGUGCCAAGCGCACGGACUUACGCCAG AGCAGGUCGUGGCAAUUGCGAGCAACGGAGGGGGAAAGCAGGCACUCGAAACCGUCC AGAGGUUGCUGCCUGUGCUGUGCCAAGCGCACGGACUAACCCCAGAGCAGGUCGUGG CAAUUGCGAGCAACGGAGGGGGAAAGCAGGCACUCGAAACCGUCCAGAGGUUGCUGC CUGUGCUGUGCCAAGCGCACGGGUUGACCCCAGAGCAGGUCGUGGCAAUUGCGAGCC AUGACGGGGGAAAGCAGGCACUCGAAACCGUCCAGAGGUUGCUGCCUGUGCUGUGCC AAGCGCACGGCCUGACCCCAGAGCAGGUCGUGGCAAUUGCGAGCCAUGACGGGGGAA AGCAGGCACUCGAAACCGUCCAGAGGUUGCUGCCUGUGCUGUGCCAAGCGCACGGAC UGACACCAGAGCAGGUCGUGGCAAUUGCGAGCAACAUCGGGGGAAAGCAGGCACUCG AAACCGUCCAGAGGUUGCUGCCUGUGCUGUGCCAAGCGCACGGACUCACGCCUGAGC AGGUAGUGGCUAUUGCAUCCAACGGAGGGGGCAGACCCGCACUGGAGUCAAUCGUG GCCCAGCUUUCGAGGCCGGACCCCGCGCUGGCCGCACUCACUAAUGAUCAUCUUGUA GCGCUGGCCUGCCUCGGCGGACGACCCGCCUUGGAUGCGGUGAAGAAGGGGCUCCCG CACGCGCCUGCAUUGAUUAAGCGGACCAACAGAAGGAUUCCCGAGAGGACAUCACAU CGAGUGGCAAAGGGUGGGCGCGCCGACCCAGCUUUCUUGUACAAAGUGGUCUCGAG AAUGGUGAGCAAGGGCGAGGAGGAUAACAUGGCCAUCAUCAAGGAGUUCAUGCGCU UCAAGGUGCACAUGGAGGGCUCCGUGAACGGCCACGAGUUCGAGAUCGAGGGCGAG GGCGAGGGCCGCCCCUACGAGGGCACCCAGACCGCCAAGCUGAAGGUGACCAAGGGU GGCCCCCUGCCCUUCGCCUGGGACAUCCUGUCCCCUCAGUUCAUGUACGGCUCCAAG GCCUACGUGAAGCACCCCGCCGACAUCCCCGACUACUUGAAGCUGUCCUUCCCCGAG GGCUUCAAGUGGGAGCGCGUGAUGAACUUCGAGGACGGCGGCGUGGUGACCGUGAC CCAGGACUCCUCCCUGCAGGACGGCGAGUUCAUCUACAAGGUGAAGCUGCGCGGCAC CAACUUCCCCUCCGACGGCCCCGUAAUGCAGAAGAAGACCAUGGGCUGGGAGGCCUC CUCCGAGCGGAUGUACCCCGAGGACGGCGCCCUGAAGGGCGAGAUCAAGCAGAGGCU GAAGCUGAAGGACGGCGGCCACUACGACGCUGAGGUCAAGACCACCUACAAGGCCAA GAAGCCCGUGCAGCUGCCCGGCGCCUACAACGUCAACAUCAAGUUGGACAUCACCUC CCACAACGAGGACUACACCAUCGUGGAACAGUACGAACGCGCCGAGGGCCGCCACUCC ACCGGCGGCAUGGACGAGCUGUACAAGAAAAGCUUAGGUUCCGCCCAUAUAGUUAUG GUGGACGCCUAUAAACCCACUAAGAGUCGACGGCCCGGGAUCCUGUACAAGUAAAGC GGCCGCUCUAGAUAAAUGAUAGCUUGAUCUGGUUACCACUAAACCAGCCUCAAGAACACCCGAA UGGAGUCUCUAAGCUACAUAAUACCAACUUACACUUUACAAAAUGUUGUCCCCCAAAAUGUAGC CAUUCGUAUCUGCUCCUAAUAAAAAGAAAGUUUCUUCACAUUCUAAAAAAAAAAAAAAAAAAAA AAAAAAAAAAAACCCCCCCCCCCCCCCCUGCAGGCGCGCCGCCGGCGAUUUAAAUUAAU |
| | > Figure 6B | |
| | >SpyTag001-TAL[hsCENTRO] -mCherry-SpyTag001 | |
| | >mRNA transcript full-length includes 5' and 3' UTRs. | |
| 64 | >TALhesin[*hsCE NITRO*]-A | MGSAHIVMVDAYKPTKTGTSLYKKAGSAAAPFTMVDLRTLGYSQQQQEKIKPKVRSTVAQHHEALVG HGFTHAHIVALSQHPAALGTVAVKYQDMIAALPEATHEAIVGVGKQWSGARALEALLTVAGELRGPPL QLDTGQLLKIAKRGGVTAVEAVHAWRNALTGAPLNLTPEQVVAIASHDGGKQALETVQRLLPVLCQA HGLTPEQVVAIASHDGGKQALETVQRLLPVLCQAHGLTPEQVVAIASNIGGKQALETVQRLLPVLCQA HGLTPEQVVAIASNGGGKQALETVQRLLPVLCQAHGLTPEQVVAIASNGGGKQALETVQRLLPVLCQA HGLTPEQVVAIASHDGGKQALETVQRLLPVLCQAHGLTPEQVVAIASHDGGKQALETVQRLLPVLCQA HGLTPEQVVAIASNIGGKQALETVQRLLPVLCQAHGLTPEQVVAIASNGGGKQALETVQRLLPVLCQA HGLTPEQVVAIASNGGGKQALETVQRLLPVLCQAHGLTPEQVVAIASHDGGKQALETVQRLLPVLCQA HGLTPEQVVAIASHDGGKQALETVQRLLPVLCQAHGLTPEQVVAIASNIGGKQALETVQRLLPVLCQA HGLTPEQVVAIASNGGGKQALETVQRLLPVLCQAHGLTPEQVVAIASNGGGKQALETVQRLLPVLCQA HGLTPEQVVAIASHDGGKQALETVQRLLPVLCQAHGLTPEQVVAIASHDGGKQALETVQRLLPVLCQA HGLTPEQVVAIASNIGGKQALETVQRLLPVLCQAHGLTPEQVVAIASNGGGRPALESIVAQLSRPDPAL AALTNDHLVALACLGGRPALDAVKKGLPHAPALIKRTNRRIPERTSHRVAKGGRADPAFLYKVVSRMV SKGEEDNMAIIKEFMRFKVHMEGSVNGHEFEIEGEGEGRPYEGTQTAKLKVTKGGPLPFAWDILSPQF MYGSKAYVKHPADIPDYLKLSFPEGFKWERVMNFEDGGVVTVTQDSSLQDGEFIYKVKLRGTNFPSD GPVMQKKTMGWEASSERMYPEDGALKGEIKQRLKLKDGGHYDAEVKTTYKAKKPVQLPGAYNVNIK LDITSHNEDYTIVEQYERAEGRHSTGGMDELYKKSLGSAHIVMVDAYKPTKSRRPGILYK- |
| | > Figure 6B | |
| | >SpyTag001-TAL[hsCENTRO] -mCherry-SpyTag001. | |
| | >Full Length Protein Sequence | |
| 65 | >TALhesin[hsCE *NTRO*]-B | GCGAAUUAAUUCGAGCUCGGUACCCAGCUUGCUUGUUCUUUUUGCAGAAGCUCAGA AUAAACGCUCAACUUUGGCAGAUCAAUUCCCCGGUGCUAGCAUGGAUAGUGCUACCC AUAUUAAAUUCUCAAAACGUGAUGAGGACGGCAAAGAGUUAGCUGGUGCAACUAUG GAGUUGCGUGAUUCAUCUGGUAAAACUAUUAGUACAUGGAUUUCAGAUGGACAAGU GAAAGAUUUCUACCUGUAUCCAGGAAAAUAUACAUUUGUCGAAACCGCAGCACCAGA CGGUUAUGAGGUAGCAACUGCUAUUACCUUUACAGUUAAUGAGCAAGGUCAGGUUA CUGUAAAUGGCAAAGCAACUAAAGGUGACGCUCAUAUUGGAUCCACCGGUACAAGUU UGUACAAAAAAGCAGGCUCCGCGGCCGCCCCCUUCACCAUGGUAGAUUUGAGAACUU UGGGAUAUUCACAGCAGCAGCAGGAAAAGAUCAAGCCCAAAGUGAGGUCGACAGUCG CGCAGCAUCACGAAGCGCUGGUGGGUCAUGGGUUUACACAUGCCCACAUCGUAGCCU UGUCGCAGCACCCUGCAGCCCUUGGCACGGUCGCCGUCAAGUACCAGGACAUGAUUG CGGCGUUGCCGGAAGCCACACAUGAGGCGAUCGUCGGUGUGGGGAAACAGUGGAGC GGAGCCCGAGCGCUUGAGGCCCUGUUGACGGUCGCGGGAGAGCUGAGAGGGCCUCC CCUUCAGCUGGACACGGGCCAGUUGCUGAAGAUCGCGAAGCGGGGAGGAGUCACGGC GGUCGAGGCGGUGCACGCGUGGCGCAAUGCGCUCACGGGAGCACCCCUCAACCUGAC CCCAGAGCAGGUCGUGGCAAUUGCGAGCCAUGACGGGGGAAAGCAGGCACUCGAAAC CGUCCAGAGGUUGCUGCCUGUGCUGUGCCAAGCGCACGGACUUACGCCAGAGCAGGU CGUGGCAAUUGCGAGCCAUGACGGGGGAAAGCAGGCACUCGAAACCGUCCAGAGGUU GCUGCCUGUGCUGUGCCAAGCGCACGGACUAACCCCAGAGCAGGUCGUGGCAAUUGC GAGCAACAUCGGGGGAAAGCAGGCACUCGAAACCGUCCAGAGGUUGCUGCCUGUGCU GUGCCAAGCGCACGGGUUGACCCCAGAGCAGGUCGUGGCAAUUGCGAGCAACGGAGG GGGAAAGCAGGCACUCGAAACCGUCCAGAGGUUGCUGCCUGUGCUGUGCCAAGCGCA CGGGUUGACCCCAGAGCAGGUCGUGGCAAUUGCGAGCAACGGAGGGGGAAAGCAGG CACUCGAAACCGUCCAGAGGUUGCUGCCUGUGCUGUGCCAAGCGCACGGACUGACAC CAGAGCAGGUCGUGGCAAUUGCGAGCCAUGACGGGGGAAAGCAGGCACUCGAAACCG UCCAGAGGUUGCUGCCUGUGCUGUGCCAAGCGCACGGACUUACACCCGAACAAGUCG UGGCAAUUGCGAGCCAUGACGGGGGAAAGCAGGCACUCGAAACCGUCCAGAGGUUGC UGCCUGUGCUGUGCCAAGCGCACGGACUAACCCCAGAGCAGGUCGUGGCAAUUGCGA GCAACAUCGGGGGAAAGCAGGCACUCGAAACCGUCCAGAGGUUGCUGCCUGUGCUGU GCCAAGCGCACGGGUUGACCCCAGAGCAGGUCGUGGCAAUUGCGAGCAACGGAGGGG GAAAGCAGGCACUCGAAACCGUCCAGAGGUUGCUGCCUGUGCUGUGCCAAGCGCACG GGUUGACCCCAGAGCAGGUCGUGGCAAUUGCGAGCAACGGAGGGGGAAAGCAGGCAC UCGAAACCGUCCAGAGGUUGCUGCCUGUGCUGUGCCAAGCGCACGGACUGACACCAG AGCAGGUCGUGGCAAUUGCGAGCCAUGACGGGGGAAAGCAGGCACUCGAAACCGUCC AGAGGUUGCUGCCUGUGCUGUGCCAAGCGCACGGACUUACACCCGAACAAGUCGUGG CAAUUGCGAGCCAUGACGGGGGAAAGCAGGCACUCGAAACCGUCCAGAGGUUGCUGC CUGUGCUGUGCCAAGCGCACGGCCUCACCCCAGAGCAGGUCGUGGCAAUUGCGAGCA ACAUCGGGGGAAAGCAGGCACUCGAAACCGUCCAGAGGUUGCUGCCUGUGCUGUGCC AAGCGCACGGACUUACGCCAGAGCAGGUCGUGGCAAUUGCGAGCAACGGAGGGGGAA AGCAGGCACUCGAAACCGUCCAGAGGUUGCUGCCUGUGCUGUGCCAAGCGCACGGAC UAACCCCAGAGCAGGUCGUGGCAAUUGCGAGCAACGGAGGGGGAAAGCAGGCACUCG AAACCGUCCAGAGGUUGCUGCCUGUGCUGUGCCAAGCGCACGGGUUGACCCCAGAGC AGGUCGUGGCAAUUGCGAGCCAUGACGGGGGAAAGCAGGCACUCGAAACCGUCCAGA GGUUGCUGCCUGUGCUGUGCCAAGCGCACGGCCUGACCCCAGAGCAGGUCGUGGCAA UUGCGAGCCAUGACGGGGGAAAGCAGGCACUCGAAACCGUCCAGAGGUUGCUGCCUG UGCUGUGCCAAGCGCACGGACUGACACCAGAGCAGGUCGUGGCAAUUGCGAGCAACA UCGGGGGAAAGCAGGCACUCGAAACCGUCCAGAGGUUGCUGCCUGUGCUGUGCCAAG CGCACGGACUCACGCCUGAGCAGGUAGUGGCUAUUGCAUCCAACGGAGGGGGCAGAC CCGCACUGGAGUCAAUCGUGGCCCAGCUUUCGAGGCCGGACCCCGCGCUGGCCGCAC UCACUAAUGAUCAUCUUGUAGCGCUGGCCUGCCUCGGCGGACGACCCGCCUUGGAUG CGGUGAAGAAGGGGCUCCCGCACGCGCCUGCAUUGAUUAAGCGGACCAACAGAAGGA UUCCCGAGAGGACAUCACAUCGAGUGGCAAAGGGUGGGCGCGCCGACCCAGCUUUCU UGUACAAAGUGGUCUCGAGCUCAAGCUUCGAAUUCAUGGUGAGCAAGGGCGAGGAG CUGUUCACCGGGGUGGUGCCCAUCCUGGUCGAGCUGGACGGCGACGUAAACGACCAC AAGUUCAGCGUCCGCGGCGAGGGCGAGGGCGAUGCCACCAACGGCAAGCUGACCCUG AAGUUCAUCUGCACCACCGGCAAGCUGCCCGUGCCCUGGCCCACCCUCGUGACCACCU UCGGCUACGGCGUGGCCUGCUUCAGCCGCUACCCCGACCACAUGAAGCAGCACGACU UCUUCAAGUCCGCCAUGCCCGAAGGCUACGUCCAGGAGCGCACCAUCUCUUUCAAGG ACGACGGUACCUACAAGACCCGCGCCGAGGUGAAGUUCGAGGGCGACACCCUGGUGA ACCGCAUCGAGCUGAAGGGCAUCGACUUCAAGGAGGACGGCAACAUCCUGGGGCACA AGCUGGAGUACAACUUCAACAGCCACAACGUCUAUAUCACGGCCGACAAGCAGAAGA ACGGCAUCAAGGCUAACUUCAAGAUCCGCCACAACGUUGAGGACGGCAGCGUGCAGC UCGCCGACCACUACCAGCAGAACACCCCCAUCGGCGACGGCCCCGUGCUGCUGCCCGA CAACCACUACCUGAGCCAUCAGUCCAAGCUGAGCAAAGACCCCAACGAGAAGCGCGAU CACAUGGUCCUGCUGGAGUUCGUGACCGCCGCCGGGAUUACACAUGGCAUGGACGAG CUGUACAAGGUCGACAUGGAUAGUGCUACCCAUAUUAAAUUCUCAAAACGUGAUGAG GACGGCAAAGAGUUAGCUGGUGCAACUAUGGAGUUGCGUGAUUCAUCUGGUAAAAC UAUUAGUACAUGGAUUUCAGAUGGACAAGUGAAAGAUUUCUACCUGUAUCCAGGAAA AUAUACAUUUGUCGAAACCGCAGCACCAGACGGUUAUGAGGUAGCAACUGCUAUUAC CUUUACAGUUAAUGAGCAAGGUCAGGUUACUGUAAAUGGCAAAGCAACUAAAGGUGA CGCUCAUAUUGGAUCCUAAGCGGCCGCUCUAGAUAAAUGAUAGCUUGAUCUGGUUACCACU AAACCAGCCUCAAGAACACCCGAAUGGAGUCUCUAAGCUACAUAAUACCAACUUACACUUUACAA AAUGUUGUCCCCCAAAAUGUAGCCAUUCGUAUCUGCUCCUAAUAAAAAGAAAGUUUCUUCACAU UCUAAAAAAAAAAAAAAAAAAAAAAAAAAAAAAAAAACCCCCCCCCCCCCCCCCUGCAGGCGCGCC GCCGGCGAUUUAAAUUAAU |
| | > Figure 6B | |
| | > SpyCatcher001-TAL[hsCENTRO] -mClover-SpyCatcher001 | |
| | >mRNA transcript full-length includes 5' and 3' UTRs. | |
| **66** | >TALhesin[*hsCE NTRO*]*-B* | MDSATHIKFSKRDEDGKELAGATMELRDSSGKTISTWISDGQVKDFYLYPGKYTFVETAAPDGYEVA TAITFTVNEQGQVTVNGKATKGDAHIGSTGTSLYKKAGSAAAPFTMVDLRTLGYSQQQQEKIKPKVR STVAQHHEALVGHGFTHAHIVALSQHPAALGTVAVKYQDMIAALPEATHEAIVGVGKQWSGARALEA LLTVAGELRGPPLQLDTGQLLKIAKRGGVTAVEAVHAWRNALTGAPLNLTPEQVVAIASHDGGKQALE TVQRLLPVLCQAHGLTPEQVVAIASHDGGKQALETVQRLLPVLCQAHGLTPEQVVAIASNIGGKQALE TVQRLLPVLCQAHGLTPEQVVAIASNGGGKQALETVQRLLPVLCQAHGLTPEQVVAIASNGGGKQALE TVQRLLPVLCQAHGLTPEQVVAIASHDGGKQALETVQRLLPVLCQAHGLTPEQVVAIASHDGGKQALE TVQRLLPVLCQAHGLTPEQVVAIASNIGGKQALETVQRLLPVLCQAHGLTPEQVVAIASNGGGKQALE TVQRLLPVLCQAHGLTPEQVVAIASNGGGKQALETVQRLLPVLCQAHGLTPEQVVAIASHDGGKQALE TVQRLLPVLCQAHGLTPEQVVAIASHDGGKQALETVQRLLPVLCQAHGLTPEQVVAIASNIGGKQALE TVQRLLPVLCQAHGLTPEQVVAIASNGGGKQALETVQRLLPVLCQAHGLTPEQVVAIASNGGGKQALE TVQRLLPVLCQAHGLTPEQVVAIASHDGGKQALETVQRLLPVLCQAHGLTPEQVVAIASHDGGKQALE TVQRLLPVLCQAHGLTPEQVVAIASNIGGKQALETVQRLLPVLCQAHGLTPEQVVAIASNGGGRPALE SIVAQLSRPDPALAALTNDHLVALACLGGRPALDAVKKGLPHAPALIKRTNRRIPERTSHRVAKGGRAD PAFLYKVVSSSSFEFMVSKGEELFTGVVPILVELDGDVNDHKFSVRGEGEGDATNGKLTLKFICTTGKL PVPWPTLVTTFGYGVACFSRYPDHMKQHDFFKSAMPEGYVQERTISFKDDGTYKTRAEVKFEGDTLV NRIELKGIDFKEDGNILGHKLEYNFNSHNVYITADKQKNGIKANFKIRHNVEDGSVQLADHYQQNTPI GDGPVLLPDNHYLSHQSKLSKDPNEKRDHMVLLEFVTAAGITHGMDELYKVDMDSATHIKFSKRDED GKELAGATMELRDSSGKTISTWISDGQVKDFYLYPGKYTFVETAAPDGYEVATAITFTVNEQGQVTV NGKATKGDAHIGS- |
| | > Figure 6B | |
| | > SpyCatcher001-TAL[hsCENTRO] -mClover-SpyCatcher001 | |
| | >Full Length Protein Sequence | |
| 67 | >TALhe-sin[*hsCEN-TRO*](hRec8)-A | GCGAAUUAAUUCGAGCUCGGUACCCAGCUUGCUUGUUCUUUUUGCAGAAGCUCAGAAUAAACGC UCAACUUUGGCAGAUCAAUUCCCCGGUGCUAGCAUGGGUUCCGCCCACAUCGUGAUGGUG GACGCCUACAAGCCGACGAAGACCGGUACAAGUUUGUACAAAAAAGCAGGCUCCGCG GCCGCCCCCUUCACCAUGGUAGAUUUGAGAACUUUGGGAUAUUCACAGCAGCAGCAG GAAAAGAUCAAGCCCAAAGUGAGGUCGACAGUCGCGCAGCAUCACGAAGCGCUGGUG GGUCAUGGGUUUACACAUGCCCACAUCGUAGCCUUGUCGCAGCACCCUGCAGCCCUU GGCACGGUCGCCGUCAAGUACCAGGACAUGAUUGCGGCGUUGCCGGAAGCCACACAU GAGGCGAUCGUCGGUGUGGGGAAACAGUGGAGCGGAGCCCGAGCGCUUGAGGCCCU GUUGACGGUCGCGGGAGAGCUGAGAGGGCCUCCCCUUCAGCUGGACACGGGCCAGU UGCUGAAGAUCGCGAAGCGGGGAGGAGUCACGGCGGUCGAGGCGGUGCACGCGUGG CGCAAUGCGCUCACGGGAGCACCCCUCAACCUGACCCCAGAGCAGGUCGUGGCAAUU GCGAGCCAUGACGGGGGAAAGCAGGCACUCGAAACCGUCCAGAGGUUGCUGCCUGUG CUGUGCCAAGCGCACGGACUUACGCCAGAGCAGGUCGUGGCAAUUGCGAGCCAUGAC GGGGGAAAGCAGGCACUCGAAACCGUCCAGAGGUUGCUGCCUGUGCUGUGCCAAGCG CACGGACUAACCCCAGAGCAGGUCGUGGCAAUUGCGAGCAACAUCGGGGGAAAGCAG GCACUCGAAACCGUCCAGAGGUUGCUGCCUGUGCUGUGCCAAGCGCACGGGUUGACC CCAGAGCAGGUCGUGGCAAUUGCGAGCAACGGAGGGGGAAAGCAGGCACUCGAAACC GUCCAGAGGUUGCUGCCUGUGCUGUGCCAAGCGCACGGGUUGACCCCAGAGCAGGU CGUGGCAAUUGCGAGCAACGGAGGGGGAAAGCAGGCACUCGAAACCGUCCAGAGGUU GCUGCCUGUGCUGUGCCAAGCGCACGGACUGACACCAGAGCAGGUCGUGGCAAUUGC GAGCCAUGACGGGGGAAAGCAGGCACUCGAAACCGUCCAGAGGUUGCUGCCUGUGCU GUGCCAAGCGCACGGACUUACACCCGAACAAGUCGUGGCAAUUGCGAGCCAUGACGG GGGAAAGCAGGCACUCGAAACCGUCCAGAGGUUGCUGCCUGUGCUGUGCCAAGCGCA CGGACUAACCCCAGAGCAGGUCGUGGCAAUUGCGAGCAACAUCGGGGGAAAGCAGGC ACUCGAAACCGUCCAGAGGUUGCUGCCUGUGCUGUGCCAAGCGCACGGGUUGACCCC AGAGCAGGUCGUGGCAAUUGCGAGCAACGGAGGGGGAAAGCAGGCACUCGAAACCGU CCAGAGGUUGCUGCCUGUGCUGUGCCAAGCGCACGGGUUGACCCCAGAGCAGGUCG UGGCAAUUGCGAGCAACGGAGGGGGAAAGCAGGCACUCGAAACCGUCCAGAGGUUGC UGCCUGUGCUGUGCCAAGCGCACGGACUGACACCAGAGCAGGUCGUGGCAAUUGCGA GCCAUGACGGGGGAAAGCAGGCACUCGAAACCGUCCAGAGGUUGCUGCCUGUGCUGU GCCAAGCGCACGGACUUACACCCGAACAAGUCGUGGCAAUUGCGAGCCAUGACGGGG GAAAGCAGGCACUCGAAACCGUCCAGAGGUUGCUGCCUGUGCUGUGCCAAGCGCACG GCCUCACCCCAGAGCAGGUCGUGGCAAUUGCGAGCAACAUCGGGGGAAAGCAGGCAC UCGAAACCGUCCAGAGGUUGCUGCCUGUGCUGUGCCAAGCGCACGGACUUACGCCAG AGCAGGUCGUGGCAAUUGCGAGCAACGGAGGGGGAAAGCAGGCACUCGAAACCGUCC AGAGGUUGCUGCCUGUGCUGUGCCAAGCGCACGGACUAACCCCAGAGCAGGUCGUGG CAAUUGCGAGCAACGGAGGGGGAAAGCAGGCACUCGAAACCGUCCAGAGGUUGCUGC CUGUGCUGUGCCAAGCGCACGGGUUGACCCCAGAGCAGGUCGUGGCAAUUGCGAGCC AUGACGGGGGAAAGCAGGCACUCGAAACCGUCCAGAGGUUGCUGCCUGUGCUGUGCC AAGCGCACGGCCUGACCCCAGAGCAGGUCGUGGCAAUUGCGAGCCAUGACGGGGGAA AGCAGGCACUCGAAACCGUCCAGAGGUUGCUGCCUGUGCUGUGCCAAGCGCACGGAC UGACACCAGAGCAGGUCGUGGCAAUUGCGAGCAACAUCGGGGGAAAGCAGGCACUCG AAACCGUCCAGAGGUUGCUGCCUGUGCUGUGCCAAGCGCACGGACUCACGCCUGAGC AGGUAGUGGCUAUUGCAUCCAACGGAGGGGGCAGACCCGCACUGGAGUCAAUCGUG GCCCAGCUUUCGAGGCCGGACCCCGCGCUGGCCGCACUCACUAAUGAUCAUCUUGUA GCGCUGGCCUGCCUCGGCGGACGACCCGCCUUGGAUGCGGUGAAGAAGGGGCUCCCG CACGCGCCUGCAUUGAUUAAGCGGACCAACAGAAGGAUUCCCGAGAGGACAUCACAU CGAGUGGCAAAGGGUGGGCGCGCCGACCCAGCUUUCUUGUACAAAGUGGUCCAUUG UGCCCAGCCACCCCCAAAAGCCCUCAGGCGAGAGCUGCCUGAGGAGGCAGCCGCUGA GGAGGAAAGGAGAAAGAUUGAAGUUCCAAGUGAGAUUGAGGUCCCGAGGGAGGCCC UGGAGCCCAGUGUUCCCCUUAUGGUGUCUUUAGAGAUCUCCCUAGAGGCAGCUGAA GAGGAGAAGUCCCGCAUCAGCCUCAUCCCACCAGAAGAACGGUGGGCCUGGCCUGAG GUGGAGGCGCCAGAAGCUCCUGCAUUGCCCGUGGUGCCUGAACUCCCUGAGGUGCCC AUGGAGAUGCCUUUGGUGCUGCCCCCAGAGCUCGAGCUGCUCAGAAUGGUGAGCAA GGGCGAGGAGGAUAACAUGGCCAUCAUCAAGGAGUUCAUGCGCUUCAAGGUGCACAU GGAGGGCUCCGUGAACGGCCACGAGUUCGAGAUCGAGGGCGAGGGCGAGGGCCGCC CCUACGAGGGCACCCAGACCGCCAAGCUGAAGGUGACCAAGGGUGGCCCCCUGCCCU UCGCCUGGGACAUCCUGUCCCCUCAGUUCAUGUACGGCUCCAAGGCCUACGUGAAGC ACCCCGCCGACAUCCCCGACUACUUGAAGCUGUCCUUCCCCGAGGGCUUCAAGUGGG AGCGCGUGAUGAACUUCGAGGACGGCGGCGUGGUGACCGUGACCCAGGACUCCUCCC UGCAGGACGGCGAGUUCAUCUACAAGGUGAAGCUGCGCGGCACCAACUUCCCCUCCG ACGGCCCCGUAAUGCAGAAGAAGACCAUGGGCUGGGAGGCCUCCUCCGAGCGGAUGU ACCCCGAGGACGGCGCCCUGAAGGGCGAGAUCAAGCAGAGGCUGAAGCUGAAGGACG GCGGCCACUACGACGCUGAGGUCAAGACCACCUACAAGGCCAAGAAGCCCGUGCAGC UGCCCGGCGCCUACAACGUCAACAUCAAGUUGGACAUCACCUCCCACAACGAGGACUA CACCAUCGUGGAACAGUACGAACGCGCCGAGGGCCGCCACUCCACCGGCGGCAUGGA CGAGCUGUACAAGAAAAGCUUAGGUUCCGCCCAUAUAGUUAUGGUGGACGCCUAUAA ACCCACUAAGAGUCGACGGCCCGGGAUCCUGUACAAGUAAAGCGGCCGCUCUAGAUAAA UGAUAGCUUGAUCUGGUUACCACUAAACCAGCCUCAAGAACACCCGAAUGGAGUCUCUAAGCUA CAUAAUACCAACUUACACUUUACAAAAUGUUGUCCCCCAAAAUGUAGCCAUUCGUAUCUGCUCCU AAUAAAAAGAAAGUUUCUUCACAUUCUAAAAAAAAAAAAAAAAAAAAAAAAAAAAAAAAAACCCCC CCCCCCCCCCCCUGCAGGCGCGCCGCCGGCGAUUUAAAUUAAU |
| | > Figure 8B | |
| | >SpyTag001-TAL[hsCENTRO] -h Rec8-mClover-SpyTag001 mRNA | |
| | >mRNA transcript full-length includes 5' and 3' UTRs. | |
| | >Full Length Protein Sequence | |
| 68 | >TALhesin[hsCE *NTRO*](hRec8)-A | MGSAHIVMVDAYKPTKTGTSLYKKAGSAAAPFTMVDLRTLGYSQQQQEKIKPKVRSTVAQHHEALVG HGFTHAHIVALSQHPAALGTVAVKYQDMIAALPEATHEAIVGVGKQWSGARALEALLTVAGELRGPPL QLDTGQLLKIAKRGGVTAVEAVHAWRNALTGAPLNLTPEQVVAIASHDGGKQALETVQRLLPVLCQA HGLTPEQVVAIASHDGGKQALETVQRLLPVLCQAHGLTPEQVVAIASNIGGKQALETVQRLLPVLCQA HGLTPEQVVAIASNGGGKQALETVQRLLPVLCQAHGLTPEQVVAIASNGGGKQALETVQRLLPVLCQA HGLTPEQVVAIASHDGGKQALETVQRLLPVLCQAHGLTPEQVVAIASHDGGKQALETVQRLLPVLCQA HGLTPEQVVAIASNIGGKQALETVQRLLPVLCQAHGLTPEQVVAIASNGGGKQALETVQRLLPVLCQA HGLTPEQVVAIASNGGGKQALETVQRLLPVLCQAHGLTPEQVVAIASHDGGKQALETVQRLLPVLCQA HGLTPEQVVAIASHDGGKQALETVQRLLPVLCQAHGLTPEQVVAIASNIGGKQALETVQRLLPVLCQA HGLTPEQVVAIASNGGGKQALETVQRLLPVLCQAHGLTPEQVVAIASNGGGKQALETVQRLLPVLCQA HGLTPEQVVAIASHDGGKQALETVQRLLPVLCQAHGLTPEQVVAIASHDGGKQALETVQRLLPVLCQA HGLTPEQVVAIASNIGGKQALETVQRLLPVLCQAHGLTPEQVVAIASNGGGRPALESIVAQLSRPDPAL AALTNDHLVALACLGGRPALDAVKKGLPHAPALIKRTNRRIPERTSHRVAKGGRADPAFLYKVVHCAQ PPPKALRRELPEEAAAEEERRKIEVPSEIEVPREALEPSVPLMVSLEISLEAAEEEKSRISLIPPEERWAW PEVEAPEAPALPVVPELPEVPMEMPLVLPPELELLRMVSKGEEDNMAIIKEFMRFKVHMEGSVNGHEFE IEGEGEGRPYEGTQTAKLKVTKGGPLPFAWDILSPQFMYGSKAYVKHPADIPDYLKLSFPEGFKWERV MNFEDGGVVTVTQDSSLQDGEFIYKVKLRGTNFPSDGPVMQKKTMGWEASSERMYPEDGALKGEIK QRLKLKDGGHYDAEVKTTYKAKKPVQLPGAYNVNIKLDITSHNEDYTIVEQYERAEGRHSTGGMDEL YKKSLGSAHIVMVDAYKPTKSRRPGILYK- |
| | > Figure 8B | |
| | >SpyTag001-TAL[hsCENTRO] -h Rec8-mClover-SpyTag001 mRNA | |
| | >Full Length Protein Sequence | |
| 69 | >TALhesin[*hsCE NTRO*](hRec8)-B | GCGAAUUAAUUCGAGCUCGGUACCCAGCUUGCUUGUUCUUUUUGCAGAAGCUCAGAAUAAACGC UCAACUUUGGCAGAUCAAUUCCCCGGUGCUAGCAUGGAUAGUGCUACCCAUAUUAAAUUC UCAAAACGUGAUGAGGACGGCAAAGAGUUAGCUGGUGCAACUAUGGAGUUGCGUGA UUCAUCUGGUAAAACUAUUAGUACAUGGAUUUCAGAUGGACAAGUGAAAGAUUUCU ACCUGUAUCCAGGAAAAUAUACAUUUGUCGAAACCGCAGCACCAGACGGUUAUGAGG UAGCAACUGCUAUUACCUUUACAGUUAAUGAGCAAGGUCAGGUUACUGUAAAUGGCA AAGCAACUAAAGGUGACGCUCAUAUUGGAUCCACCGGUACAAGUUUGUACAAAAAAG CAGGCUCCGCGGCCGCCCCCUUCACCAUGGUAGAUUUGAGAACUUUGGGAUAUUCAC AGCAGCAGCAGGAAAAGAUCAAGCCCAAAGUGAGGUCGACAGUCGCGCAGCAUCACG AAGCGCUGGUGGGUCAUGGGUUUACACAUGCCCACAUCGUAGCCUUGUCGCAGCACC CUGCAGCCCUUGGCACGGUCGCCGUCAAGUACCAGGACAUGAUUGCGGCGUUGCCGG AAGCCACACAUGAGGCGAUCGUCGGUGUGGGGAAACAGUGGAGCGGAGCCCGAGCGC UUGAGGCCCUGUUGACGGUCGCGGGAGAGCUGAGAGGGCCUCCCCUUCAGCUGGAC ACGGGCCAGUUGCUGAAGAUCGCGAAGCGGGGAGGAGUCACGGCGGUCGAGGCGGU GCACGCGUGGCGCAAUGCGCUCACGGGAGCACCCCUCAACCUGACCCCAGAGCAGGU CGUGGCAAUUGCGAGCCAUGACGGGGGAAAGCAGGCACUCGAAACCGUCCAGAGGUU GCUGCCUGUGCUGUGCCAAGCGCACGGACUUACGCCAGAGCAGGUCGUGGCAAUUGC GAGCCAUGACGGGGGAAAGCAGGCACUCGAAACCGUCCAGAGGUUGCUGCCUGUGCU GUGCCAAGCGCACGGACUAACCCCAGAGCAGGUCGUGGCAAUUGCGAGCAACAUCGG GGGAAAGCAGGCACUCGAAACCGUCCAGAGGUUGCUGCCUGUGCUGUGCCAAGCGCA CGGGUUGACCCCAGAGCAGGUCGUGGCAAUUGCGAGCAACGGAGGGGGAAAGCAGG CACUCGAAACCGUCCAGAGGUUGCUGCCUGUGCUGUGCCAAGCGCACGGGUUGACCC CAGAGCAGGUCGUGGCAAUUGCGAGCAACGGAGGGGGAAAGCAGGCACUCGAAACCG UCCAGAGGUUGCUGCCUGUGCUGUGCCAAGCGCACGGACUGACACCAGAGCAGGUCG UGGCAAUUGCGAGCCAUGACGGGGGAAAGCAGGCACUCGAAACCGUCCAGAGGUUGC UGCCUGUGCUGUGCCAAGCGCACGGACUUACACCCGAACAAGUCGUGGCAAUUGCGA GCCAUGACGGGGGAAAGCAGGCACUCGAAACCGUCCAGAGGUUGCUGCCUGUGCUGU GCCAAGCGCACGGACUAACCCCAGAGCAGGUCGUGGCAAUUGCGAGCAACAUCGGGG GAAAGCAGGCACUCGAAACCGUCCAGAGGUUGCUGCCUGUGCUGUGCCAAGCGCACG GGUUGACCCCAGAGCAGGUCGUGGCAAUUGCGAGCAACGGAGGGGGAAAGCAGGCAC UCGAAACCGUCCAGAGGUUGCUGCCUGUGCUGUGCCAAGCGCACGGGUUGACCCCAG AGCAGGUCGUGGCAAUUGCGAGCAACGGAGGGGGAAAGCAGGCACUCGAAACCGUCC AGAGGUUGCUGCCUGUGCUGUGCCAAGCGCACGGACUGACACCAGAGCAGGUCGUG |
| | > Figure 8B | |
| | > SpyCatcher001-TAL[hsCENTRO] -h Rec8-mClover-SpyCatcher001 | |
| | >mRNA transcript full-length includes 5' and 3' UTRs. | |
| | | GCAAUUGCGAGCCAUGACGGGGGAAAGCAGGCACUCGAAACCGUCCAGAGGUUGCUG CCUGUGCUGUGCCAAGCGCACGGACUUACACCCGAACAAGUCGUGGCAAUUGCGAGC CAUGACGGGGGAAAGCAGGCACUCGAAACCGUCCAGAGGUUGCUGCCUGUGCUGUGC CAAGCGCACGGCCUCACCCCAGAGCAGGUCGUGGCAAUUGCGAGCAACAUCGGGGGA AAGCAGGCACUCGAAACCGUCCAGAGGUUGCUGCCUGUGCUGUGCCAAGCGCACGGA CUUACGCCAGAGCAGGUCGUGGCAAUUGCGAGCAACGGAGGGGGAAAGCAGGCACUC GAAACCGUCCAGAGGUUGCUGCCUGUGCUGUGCCAAGCGCACGGACUAACCCCAGAG CAGGUCGUGGCAAUUGCGAGCAACGGAGGGGGAAAGCAGGCACUCGAAACCGUCCAG AGGUUGCUGCCUGUGCUGUGCCAAGCGCACGGGUUGACCCCAGAGCAGGUCGUGGC AAUUGCGAGCCAUGACGGGGGAAAGCAGGCACUCGAAACCGUCCAGAGGUUGCUGCC UGUGCUGUGCCAAGCGCACGGCCUGACCCCAGAGCAGGUCGUGGCAAUUGCGAGCCA UGACGGGGGAAAGCAGGCACUCGAAACCGUCCAGAGGUUGCUGCCUGUGCUGUGCCA AGCGCACGGACUGACACCAGAGCAGGUCGUGGCAAUUGCGAGCAACAUCGGGGGAAA GCAGGCACUCGAAACCGUCCAGAGGUUGCUGCCUGUGCUGUGCCAAGCGCACGGACU CACGCCUGAGCAGGUAGUGGCUAUUGCAUCCAACGGAGGGGGCAGACCCGCACUGGA GUCAAUCGUGGCCCAGCUUUCGAGGCCGGACCCCGCGCUGGCCGCACUCACUAAUGA UCAUCUUGUAGCGCUGGCCUGCCUCGGCGGACGACCCGCCUUGGAUGCGGUGAAGAA GGGGCUCCCGCACGCGCCUGCAUUGAUUAAGCGGACCAACAGAAGGAUUCCCGAGAG GACAUCACAUCGAGUGGCAAAGGGUGGGCGCGCCGACCCAGCUUUCUUGUACAAAGU GGUCUCGAGCUCACAUUGUGCCCAGCCACCCCCAAAAGCCCUCAGGCGAGAGCUGCC UGAGGAGGCAGCCGCUGAGGAGGAAAGGAGAAAGAUUGAAGUUCCAAGUGAGAUUG AGGUCCCGAGGGAGGCCCUGGAGCCCAGUGUUCCCCUUAUGGUGUCUUUAGAGAUC UCCCUAGAGGCAGCUGAAGAGGAGAAGUCCCGCAUCAGCCUCAUCCCACCAGAAGAA CGGUGGGCCUGGCCUGAGGUGGAGGCGCCAGAAGCUCCUGCAUUGCCCGUGGUGCC UGAACUCCCUGAGGUGCCCAUGGAGAUGCCUUUGGUGCUGCCCCCAGAGCUCGAGCU GCUCAUCGAAUUCAUGGUGAGCAAGGGCGAGGAGCUGUUCACCGGGGUGGUGCCCA UCCUGGUCGAGCUGGACGGCGACGUAAACGACCACAAGUUCAGCGUCCGCGGCGAGG GCGAGGGCGAUGCCACCAACGGCAAGCUGACCCUGAAGUUCAUCUGCACCACCGGCA AGCUGCCCGUGCCCUGGCCCACCCUCGUGACCACCUUCGGCUACGGCGUGGCCUGCU UCAGCCGCUACCCCGACCACAUGAAGCAGCACGACUUCUUCAAGUCCGCCAUGCCCGA AGGCUACGUCCAGGAGCGCACCAUCUCUUUCAAGGACGACGGUACCUACAAGACCCG CGCCGAGGUGAAGUUCGAGGGCGACACCCUGGUGAACCGCAUCGAGCUGAAGGGCAU CGACUUCAAGGAGGACGGCAACAUCCUGGGGCACAAGCUGGAGUACAACUUCAACAG CCACAACGUCUAUAUCACGGCCGACAAGCAGAAGAACGGCAUCAAGGCUAACUUCAA GAUCCGCCACAACGUUGAGGACGGCAGCGUGCAGCUCGCCGACCACUACCAGCAGAA CACCCCCAUCGGCGACGGCCCCGUGCUGCUGCCCGACAACCACUACCUGAGCCAUCAG UCCAAGCUGAGCAAAGACCCCAACGAGAAGCGCGAUCACAUGGUCCUGCUGGAGUUC GUGACCGCCGCCGGGAUUACACAUGGCAUGGACGAGCUGUACAAGGUCGACAUGGAU AGUGCUACCCAUAUUAAAUUCUCAAAACGUGAUGAGGACGGCAAAGAGUUAGCUGGU GCAACUAUGGAGUUGCGUGAUUCAUCUGGUAAAACUAUUAGUACAUGGAUUUCAGA UGGACAAGUGAAAGAUUUCUACCUGUAUCCAGGAAAAUAUACAUUUGUCGAAACCGC AGCACCAGACGGUUAUGAGGUAGCAACUGCUAUUACCUUUACAGUUAAUGAGCAAGG UCAGGUUACUGUAAAUGGCAAAGCAACUAAAGGUGACGCUCAUAUUGGAUCCUAAGC GGCCGCUCUAGAUAAAUGAUAGCUUGAUCUGGUUACCACUAAACCAGCCUCAAGAACACCCGAA UGGAGUCUCUAAGCUACAUAAUACCAACUUACACUUUACAAAAUGUUGUCCCCCAAAAUGUAGC CAUUCGUAUCUGCUCCUAAUAAAAAGAAAGUUUCUUCACAUUCUAAAAAAAAAAAAAAAAAAA AAAAAAAAAAAAAAACCCCCCCCCCCCCCCCCUGCAGGCGCGCCGCCGGCGAUUUAAAUUAAU |
| 70 | >TALhesin[*hsCE NTRO*](hRec8)-B | MDSATHIKFSKRDEDGKELAGATMELRDSSGKTISTWISDGQVKDFYLYPGKYTFVETAAPDGYEVA TAITFTVNEQGQVTVNGKATKGDAHIGSTGTSLYKKAGSAAAPFTMVDLRTLGYSQQQQEKIKPKVR STVAQHHEALVGHGFTHAHIVALSQHPAALGTVAVKYQDMIAALPEATHEAIVGVGKQWSGARALEA LLTVAGELRGPPLQLDTGQLLKIAKRGGVTAVEAVHAWRNALTGAPLNLTPEQVVAIASHDGGKQALE TVQRLLPVLCQAHGLTPEQVVAIASHDGGKQALETVQRLLPVLCQAHGLTPEQVVAIASNIGGKQALE TVQRLLPVLCQAHGLTPEQVVAIASNGGGKQALETVQRLLPVLCQAHGLTPEQVVAIASNGGGKQALE TVQRLLPVLCQAHGLTPEQVVAIASHDGGKQALETVQRLLPVLCQAHGLTPEQVVAIASHDGGKQALE TVQRLLPVLCQAHGLTPEQVVAIASNIGGKQALETVQRLLPVLCQAHGLTPEQVVAIASNGGGKQALE TVQRLLPVLCQAHGLTPEQVVAIASNGGGKQALETVQRLLPVLCQAHGLTPEQVVAIASHDGGKQALE TVQRLLPVLCQAHGLTPEQVVAIASHDGGKQALETVQRLLPVLCQAHGLTPEQVVAIASNIGGKQALE TVQRLLPVLCQAHGLTPEQVVAIASNGGGKQALETVQRLLPVLCQAHGLTPEQVVAIASNGGGKQALE TVQRLLPVLCQAHGLTPEQVVAIASHDGGKQALETVQRLLPVLCQAHGLTPEQVVAIASHDGGKQALE TVQRLLPVLCQAHGLTPEQVVAIASNIGGKQALETVQRLLPVLCQAHGLTPEQVVAIASNGGGRPALE SIVAQLSRPDPALAALTNDHLVALACLGGRPALDAVKKGLPHAPALIKRTNRRIPERTSHRVAKGGRAD PAFLYKVVSSSHCAQPPPKALRRELPEEAAAEEERRKIEVPSEIEVPREALEPSVPLMVSLEISLEAAEEE KSRISLIPPEERWAWPEVEAPEAPALPVVPELPEVPMEMPLVLPPELELLIEFMVSKGEELFTGVVPILVE |
| | > Figure 8B | |
| | > SpyCatcher001-TAL[hsCENTRO] -h Rec8-mClover-SpyCatcher001 | |
| | >Full Length Protein Sequence | |
| | | LDGDVNDHKFSVRGEGEGDATNGKLTLKFICTTGKLPVPWPTLVTTFGYGVACFSRYPDHMKQHDFF KSAMPEGYVQERTISFKDDGTYKTRAEVKFEGDTLVNRIELKGIDFKEDGNILGHKLEYNFNSHNVYIT ADKQKNGIKANFKIRHNVEDGSVQLADHYQQNTPIGDGPVLLPDNHYLSHQSKLSKDPNEKRDHMV LLEFVTAAGITHGMDELYKVDMDSATHIKFSKRDEDGKELAGATMELRDSSGKTISTWISDGQVKDF YLYPGKYTFVETAAPDGYEVATAITFTVNEQGQVTVNGKATKGDAHIGS- |
| 71 | >TAL[mmMajSat ] | AUGCAGGUGGAUCUACGCACGCUCGGCUACAGUCAGCAGCAAGAGAAGAUCAAA CCGAAGGUGCGUUCGACAGUGGCGCAGCACCACGAGGCACUGGUGGGCCAUGGGUU UACACACGCGCACAUCGUUGCGCUCAGCCAACACCCGGCAGCGUUAGGGACCGUCGC UGUCACGUAUCAGCACAUAAUCACGGCGUUGCCAGAGGCGACACACGAAGACAUCGU UGGCGUCGGCAAACAGUGGUCCGGCGCACGCGCCCUGGAGGCCUUGCUCACGGAUGC GGGGGAGUUGAGAGGUCCGCCGUUACAGUUGGACACAGGCCAACUUGUGAAGAUUG CAAAACGUGGCGGCGUGACCGCAAUGGAGGCAGUGCAUGCAUCGCGCAAUGCACUGA CGGGUGCCCCCCUGAACCUGACCCCGGACCAAGUGGUGGCUAUCGCCAGCAACGGUG GCGGCAAGCAAGCGCUCGAAACGGUGCAGCGGCUGUUGCCGGUGCUGUGCCAGGACC AUGGCCUGACCCCGGACCAAGUGGUGGCUAUCGCCAGCAACAAGGGCGGCAAGCAAG CGCUCGAAACGGUGCAGCGGCUGUUGCCGGUGCUGUGCCAGGACCAUGGCCUGACUC CGGACCAAGUGGUGGCUAUCGCCAGCCACGAUGGCGGCAAGCAAGCGCUCGAAACGG UGCAGCGGCUGUUGCCGGUGCUGUGCCAGGACCAUGGCCUGACUCCGGACCAAGUG GUGGCUAUCGCCAGCCACGAUGGCGGCAAGCAAGCGCUCGAAACGGUGCAGCGGCUG UUGCCGGUGCUGUGCCAGGACCAUGGCCUGACCCCGGACCAAGUGGUGGCUAUCGCC AGCAACAUUGGCGGCAAGCAAGCGCUCGAAACGGUGCAGCGGCUGUUGCCGGUGCUG UGCCAGGACCAUGGCCUGACCCCGGACCAAGUGGUGGCUAUCGCCAGCAACGGUGGC GGCAAGCAAGCGCUCGAAACGGUGCAGCGGCUGUUGCCGGUGCUGUGCCAGGACCAU GGCCUGACCCCGGACCAAGUGGUGGCUAUCGCCAGCAACAUUGGCGGCAAGCAAGCG CUCGAAACGGUGCAGCGGCUGUUGCCGGUGCUGUGCCAGGACCAUGGCCUGACCCCG GACCAAGUGGUGGCUAUCGCCAGCAACGGUGGCGGCAAGCAAGCGCUCGAAACGGUG CAGCGGCUGUUGCCGGUGCUGUGCCAGGACCAUGGCCUGACCCCGGACCAAGUGGUG GCUAUCGCCAGCAACGGUGGCGGCAAGCAAGCGCUCGAAACGGUGCAGCGGCUGUUG CCGGUGCUGUGCCAGGACCAUGGCCUGACUCCGGACCAAGUGGUGGCUAUCGCCAGC CACGAUGGCGGCAAGCAAGCGCUCGAAACGGUGCAGCGGCUGUUGCCGGUGCUGUG CCAGGACCAUGGCCUGACCCCGGACCAAGUGGUGGCUAUCGCCAGCCACGAUGGCGG CAAGCAAGCGCUCGAAACGGUGCAGCGGCUGUUGCCGGUGCUGUGCCAGGACCAUGG CCUGACCCCGGACCAAGUGGUGGCUAUCGCCAGCAACAUUGGCGGCAAGCAAGCGCU CGAAACGGUGCAGCGGCUGUUGCCGGUGCUGUGCCAGGACCAUGGCCUGACUCCGG ACCAAGUGGUGGCUAUCGCCAGCCACGAUGGCGGCAAGCAAGCGCUCGAAACGGUGC AGCGGCUGUUGCCGGUGCUGUGCCAGGACCAUGGCCUGACCCCGGACCAAGUGGUG GCUAUCGCCAGCAACAAGGGCGGCAAGCAAGCGCUCGAAACGGUGCAGCGGCUGUUG CCGGUGCUGUGCCAGGACCAUGGCCUGACCCCGGACCAAGUGGUGGCUAUCGCCAGC AACGGUGGCGGCAAGCAAGCGCUCGAAAGCAUUGUGGCCCAGCUGAGCCGGCCUGAU CCGGCGUUGGCCGCGUUGACCAACGACCACCUCGUCGCCUUGGCCUGCCUCGGCGGA CGUCCUGCCAUGGAUGCAGUGAAAAAGGGAUUGCCGCACGCGCCGGAAUUGAUCAGA AGAGUCAAUCGCCGUAUUGGCGAACGCACGUCCCAUCGCGUUGCGGGAUCCACUGGA GGCGGAGGUGGCUCCGGCACCGCCUCCUCC |
| | >mRNA transcript | |
| 72 | >TAL[mmMajSat ] | MQVDLRTLGYSQQQQEKIKPKVRSTVAQHHEALVGHGFTHAHIVALSQHPAALGTVAVTYQHIITAL PEATHEDIVGVGKQWSGARALEALLTDAGELRGPPLQLDTGQLVKIAKRGGVTAMEAVHASRNALTG APLNLTPDQVVAIASNGGGKQALETVQRLLPVLCQDHGLTPDQVVAIASNKGGKQALETVQRLLPVLC QDHGLTPDQVVAIASHDGGKQALETVQRLLPVLCQDHGLTPDQVVAIASHDGGKQALETVQRLLPVL CQDHGLTPDQVVAIASNIGGKQALETVQRLLPVLCQDHGLTPDQVVAIASNGGGKQALETVQRLLPV LCQDHGLTPDQVVAIASNIGGKQALETVQRLLPVLCQDHGLTPDQVVAIASNGGGKQALETVQRLLP VLCQDHGLTPDQVVAIASNGGGKQALETVQRLLPVLCQDHGLTPDQVVAIASHDGGKQALETVQRLL PVLCQDHGLTPDQVVAIASHDGGKQALETVQRLLPVLCQDHGLTPDQVVAIASNIGGKQALETVQRL LPVLCQDHGLTPDQVVAIASHDGGKQALETVQRLLPVLCQDHGLTPDQVVAIASNKGGKQALETVQR LLPVLCQDHGLTPDQVVAIASNGGGKQALESIVAQLSRPDPALAALTNDHLVALACLGGRPAMDAVKK GLPHAPELIRRVNRRIGERTSHRVAGSTGGGGGSGTASS |
| | > Protein Sequence | |
| 73 | > N-Securin(aa1-101)-mClover-TAL[mmMajSat] | GCGAAUUAAUUCGAGCUCGGUACCCAGCUUGCUUGUUCUUUUUGCAGAAGCUCAGAAUAAACGC UCAACUUUGGCAGAUCAAUUCCCCGGUGCUAGCACAAGUUUGUACAAAAAAGCAGGCUCCGCGG CCGCCCCCUUCACCAUGGCUACUCUUAUCUUUGUUGAUAAGGAUAAUGAAGAACCCGG CCGCCGUUUGGCAUCUAAGGAUGGGUUGAAGCUGGGCACUGGUGUCAAGGCCUUAG AUGGGAAAUUGCAGGUUUCAACGCCUCGAGUCGGCAAAGUGUUCAAUGCUCCAGCCG UGCCUAAAGCCAGCAGAAAGGCUUUGGGGACAGUCAACAGAGUUGCCGAAAAGCCUA UGAAGACUGGCAAACCCCUCCAACCAAAACAGCCGACCUUGACUGGGAAAAAGAUCAC CGAGAAGUCUACUAAGACACAAAGUUCUGUUAAGGGUGGGCGCGCCGACCCAGCUUU CUUGUACAAAGUGGUAAGCUUCGAAUUCAUGGUGAGCAAGGGCGAGGAGCUGUUCA |
| | >mRNA Transcript | |
| | | CCGGGGUGGUGCCCAUCCUGGUCGAGCUGGACGGCGACGUAAACGACCACAAGUUCA GCGUCCGCGGCGAGGGCGAGGGCGAUGCCACCAACGGCAAGCUGACCCUGAAGUUCA UCUGCACCACCGGCAAGCUGCCCGUGCCCUGGCCCACCCUCGUGACCACCUUCGGCU ACGGCGUGGCCUGCUUCAGCCGCUACCCCGACCACAUGAAGCAGCACGACUUCUUCA AGUCCGCCAUGCCCGAAGGCUACGUCCAGGAGCGCACCAUCUCUUUCAAGGACGACG GUACCUACAAGACCCGCGCCGAGGUGAAGUUCGAGGGCGACACCCUGGUGAACCGCA UCGAGCUGAAGGGCAUCGACUUCAAGGAGGACGGCAACAUCCUGGGGCACAAGCUGG AGUACAACUUCAACAGCCACAACGUCUAUAUCACGGCCGACAAGCAGAAGAACGGCAU CAAGGCUAACUUCAAGAUCCGCCACAACGUUGAGGACGGCAGCGUGCAGCUCGCCGA CCACUACCAGCAGAACACCCCCAUCGGCGACGGCCCCGUGCUGCUGCCCGACAACCAC UACCUGAGCCAUCAGUCCAAGCUGAGCAAAGACCCCAACGAGAAGCGCGAUCACAUG GUCCUGCUGGAGUUCGUGACCGCCGCCGGGAUUACACAUGGCAUGGACGAGCUGUA CAAGGUCGACAUGCAGGUGGAUCUACGCACGCUCGGCUACAGUCAGCAGCAGCAAGA GAAGAUCAAACCGAAGGUGCGUUCGACAGUGGCGCAGCACCACGAGGCACUGGUGGG CCAUGGGUUUACACACGCGCACAUCGUUGCGCUCAGCCAACACCCGGCAGCGUUAGG GACCGUCGCUGUCACGUAUCAGCACAUAAUCACGGCGUUGCCAGAGGCGACACACGA AGACAUCGUUGGCGUCGGCAAACAGUGGUCCGGCGCACGCGCCCUGGAGGCCUUGCU CACGGAUGCGGGGGAGUUGAGAGGUCCGCCGUUACAGUUGGACACAGGCCAACUUG UGAAGAUUGCAAAACGUGGCGGCGUGACCGCAAUGGAGGCAGUGCAUGCAUCGCGCA AUGCACUGACGGGUGCCCCCCUGAACCUGACCCCGGACCAAGUGGUGGCUAUCGCCA GCAACGGUGGCGGCAAGCAAGCGCUCGAAACGGUGCAGCGGCUGUUGCCGGUGCUG UGCCAGGACCAUGGCCUGACCCCGGACCAAGUGGUGGCUAUCGCCAGCAACAAGGGC GGCAAGCAAGCGCUCGAAACGGUGCAGCGGCUGUUGCCGGUGCUGUGCCAGGACCAU GGCCUGACUCCGGACCAAGUGGUGGCUAUCGCCAGCCACGAUGGCGGCAAGCAAGCG CUCGAAACGGUGCAGCGGCUGUUGCCGGUGCUGUGCCAGGACCAUGGCCUGACUCCG GACCAAGUGGUGGCUAUCGCCAGCCACGAUGGCGGCAAGCAAGCGCUCGAAACGGUG CAGCGGCUGUUGCCGGUGCUGUGCCAGGACCAUGGCCUGACCCCGGACCAAGUGGUG GCUAUCGCCAGCAACAUUGGCGGCAAGCAAGCGCUCGAAACGGUGCAGCGGCUGUUG CCGGUGCUGUGCCAGGACCAUGGCCUGACCCCGGACCAAGUGGUGGCUAUCGCCAGC AACGGUGGCGGCAAGCAAGCGCUCGAAACGGUGCAGCGGCUGUUGCCGGUGCUGUG CCAGGACCAUGGCCUGACCCCGGACCAAGUGGUGGCUAUCGCCAGCAACAUUGGCGG CAAGCAAGCGCUCGAAACGGUGCAGCGGCUGUUGCCGGUGCUGUGCCAGGACCAUGG CCUGACCCCGGACCAAGUGGUGGCUAUCGCCAGCAACGGUGGCGGCAAGCAAGCGCU CGAAACGGUGCAGCGGCUGUUGCCGGUGCUGUGCCAGGACCAUGGCCUGACCCCGGA CCAAGUGGUGGCUAUCGCCAGCAACGGUGGCGGCAAGCAAGCGCUCGAAACGGUGCA GCGGCUGUUGCCGGUGCUGUGCCAGGACCAUGGCCUGACUCCGGACCAAGUGGUGG CUAUCGCCAGCCACGAUGGCGGCAAGCAAGCGCUCGAAACGGUGCAGCGGCUGUUGC CGGUGCUGUGCCAGGACCAUGGCCUGACCCCGGACCAAGUGGUGGCUAUCGCCAGCC ACGAUGGCGGCAAGCAAGCGCUCGAAACGGUGCAGCGGCUGUUGCCGGUGCUGUGC CAGGACCAUGGCCUGACCCCGGACCAAGUGGUGGCUAUCGCCAGCAACAUUGGCGGC AAGCAAGCGCUCGAAACGGUGCAGCGGCUGUUGCCGGUGCUGUGCCAGGACCAUGGC CUGACUCCGGACCAAGUGGUGGCUAUCGCCAGCCACGAUGGCGGCAAGCAAGCGCUC GAAACGGUGCAGCGGCUGUUGCCGGUGCUGUGCCAGGACCAUGGCCUGACCCCGGAC CAAGUGGUGGCUAUCGCCAGCAACAAGGGCGGCAAGCAAGCGCUCGAAACGGUGCAG CGGCUGUUGCCGGUGCUGUGCCAGGACCAUGGCCUGACCCCGGACCAAGUGGUGGC UAUCGCCAGCAACGGUGGCGGCAAGCAAGCGCUCGAAAGCAUUGUGGCCCAGCUGAG CCGGCCUGAUCCGGCGUUGGCCGCGUUGACCAACGACCACCUCGUCGCCUUGGCCUG CCUCGGCGGACGUCCUGCCAUGGAUGCAGUGAAAAAGGGAUUGCCGCACGCGCCGGA AUUGAUCAGAAGAGUCAAUCGCCGUAUUGGCGAACGCACGUCCCAUCGCGUUGCGGG AUCCACUGGAGGCGGAGGUGGCUCCGGCACCGCCUCCUCCUAAGCGGCCGCUCUAGAUA AAUGAUAGCUUGAUCUGGUUACCACUAAACCAGCCUCAAGAACACCCGAAUGGAGUCUCUAAGC UACAUAAUACCAACUUACACUUUACAAAAUGUUGUCCCCCAAAAUGUAGCCAUUCGUAUCUGCUC CUAAUAAAAAGAAAGUUUCUUCACAUUCUAAAAAAAAAAAAAAAAAAAAAAAAAAAAAAAAAACCC CCCCCCCCCCCCCCUGCAGGCGCGCCGCCGGCGAUUUAAAUUAAU |
| 74 | > N-Securin(aa1-101)-mClover-TAL[mmMajSat] | MATLIFVDKDNEEPGRRLASKDGLKLGTGVKALDGKLQVSTPRVGKVFNAPAVPKASRKALGTVNRVA EKPMKTGKPLQPKQPTLTGKKITEKSTKTQSSVKGGRADPAFLYKVVSFEFMVSKGEELFTGVVPILVE LDGDVNDHKFSVRGEGEGDATNGKLTLKFICTTGKLPVPWPTLVTTFGYGVACFSRYPDHMKQHDFF KSAMPEGYVQERTISFKDDGTYKTRAEVKFEGDTLVNRIELKGIDFKEDGNILGHKLEYNFNSHNVYIT ADKQKNGIKANFKIRHNVEDGSVQLADHYQQNTPIGDGPVLLPDNHYLSHQSKLSKDPNEKRDHMV LLEFVTAAGITHGMDELYKVDMQVDLRTLGYSQQQQEKIKPKVRSTVAQHHEALVGHGFTHAHIVAL SQHPAALGTVAVTYQHIITALPEATHEDIVGVGKQWSGARALEALLTDAGELRGPPLQLDTGQLVKIA KRGGVTAMEAVHASRNALTGAPLNLTPDQVVAIASNGGGKQALETVQRLLPVLCQDHGLTPDQVVAI ASNKGGKQALETVQRLLPVLCQDHGLTPDQVVAIASHDGGKQALETVQRLLPVLCQDHGLTPDQVVA |
| | >Protein Sequence | |
| | | IASHDGGKQALETVQRLLPVLCQDHGLTPDQVVAIASNIGGKQALETVQRLLPVLCQDHGLTPDQVV AIASNGGGKQALETVQRLLPVLCQDHGLTPDQVVAIASNIGGKQALETVQRLLPVLCQDHGLTPDQV VAIASNGGGKQALETVQRLLPVLCQDHGLTPDQVVAIASNGGGKQALETVQRLLPVLCQDHGLTPDQ VVAIASHDGGKQALETVQRLLPVLCQDHGLTPDQVVAIASHDGGKQALETVQRLLPVLCQDHGLTPD QVVAIASNIGGKQALETVQRLLPVLCQDHGLTPDQVVAIASHDGGKQALETVQRLLPVLCQDHGLTP DQVVAIASNKGGKQALETVQRLLPVLCQDHGLTPDQVVAIASNGGGKQALESIVAQLSRPDPALAALT NDHLVALACLGGRPAMDAVKKGLPHAPELIRRVNRRIGERTSHRVAGSTGGGGGSGTASS |
| 75 | > N-Cy-clinB1(aa1-79)-mClover-TAL[mmMajSat] | GCGAAUUAAUUCGAGCUCGGUACCCAGCUUGCUUGUUCUUUUUGCAGAAGCUCAGAAUAAACGC UCAACUUUGGCAGAUCAAUUCCCCGGUGCUAGCACAAGUUUGUACAAAAAAGCAGGCUCCGCGG CCGCCCCCUUCACCAUGGCGCUCAGGGUCACUAGGAACACGAAAAUUAACGCAGAAAAU AAGGCCAAGGUCAGUAUGGCAGGCGCCAAGCGUGUGCCUGUGACAGUUACUGCUGC UUCCAAGCCCGGGCUGAGACCGAGAACUGCUCUUGGAGACAUUGGUAAUAAAGUCAG CGAAGAGCUACAGGCAAGAGUGCCUCUGAAAAGGGAAGCAAAAACGCUAGGUACUGG AAAAGGUACUGUUAAAGCCCUAAAGGGUGGGCGCGCCGACCCAGCUUUCUUGUACAA AGUGGUAAGCUUCGAAUUCAUGGUGAGCAAGGGCGAGGAGCUGUUCACCGGGGUGG UGCCCAUCCUGGUCGAGCUGGACGGCGACGUAAACGACCACAAGUUCAGCGUCCGCG GCGAGGGCGAGGGCGAUGCCACCAACGGCAAGCUGACCCUGAAGUUCAUCUGCACCA CCGGCAAGCUGCCCGUGCCCUGGCCCACCCUCGUGACCACCUUCGGCUACGGCGUGG CCUGCUUCAGCCGCUACCCCGACCACAUGAAGCAGCACGACUUCUUCAAGUCCGCCAU GCCCGAAGGCUACGUCCAGGAGCGCACCAUCUCUUUCAAGGACGACGGUACCUACAA GACCCGCGCCGAGGUGAAGUUCGAGGGCGACACCCUGGUGAACCGCAUCGAGCUGAA GGGCAUCGACUUCAAGGAGGACGGCAACAUCCUGGGGCACAAGCUGGAGUACAACUU CAACAGCCACAACGUCUAUAUCACGGCCGACAAGCAGAAGAACGGCAUCAAGGCUAAC UUCAAGAUCCGCCACAACGUUGAGGACGGCAGCGUGCAGCUCGCCGACCACUACCAG CAGAACACCCCCAUCGGCGACGGCCCCGUGCUGCUGCCCGACAACCACUACCUGAGCC AUCAGUCCAAGCUGAGCAAAGACCCCAACGAGAAGCGCGAUCACAUGGUCCUGCUGG AGUUCGUGACCGCCGCCGGGAUUACACAUGGCAUGGACGAGCUGUACAAGGUCGACA UGCAGGUGGAUCUACGCACGCUCGGCUACAGUCAGCAGCAGCAAGAGAAGAUCAAAC CGAAGGUGCGUUCGACAGUGGCGCAGCACCACGAGGCACUGGUGGGCCAUGGGUUU ACACACGCGCACAUCGUUGCGCUCAGCCAACACCCGGCAGCGUUAGGGACCGUCGCU GUCACGUAUCAGCACAUAAUCACGGCGUUGCCAGAGGCGACACACGAAGACAUCGUU GGCGUCGGCAAACAGUGGUCCGGCGCACGCGCCCUGGAGGCCUUGCUCACGGAUGCG GGGGAGUUGAGAGGUCCGCCGUUACAGUUGGACACAGGCCAACUUGUGAAGAUUGC AAAACGUGGCGGCGUGACCGCAAUGGAGGCAGUGCAUGCAUCGCGCAAUGCACUGAC GGGUGCCCCCCUGAACCUGACCCCGGACCAAGUGGUGGCUAUCGCCAGCAACGGUGG CGGCAAGCAAGCGCUCGAAACGGUGCAGCGGCUGUUGCCGGUGCUGUGCCAGGACCA UGGCCUGACCCCGGACCAAGUGGUGGCUAUCGCCAGCAACAAGGGCGGCAAGCAAGC GCUCGAAACGGUGCAGCGGCUGUUGCCGGUGCUGUGCCAGGACCAUGGCCUGACUCC GGACCAAGUGGUGGCUAUCGCCAGCCACGAUGGCGGCAAGCAAGCGCUCGAAACGGU GCAGCGGCUGUUGCCGGUGCUGUGCCAGGACCAUGGCCUGACUCCGGACCAAGUGG UGGCUAUCGCCAGCCACGAUGGCGGCAAGCAAGCGCUCGAAACGGUGCAGCGGCUGU UGCCGGUGCUGUGCCAGGACCAUGGCCUGACCCCGGACCAAGUGGUGGCUAUCGCCA GCAACAUUGGCGGCAAGCAAGCGCUCGAAACGGUGCAGCGGCUGUUGCCGGUGCUG UGCCAGGACCAUGGCCUGACCCCGGACCAAGUGGUGGCUAUCGCCAGCAACGGUGGC GGCAAGCAAGCGCUCGAAACGGUGCAGCGGCUGUUGCCGGUGCUGUGCCAGGACCAU GGCCUGACCCCGGACCAAGUGGUGGCUAUCGCCAGCAACAUUGGCGGCAAGCAAGCG CUCGAAACGGUGCAGCGGCUGUUGCCGGUGCUGUGCCAGGACCAUGGCCUGACCCCG GACCAAGUGGUGGCUAUCGCCAGCAACGGUGGCGGCAAGCAAGCGCUCGAAACGGUG CAGCGGCUGUUGCCGGUGCUGUGCCAGGACCAUGGCCUGACCCCGGACCAAGUGGUG GCUAUCGCCAGCAACGGUGGCGGCAAGCAAGCGCUCGAAACGGUGCAGCGGCUGUUG CCGGUGCUGUGCCAGGACCAUGGCCUGACUCCGGACCAAGUGGUGGCUAUCGCCAGC CACGAUGGCGGCAAGCAAGCGCUCGAAACGGUGCAGCGGCUGUUGCCGGUGCUGUG CCAGGACCAUGGCCUGACCCCGGACCAAGUGGUGGCUAUCGCCAGCCACGAUGGCGG CAAGCAAGCGCUCGAAACGGUGCAGCGGCUGUUGCCGGUGCUGUGCCAGGACCAUGG CCUGACCCCGGACCAAGUGGUGGCUAUCGCCAGCAACAUUGGCGGCAAGCAAGCGCU CGAAACGGUGCAGCGGCUGUUGCCGGUGCUGUGCCAGGACCAUGGCCUGACUCCGG ACCAAGUGGUGGCUAUCGCCAGCCACGAUGGCGGCAAGCAAGCGCUCGAAACGGUGC AGCGGCUGUUGCCGGUGCUGUGCCAGGACCAUGGCCUGACCCCGGACCAAGUGGUG GCUAUCGCCAGCAACAAGGGCGGCAAGCAAGCGCUCGAAACGGUGCAGCGGCUGUUG CCGGUGCUGUGCCAGGACCAUGGCCUGACCCCGGACCAAGUGGUGGCUAUCGCCAGC AACGGUGGCGGCAAGCAAGCGCUCGAAAGCAUUGUGGCCCAGCUGAGCCGGCCUGAU CCGGCGUUGGCCGCGUUGACCAACGACCACCUCGUCGCCUUGGCCUGCCUCGGCGGA CGUCCUGCCAUGGAUGCAGUGAAAAAGGGAUUGCCGCACGCGCCGGAAUUGAUCAGA |
| | >mRNA Transcript | |
| | | AGAGUCAAUCGCCGUAUUGGCGAACGCACGUCCCAUCGCGUUGCGGGAUCCACUGGA GGCGGAGGUGGCUCCGGCACCGCCUCCUCCUAAGCGGCCGCUCUAGAUAAAUGAUAGCUU GAUCUGGUUACCACUAAACCAGCCUCAAGAACACCCGAAUGGAGUCUCUAAGCUACAUAAUACCA ACUUACACUUUACAAAAUGUUGUCCCCCAAAAUGUAGCCAUUCGUAUCUGCUCCUAAUAAAAAGA AAGUUUCUUCACAUUCUAAAAAAAAAAAAAAAAAAAAAAAAAAAAAAAAAAAAAACCCCCCCCCCCCC CCUGCAGGCGCGCCGCCGGCGAUUUAAAUUAAU |
| 76 | > N-Cy-clinB1(aa1-79)-mClover-TAL[mmMajSat] | MALRVTRNTKINAENKAKVSMAGAKRVPVTVTAASKPGLRPRTALGDIGNKVSEELQARVPLKREAKT LGTGKGTVKALKGGRADPAFLYKVVSFEFMVSKGEELFTGVVPILVELDGDVNDHKFSVRGEGEGDAT NGKLTLKFICTTGKLPVPWPTLVTTFGYGVACFSRYPDHMKQHDFFKSAMPEGYVQERTISFKDDGT YKTRAEVKFEGDTLVNRIELKGIDFKEDGNILGHKLEYNFNSHNVYITADKQKNGIKANFKIRHNVEDG SVQLADHYQQNTPIGDGPVLLPDNHYLSHQSKLSKDPNEKRDHMVLLEFVTAAGITHGMDELYKVD MQVDLRTLGYSQQQQEKIKPKVRSTVAQHHEALVGHGFTHAHIVALSQHPAALGTVAVTYQHIITAL PEATHEDIVGVGKQWSGARALEALLTDAGELRGPPLQLDTGQLVKIAKRGGVTAMEAVHASRNALTG APLNLTPDQVVAIASNGGGKQALETVQRLLPVLCQDHGLTPDQVVAIASNKGGKQALETVQRLLPVLC QDHGLTPDQVVAIASHDGGKQALETVQRLLPVLCQDHGLTPDQVVAIASHDGGKQALETVQRLLPVL CQDHGLTPDQVVAIASNIGGKQALETVQRLLPVLCQDHGLTPDQVVAIASNGGGKQALETVQRLLPV LCQDHGLTPDQVVAIASNIGGKQALETVQRLLPVLCQDHGLTPDQVVAIASNGGGKQALETVQRLLP VLCQDHGLTPDQVVAIASNGGGKQALETVQRLLPVLCQDHGLTPDQVVAIASHDGGKQALETVQRLL PVLCQDHGLTPDQVVAIASHDGGKQALETVQRLLPVLCQDHGLTPDQVVAIASNIGGKQALETVQRL LPVLCQDHGLTPDQVVAIASHDGGKQALETVQRLLPVLCQDHGLTPDQVVAIASNKGGKQALETVQR LLPVLCQDHGLTPDQVVAIASNGGGKQALESIVAQLSRPDPALAALTNDHLVALACLGGRPAMDAVKK GLPHAPELIRRVNRRIGERTSHRVAGSTGGGGGSGTASS |
| | > Protein Sequence | |
| 77 | > SpyTag-NS34A(CS)-TAL[mmMajSat] -NS34A(CS)-mCherry-SpyTag | GCGAAUUAAUUCGAGCUCGGUACCCAGCUUGCUUGUUCUUUUUGCAGAAGCUCAGAAUAAACGC UCAACUUUGGCAGAUCAAUUCCCCGGUGCUAGCAUGGGUUCCGCCCACAUCGUGAUGGUG GACGCCUACAAGCCGACGAAGACCGGUGGUUCCAGUUCUGACACCGAGGACGUGGUG UGCUGCAGCAUGAGCUACACCUGGACCGGCAAGACUACAAGUUUGUACAAAAAAGCA GGCUCCGCGGCCGCCCCCUUCACCAUGCAGGUGGAUCUACGCACGCUCGGCUACAGU CAGCAGCAGCAAGAGAAGAUCAAACCGAAGGUGCGUUCGACAGUGGCGCAGCACCAC GAGGCACUGGUGGGCCAUGGGUUUACACACGCGCACAUCGUUGCGCUCAGCCAACAC CCGGCAGCGUUAGGGACCGUCGCUGUCACGUAUCAGCACAUAAUCACGGCGUUGCCA GAGGCGACACACGAAGACAUCGUUGGCGUCGGCAAACAGUGGUCCGGCGCACGCGCC CUGGAGGCCUUGCUCACGGAUGCGGGGGAGUUGAGAGGUCCGCCGUUACAGUUGGA CACAGGCCAACUUGUGAAGAUUGCAAAACGUGGCGGCGUGACCGCAAUGGAGGCAGU GCAUGCAUCGCGCAAUGCACUGACGGGUGCCCCCCUGAACCUGACCCCGGACCAAGU GGUGGCUAUCGCCAGCAACGGUGGCGGCAAGCAAGCGCUCGAAACGGUGCAGCGGCU GUUGCCGGUGCUGUGCCAGGACCAUGGCCUGACCCCGGACCAAGUGGUGGCUAUCGC CAGCAACAAGGGCGGCAAGCAAGCGCUCGAAACGGUGCAGCGGCUGUUGCCGGUGCU GUGCCAGGACCAUGGCCUGACUCCGGACCAAGUGGUGGCUAUCGCCAGCCACGAUGG CGGCAAGCAAGCGCUCGAAACGGUGCAGCGGCUGUUGCCGGUGCUGUGCCAGGACCA UGGCCUGACUCCGGACCAAGUGGUGGCUAUCGCCAGCCACGAUGGCGGCAAGCAAGC GCUCGAAACGGUGCAGCGGCUGUUGCCGGUGCUGUGCCAGGACCAUGGCCUGACCCC GGACCAAGUGGUGGCUAUCGCCAGCAACAUUGGCGGCAAGCAAGCGCUCGAAACGGU GCAGCGGCUGUUGCCGGUGCUGUGCCAGGACCAUGGCCUGACCCCGGACCAAGUGGU GGCUAUCGCCAGCAACGGUGGCGGCAAGCAAGCGCUCGAAACGGUGCAGCGGCUGUU GCCGGUGCUGUGCCAGGACCAUGGCCUGACCCCGGACCAAGUGGUGGCUAUCGCCAG CAACAUUGGCGGCAAGCAAGCGCUCGAAACGGUGCAGCGGCUGUUGCCGGUGCUGU GCCAGGACCAUGGCCUGACCCCGGACCAAGUGGUGGCUAUCGCCAGCAACGGUGGCG GCAAGCAAGCGCUCGAAACGGUGCAGCGGCUGUUGCCGGUGCUGUGCCAGGACCAUG GCCUGACCCCGGACCAAGUGGUGGCUAUCGCCAGCAACGGUGGCGGCAAGCAAGCGC UCGAAACGGUGCAGCGGCUGUUGCCGGUGCUGUGCCAGGACCAUGGCCUGACUCCG GACCAAGUGGUGGCUAUCGCCAGCCACGAUGGCGGCAAGCAAGCGCUCGAAACGGUG CAGCGGCUGUUGCCGGUGCUGUGCCAGGACCAUGGCCUGACCCCGGACCAAGUGGUG GCUAUCGCCAGCCACGAUGGCGGCAAGCAAGCGCUCGAAACGGUGCAGCGGCUGUUG CCGGUGCUGUGCCAGGACCAUGGCCUGACCCCGGACCAAGUGGUGGCUAUCGCCAGC AACAUUGGCGGCAAGCAAGCGCUCGAAACGGUGCAGCGGCUGUUGCCGGUGCUGUG CCAGGACCAUGGCCUGACUCCGGACCAAGUGGUGGCUAUCGCCAGCCACGAUGGCGG CAAGCAAGCGCUCGAAACGGUGCAGCGGCUGUUGCCGGUGCUGUGCCAGGACCAUGG CCUGACCCCGGACCAAGUGGUGGCUAUCGCCAGCAACAAGGGCGGCAAGCAAGCGCU CGAAACGGUGCAGCGGCUGUUGCCGGUGCUGUGCCAGGACCAUGGCCUGACCCCGGA CCAAGUGGUGGCUAUCGCCAGCAACGGUGGCGGCAAGCAAGCGCUCGAAAGCAUUGU GGCCCAGCUGAGCCGGCCUGAUCCGGCGUUGGCCGCGUUGACCAACGACCACCUCGU CGCCUUGGCCUGCCUCGGCGGACGUCCUGCCAUGGAUGCAGUGAAAAAGGGAUUGCC GCACGCGCCGGAAUUGAUCAGAAGAGUCAAUCGCCGUAUUGGCGAACGCACGUCCCA |
| | >mRNA Transcript | |
| | | UCGCGUUGCGGGAUCCACUGGAGGCGGAGGUGGCUCCGGCACCGCCUCCUCCAAGGG UGGGCGCGCCGACCCAGCUUUCUUGUACAAAGUGGUUGACACUGAGGAUGUAGUAU GCUGUAGCAUGAGUUACACUUGGACAGGCAAGGGAUCAAGUUCAUCCUCGAGAAUG GUGAGCAAGGGCGAGGAGGAUAACAUGGCCAUCAUCAAGGAGUUCAUGCGCUUCAAG GUGCACAUGGAGGGCUCCGUGAACGGCCACGAGUUCGAGAUCGAGGGCGAGGGCGA GGGCCGCCCCUACGAGGGCACCCAGACCGCCAAGCUGAAGGUGACCAAGGGUGGCCC CCUGCCCUUCGCCUGGGACAUCCUGUCCCCUCAGUUCAUGUACGGCUCCAAGGCCUA CGUGAAGCACCCCGCCGACAUCCCCGACUACUUGAAGCUGUCCUUCCCCGAGGGCUU CAAGUGGGAGCGCGUGAUGAACUUCGAGGACGGCGGCGUGGUGACCGUGACCCAGG ACUCCUCCCUGCAGGACGGCGAGUUCAUCUACAAGGUGAAGCUGCGCGGCACCAACU UCCCCUCCGACGGCCCCGUAAUGCAGAAGAAGACCAUGGGCUGGGAGGCCUCCUCCG AGCGGAUGUACCCCGAGGACGGCGCCCUGAAGGGCGAGAUCAAGCAGAGGCUGAAGC UGAAGGACGGCGGCCACUACGACGCUGAGGUCAAGACCACCUACAAGGCCAAGAAGC CCGUGCAGCUGCCCGGCGCCUACAACGUCAACAUCAAGUUGGACAUCACCUCCCACAA CGAGGACUACACCAUCGUGGAACAGUACGAACGCGCCGAGGGCCGCCACUCCACCGG CGGCAUGGACGAGCUGUACAAGAAAAGCUUAGGUUCCGCCCAUAUAGUUAUGGUGG ACGCCUAUAAACCCACUAAGAGUCGACGGCCCGGGAUCCUGUACAAGUAAAGCGGCCG CUCUAGAUAAAUGAUAGCUUGAUCUGGUUACCACUAAACCAGCCUCAAGAACACCCGAAUGGAG UCUCUAAGCUACAUAAUACCAACUUACACUUUACAAAAUGUUGUCCCCCAAAAUGUAGCCAUUCG UAUCUGCUCCUAAUAAAAAGAAAGUUUCUUCACAUUCUAAAAAAAAAAAAAAAAAAAAAAAAA AAAAAAAAACCCCCCCCCCCCCCCCCUGCAGGCGCGCCGCCGGCGAUUUAAAUUAAU |
| 78 | > SpyTag-NS34A(CS)-TAL[mmMajSat] -NS34A(CS)-mCherry-SpyTag | MGSAHIVMVDAYKPTKTGGSSSDTEDVVCCSMSYTWTGKTTSLYKKAGSAAAPFTMQVDLRTLGYS QQQQEKIKPKVRSTVAQHHEALVGHGFTHAHIVALSQHPAALGTVAVTYQHIITALPEATHEDIVGVG KQWSGARALEALLTDAGELRGPPLQLDTGQLVKIAKRGGVTAMEAVHASRNALTGAPLNLTPDQVVA IASNGGGKQALETVQRLLPVLCQDHGLTPDQVVAIASNKGGKQALETVQRLLPVLCQDHGLTPDQVV AIASHDGGKQALETVQRLLPVLCQDHGLTPDQVVAIASHDGGKQALETVQRLLPVLCQDHGLTPDQV VAIASNIGGKQALETVQRLLPVLCQDHGLTPDQVVAIASNGGGKQALETVQRLLPVLCQDHGLTPDQ VVAIASNIGGKQALETVQRLLPVLCQDHGLTPDQVVAIASNGGGKQALETVQRLLPVLCQDHGLTPD QVVAIASNGGGKQALETVQRLLPVLCQDHGLTPDQVVAIASHDGGKQALETVQRLLPVLCQDHGLTP DQVVAIASHDGGKQALETVQRLLPVLCQDHGLTPDQVVAIASNIGGKQALETVQRLLPVLCQDHGLT PDQVVAIASHDGGKQALETVQRLLPVLCQDHGLTPDQVVAIASNKGGKQALETVQRLLPVLCQDHGL TPDQVVAIASNGGGKQALESIVAQLSRPDPALAALTNDHLVALACLGGRPAMDAVKKGLPHAPELIRR VNRRIGERTSHRVAGSTGGGGGSGTASSKGGRADPAFLYKVVDTEDVVCCSMSYTWTGKGSSSSSR MVSKGEEDNMAIIKEFMRFKVHMEGSVNGHEFEIEGEGEGRPYEGTQTAKLKVTKGGPLPFAWDILS PQFMYGSKAYVKHPADIPDYLKLSFPEGFKWERVMNFEDGGVVTVTQDSSLQDGEFIYKVKLRGTNF PSDGPVMQKKTMGWEASSERMYPEDGALKGEIKQRLKLKDGGHYDAEVKTTYKAKKPVQLPGAYNV NIKLDITSHNEDYTIVEQYERAEGRHSTGGMDELYKKSLGSAHIVMVDAYKPTKSRRPGILYK |
| | > Protein Sequence | |
| 79 | > SpyCatcher-NS34A(CS)-TAL[mmMajSat] -NS34A(CS)-mClover-SpyCatcher | GUUUUCCCAGUCACGACGUUGUAAAACGACGGCCAGUGAAUUGUAAUACGACUCACUAUAGGGC GAAUUAAUUCGAGCUCGGUACCCAGCUUGCUUGUUCUUUUUGCAGAAGCUCAGAAUAAACGCUC AACUUUGGCAGAUCAAUUCCCCGGUGCUAGCAUGGAUAGUGCUACCCAUAUUAAAUUCUC AAAACGUGAUGAGGACGGCAAAGAGUUAGCUGGUGCAACUAUGGAGUUGCGUGAUU CAUCUGGUAAAACUAUUAGUACAUGGAUUUCAGAUGGACAAGUGAAAGAUUUCUACC UGUAUCCAGGAAAAUAUACAUUUGUCGAAACCGCAGCACCAGACGGUUAUGAGGUAG CAACUGCUAUUACCUUUACAGUUAAUGAGCAAGGUCAGGUUACUGUAAAUGGCAAAG CAACUAAAGGUGACGCUCAUAUUGGAUCCACCGGUGGUUCCAGUUCUGACACCGAGG ACGUGGUGUGCUGCAGCAUGAGCUACACCUGGACCGGCAAGACUACAAGUUUGUACA AAAAAGCAGGCUCCGCGGCCGCCCCCUUCACCAUGCAGGUGGAUCUACGCACGCUCG GCUACAGUCAGCAGCAGCAAGAGAAGAUCAAACCGAAGGUGCGUUCGACAGUGGCGC AGCACCACGAGGCACUGGUGGGCCAUGGGUUUACACACGCGCACAUCGUUGCGCUCA GCCAACACCCGGCAGCGUUAGGGACCGUCGCUGUCACGUAUCAGCACAUAAUCACGG CGUUGCCAGAGGCGACACACGAAGACAUCGUUGGCGUCGGCAAACAGUGGUCCGGCG CACGCGCCCUGGAGGCCUUGCUCACGGAUGCGGGGGAGUUGAGAGGUCCGCCGUUA CAGUUGGACACAGGCCAACUUGUGAAGAUUGCAAAACGUGGCGGCGUGACCGCAAUG GAGGCAGUGCAUGCAUCGCGCAAUGCACUGACGGGUGCCCCCCUGAACCUGACCCCG GACCAAGUGGUGGCUAUCGCCAGCAACGGUGGCGGCAAGCAAGCGCUCGAAACGGUG CAGCGGCUGUUGCCGGUGCUGUGCCAGGACCAUGGCCUGACCCCGGACCAAGUGGUG GCUAUCGCCAGCAACAAGGGCGGCAAGCAAGCGCUCGAAACGGUGCAGCGGCUGUUG CCGGUGCUGUGCCAGGACCAUGGCCUGACUCCGGACCAAGUGGUGGCUAUCGCCAGC CACGAUGGCGGCAAGCAAGCGCUCGAAACGGUGCAGCGGCUGUUGCCGGUGCUGUG CCAGGACCAUGGCCUGACUCCGGACCAAGUGGUGGCUAUCGCCAGCCACGAUGGCGG CAAGCAAGCGCUCGAAACGGUGCAGCGGCUGUUGCCGGUGCUGUGCCAGGACCAUGG CCUGACCCCGGACCAAGUGGUGGCUAUCGCCAGCAACAUUGGCGGCAAGCAAGCGCU CGAAACGGUGCAGCGGCUGUUGCCGGUGCUGUGCCAGGACCAUGGCCUGACCCCGGA |
| | >mRNA Transcript | |
| | | CCAAGUGGUGGCUAUCGCCAGCAACGGUGGCGGCAAGCAAGCGCUCGAAACGGUGCA GCGGCUGUUGCCGGUGCUGUGCCAGGACCAUGGCCUGACCCCGGACCAAGUGGUGG CUAUCGCCAGCAACAUUGGCGGCAAGCAAGCGCUCGAAACGGUGCAGCGGCUGUUGC CGGUGCUGUGCCAGGACCAUGGCCUGACCCCGGACCAAGUGGUGGCUAUCGCCAGCA ACGGUGGCGGCAAGCAAGCGCUCGAAACGGUGCAGCGGCUGUUGCCGGUGCUGUGC CAGGACCAUGGCCUGACCCCGGACCAAGUGGUGGCUAUCGCCAGCAACGGUGGCGGC AAGCAAGCGCUCGAAACGGUGCAGCGGCUGUUGCCGGUGCUGUGCCAGGACCAUGGC CUGACUCCGGACCAAGUGGUGGCUAUCGCCAGCCACGAUGGCGGCAAGCAAGCGCUC GAAACGGUGCAGCGGCUGUUGCCGGUGCUGUGCCAGGACCAUGGCCUGACCCCGGAC CAAGUGGUGGCUAUCGCCAGCCACGAUGGCGGCAAGCAAGCGCUCGAAACGGUGCAG CGGCUGUUGCCGGUGCUGUGCCAGGACCAUGGCCUGACCCCGGACCAAGUGGUGGC UAUCGCCAGCAACAUUGGCGGCAAGCAAGCGCUCGAAACGGUGCAGCGGCUGUUGCC GGUGCUGUGCCAGGACCAUGGCCUGACUCCGGACCAAGUGGUGGCUAUCGCCAGCCA CGAUGGCGGCAAGCAAGCGCUCGAAACGGUGCAGCGGCUGUUGCCGGUGCUGUGCC AGGACCAUGGCCUGACCCCGGACCAAGUGGUGGCUAUCGCCAGCAACAAGGGCGGCA AGCAAGCGCUCGAAACGGUGCAGCGGCUGUUGCCGGUGCUGUGCCAGGACCAUGGCC UGACCCCGGACCAAGUGGUGGCUAUCGCCAGCAACGGUGGCGGCAAGCAAGCGCUCG AAAGCAUUGUGGCCCAGCUGAGCCGGCCUGAUCCGGCGUUGGCCGCGUUGACCAACG ACCACCUCGUCGCCUUGGCCUGCCUCGGCGGACGUCCUGCCAUGGAUGCAGUGAAAA AGGGAUUGCCGCACGCGCCGGAAUUGAUCAGAAGAGUCAAUCGCCGUAUUGGCGAAC GCACGUCCCAUCGCGUUGCGGGAUCCACUGGAGGCGGAGGUGGCUCCGGCACCGCCU CCUCCAAGGGUGGGCGCGCCGACCCAGCUUUCUUGUACAAAGUGGUUGACACUGAGG AUGUAGUAUGCUGUAGCAUGAGUUACACUUGGACAGGCAAGGGAUCAAGUUCAUCC UCGAGCUCAAGCUUCGAAUUCAUGGUGAGCAAGGGCGAGGAGCUGUUCACCGGGGU GGUGCCCAUCCUGGUCGAGCUGGACGGCGACGUAAACGACCACAAGUUCAGCGUCCG CGGCGAGGGCGAGGGCGAUGCCACCAACGGCAAGCUGACCCUGAAGUUCAUCUGCAC CACCGGCAAGCUGCCCGUGCCCUGGCCCACCCUCGUGACCACCUUCGGCUACGGCGU GGCCUGCUUCAGCCGCUACCCCGACCACAUGAAGCAGCACGACUUCUUCAAGUCCGC CAUGCCCGAAGGCUACGUCCAGGAGCGCACCAUCUCUUUCAAGGACGACGGUACCUA CAAGACCCGCGCCGAGGUGAAGUUCGAGGGCGACACCCUGGUGAACCGCAUCGAGCU GAAGGGCAUCGACUUCAAGGAGGACGGCAACAUCCUGGGGCACAAGCUGGAGUACAA CUUCAACAGCCACAACGUCUAUAUCACGGCCGACAAGCAGAAGAACGGCAUCAAGGC UAACUUCAAGAUCCGCCACAACGUUGAGGACGGCAGCGUGCAGCUCGCCGACCACUA CCAGCAGAACACCCCCAUCGGCGACGGCCCCGUGCUGCUGCCCGACAACCACUACCUG AGCCAUCAGUCCAAGCUGAGCAAAGACCCCAACGAGAAGCGCGAUCACAUGGUCCUG CUGGAGUUCGUGACCGCCGCCGGGAUUACACAUGGCAUGGACGAGCUGUACAAGGUC GACAUGGAUAGUGCUACCCAUAUUAAAUUCUCAAAACGUGAUGAGGACGGCAAAGAG UUAGCUGGUGCAACUAUGGAGUUGCGUGAUUCAUCUGGUAAAACUAUUAGUACAUG GAUUUCAGAUGGACAAGUGAAAGAUUUCUACCUGUAUCCAGGAAAAUAUACAUUUGU CGAAACCGCAGCACCAGACGGUUAUGAGGUAGCAACUGCUAUUACCUUUACAGUUAA UGAGCAAGGUCAGGUUACUGUAAAUGGCAAAGCAACUAAAGGUGACGCUCAUAUUG GAUCCUAAGCGGCCGCUCUAGAUAAAUGAUAGCUUGAUCUGGUUACCACUAAACCAGCCUCAA GAACACCCGAAUGGAGUCUCUAAGCUACAUAAUACCAACUUACACUUUACAAAAUGUUGUCCCCC AAAAUGUAGCCAUUCGUAUCUGCUCCUAAUAAAAAGAAAGUUUCUUCACAUUCUAAAAAAAAAAAA AAAAAAAAAAAAAAAAAAAAAACCCCCCCCCCCCCCCCCUGCAGGCGCGCCGCCGGCGAUUUAAA UUAAU |
| 80 | > SpyCatcher-NS34A(CS)-TAL[mmMajSat] -NS34A(CS)-mClover-SpyCatcher | MDSATHIKFSKRDEDGKELAGATMELRDSSGKTISTWISDGQVKDFYLYPGKYTFVETAAPDGYEVA TAITFTVNEQGQVTVNGKATKGDAHIGSTGGSSSDTEDVVCCSMSYTWTGKTTSLYKKAGSAAAPFT MQVDLRTLGYSQQQQEKIKPKVRSTVAQHHEALVGHGFTHAHIVALSQHPAALGTVAVTYQHIITAL PEATHEDIVGVGKQWSGARALEALLTDAGELRGPPLQLDTGQLVKIAKRGGVTAMEAVHASRNALTG APLNLTPDQVVAIASNGGGKQALETVQRLLPVLCQDHGLTPDQVVAIASNKGGKQALETVQRLLPVLC QDHGLTPDQVVAIASHDGGKQALETVQRLLPVLCQDHGLTPDQVVAIASHDGGKQALETVQRLLPVL CQDHGLTPDQVVAIASNIGGKQALETVQRLLPVLCQDHGLTPDQVVAIASNGGGKQALETVQRLLPV LCQDHGLTPDQVVAIASNIGGKQALETVQRLLPVLCQDHGLTPDQVVAIASNGGGKQALETVQRLLP VLCQDHGLTPDQVVAIASNGGGKQALETVQRLLPVLCQDHGLTPDQVVAIASHDGGKQALETVQRLL PVLCQDHGLTPDQVVAIASHDGGKQALETVQRLLPVLCQDHGLTPDQVVAIASNIGGKQALETVQRL LPVLCQDHGLTPDQVVAIASHDGGKQALETVQRLLPVLCQDHGLTPDQVVAIASNKGGKQALETVQR LLPVLCQDHGLTPDQVVAIASNGGGKQALESIVAQLSRPDPALAALTNDHLVALACLGGRPAMDAVKK GLPHAPELIRRVNRRIGERTSHRVAGSTGGGGGSGTASSKGGRADPAFLYKVVDTEDVVCCSMSYTW TGKGSSSSSSSSFEFMVSKGEELFTGVVPILVELDGDVNDHKFSVRGEGEGDATNGKLTLKFICTTGKL PVPWPTLVTTFGYGVACFSRYPDHMKQHDFFKSAMPEGYVQERTISFKDDGTYKTRAEVKFEGDTLV NRIELKGIDFKEDGNILGHKLEYNFNSHNVYITADKQKNGIKANFKIRHNVEDGSVQLADHYQQNTPI GDGPVLLPDNHYLSHQSKLSKDPNEKRDHMVLLEFVTAAGITHGMDELYKVDMDSATHIKFSKRDED |
| | >Protein Sequence | |
| | | GKELAGATMELRDSSGKTISTWISDGQVKDFYLYPGKYTFVETAAPDGYEVATAITFTVNEQGQVTV NGKATKGDAHIGS |

### References

1 Gruhn, J. R. et al. Chromosome errors in human eggs shape natural fertility over reproductive life span. Science 365, 1466-1469, doi:10.1126/science.aav7321 (2019).
2 Herbert, M., Kalleas, D., Cooney, D., Lamb, M. & Lister, L. Meiosis and maternal aging: insights from aneuploid oocytes and trisomy births. Cold Spring Harb Perspect Biol 7, a017970, doi:10.1101/cshperspect.a017970 (2015).
3 Charalambous, C., Webster, A. & Schuh, M. Aneuploidy in mammalian oocytes and the impact of maternal ageing. Nat Rev Mol Cell Biol, doi:10.1038/s41580-022-00517-3 (2022).
4 Kuliev, A., Zlatopolsky, Z., Kirillova, I., Spivakova, J. & Cieslak Janzen, J. Meiosis errors in over 20,000 oocytes studied in the practice of preimplantation aneuploidy testing. Reprod Biomed Online 22, 2-8, doi:10.1016/j.rbmo.2010.08.014 (2011).
5 Hassold, T. & Chiu, D. Maternal age-specific rates of numerical chromosome abnormalities with special reference to trisomy. Hum Genet 70, 11-17, doi:10.1007/BF00389450 (1985).
6 Burkhardt, S. et al. Chromosome Cohesion Established by Rec8-Cohesin in Fetal Oocytes Is Maintained without Detectable Turnover in Oocytes Arrested for Months in Mice. Curr Biol 26, 678-685, doi:10.1016/j.cub.2015.12.073 (2016).
7 Jessberger, R. Deterioration without replenishment--the misery of oocyte cohesin. Genes Dev 24, 2587-2591, doi:10.1101/gad.2000610 (2010).
8 Revenkova, E., Herrmann, K., Adelfalk, C. & Jessberger, R. Oocyte cohesin expression restricted to predictyate stages provides full fertility and prevents aneuploidy. Curr Biol 20, 1529-1533, doi:10.1016/j.cub.2010.08.024 (2010).
9 Tachibana-Konwalski, K. et al. Rec8-containing cohesin maintains bivalents without turnover during the growing phase of mouse oocytes. Genes Dev 24, 2505-2516, doi:10.1101/gad.605910 (2010).
10 Angell, R. R., Xian, J. & Keith, J. Chromosome anomalies in human oocytes in relation to age. Hum Reprod 8, 1047-1054, doi:10.1093joxfordjournals.humrep.a138190 (1993).
11 Howles, C. M., Kim, C. H. & Elder, K. Treatment strategies in assisted reproduction for women of advanced maternal age. Int Surg 91, S37-54 (2006).
12 Chiang, T., Duncan, F. E., Schindler, K., Schultz, R. M. & Lampson, M. A. Evidence that weakened centromere cohesion is a leading cause of age-related aneuploidy in oocytes. Curr Biol 20, 1522-1528, doi:10.1016/j.cub.2010.06.069 (2010).
13 Liu, L. & Keefe, D. L. Defective cohesin is associated with age-dependent misaligned chromosomes in oocytes. Reprod Biomed Online 16, 103-112, doi:10.1016/s1472-6483(10)60562-7 (2008).
14 Lister, L. M. et al. Age-related meiotic segregation errors in mammalian oocytes are preceded by depletion of cohesin and Sgo2. Curr Biol 20, 1511-1521, doi:10.1016/j.cub.2010.08.023 (2010).
15 Merriman, J. A., Jennings, P. C., McLaughlin, E. A. & Jones, K. T. Effect of aging on superovulation efficiency, aneuploidy rates, and sister chromatid cohesion in mice aged up to 15 months. Biol Reprod 86, 49, doi:10.1095/biolreprod.111.095711 (2012).
16 Wolstenholme, J. & Angell, R. R. Maternal age and trisomy--a unifying mechanism of formation. Chromosoma 109, 435-438, doi:10.1007/s004120000088 (2000).
17 Danylevska, A., Kovacovicova, K., Awadova, T. & Anger, M. The frequency of precocious segregation of sister chromatids in mouse female meiosis I is affected by genetic background. Chromosome Res 22, 365-373, doi:10.1007js10577-014-9428-6 (2014).
18 Yun, Y., Lane, S. I. & Jones, K. T. Premature dyad separation in meiosis II is the major segregation error with maternal age in mouse oocytes. Development 141, 199-208, doi:10.1242/dev.100206 (2014).
19 Ottolini, C. S. et al. Genome-wide maps of recombination and chromosome segregation in human oocytes and embryos show selection for maternal recombination rates. Nat Genet 47, 727-735, doi:10.1038/ng.3306 (2015).
20 Zielinska, A. P., Holubcova, Z., Blayney, M., Elder, K. & Schuh, M. Sister kinetochore splitting and precocious disintegration of bivalents could explain the maternal age effect. Elife 4, e11389, doi:10.7554/eLife.11389 (2015).
21 Shomper, M., Lappa, C. & FitzHarris, G. Kinetochore microtubule establishment is defective in oocytes from aged mice. Cell Cycle 13, 1171-1179, doi:10.4161/cc.28046 (2014).
22 Miyanari, Y., Ziegler-Birling, C. & Torres-Padilla, M. E. Live visualization of chromatin dynamics with fluorescent TALEs. Nat Struct Mol Biol 20, 1321-1324, doi:10.1038/nsmb.2680 (2013).
23 Mihajlovic, A. I., Haverfield, J. & FitzHarris, G. Distinct classes of lagging chromosome underpin age-related oocyte aneuploidy in mouse. Dev Cell 56, 2273-2283 e2273, doi:10.1016/j.devcel.2021.07.022 (2021).
24 Zakeri, B. et al. Peptide tag forming a rapid covalent bond to a protein, through engineering a bacterial adhesin. Proc Natl Acad Sci U S A 109, E690-697, doi:10.1073/pnas.1115485109 (2012).
25 Gryaznova, Y. et al. Kinetochore individualization in meiosis I is required for centromeric cohesin removal in meiosis II. EMBO J 40, e106797, doi:10.15252/embj.2020106797 (2021).
26 Ogushi, S. et al. Loss of sister kinetochore co-orientation and peri-centromeric cohesin protection after meiosis I depends on cleavage of centromeric REC8. Dev Cell 56, 3100-3114 e3104, doi:10.1016/j.devcel.2021.10.017 (2021).
27 Holzmann, J. et al. Absolute quantification of cohesin, CTCF and their regulators in human cells. Elife 8, doi:10.7554/eLife.46269 (2019).
28 Duncan, F. E. et al. Chromosome cohesion decreases in human eggs with advanced maternal age. Aging Cell 11, 1121-1124, doi:10.1111/j.1474-9726.2012.00866.x (2012).
29 Clift, D., So, C., McEwan, W. A., James, L. C. & Schuh, M. Acute and rapid degradation of endogenous proteins by Trim-Away. Nat Protoc 13, 2149-2175, doi:10.1038/s41596-018-0028-3 (2018).
30 Ren, R. et al. Visualization of aging-associated chromatin alterations with an engineered TALE system. Cell Res 27, 483-504, doi:10.1038/cr.2017.18 (2017).
31 Keeble, A. H. et al. Evolving Accelerated Amidation by SpyTag/SpyCatcher to Analyze Membrane Dynamics. Angew Chem Int Ed Engl 56, 16521-16525, doi:10.1002/anie.201707623 (2017).
32 McNulty, S. M. & Sullivan, B. A. Alpha satellite DNA biology: finding function in the recesses of the genome. Chromosome Res 26, 115-138, doi:10.1007/s10577-018-9582-3 (2018).
33 Pampalona, J. et al. Chromosome Bridges Maintain Kinetochore-Microtubule Attachment throughout Mitosis and Rarely Break during Anaphase. PLoS One 11, e0147420, doi:10.1371/journal.pone.0147420 (2016).
34 Ma, S. F. et al. Parthenogenetic activation of mouse oocytes by strontium chloride: a search for the best conditions. Theriogenology 64, 1142-1157, doi:10.1016/j.theriogenology.2005.03.002 (2005).
35 Amargant, F. et al. The human sperm basal body is a complex centrosome important for embryo preimplantation development. Mol Hum Reprod 27, doi:10.1093/molehr/gaab062 (2021).
36 Kudo, N. R. et al. Role of cleavage by separase of the Rec8 kleisin subunit of cohesin during mammalian meiosis I. J Cell Sci 122, 2686-2698, doi:10.1242/jcs.035287 (2009).
37 Rosen, L. E. et al. Cohesin cleavage by separase is enhanced by a substrate motif distinct from the cleavage site. Nat Commun 10, 5189, doi:10.1038/s41467-019-13209-y (2019).
38 Ding, Y., Kaido, M., Llano, E., Pendas, A. M. & Kitajima, T. S. The Post-anaphase SUMO Pathway Ensures the Maintenance of Centromeric Cohesion through Meiosis I-II Transition in Mammalian Oocytes. Curr Biol 28, 1661-1669 e1664, doi:10.1016/j.cub.2018.04.019 (2018).
39 Hassold, T. & Hunt, P. Maternal age and chromosomally abnormal pregnancies: what we know and what we wish we knew. Curr Opin Pediatr 21, 703-708, doi:10.1097/MOP.0b013e328332c6ab (2009).
40 Chatzidaki, E. E. et al. Ovulation suppression protects against chromosomal abnormalities in mouse eggs at advanced maternal age. Curr Biol, doi:10.1016/j.cub.2021.06.076 (2021).
41 Patel, J., Tan, S. L., Hartshorne, G. M. & McAinsh, A. D. Unique geometry of sister kinetochores in human oocytes during meiosis I may explain maternal age-associated increases in chromosomal abnormalities. Biol Open 5, 178-184, doi:10.1242/bio.016394 (2015).
42 Sakakibara, Y. et al. Bivalent separation into univalents precedes age-related meiosis I errors in oocytes. Nat Commun 6, 7550, doi:10.1038/ncomms8550 (2015).
43 So, C. et al. Mechanism of spindle pole organization and instability in human oocytes. Science 375, eabj3944, doi:10.1126/science.abj3944 (2022).
44 Liman, E. R., Tytgat, J. & Hess, P. Subunit stoichiometry of a mammalian K+ channel determined by construction of multimeric cDNAs. Neuron 9, 861-871, doi:10.1016/0896-6273(92)90239-a (1992).
45 Maier, N. K., Ma, J., Lampson, M. A. & Cheeseman, I. M. Separase cleaves the kinetochore protein Meikin at the meiosis I/II transition. Dev Cell, doi:10.1016/j.devcel.2021.06.019 (2021).
46 Schuh, M. & Ellenberg, J. Self-organization of MTOCs replaces centrosome function during acentrosomal spindle assembly in live mouse oocytes. Cell 130, 484-498, doi:10.1016/j.cell.2007.06.025 (2007).
47 Eijpe, M., Offenberg, H., Jessberger, R., Revenkova, E. & Heyting, C. Meiotic cohesin REC8 marks the axial elements of rat synaptonemal complexes before cohesins SMC1beta and SMC3. J Cell Biol 160, 657-670, doi:10.1083/jcb.200212080 (2003).
48 Webster, A. et al. Aub and Ago3 Are Recruited to Nuage through Two Mechanisms to Form a Ping-Pong Complex Assembled by Krimper. Mol Cell 59, 564-575, doi:10.1016/j.molcel.2015.07.017 (2015).
49 Webster, A. & Schuh, M. Mechanisms of Aneuploidy in Human Eggs. Trends Cell Biol 27, 55-68, doi:10.1016/j.tcb.2016.09.002 (2017).
50 Mak, A. N., Bradley, P., Cernadas, R. A., Bogdanove, A. J. & Stoddard, B. L. The crystal structure of TAL effector PthXo1 bound to its DNA target. Science 335, 716-719, doi:10.1126/science.1216211 (2012).
51 Li, L., Fierer, J. O., Rapoport, T. A. & Howarth, M. Structural analysis and optimization of the covalent association between SpyCatcher and a peptide Tag. J Mol Biol 426, 309-317, doi:10.1016/j.jmb.2013.10.021 (2014).
52 Pinto, F., Thornton, E.L. & Wang, B. An expanded library of orthogonal split inteins enables modular multi-peptide assemblies. Nat Commun 11, 1529 (2020).
53 Hartzell E.J., Terr J., and Chen W. Engineering a Blue Light Inducible SpyTag System (BLISS). Journal of the American Chemical Society 2021 143 (23), 8572-8577.
54 Stanton BZ, Chory EJ, Crabtree GR. Chemically induced proximity in biology and medicine. Science. 2018 Mar 9;359(6380):eaao5902.
55 Lesne, J., Chang, HJ., De Visch, A. et al. Structural basis for chemically-induced homodimerization of a single domain antibody. Sci Rep 9, 1840 (2019).
56 Karginov AV, Zou Y, Shirvanyants D, Kota P, Dokholyan NV, Young DD, Hahn KM, Deiters A. Light regulation of protein dimerization and kinase activity in living cells using photocaged rapamycin and engineered FKBP. J Am Chem Soc. 2011 Jan 26;133(3):420-3.
57 Kudo NR, Anger M, Peters AH, Stemmann O, Theussl HC, Helmhart W, Kudo H, Heyting C, Nasmyth K. Role of cleavage by separase of the Rec8 kleisin subunit of cohesin during mammalian meiosis I. J Cell Sci. 2009 Aug 1;122(Pt 15):2686-98.
58 Békés M, Langley DR, Crews CM. PROTAC targeted protein degraders: the past is prologue. Nat Rev Drug Discov. 2022 Mar;21(3):181-200. doi: 10.1038/s41573-021-00371-6. Epub 2022 Jan 18.
59 Zimmermann M, Cal R, Janett E, Hoffmann V, Bochet CG, Constable E, Beaufils F, Wymann MP. Cell-permeant and photocleavable chemical inducer of dimerization. Angew Chem Int Ed Engl. 2014 Apr 25;53(18):4717-20.
60 Chen X., Wu Y.-W., Angew. Chem. Int. Ed. 2018, 57, 6796.
61 Kang S, Davidsen K, Gomez-Castillo L, Jiang H, Fu X, Li Z, Liang Y, Jahn M, Moussa M, DiMaio F, Gu L. COMBINES-CID: An Efficient Method for De Novo Engineering of Highly Specific Chemically Induced Protein Dimerization Systems. J Am Chem Soc. 2019 Jul 17;141(28):10948-10952.
62 Guntas G, Hallett RA, Zimmerman SP, Williams T, Yumerefendi H, Bear JE, Kuhlman B. Engineering an improved light-induced dimer (iLID) for controlling the localization and activity of signaling proteins. Proc Natl Acad Sci USA. 2015 Jan 6;112(1):112-7. doi: 10.1073/pnas.1417910112. Epub 2014 Dec 22.
63 Thomas C, Wetherall B, Levasseur MD, Harris RJ, Kerridge ST, Higgins JMG, Davies OR, Madgwick S. A prometaphase mechanism of securin destruction is essential for meiotic progression in mouse oocytes. Nat Commun. 2021 Jul 14;12(1):4322.
64 Levasseur MD, Thomas C, Davies OR, Higgins JMG, Madgwick S. Aneuploidy in Oocytes Is Prevented by Sustained CDK1 Activity through Degron Masking in Cyclin B1. Dev Cell. 2019 Mar 11;48(5):672-684.e5.
65 Lamarre D, Anderson PC, Bailey M, Beaulieu P, Bolger G, Bonneau P, Bös M, Cameron DR, Cartier M, Cordingley MG, Faucher AM, Goudreau N, Kawai SH, Kukolj G, Lagacé L, LaPlante SR, Narjes H, Poupart MA, Rancourt J, Sentjens RE, St George R, Simoneau B, Steinmann G, Thibeault D, Tsantrizos YS, Weldon SM, Yong CL, Llinàs-Brunet M. An NS3 protease inhibitor with antiviral effects in humans infected with hepatitis C virus. Nature. 2003 Nov 13;426(6963):186-9. doi: 10.1038/nature02099. Epub 2003 Oct 26. Erratum in: Nature. 2003 Nov 20;246.
66 Boni A, Politi AZ, Strnad P, Xiang W, Hossain MJ, Ellenberg J. Live imaging and modeling of inner nuclear membrane targeting reveals its molecular requirements in mammalian cells. J Cell Biol. 2015 Jun 8;209(5):705-20.
67 Ballister ER, Aonbangkhen C, Mayo AM, Lampson MA, Chenoweth DM. Localized light-induced protein dimerization in living cells using a photocaged dimerizer. Nat Commun. 2014 Nov 17;5:5475.

## Claims

1. A complex suitable for cohesion of chromatids or chromosomes comprising (I) one or more first protein(s) and (II) one or more second protein(s),
wherein the (I) first and the (II) second protein(s) each comprise (**i**) a chromatin-binding component, (**ii**) a protein-binding region being N-terminal of the chromatin-binding component (PBN region), (**iii**) a protein-binding region being C-terminal of the chromatin-binding component (PBC region);
wherein (i) the chromatin binding components of the first and the second protein(s) are capable of binding to a target sequence on chromatids or chromosomes; and
wherein (I)(ii) the PBN region of the first protein(s) is/are capable of binding to (II)(ii) the PBN region of the second protein(s) and wherein (I)(iii) the PBC region of the first protein(s) is/are capable of binding to (II)(iii) the PBC region of the second protein(s), or
wherein (I)(ii) the PBN region of the first protein(s) is/are capable of binding to (II)(iii) the PBC region of the second protein(s) and wherein (I)(iii) the PBC region of the first protein(s) is/are capable of binding to (II)(ii) the PBN region of the second protein(s); and
wherein the binding to the chromatids or chromosomes and the binding of the PBN and PBC regions of the first and second protein(s) reversibly tether chromatids or chromosomes.

2. The complex of claim 1, wherein (I)(ii) the PBN region of the first protein(s) is/are capable of binding to (II)(ii) the PBN region of the second protein(s) and wherein (I)(iii) the PBC region of the first protein(s) is/are capable of binding to (II)(iii) the PBC region of the second protein(s); and/or
wherein the (I)(ii) PBN region of the first protein(s) and (I)(iii) PBC region of the first protein(s) have at least 90% sequence identity, preferably 100% sequence identity.

3. The complex of claim 1 or 2, wherein (I)(ii) the PBN region of the first protein(s) and/or (I)(iii) the PBC region of the first protein(s) are selected from a spy-tag, a snoop-tag, a Dog-Tag, and a split intein N-terminal fragment, preferably wherein the (I)(ii) PBN regions of the first protein(s) and/or the (I)(iii) PBC region of the first protein(s) are a spy-tag.

4. The complex of claim 1, wherein (I)(ii) the PBN region of the first protein(s) is/are capable of binding to the (II)(iii) the PBC region of the second protein(s) and wherein the (I)(iii) the PBC region of the first protein(s) is/are capable of binding to the (II)(ii) the PBN region of the second protein(s); and
wherein said binding of the PBN and PBC regions is **inducible,** preferably wherein the binding of the PBN and PBC regions is inducible by light and/or by chemically induced proximity.

5. The complex of claim 4, wherein the chemically induced proximity is inducible by a compound, preferably wherein the compound is a small molecule compound, more preferably wherein the small molecule compound is selected from one or two, preferably one, of rapamycin, FK506, FK506-cyclosporin, FK1012, coumermycin, gibberellin, S-(+)-abscisic acid, BisMTX, TMP-Halo (trimethoprim fused to Halo ligand), caffeine and cannabidiol.

6. The complex of any of the preceding claims, wherein the tethering is reversible by
(a) cleavage of the complex, wherein the first and the second protein(s) of complex additionally comprise a protease cleavage site; and/or
(b) degradation of the complex, wherein the first and/or the second protein(s) of complex additionally comprise a ubiquitination site; and/or
(c) disruption of the binding of the PBN and PBC regions of the first and the second protein(s), wherein said binding is mediated by chemically induced proximity.

7. The complex of claim 6, wherein the (a) protease cleavage site is located between the (ii) PBN region and the (i) chromatin binding component or between the (i) chromatin binding component and the (iii) PBC region of the first and/or the second protein, preferably wherein the protease cleavage site is cleavable by a naturally occurring enzyme in a fertilized zygote, more preferably wherein the protease cleavage site is a separase cleavable site, even more preferably wherein said separase cleavable site is a Rec8 site.

8. The complex of claim 6, wherein the first and the second protein(s) of complex additionally comprise (b) a ubiquitination site, preferably wherein the ubiquitination site is an amino acid sequence of securin or cyclin being APC/C cleavable, preferably wherein said amino acid sequence of securin or cyclin has at least 25 amino acids, preferably at least 40 amino acids, more preferably at least 60 amino acids, more preferably wherein at least 80 amino acids, even more preferably from 80 amino acids to 150 amino acids of securin or cyclin, and most preferably wherein the amino acid sequences of securin or cyclin is located at the N-terminus of securin or cyclin.

9. The complex of claim 6, wherein the tethering is reversible by targeted protein degradation, preferably wherein the complex further comprises a PROTAC binding site.

10. The complex of claim 6, the binding of the PBN and PBC regions of the first protein(s) to the PBN and PBC regions of the second protein(s) is mediated by chemically induced proximity and wherein the binding of the PBN and PBC regions of the first and the second proteins is disrupted by the absence of a small molecule compound or functionally inactivating said small molecule compound.

11. The complex of any of the preceding claims, wherein the chromatin-binding component is a DNA binding domain and/or wherein the chromatin-binding component is capable of binding to a DNA-associated protein, preferably wherein the chromatin-binding domain is a DNA binding domain, preferably wherein the DNA binding domain is selected from a group consisting of a TAL protein domain, a Zinc finger protein, a leucine zipper, and a dCas9 complexed with a gRNA, preferably wherein the DNA-binding component is individually selected from a group consisting of a TAL protein domain and a Zinc finger protein.

12. A nucleic acid molecule or two nucleic acid molecules encoding the complex according to any of claims 1 to 11, preferably wherein the nucleic acid molecule(s) are RNA molecule(s), more preferably mRNA molecule(s).

13. A complex of any of claims 1 to 11 or a nucleic acid molecule(s) of claim 12 suitable for use in reproductive medicine, preferably assisted reproductive technology, and more preferably for use in reducing the risk of aneuploidy in a zygote.

14. An *in vitro* method for stabilizing chromatid or chromosome cohesion of an oocyte or an egg cell comprising the step of delivering the complex of any of claims 1 to 11 or the nucleic acid molecule(s) of claim 12 into the oocyte or the egg cell, wherein said RNA molecule(s) are translated into the complex of any of claims 1 to 11,
whereby the complex stabilizes the chromatid or chromosome cohesion in the oocyte or the egg cell and wherein the complex is delivered before metaphase II arrest.

## Patentansprüche

1. Komplex, der für die Kohäsion von Chromatiden oder Chromosomen geeignet ist, umfassend (I) ein oder mehrere erste(s) Protein(e) und (II) ein oder mehrere zweite(s) Protein(e),
wobei das/die (I) erste(n) und das/die (II) zweite(n) Protein(e) jeweils (**i**) eine Chromatin-bindende Komponente, (**ii**) eine Protein-bindende Region die N-terminal der Chromatin-bindende Komponente ist (PBN-Region), (**iii**) eine Protein-bindende Region, die C-terminal der Chromatin-bindenden Komponente ist (PBC-Region) umfassen;
wobei (i) die Chromatin-bindenden Komponente des/der ersten und des/der zweiten Proteins/Proteine in der Lage sind, an eine Zielsequenz auf Chromatiden oder Chromosomen zu binden; und
wobei (I)(ii) die PBN-Region des/der ersten Proteins/Proteine in der Lage ist/sind, an (II)(ii) die PBN-Region des/der zweiten Proteins/Proteine zu binden und wobei (I)(iii) die PBC-Region des/der ersten Proteins/Proteine in der Lage ist/sind, an (II)(iii) die PBC-Region des/der zweiten Proteins/Proteine zu binden, oder
wobei (I)(ii) die PBN-Region des/der ersten Proteins/Proteine in der Lage ist/sind, an (II)(iii) die PBC-Region des/der zweiten Proteins/Proteine zu binden, und wobei (I)(iii) die PBC-Region des/der ersten Proteins/Proteine in der Lage ist/sind, an (II)(ii) die PBN-Region des/der zweiten Proteins/Proteine zu binden; und
wobei die Bindung an die Chromatiden oder Chromosomen und die Bindung der PBN- und PBC-Regionen des/der ersten und zweiten Proteins/Proteine die Chromatiden oder Chromosomen reversibel zusammenhält.

2. Komplex nach Anspruch 1, wobei (I)(ii) die PBN-Region des/der ersten Proteins/Proteine in der Lage ist/sind, an (II)(ii) die PBN-Region des/der zweiten Proteins/Proteine zu binden und wobei (I)(iii) die PBC-Region des/der ersten Proteins/Proteine in der Lage ist/sind, an (II)(iii) die PBC-Region des/der zweiten Proteins/Proteine zu binden; und/oder
wobei die (I)(ii) PBN-Region des/der ersten Proteins/Proteine und die (I)(iii) PBC-Region des/der ersten Proteins/Proteine mindestens 90 % Sequenzidentität, vorzugsweise 100 % Sequenzidentität, aufweisen.

3. Komplex nach Anspruch 1 oder 2, wobei (I)(ii) die PBN-Region des/der ersten Proteins/Proteine und/oder (I)(iii) die PBC-Region des/der ersten Proteins/Proteine aus einem Spy-Tag, einem Snoop-Tag, einem Dog-Tag und einem gespaltenen N-terminalen Intein-Fragment ausgewählt sind, wobei vorzugsweise die (I)(ii) PBN-Regionen des/der ersten Protein/Proteine und/oder die (I)(iii) PBC-Region des/der ersten Proteins/Proteine ein Spy-Tag sind.

4. Komplex nach Anspruch 1, wobei (I)(ii) die PBN-Region des/der ersten Proteins/Proteine in der Lage ist/sind, an (II)(iii) die PBC-Region des/der zweiten Proteins/Proteine zu binden, und wobei (I)(iii) die PBC-Region des/der ersten Proteins/Proteine in der Lage ist/sind, an (II)(ii) die PBN-Region des/der zweiten Proteins/Proteine zu binden; und
wobei diese Bindung der PBN- und PBC-Regionen induzierbar ist, vorzugsweise wobei die Bindung der PBN- und PBC-Regionen durch Licht und/oder durch chemisch induzierte Nähe induzierbar ist.

5. Komplex nach Anspruch 4, wobei die chemisch induzierte Nähe durch eine Verbindung induzierbar ist, wobei die Verbindung vorzugsweise eine niedermolekulare Verbindung ist, wobei die niedermolekulare Verbindung vorzugsweise ausgewählt ist aus einer oder zwei, vorzugsweise einer, von Rapamycin, FK506, FK506-Cyclosporin, FK1012, Coumermycin, Gibberellin, S-(+)-Abscisinsäure, BisMTX, TMP-Halo (Trimethoprim fusioniert mit Halo-Ligand), Koffein und Cannabidiol.

6. Der Komplex nach einem der vorhergehenden Ansprüche, wobei die Kohäsion reversibel ist durch
(a) Spaltung des Komplexes, wobei das/die erste(n) und das/die zweite(n) Protein(e) des Komplexes zusätzlich eine Protease-Spaltstelle aufweisen; und/oder
(b) Abbau des Komplexes, wobei das/die erste(n) und das/die zweite(n) Protein(e) des Komplexes zusätzlich eine Ubiquitinierungsstelle aufweisen; und/oder
(c) Unterbrechung der Bindung der PBN- und PBC-Regionen des/der ersten und des/der zweiten Proteins/Proteine, wobei diese Bindung durch chemisch induzierte Nähe vermittelt wird.

7. Komplex nach Anspruch 6, wobei die (a) Proteaseschnittstelle zwischen der (ii) PBN-Region und der (i) Chromatin-bindenden Komponente oder zwischen der (i) Chromatin-bindenden Komponente und der (iii) PBC-Region des ersten und/oder des zweiten Proteins angeordnet ist, wobei die Proteaseschnittstelle vorzugsweise durch ein natürlich vorkommendes Enzym in einer befruchteten Zygote spaltbar ist, wobei die Proteaseschnittstelle vorzugsweise eine Separaseschnittstelle ist, noch bevorzugter, wobei die Separaseschnittstelle eine Rec8-Stelle ist.

8. Komplex nach Anspruch 6, wobei das/die erste(n) und das/die zweite(n) Protein(e) des Komplexes zusätzlich (b) eine Ubiquitinierungsstelle umfassen, vorzugsweise wobei die Ubiquitinierungsstelle eine Aminosäuresequenz von Securin oder Cyclin ist, die APC/C-spaltbar ist, vorzugsweise wobei die Aminosäuresequenz von Securin oder Cyclin mindestens 25 Aminosäuren aufweist, vorzugsweise mindestens 40 Aminosäuren, noch bevorzugter mindestens 60 Aminosäuren, noch bevorzugter mindestens 80 Aminosäuren, noch bevorzugter von 80 Aminosäuren bis 150 Aminosäuren von Securin oder Cyclin, und am meisten bevorzugt, wobei die Aminosäuresequenzen von Securin oder Cyclin am N-Terminus von Securin oder Cyclin angeordnet sind.

9. Komplex nach Anspruch 6, wobei die Köhasion durch gezielten Proteinabbau reversibel ist, wobei der Komplex vorzugsweise ferner eine PROTAC-Bindungsstelle umfasst.

10. Komplex nach Anspruch 6, wobei die Bindung der PBN- und PBC-Regionen des/der ersten Proteins/Proteine an die PBN- und PBC-Regionen des/der zweiten Proteins/Proteine durch chemisch induzierte Nähe vermittelt wird und wobei die Bindung der PBN- und PBC-Regionen des ersten und des zweiten Proteins durch die Abwesenheit einer niedermolekularen Verbindung oder die funktionelle Inaktivierung der niedermolekularen Verbindung unterbrochen wird.

11. Komplex nach einem der vorhergehenden Ansprüche, wobei die Chromatin-bindenden Komponente eine DNA-Bindungsdomäne ist und/oder wobei die Chromatin-bindenden Komponente in der Lage ist, an ein DNA-assoziiertes Protein zu binden, wobei die Chromatin-bindenden Komponente vorzugsweise eine DNA-Bindungsdomäne ist, wobei die DNA-Bindungsdomäne vorzugsweise aus einer Gruppe ausgewählt ist, die aus einer TAL-Proteindomäne, einem Zinkfingerprotein, einem Leucinzipper und einem mit einer gRNA komplexierten dCas9 besteht, wobei die DNA-Bindungskomponente vorzugsweise individuell aus einer Gruppe ausgewählt ist, die aus einer TAL-Proteindomäne und einem Zinkfingerprotein besteht.

12. Nukleinsäuremolekül oder zwei Nukleinsäuremoleküle, die den Komplex nach einem der Ansprüche 1 bis 11 kodieren, wobei es sich bei dem/den Nukleinsäuremolekül(en) vorzugsweise um RNA-Moleküle, insbesondere mRNA-Moleküle, handelt.

13. Komplex nach einem der Ansprüche 1 bis 11 oder ein Nukleinsäuremolekül bzw. Nukleinsäuremoleküle nach Anspruch 12, das/die zur Verwendung in der Reproduktionsmedizin geeignet ist /sind, vorzugsweise in der assistierten Reproduktionstechnologie, und noch bevorzugter zur Verwendung bei der Verringerung des Risikos der Aneuploidie in einer Zygote.

14. In-vitro-Verfahren zur Stabilisierung der Chromatiden- oder Chromosomenkohäsion einer Oozyte oder einer Eizelle, umfassend den Schritt der Zuführung des Komplexes nach einem der Ansprüche 1 bis 11 oder des/der Nukleinsäuremoleküls/Nukleinsäuremoleküle nach Anspruch 12 in die Oozyte oder die Eizelle, wobei das/die RNA-Molekül(e) in den Komplex nach einem der Ansprüche 1 bis 11 übersetzt wird/werden,
wobei der Komplex die Chromatiden- oder Chromosomenkohäsion in der Oozyte oder der Eizelle stabilisiert und wobei der Komplex vor dem Metaphase-II-Arrest zugeführt wird.

## Revendications

1. Complexe approprié pour la cohésion de chromatides ou de chromosomes comprenant (I) une ou plusieurs premières protéines et (II) une ou plusieurs secondes protéines,
dans lequel la ou les (I) première(s) et (II) seconde(s) protéine(s) comprennent chacune (i) un composant de liaison à la chromatine, (ii) une région de liaison aux protéines étant N-terminale du composant de liaison à la chromatine (région PBN), (iii) une région de liaison aux protéines étant C-terminale du composant de liaison à la chromatine (région PBC) ;
dans lequel (i) les composants de liaison à la chromatine de la ou des première(s) et de la ou des seconde(s) protéine(s) sont capables de se lier à une séquence cible sur les chromatides ou les chromosomes ; et
dans lequel (I)(ii) la région PBN de la ou des première(s) protéine(s) est/sont capable(s) de se lier à (II)(ii) la région PBN de la ou des seconde(s) protéine(s) et dans lequel (I)(iii) la région PBC de la ou des première(s) protéine(s) est/sont capable(s) de se lier à (II)(iii) la région PBC de la ou des seconde(s) protéine(s), ou
dans lequel (I)(ii) la région PBN de la ou des première(s) protéine(s) est/sont capable(s) de se lier à (II)(iii) la région PBC de la ou des seconde(s) protéine(s) et dans lequel (I)(iii) la région PBC de la ou des première(s) protéine(s) est/sont capable(s) de se lier à (II)(ii) la région PBN de la ou des seconde(s) protéine(s) ; et
dans lequel la liaison aux chromatides ou aux chromosomes et la liaison des régions PBN et PBC de la ou des première(s) et seconde(s) protéine(s) fixent de manière réversible les chromatides ou les chromosomes.

2. Complexe selon la revendication 1, dans lequel (I)(ii) la région PBN de la ou des première(s) protéine(s) est/sont capable(s) de se lier à (II)(ii) la région PBN de la ou des seconde(s) protéine(s) et dans lequel (I)(iii) la région PBC de la ou des première(s) protéine(s) est/sont capable(s) de se lier à (II)(iii) la région PBC de la ou des seconde(s) protéine(s) ; et/ou
dans lequel la région PBN (I)(ii) de la ou des première(s) protéine(s) et la région PBC (I)(iii) de la ou des première(s) protéine(s) ont au moins 90 % d'identité de séquence, de préférence 100 % d'identité de séquence.

3. Complexe selon la revendication 1 ou 2, dans lequel (I)(ii) la région PBN de la ou des première(s) protéine(s) et/ou (I)(iii) la région PBC de la ou des première(s) protéine(s) sont choisies parmi un marqueur spy, un marqueur snoop, un marqueur Dog et un fragment N-terminal d'intéine divisée, de préférence dans lequel les régions PBN (I)(ii) de la ou des première(s) protéine(s) et/ou la région PBC (I)(iii) de la ou des première(s) protéine(s) sont un marqueur spy.

4. Complexe selon la revendication 1, dans lequel (I)(ii) la région PBN de la ou des première(s) protéine(s) est/sont capable(s) de se lier à la région PBC (II)(iii) de la ou des seconde(s) protéine(s) et dans lequel la région PBC (I)(iii) de la ou des première(s) protéine(s) est/sont capable(s) de se lier à la région PBN (II)(ii) de la ou des seconde(s) protéine(s) ; et
dans lequel ladite liaison des régions PBN et PBC est inductible, de préférence dans lequel la liaison des régions PBN et PBC est inductible par la lumière et/ou par une proximité induite chimiquement.

5. Complexe selon la revendication 4, dans lequel la proximité induite chimiquement est inductible par un composé, de préférence dans lequel le composé est un composé à petite molécule, plus préférablement dans lequel le composé à petite molécule est choisi parmi un ou deux, de préférence un composé, parmi la rapamy-cine, le FK506, le FK506-cyclosporine, le FK1012, la coumermycine, la gibbérelline, l'acide S-(+)-abscisique, le BisMTX, le TMP-Halo (triméthoprime fusionné à un ligand Halo), la caféine et le cannabidiol.

6. Complexe selon l'une quelconque des revendications précédentes, dans lequel la fixation est réversible par
(a) clivage du complexe, dans lequel la ou les première(s) et la ou les seconde(s) protéine(s) du complexe comprennent en outre un site de clivage par protéase ; et/ou
(b) dégradation du complexe, dans lequel la ou les première(s) et la ou les seconde(s) protéine(s) du complexe comprennent en outre un site d'ubiquitination ; et/ou
(c) rupture de la liaison des régions PBN et PBC de la ou des première(s) et de la ou des seconde(s) protéine(s), dans laquelle ladite liaison est médiée par une proximité induite chimiquement.

7. Complexe selon la revendication 6, dans lequel le site de clivage par protéase (a) est situé entre la région PBN (ii) et le composant de liaison à la chromatine (i) ou entre le composant de liaison à la chromatine (i) et la région PBC (iii) de la première et/ou de la seconde protéine, de préférence dans lequel le site de clivage par protéase est clivable par une enzyme naturelle chez un zygote fécondé, plus préférablement dans lequel le site de clivage par protéase est un site clivable par séparase, encore plus préférablement dans lequel ledit site clivable par séparase est un site Rec8.

8. Complexe selon la revendication 6, dans lequel la ou les première(s) et la ou les seconde(s) protéine(s) du complexe comprennent en outre (b) un site d'ubiquitination, de préférence dans lequel le site d'ubiquitination est une séquence d'acides aminés de sécurine ou de cycline pouvant être clivée par APC/C, de préférence dans lequel ladite séquence d'acides aminés de sécurine ou de cycline comporte au moins 25 acides aminés, de préférence au moins 40 acides aminés, plus préférablement au moins 60 acides aminés, plus préférablement dans lequel au moins 80 acides aminés, encore plus préférablement de 80 acides aminés à 150 acides aminés de sécurine ou de cycline, et de manière préférée entre toutes dans lequel les séquences d'acides aminés de sécurine ou de cycline sont situées à l'extrémité N-terminale de la sécurine ou de la cycline.

9. Complexe selon la revendication 6, dans lequel la fixation est réversible par dégradation ciblée de protéine, de préférence dans lequel le complexe comprend en outre un site de liaison PROTAC.

10. Complexe selon la revendication 6, dans lequel la liaison des régions PBN et PBC de la ou des première(s) protéine(s) aux régions PBN et PBC de la ou des seconde(s) protéine(s) est médiée par une proximité induite chimiquement et dans lequel la liaison des régions PBN et PBC de la ou des première(s) et de la ou des seconde(s) protéines est interrompue par l'absence d'un composé à petite molécule ou par l'inactivation fonctionnelle dudit composé à petite molécule.

11. Complexe selon l'une quelconque des revendications précédentes, dans lequel le composant de liaison à la chromatine est un domaine de liaison à l'ADN et/ou dans lequel le composant de liaison à la chromatine est capable de se lier à une protéine associée à l'ADN, de préférence dans lequel le domaine de liaison à la chromatine est un domaine de liaison à l'ADN, de préférence dans lequel le domaine de liaison à l'ADN est choisi dans un groupe constitué d'un domaine protéique TAL, d'une protéine à doigts de zinc, d'une glissière à leucine et d'une dCas9 complexée avec un ARNg, de préférence dans lequel le composant de liaison à l'ADN est choisi individuellement dans un groupe constitué par un domaine protéique TAL et une protéine à doigts de zinc.

12. Molécule d'acide nucléique ou deux molécules d'acide nucléique codant pour le complexe selon l'une quelconque des revendications 1 à 11, de préférence dans lequel la ou les molécules d'acide nucléique sont une ou des molécule(s) d'ARN, plus préférablement une ou des molécule(s) d'ARNm.

13. Complexe selon l'une quelconque des revendications 1 à 11 ou molécule(s) d'acide nucléique selon la revendication 12 approprié(es) à une utilisation en médecine de la reproduction, de préférence en technologie de reproduction assistée, et plus préférablement à une utilisation dans la réduction du risque d'aneuploïdie chez un zygote.

14. Procédé *in vitro* de stabilisation de la cohésion des chromatides ou des chromosomes d'un ovocyte ou d'un ovule comprenant l'étape d'administration du complexe selon l'une quelconque des revendications 1 à 11 ou de la ou des molécule(s) d'acide nucléique selon la revendication 12 dans l'ovocyte ou l'ovule, dans lequel ladite ou lesdites molécule(s) d'ARN sont traduites en complexe selon l'une quelconque des revendications 1 à 11,
ce par quoi le complexe stabilise la cohésion des chromatides ou des chromosomes dans l'ovocyte ou l'ovule et dans lequel le complexe est administré avant l'arrêt de la métaphase II.
